# EUROPEAN PATENT APPLICATION

(11) **EP 4 332 105 A1**
(43) Date of publication of application: **06.03.2024**
(21) Application number: 22795037.5
(22) Date of filing: 29.04.2022
(51) Int. Cl.: C07D 487/08, C07D 519/00, A61K 31/517, A61K 31/5377, A61P 35/00

(54) **PYRIDINO- OR PYRIMIDO-CYCLIC COMPOUND, PREPARATION METHOD THEREFOR AND MEDICAL USE THEREOF**

(30) Priority: 30.04.2021 CN 202110483687; 14.05.2021 CN 202110528325; 21.06.2021 CN 202110686065; 11.08.2021 CN 202110919898; 14.01.2022 CN 202210044029
(71) Applicant: Genfleet Therapeutics (Shanghai) Inc., Shanghai 201203 (CN); Zhejiang Genfleet Therapeutics Co., Ltd., Shaoxing Binhai New City Shaoxing, Zhejiang 312000 (CN)
(72) Inventor: JIANG, Tao, Shanghai 201203 (CN); ZHOU, Fusheng, Shanghai 201203 (CN); ZHANG, Leitao, Shanghai 201203 (CN); CAI, Lijian, Shanghai 201203 (CN); XU, Xiaoming, Shanghai 201203 (CN); MA, Kai, Shanghai 201203 (CN); LIU, Zhubo, Shanghai 201203 (CN); LAN, Jiong, Shanghai 201203 (CN)
(74) Representative: Elzaburu S.L.P.
(86) International application number: PCT/CN2022/090470
(87) International publication number: WO 2022/228568

(57) **Abstract**

Provided is a pyridino- or pyrimido-cyclic compound having an inhibitory effect on a KRAS gene mutation, or a pharmaceutically acceptable salt, a stereoisomer or a solvate thereof, wherein the structure of the compound is as shown in formula (AI) or (AII), and the definition of each group in the formula is detailed in the description. In addition, also disclosed are a pharmaceutical composition comprising the compound, and the use thereof in the preparation of a cancer drug.

## Description

### TECHNICAL FIELD

The present disclosure relates to the field of medicine, in particular, relates to a class of pyridino- or pyrimido-cyclic compound, preparation method therefor and medical use thereof.

### BACKGROUND

KRAS is a 21kD member of the Ras family of GTPase proteins and is an essential component for cell signaling. The role of KRAS in malignant tumors and mutations in various tumor types (e.g. G12C mutation, G12D mutation, G12V mutation, etc.) are known to the public, and thus KRAS has become a very attractive target for cancer therapy in the pharmaceutical industry. Compounds that inhibit KRAS activity are highly desirable to those skilled in the art and are under hot research. At present, breakthroughs have been made in the art for KRAS G12C, e.g. KRAS G12C inhibitors of AMG, and MIRATI have shown adequate safety and efficacy, however, there is a sustaining interest and effort to develop inhibitors of KRAS, in particular activating KRAS mutants, in particular KRAS G12D.

### SUMMARY OF THE INVENTION

The present disclosure provides a new class of pyridino- or pyrimido-cyclic compounds with high activity, good selectivity, and low toxicity as KRAS G12D inhibitors.

In a first aspect, the present disclosure provides a compound of Formula (AI) or Formula (AII), or a pharmaceutically acceptable salt, a stereoisomer, or a solvate thereof.

In one embodiment, the present disclosure provides a compound of Formula (AI) or a pharmaceutically acceptable salt, a stereoisomer, or a solvate thereof: 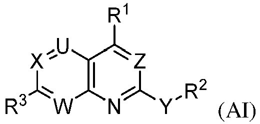
in the formula,
R¹ is selected from the group consisting of the following formulae and stereoisomers thereof: 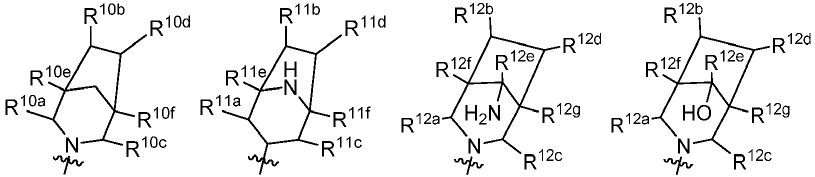 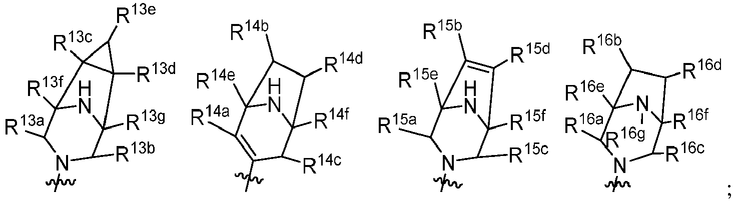 wherein,
R^{10a}, R^{10b}, R^{10c}, R^{10d}, R^{10e}, and R^{10f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, - CH₂CH₂-, -CH₂OCH₂CH₂-, or -CH₂CH₂CH₂-;
R^{11a}, R^{11b}, R^{11c}, R^{11d}, R^{11e}, and R^{11f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, - CH₂CH₂-, -CH₂OCH₂CH₂-, or -CH₂CH₂CH₂-;
each R^{12a}, R^{12b}, R^{12c}, R^{12d}, R^{12e}, R^{12f}, and R^{12g} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, -CH₂CH₂-, -CH₂OCH₂CH₂-, or -CH₂CH₂CH₂-;
R^{13a}, R^{13b}, R^{13c}, R^{13d}, R^{13e}, R^{13f}, and R^{13g} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl;
R^{14a}, R^{14b}, R^{14c}, R^{14d}, R^{14e}, and R^{14f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl; R^{15a}, R^{15b}, R^{15c}, R^{15d}, R^{15e}, and R^{15f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl; R^{16a}, R^{16b}, R^{16c}, R^{16d}, R^{16e}, and R^{16f} are each independently hydrogen, C2-C6 alkenyl, C2-C6 alkynyl, cyano, -CH₂CH₂-, -CH₂OCH₂CH₂-, -CH₂CH₂CH₂-, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R₅)₂, -C(O)H, -CO₂R⁵, -CON(R⁵)₂, -CO₂N(R⁵)₂, or 5- or 6-membered heteroaryl;
R^{16g} is hydrogen, hydroxy, or C1-C6 alkyl;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
U is N or C(R⁴⁴); wherein R⁴⁴ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl;
the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl;
the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4, or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl and the 7- to 11-membered spiroheterocyclyl are each independently saturated or partially unsaturated; when the 6- to 10-membered fused heterocyclyl or the 7- to 11-membered spiroheterocyclyl is partially unsaturated, the ring contain 1 or 2 double bonds;
two hydrogen atoms on the same carbon atom of any one or two carbon atoms of the 6- to 10-membered fused heterocyclyl are optionally simultaneously substituted with =CR^{2a}R^{2b}; the 6- to 10-membered fused heterocyclyl is further optionally substituted with one or more (e.g. 1, 2, 3, 4 or 5) R⁶;
two hydrogen atoms on the same carbon atom of any one or two carbon atoms of the 7- to 11-membered spiroheterocyclyl are optionally simultaneously substituted with =CR^{2a}R^{2b}; the 7- to 11-membered spiroheterocyclyl is further optionally substituted with one or more (e.g. 1, 2, 3, 4 or 5) R6;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is independently 2, 3, or 4;
each m4, m5, m6, m7 is independently 0, 1, 2 or 3; m4 and m5 are not simultaneously 0; m6 and m7 are not simultaneously 0;
   or
R² is hydrogen, -N(R⁵)₂, heterocyclyl, C1-C6 alkyl, -L-heterocyclyl, -L-aryl, -L-heteroaryl, -L-cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-aryl, -L-COOH, or -L-C(O)OC1-C6 alkyl; wherein n is 1, 2, 3, 4 or 5; each of the heterocyclyl, aryl of the -L-NR⁵C(O)-aryl, heterocyclyl of the -L-heterocyclyl, cycloalkyl of the -L-cycloalkyl may be optionally substituted with one or more (e.g. 1, 2, 3, 4 or 5) R⁶; each aryl of the -L-aryl, heteroaryl of the -L-heteroaryl may be optionally substituted with one or more (e.g. 1, 2, 3, 4 or 5) R⁷;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C2-C6 haloalkenyl, C2-C6 haloalkynyl, C1-C6 alkoxy, C1-C6 haloalkoxy, cyano, -Q-phenyl, -Q-phenyl-SO₂F, -NHC(O) phenyl, -NHC(O) phenyl-SO₂F, C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl)C(O)-, oxo, (C1-C3 haloalkyl)C(O)-, -SO₂F, (C1-C3 alkoxy) C1-C3 alkoxy, -CH₂OC(O)N(R⁵)₂, -CH₂NHC(O)OC1-C6 alkyl, -CH₂NHC(O)N(R⁵)₂, - CH₂NHC(O)C1-C6 alkyl, -CH₂ (pyrazolyl), -CH₂NHSO₂C1-C6 alkyl, -C1-C3 alkyl-OC(O) heterocyclyl, -OC(O)N(R⁵)₂, -OC(O)NH (C1-C3 alkyl)O (C1-C3 alkyl)), -OC(O)NH (C1-C3 alkyl)O (C1-C3 alkyl)phenyl (C1-C3 alkyl)N(CH₃)₂, -OC(O)NH (C1-C3 alkyl)O (C1-C3 alkyl)phenyl, -OC(O) heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂ heterocyclyl; wherein the phenyl referred to in the above groups are optionally substituted by-C(O)H or OH; the heterocyclyl in-C1-C3 alkyl-heterocyclyl is optionally substituted with oxo; Q is a bond or O;
each R⁷ is each independently halogen, hydroxy, -C(O)H, C1-C6 alkyl, C1-C6 alkoxy, C1-C6 haloalkyl, C1-C6 hydroxyalkyl, or -N(R⁵)₂;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl, the 3- to 6-membered nitrogen-containing heterocyclyl is optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

In one embodiment, the present disclosure provides a compound of Formula (AII) or a pharmaceutically acceptable salt, a stereoisomer, or a solvate thereof: 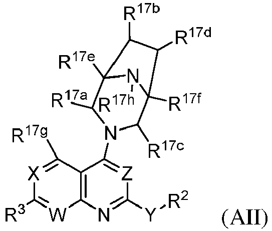 wherein:
R^{17a}, R^{17b}, R^{17c}, R^{17d}, R^{17e}, and R^{17f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, - CH₂CH₂-, or -CH₂CH₂CH₂-;
R^{17g} is hydrogen, hydroxy, halogen, C1-C6-alkyl, C1-C6-alkoxy, C1-C6-haloalkyl or C1-C6-haloalkoxy;
R^{17h} is hydrogen, hydroxy, or C1-C6 alkyl;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl;
the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl;
the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4, or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl and the 7- to 11-membered spiroheterocyclyl are each independently saturated or partially unsaturated; when the 6- to 10-membered fused heterocyclyl or the 7- to 11-membered spiroheterocyclyl is partially unsaturated, the ring contain 1 or 2 double bonds;
when R² is -L-6- to 10-membered fused heterocyclyl and R^{17g} is hydrogen, two hydrogen atoms on the same carbon atom of any one or two carbon atoms of the 6- to 10-membered fused heterocyclyl must both be substituted with =CR^{2a}R^{2b} simultaneously; and/or the 6- to 10-membered fused heterocyclyl is optionally substituted with one or more (e.g. 1, 2, 3, 4 or 5) R⁶;
when R² is -L-6- to 10-membered fused heterocyclyl and R^{17g} is not hydrogen, two hydrogen atoms on the same carbon atom of any one or two carbon atoms of the 6- to 10-membered fused heterocyclyl are optionally simultaneously substituted with =CR^{2a}R^{2b}; and/or the 6- to 10-membered fused heterocyclyl is optionally substituted with one or more (e.g. 1, 2, 3, 4 or 5) R⁶;
when R² is -L-7- to 11-membered spiroheterocyclyl and R^{17g} is hydrogen, two hydrogen atoms on the same carbon atom of any one or two carbon atoms of the 7- to 11-membered spiroheterocyclyl must both be substituted with =CR^{2a}R^{2b} simultaneously; and/or the 7- to 11-membered spiroheterocyclyl is optionally substituted with one or more (e.g. 1, 2, 3, 4 or 5) R⁶;
when R² is -L-7- to 11-membered spiroheterocyclyl and R^{17g} is not hydrogen, two hydrogen atoms on the same carbon atom of any one or two carbon atoms of the 7- to 11-membered spiroheterocyclyl are optionally simultaneously substituted with =CR^{2a}R^{2b}; and/or the 7- to 11-membered spiroheterocyclyl is optionally substituted with one or more (e.g. 1, 2, 3, 4 or 5) R⁶;
when R² is -L-spirocyclic ring substituted 6- to 10-membered fused heterocyclyl and R^{17g} is hydrogen and the spirocyclic ring substituted 6- to 10-membered fused heterocyclyl is 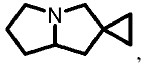 two hydrogen atoms on the same carbon atom of any one or two carbon atoms of the 6- to 10-membered fused heterocyclyl must both be substituted with =CR^{2a}R^{2b} simultaneously; and/or the spirocyclic ring substituted 6- to 10-membered fused heterocyclyl is optionally substituted with one or more (e.g. 1, 2, 3, 4 or 5) R⁶;
when R² is -L-spirocyclic ring substituted 6- to 10-membered fused heterocyclyl and R^{17g} is not hydrogen or the spirocyclic ring substituted 6- to 10-membered fused heterocyclyl is not 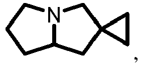 two hydrogen atoms on the same carbon atom of one or two carbon atoms of the spirocyclic ring substituted 6- to 10-membered fused heterocyclyl are optionally simultaneously substituted with =CR^{2a}R^{2b}; and/or the spirocyclic ring substituted 6- to 10-membered fused heterocyclyl is optionally substituted with one or more (e.g. 1, 2, 3, 4 or 5) R⁶;
when R² is -L-spirocyclic ring substituted 7- to 11-membered spiroheterocyclyl, two hydrogen atoms on the same carbon atom of any one or two carbon atoms of the spirocyclic ring substituted 7- to 11-membered spiroheterocyclyl is optionally simultaneously substituted with =CR^{2a}R^{2b}; and/or the spirocyclic ring substituted 7- to 11-membered spiroheterocyclyl is optionally substituted with one or more (e.g. 1, 2, 3, 4 or 5) R⁶;
when R² is -L-fused ring substituted 6- to 10-membered fused heterocyclyl, two hydrogen atoms on the same carbon atom of any one or two carbon atoms of the fused ring substituted 6- to 10-membered fused heterocyclyl are optionally simultaneously substituted with =CR^{2a}R^{2b}; and/or the fused ring substituted 6- to 10-membered fused heterocyclyl is optionally substituted with one or more (e.g. 1, 2, 3, 4 or 5) R⁶;
when R² is -L-fused ring substituted 7- to 11-membered spiroheterocyclyl, two hydrogen atoms on the same carbon atom of any one or two carbon atoms of the fused ring substituted 7- to 11-membered spiroheterocyclyl is optionally simultaneously substituted with =CR^{2a}R^{2b}; and/or the fused ring substituted 7- to 11-membered spiroheterocyclyl is optionally substituted with one or more (e.g. 1, 2, 3, 4 or 5) R⁶;
when R² is -L-spirocyclic and fused ring substituted 6- to 10-membered fused heterocyclyl, two hydrogen atoms on the same carbon atom of any one or two carbon atoms of the spirocyclic and fused ring substituted 6- to 10-membered fused heterocyclyl are optionally simultaneously substituted with =CR^{2a}R^{2b}; and/or the spirocyclic and fused ring substituted 6- to 10-membered fused heterocyclyl is optionally substituted with one or more (e.g. 1, 2, 3, 4 or 5) R⁶;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is independently 2, 3, or 4;
each m4, m5, m6, m7 is independently 0, 1, 2 or 3; m4 and m5 are not simultaneously 0; m6 and m7 are not simultaneously 0;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C2-C6 haloalkenyl, C2-C6 haloalkynyl, C1-C6 alkoxy, C1-C6 haloalkoxy, cyano, -Q-phenyl, -Q-phenyl-SO₂F, -NHC(O) phenyl, -NHC(O) phenyl-SO₂F, C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl)C(O)-, oxo, (C1-C3 haloalkyl)C(O)-, -SO₂F, (C1-C3 alkoxy) C1-C3 alkoxy, -CH₂OC(O)N(R⁵)₂, -CH₂NHC(O)OC1-C6 alkyl, -CH₂NHC(O)N(R⁵)₂, - CH₂NHC(O)C1-C6 alkyl, -CH₂ (pyrazolyl), -CH₂NHSO₂C1-C6 alkyl, -C1-C3 alkyl-OC(O) heterocyclyl, -OC(O)N(R⁵)₂, -OC(O)NH (C1-C3 alkyl)O (C1-C3 alkyl)), -OC(O)NH (C1-C3 alkyl)O (C1-C3 alkyl)phenyl (C1-C3 alkyl)N(CH₃)₂, -OC(O)NH (C1-C3 alkyl)O (C1-C3 alkyl)phenyl, -OC(O) heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂ heterocyclyl; wherein the phenyl referred to in the above groups are optionally substituted by-C(O)H or OH; the heterocyclyl in-C1-C3 alkyl-heterocyclyl is optionally substituted with oxo; Q is a bond or O;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl, the 3- to 6-membered nitrogen-containing heterocyclyl is optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

In one embodiment, 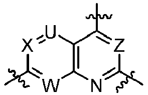 is selected from the group consisting of: 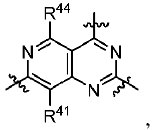 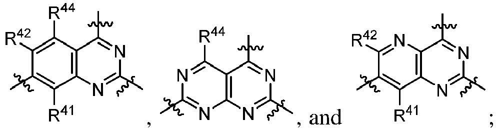 in each formula, each of R⁴¹, R⁴², and R⁴⁴ is as defined above.

In one embodiment, the present disclosure provides a compound of Formula (I) or Formula (II), or a pharmaceutically acceptable salt, a stereoisomer, or a solvate thereof:

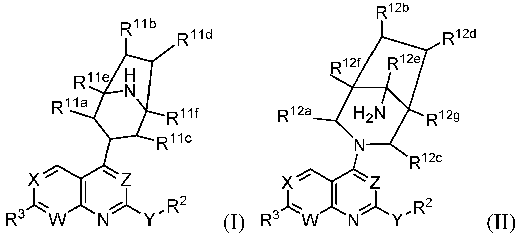

In each formula,
R^{11a}, R^{11b}, R^{11c}, R^{11d}, R^{11e}, and R^{11f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, - CH₂CH₂-, -CH₂OCH₂CH₂-, or -CH₂CH₂CH₂-;
R^{12a}, R^{12b}, R^{12c}, R^{12d}, R^{12e}, R^{12f}, and R^{12g} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, -CH₂CH₂-, -CH₂OCH₂CH₂-, or -CH₂CH₂CH₂-;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl; the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl;
the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4, or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl or the 7- to 11-membered spiroheterocyclyl are saturated or partially unsaturated; when the 6- to 10-membered fused heterocyclyl or the 7- to 11-membered spiroheterocyclyl is partially unsaturated, the ring contain 1 or 2 double bonds;
two hydrogen atoms on the same carbon atom of any one or two carbon atoms of the 6- to 10-membered fused heterocyclyl or the 7- to 11-membered spiroheterocyclyl are optionally simultaneously substituted with =CR^{2a}R^{2b}; the 6- to 10-membered fused heterocyclyl or the 7- to 11-membered spiroheterocyclyl is further optionally substituted with one or more R⁶;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is independently 2, 3, or 4;
each m4, m5, m6, m7 is independently 0, 1, 2 or 3; m4 and m5 are not simultaneously 0; m6 and m7 are not simultaneously 0;
   or
R² is hydrogen, -N(R⁵)₂, heterocyclyl, C1-C6 alkyl, -L-heterocyclyl, -L-aryl, -L-heteroaryl, -L-cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-aryl, -L-COOH, or -L-C(O)OC1-C6 alkyl; wherein n is 1, 2, 3, 4 or 5; each of the heterocyclyl, aryl of the -L-NR⁵C(O)-aryl, heterocyclyl of the -L-heterocyclyl, cycloalkyl of the -L-cycloalkyl may be optionally substituted with one or more (e.g. 1, 2, 3, 4 or 5) R⁶; each aryl of the -L-aryl, heteroaryl of the -L-heteroaryl may be optionally substituted with one or more (e.g. 1, 2, 3, 4 or 5) R⁷;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C1-C6 alkoxy, C1-C6 haloalkoxy, cyano, -Q-phenyl, -Q-phenyl-SO₂F, -NHC(O) phenyl, -NHC(O) phenyl-SO₂F_{,} C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl)C(O)-, oxo, (C1-C3 haloalkyl)C(O)-, -SO₂F, (C1-C3 alkoxy) C1-C3 alkoxy, -CH₂OC(O)N(R⁵)₂, - CH₂NHC(O)OC1-C6 alkyl, -CH₂NHC(O)N(R⁵)₂, -CH₂NHC(O)C1-C6 alkyl, -CH₂ (pyrazolyl), - CH₂NHSO₂C1-C6 alkyl, -C1-C3 alkyl-OC(O) heterocyclyl, -OC(O)N(R⁵)₂, -OC(O)NH (C1-C3 alkyl)O (C1-C3 alkyl)), -OC(O)NH (C1-C3 alkyl)O (C1-C3 alkyl)phenyl (C1-C3 alkyl)N(CH₃)₂, - OC(O)NH (C1-C3 alkyl)O (C1-C3 alkyl)phenyl, -OC(O) heterocyclyl, -OC(O)C1-C6 alkyl, - OC(O)C1-C6 haloalkyl, -C1-C3 alkyl-OC(O)C1-C6 alkyl, -C1-C3 alkyl-OC(O)C1-C6 haloalkyl, - OC(O)phenyl, -C1-C3 alkyl-OC(O)phenyl or -CH₂ heterocyclyl; wherein the phenyl referred to in the above groups are optionally substituted by-C(O)H or OH; the heterocyclyl in-C1-C3 alkyl-heterocyclyl is optionally substituted with oxo; Q is a bond or O;
each R⁷ is each independently halogen, hydroxy, -C(O)H, C1-C6 alkyl, C1-C6 alkoxy, C1-C6 haloalkyl, C1-C6 hydroxyalkyl, or -N(R⁵)₂;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl, the 3- to 6-membered nitrogen-containing heterocyclyl is optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

In one embodiment, 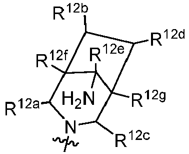 is selected from the group consisting of:, 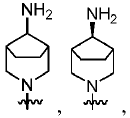 and

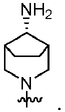

In one embodiment, the present disclosure provides a compound of Formula (III) or Formula (IV), or Formula (V), or a pharmaceutically acceptable salt, a stereoisomer, or a solvate thereof:

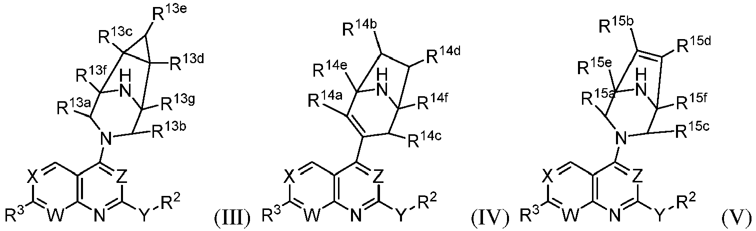

In each formula,
R^{13a}, R^{13b}, R^{13c}, R^{13d}, R^{13e}, R^{13f}, and R^{13g} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl;
R^{14a}, R^{14b}, R^{14c}, R^{14d}, R^{14e}, and R^{14f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl; R^{15a}, R^{15b}, R^{15c}, R^{15d}, R^{15e}, and R^{15f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl; W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl;
the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl;
the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4 or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl or the 7- to 11-membered spiroheterocyclyl are saturated or partially unsaturated; when the 6- to 10-membered fused heterocyclyl or the 7- to 11-membered spiroheterocyclyl is partially unsaturated, the ring contain 1 or 2 double bonds;
two hydrogen atoms on the same carbon atom of any one or two carbon atoms of the 6- to 10-membered fused heterocyclyl or the 7- to 11-membered spiroheterocyclyl are optionally simultaneously substituted with =CR^{2a}R^{2b}; the 6- to 10-membered fused heterocyclyl or the 7- to 11-membered spiroheterocyclyl is further optionally substituted with one or more R⁶;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is 2, 3, or 4;
each m4, m5, m6, m7 is independently 0, 1, 2 or 3; m4 and m5 are not simultaneously 0; m6 and m7 are not simultaneously 0;
   or
R² is hydrogen, -N(R⁵)₂, heterocyclyl, C1-C6 alkyl, -L-heterocyclyl, -L-aryl, -L-heteroaryl, -L-cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-aryl, -L-COOH, or -L-C(O)OC1-C6 alkyl; wherein n is 1, 2, 3, 4 or 5; each of the heterocyclyl, aryl of the -L-NR⁵C(O)-aryl, heterocyclyl of the -L-heterocyclyl, cycloalkyl of the -L-cycloalkyl may be optionally substituted with one or more (e.g. 1, 2, 3, 4 or 5) R⁶; each aryl of the -L-aryl, heteroaryl of the -L-heteroaryl may be optionally substituted with one or more (e.g. 1, 2, 3, 4 or 5) R⁷;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C1-C6 alkoxy, C1-C6 haloalkoxy, cyano, -Q-phenyl, -Q-phenyl-SO₂F, -NHC(O) phenyl, -NHC(O) phenyl-SO₂F_{,} C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl)C(O)-, oxo, (C1-C3 haloalkyl)C(O)-, -SO₂F, (C1-C3 alkoxy) C1-C3 alkoxy, -CH₂OC(O)N(R⁵)₂, - CH₂NHC(O)OC1-C6 alkyl, -CH₂NHC(O)N(R⁵)₂, -CH₂NHC(O)C1-C6 alkyl, -CH₂ (pyrazolyl), - CH₂NHSO₂C1-C6 alkyl, -C1-C3 alkyl-OC(O) heterocyclyl, -OC(O)N(R⁵)₂, -OC(O)NH (C1-C3 alkyl)O (C1-C3 alkyl)), -OC(O)NH (C1-C3 alkyl)O (C1-C3 alkyl)phenyl (C1-C3 alkyl)N(CH₃)₂, - OC(O)NH (C1-C3 alkyl)O (C1-C3 alkyl)phenyl, -OC(O) heterocyclyl, -OC(O)C1-C6 alkyl, - OC(O)C1-C6 haloalkyl, -C1-C3 alkyl-OC(O)C1-C6 alkyl, -C1-C3 alkyl-OC(O)C1-C6 haloalkyl, - OC(O)phenyl, -C1-C3 alkyl-OC(O)phenyl or -CH₂ heterocyclyl; wherein the phenyl referred to in the above groups are optionally substituted by-C(O)H or OH; the heterocyclyl in-C1-C3 alkyl-heterocyclyl is optionally substituted with oxo; Q is a bond or O;
each R⁷ is each independently halogen, hydroxy, -C(O)H, C1-C6 alkyl, C1-C6 alkoxy, C1-C6 haloalkyl, C1-C6 hydroxyalkyl, or -N(R⁵)₂;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

In one embodiment, the present disclosure provides a compound of Formula (VI) or a pharmaceutically acceptable salt, a stereoisomer, or a solvate thereof:

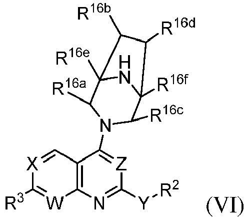

In each formula,
R^{16a}, R^{16b}, R^{16c}, R^{16d}, R^{16e}, and R^{16f} are each independently hydrogen, C2-C6 alkenyl, C2-C6 alkynyl, cyano, -CH₂CH₂-, -CH₂OCH₂CH₂-, or -CH₂CH₂CH₂-;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl; the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl;
the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4 or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl or the 7- to 11-membered spiroheterocyclyl are saturated or partially unsaturated; when the 6- to 10-membered fused heterocyclyl or the 7- to 11-membered spiroheterocyclyl is partially unsaturated, the ring contain 1 or 2 double bonds;
two hydrogen atoms on the same carbon atom of any one or two carbon atoms of the 6- to 10-membered fused heterocyclyl or the 7- to 11-membered spiroheterocyclyl are optionally simultaneously substituted with =CR^{2a}R^{2b}; the 6- to 10-membered fused heterocyclyl or the 7- to 11-membered spiroheterocyclyl is further optionally substituted with one or more R⁶;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is 2, 3, or 4;
each m4, m5, m6, m7 is independently 0, 1, 2 or 3; m4 and m5 are not simultaneously 0; m6 and m7 are not simultaneously 0;
   or
R² is hydrogen, -N(R⁵)₂, heterocyclyl, C1-C6 alkyl, -L-heterocyclyl, -L-aryl, -L-heteroaryl, -L-cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-aryl, -L-COOH, or -L-C(O)OC1-C6 alkyl; wherein n is 1, 2, 3, 4 or 5; each of the heterocyclyl, aryl of the -L-NR⁵C(O)-aryl, heterocyclyl of the -L-heterocyclyl, cycloalkyl of the -L-cycloalkyl may be optionally substituted with one or more (e.g. 1, 2, 3, 4 or 5) R⁶; each aryl of the -L-aryl, heteroaryl of the -L-heteroaryl may be optionally substituted with one or more (e.g. 1, 2, 3, 4 or 5) R⁷;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C1-C6 alkoxy, C1-C6 haloalkoxy, cyano, -Q-phenyl, -Q-phenyl-SO₂F, -NHC(O) phenyl, -NHC(O) phenyl-SO₂F, C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl)C(O)-, oxo, (C1-C3 haloalkyl)C(O)-, -SO₂F, (C1-C3 alkoxy) C1-C3 alkoxy, -CH₂OC(O)N(R⁵)₂, - CH₂NHC(O)OC1-C6 alkyl, -CH₂NHC(O)N(R⁵)₂, -CH₂NHC(O)C1-C6 alkyl, -CH₂ (pyrazolyl), - CH₂NHSO₂C1-C6 alkyl, -C1-C3 alkyl-OC(O) heterocyclyl, -OC(O)N(R⁵)₂, -OC(O)NH (C1-C3 alkyl)O (C1-C3 alkyl)), -OC(O)NH (C1-C3 alkyl)O (C1-C3 alkyl)phenyl (C1-C3 alkyl)N(CH₃)₂, - OC(O)NH (C1-C3 alkyl)O (C1-C3 alkyl)phenyl, -OC(O) heterocyclyl, -OC(O)C1-C6 alkyl, - OC(O)C1-C6 haloalkyl, -C1-C3 alkyl-OC(O)C1-C6 alkyl, -C1-C3 alkyl-OC(O)C1-C6 haloalkyl, - OC(O)phenyl, -C1-C3 alkyl-OC(O)phenyl or -CH₂ heterocyclyl; wherein the phenyl referred to in the above groups are optionally substituted by-C(O)H or OH; the heterocyclyl in-C1-C3 alkyl-heterocyclyl is optionally substituted with oxo; Q is a bond or O;
each R⁷ is each independently halogen, hydroxy, -C(O)H, C1-C6 alkyl, C1-C6 alkoxy, C1-C6 haloalkyl, C1-C6 hydroxyalkyl, or -N(R⁵)₂;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

In one embodiment, 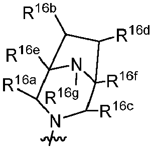 is selected from the group consisting of: 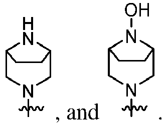 In one embodiment, the present disclosure provides a compound of Formula (VII) or a pharmaceutically acceptable salt, a stereoisomer, or a solvate thereof: 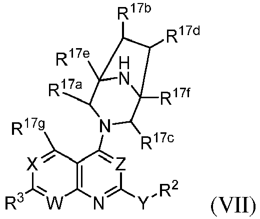 wherein:
R^{17a}, R^{17b}, R^{17c}, R^{17d}, R^{17e}, and R^{17f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl,
C1-C6 alkyl-N(R⁵)₂, -C(O)H, -CO₂R⁵, -CON(R⁵)₂, -CO₂N(R⁵)₂, 5- or 6-membered heteroaryl, - CH₂CH₂-, or -CH₂CH₂CH₂-;
R^{17g} is hydrogen, hydroxy, halogen, C1-C6-alkyl, C1-C6-alkoxy, C1-C6-haloalkyl or C1-C6-haloalkoxy;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl; the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl; the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4 or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl or the 7- to 11-membered spiroheterocyclyl are saturated or partially unsaturated; when the 6- to 10-membered fused heterocyclyl or the 7- to 11-membered spiroheterocyclyl is partially unsaturated, the ring contain 1 or 2 double bonds;
when R² is -L-6- to 10-membered fused heterocyclyl and R^{17g} is hydrogen, two hydrogen atoms on the same carbon atom of any one or two carbon atoms of the 6- to 10-membered fused heterocyclyl must both be substituted with =CR^{2a}R^{2b} simultaneously; the 6- to 10-membered fused heterocyclyl is further optionally substituted with one or more R⁶;
when R² is -L-6- to 10-membered fused heterocyclyl and R^{17g} is not hydrogen, two hydrogen atoms on the same carbon atom of any one or two carbon atoms of the 6- to 10-membered fused heterocyclyl are optionally simultaneously substituted with =CR^{2a}R^{2b}; the 6- to 10-membered fused heterocyclyl is further optionally substituted with one or more R⁶;
when R² is -L-7- to 11-membered spiroheterocyclyl and R^{17g} is hydrogen, two hydrogen atoms on the same carbon atom of any one or two carbon atoms of the 7- to 11-membered spiroheterocyclyl must both be substituted with =CR^{2a}R^{2b} simultaneously; the 7- to 11-membered spiroheterocyclyl is further optionally substituted with one or more R⁶;
when R² is -L-7- to 11-membered spiroheterocyclyl and R^{17g} is not hydrogen, two hydrogen atoms on the same carbon atom of any one or two carbon atoms of the 7- to 11-membered spiroheterocyclyl are optionally simultaneously substituted with =CR^{2a}R^{2b}; the 7- to 11-membered spiroheterocyclyl is further optionally substituted with one or more R⁶;
when R² is -L-spirocyclic ring substituted 6- to 10-membered fused heterocyclyl and R^{17g} is hydrogen and the spirocyclic ring substituted 6- to 10-membered fused heterocyclyl is 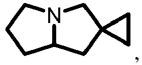 two hydrogen atoms on the same carbon atom of any one or two carbon atoms of the 6- to 10-membered fused heterocyclyl must both be substituted with =CR^{2a}R^{2b} simultaneously; the spirocyclic ring substituted 6- to 10-membered fused heterocyclyl is further optionally substituted with one or more R⁶;
when R² is -L-spirocyclic ring substituted 6- to 10-membered fused heterocyclyl and R^{17g} is not hydrogen or the spirocyclic ring substituted 6- to 10-membered fused heterocyclyl is not 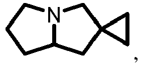 hydrogen atoms of one or two carbon atoms of the spirocyclic ring substituted 6- to 10-membered fused heterocyclyl are optionally simultaneously substituted with =CR^{2a}R^{2b}; the spirocyclic ring substituted 6- to 10-membered fused heterocyclyl is further optionally substituted with one or more R⁶;
when R² is -L-spirocyclic ring substituted 7- to 11-membered spiroheterocyclyl, two hydrogen atoms on the same carbon atom of any one or two carbon atoms of the spirocyclic ring substituted 7- to 11-membered spiroheterocyclyl is optionally simultaneously substituted with =CR^{2a}R^{2b}; the spirocyclic ring substituted 7- to 11-membered spiroheterocyclyl is further optionally substituted with one or more R⁶;
when R² is -L-fused ring substituted 6- to 10-membered fused heterocyclyl, two hydrogen atoms on the same carbon atom of any one or two carbon atoms of the fused ring substituted 6- to 10-membered fused heterocyclyl are optionally simultaneously substituted with =CR^{2a}R^{2b}; the fused ring substituted 6- to 10-membered fused heterocyclyl is further optionally substituted with one or more R⁶;
when R² is -L-fused ring substituted 7- to 11-membered spiroheterocyclyl, two hydrogen atoms on the same carbon atom of any one or two carbon atoms of the fused ring substituted 7- to 11-membered spiroheterocyclyl is optionally simultaneously substituted with =CR^{2a}R^{2b}; the fused ring substituted 7- to 11-membered spiroheterocyclyl is further optionally substituted with one or more R6;
when R² is -L-spirocyclic and fused ring substituted 6- to 10-membered fused heterocyclyl, two hydrogen atoms on the same carbon atom of any one or two carbon atoms of the spirocyclic and fused ring substituted 6- to 10-membered fused heterocyclyl are optionally simultaneously substituted with =CR^{2a}R^{2b}; the spirocyclic and fused ring substituted 6- to 10-membered fused heterocyclyl is further optionally substituted with one or more R⁶;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is 2, 3, or 4;
each m4, m5, m6, m7 is independently 0, 1, 2 or 3; m4 and m5 are not simultaneously 0; m6 and m7 are not simultaneously 0;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C1-C6 alkoxy, C1-C6 haloalkoxy, cyano, -Q-phenyl, -Q-phenyl-SO₂F, -NHC(O) phenyl, -NHC(O) phenyl-SO₂F_{,} C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl)C(O)-, oxo, (C1-C3 haloalkyl)C(O)-, -SO₂F, (C1-C3 alkoxy) C1-C3 alkoxy, -CH₂OC(O)N(R⁵)₂, - CH₂NHC(O)OC1-C6 alkyl, -CH₂NHC(O)N(R⁵)₂, -CH₂NHC(O)C1-C6 alkyl, -CH₂ (pyrazolyl), - CH₂NHSO₂C1-C6 alkyl, -C1-C3 alkyl-OC(O) heterocyclyl, -OC(O)N(R⁵)₂, -OC(O)NH (C1-C3 alkyl)O (C1-C3 alkyl)), -OC(O)NH (C1-C3 alkyl)O (C1-C3 alkyl)phenyl (C1-C3 alkyl)N(CH₃)₂, - OC(O)NH (C1-C3 alkyl)O (C1-C3 alkyl)phenyl, -OC(O) heterocyclyl, -OC(O)C1-C6 alkyl, - OC(O)C1-C6 haloalkyl, -C1-C3 alkyl-OC(O)C1-C6 alkyl, -C1-C3 alkyl-OC(O)C1-C6 haloalkyl, - OC(O)phenyl, -C1-C3 alkyl-OC(O)phenyl or -CH₂ heterocyclyl; wherein the phenyl referred to in the above groups are optionally substituted by-C(O)H or OH; the heterocyclyl in-C1-C3 alkyl-heterocyclyl is optionally substituted with oxo; Q is a bond or O;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

In one embodiment, at least three of R^{11a}, R^{11b}, R^{11c}, R^{11d}, R^{11e}, and R^{11f} are hydrogen.

In one embodiment, at least four of R^{11a}, R^{11b}, R^{11c}, R^{11d}, R^{11e}, and R^{11f} are hydrogen.

In one embodiment, at least five of R^{11a}, R^{11b}, R^{11c}, R^{11d}, R^{11e}, and R^{11f} are hydrogen.

In one embodiment, six of R^{11a}, R^{11b}, R^{11c}, R^{11d}, R^{11e}, and R^{11f} are all hydrogen.

In one embodiment, at least five of R^{11a}, R^{11b}, R^{11c}, R^{11d}, R^{11e}, and R^{11f} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂- or -CH₂CH₂CH₂-.

In one embodiment, at least five of R^{11a}, R^{11b}, R^{11c}, R^{11d}, R^{11e}, and R^{11f} are hydrogen and the other is -CH₂CH₂-, -CH₂OCH₂CH₂- or -CH₂CH₂CH₂-.

In one embodiment, at least five of R^{11a}, R^{11b}, R^{11c}, R^{11d}, R^{11e}, and R^{11f} are hydrogen and the other is hydroxy.

In one embodiment, at least five of R^{11a}, R^{11b}, R^{11c}, R^{11d}, R^{11e}, and R^{11f} are hydrogen and the other is -CO₂R⁵. Preferably, R⁵ is hydrogen or C1-C3 alkyl.

In one embodiment, at least five of R^{11a}, R^{11b}, R^{11c}, R^{11d}, R^{11e}, and R^{11f} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, at least four of R^{12a}, R^{12b}, R^{12c}, R^{12d}, R^{12e}, R^{12f}, and R^{12g} are hydrogen.

In one embodiment, at least five of R^{12a}, R^{12b}, R^{12c}, R^{12d}, R^{12e}, R^{12f}, and R^{12g} are hydrogen.

In one embodiment, at least six of R^{12a}, R^{12b}, R^{12c}, R^{12d}, R^{12e}, R^{12f}, and R^{12g} are hydrogen.

In one embodiment, seven of R^{12a}, R^{12b}, R^{12c}, R^{12d}, R^{12e}, R^{12f}, and R^{12g} are all hydrogen.

In one embodiment, at least six of R^{12a}, R^{12b}, R^{12c}, R^{12d}, R^{12e}, R^{12f}, and R^{12g} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂-, or -CH₂CH₂CH₂-.

In one embodiment, at least six of R^{12a}, R^{12b}, R^{12c}, R^{12d}, R^{12e}, R^{12f}, and R^{12g} are hydrogen and the other is -CH₂CH₂-, -CH₂OCH₂CH₂-, or -CH₂CH₂CH₂-.

In one embodiment, at least six of R^{12a}, R^{12b}, R^{12c}, R^{12d}, R^{12e}, R^{12f}, and R^{12g} are hydrogen and the other is hydroxy.

In one embodiment, at least six of R^{12a}, R^{12b}, R^{12c}, R^{12d}, R^{12e}, R^{12f}, and R^{12g} are hydrogen and the other is -CO₂R⁵. Preferably, R⁵ is hydrogen or C1-C3 alkyl.

In one embodiment, at least six of R^{12a}, R^{12b}, R^{12c}, R^{12d}, R^{12e}, R^{12f}, and R^{12g} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, at least four of R^{13a}, R^{13b}, R^{13c}, R^{13d}, R^{13e}, R^{13f}, and R^{13g} are hydrogen.

In one embodiment, at least five of R^{13a}, R^{13b}, R^{13c}, R^{13d}, R^{13e}, R^{13f}, and R^{13g} are hydrogen.

In one embodiment, at least six of R^{13a}, R^{13b}, R^{13c}, R^{13d}, R^{13e}, R^{13f}, and R^{13g} are hydrogen.

In one embodiment, seven of R^{13a}, R^{13b}, R^{13c}, R^{13d}, R^{13e}, R^{13f}, and R^{13g} are all hydrogen.

In one embodiment, at least six of R^{13a}, R^{13b}, R^{13c}, R^{13d}, R^{13e}, R^{13f}, and R^{13g} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂-, or -CH₂CH₂CH₂-.

In one embodiment, at least six of R^{13a}, R^{13b}, R^{13c}, R^{13d}, R^{13e}, R^{13f}, and R^{13g} are hydrogen and the other is -CH₂CH₂-, -CH₂OCH₂CH₂-, or -CH₂CH₂CH₂-.

In one embodiment, at least six of R^{13a}, R^{13b}, R^{13c}, R^{13d}, R^{13e}, R^{13f}, and R^{13g} are hydrogen and the other is hydroxy.

In one embodiment, at least six of R^{13a}, R^{13b}, R^{13c}, R^{13d}, R^{13e}, R^{13f}, and R^{13g} are hydrogen and the other is -CO₂R⁵. Preferably, R⁵ is hydrogen or C1-C3 alkyl.

In one embodiment, at least six of R^{13a}, R^{13b}, R^{13c}, R^{13d}, R^{13e}, R^{13f}, and R^{13g} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, at least three of R^{14a}, R^{14b}, R^{14c}, R^{14d}, R^{14e}, and R^{14f} are hydrogen.

In one embodiment, at least four of R^{14a}, R^{14b}, R^{14c}, R^{14d}, R^{14e}, and R^{14f} are hydrogen.

In one embodiment, at least five of R^{14a}, R^{14b}, R^{14c}, R^{14d}, R^{14e}, and R^{14f} are hydrogen.

In one embodiment, six of R^{14a}, R^{14b}, R^{14c}, R^{14d}, R^{14e}, and R^{14f} are all hydrogen.

In one embodiment, at least five of R^{14a}, R^{14b}, R^{14c}, R^{14d}, R^{14e}, and R^{14f} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂-, or -CH₂CH₂CH₂-.

In one embodiment, at least five of R^{14a}, R^{14b}, R^{14c}, R^{14d}, R^{14e}, and R^{14f} are hydrogen and the other is -CH₂CH₂-, -CH₂OCH₂CH₂- or -CH₂CH₂CH₂-.

In one embodiment, at least five of R^{14a}, R^{14b}, R^{14c}, R^{14d}, R^{14e}, and R^{14f} are hydrogen and the other is hydroxy.

In one embodiment, at least five of R^{14a}, R^{14b}, R^{14c}, R^{14d}, R^{14e}, and R^{14f} are hydrogen and the other is -CO₂R⁵. Preferably, R⁵ is hydrogen or C1-C3 alkyl.

In one embodiment, at least five of R^{14a}, R^{14b}, R^{14c}, R^{14d}, R^{14e}, and R^{14f} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, at least three of R^{15a}, R^{15b}, R^{15c}, R^{15d}, R^{15c}, and R^{15f} are hydrogen.

In one embodiment, at least four of R^{15a}, R^{15b}, R^{15c}, R^{15d}, R^{15c}, and R^{15f} are hydrogen.

In one embodiment, at least five of R^{15a}, R^{15b}, R^{15c}, R^{15d}, R^{15c}, and R^{15f} are hydrogen.

In one embodiment, six of R^{15a}, R^{15b}, R^{15c}, R^{15d}, R^{15c}, and R^{15f} are all hydrogen.

In one embodiment, at least five of R^{15a}, R^{15b}, R^{15c}, R^{15d}, R^{15c}, and R^{15f} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂-, or -CH₂CH₂CH₂-.

In one embodiment, at least five of R^{15a}, R^{15b}, R^{15c}, R^{15d}, R^{15c}, and R^{15f} are hydrogen and the other is -CH₂CH₂-, -CH₂OCH₂CH₂- or -CH₂CH₂CH₂-.

In one embodiment, at least five of R^{15a}, R^{15b}, R^{15c}, R^{15d}, R^{15c}, and R^{15f} are hydrogen and the other is hydroxy.

In one embodiment, at least five of R^{15a}, R^{15b}, R^{15c}, R^{15d}, R^{15c}, and R^{15f} are hydrogen and the other is -CO₂R⁵. Preferably, R⁵ is hydrogen or C1-C3 alkyl.

In one embodiment, at least five of R^{15a}, R^{15b}, R^{15c}, R^{15d}, R^{15c}, and R^{15f} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, at least three of R^{16a}, R^{16b}, R^{16c}, R^{16d}, R^{16e}, and R^{16f} are hydrogen.

In one embodiment, at least four of R^{16a}, R^{16b}, R^{16c}, R^{16d}, R^{16e}, and R^{16f} are hydrogen.

In one embodiment, at least five of R^{16a}, R^{16b}, R^{16c}, R^{16d}, R^{16e}, and R^{16f} are hydrogen.

In one embodiment, six of R^{16a}, R^{16b}, R^{16c}, R^{16d}, R^{16e}, and R^{16f} are all hydrogen.

In one embodiment, at least five of R^{16a}, R^{16b}, R^{16c}, R^{16d}, R^{16e}, and R^{16f} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂-, or -CH₂CH₂CH₂-.

In one embodiment, at least five of R^{16a}, R^{16b}, R^{16c}, R^{16d}, R^{16e}, and R^{16f} are hydrogen and the other is -CH₂CH₂-, -CH₂OCH₂CH₂- or -CH₂CH₂CH₂-.

In one embodiment, at least five of R^{16a}, R^{16b}, R^{16c}, R^{16d}, R^{16e}, and R^{16f} are hydrogen and the other is hydroxy.

In one embodiment, at least five of R^{16a}, R^{16b}, R^{16c}, R^{16d}, R^{16e}, and R^{16f} are hydrogen and the other is -CO₂R⁵. Preferably, R⁵ is hydrogen or C1-C3 alkyl.

In one embodiment, at least five of R^{16a}, R^{16b}, R^{16c}, R^{16d}, R^{16e}, and R^{16f} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, at least three of R^{17a}, R^{17b}, R^{17c}, R^{17d}, R^{17e}, and R^{17f} are hydrogen.

In one embodiment, at least four of R^{17a}, R^{17b}, R^{17c}, R^{17d}, R^{17e}, and R^{17f} are hydrogen.

In one embodiment, at least five of R^{17a}, R^{17b}, R^{17c}, R^{17d}, R^{17e}, and R^{17f} are hydrogen.

In one embodiment, six of R^{17a}, R^{17b}, R^{17c}, R^{17d}, R^{17e}, and R^{17f} are all hydrogen.

In one embodiment, at least five of R^{17a}, R^{17b}, R^{17c}, R^{17d}, R^{17e}, and R^{17f} are hydrogen, and the other is hydrogen, halogen, hydroxy, C1-C3 alkyl, C1-C3 cyanoalkyl, C1-C3 hydroxyalkyl, C1-C3 alkyl-N(R⁵)₂, -C(O)H, -CO₂R⁵, -CO₂N(R⁵)₂, -CH₂CH₂-, -CH₂OCH₂CH₂-, or -CH₂CH₂CH₂-.

In one embodiment, at least five of R^{17a}, R^{17b}, R^{17c}, R^{17d}, R^{17e}, and R^{17f} are hydrogen and the other is -CH₂CH₂-, -CH₂OCH₂CH₂- or -CH₂CH₂CH₂-.

In one embodiment, at least five of R^{17a}, R^{17b}, R^{17c}, R^{17d}, R^{17e}, and R^{17f} are hydrogen and the other is hydroxy.

In one embodiment, at least five of R^{17a}, R^{17b}, R^{17c}, R^{17d}, R^{17e}, and R^{17f} are hydrogen and the other is -CO₂R⁵. Preferably, R⁵ is hydrogen or C1-C3 alkyl.

In one embodiment, at least five of R^{17a}, R^{17b}, R^{17c}, R^{17d}, R^{17e}, and R^{17f} are hydrogen and the other is -CO₂N(R⁵)₂. Preferably, each R⁵ is independently hydrogen or C1-C3 alkyl; or one R⁵ is hydrogen and the other R⁵ is C1-C3 alkyl.

In one embodiment, R^{17g} is hydrogen or methyl.

In one embodiment, W is N.

In one embodiment, W is C(R⁴¹); wherein R⁴¹ is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

In one embodiment, R⁴¹ is cyclopropyl, cyclopropyl-O-, cyclobutyl, cyclobutyl-O-, cyclopentyl, cyclopentyl-O-, tetrahydrofuranyl, or tetrahydrofuran-O-; wherein the cyclopropyl, cyclobutyl, cyclopentyl, tetrahydrofuranyl are each independently substituted with halogen.

In one embodiment, R⁴¹ is halogen.

In one embodiment, R⁴¹ is C1-C3 alkyl.

In one embodiment, R⁴¹ is fluoro.

In one embodiment, R⁴¹ is methyl.

In one embodiment, X is N.

In one embodiment, X is C(R⁴²); wherein R⁴² is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

In one embodiment, R⁴² is cyclopropyl, cyclopropyl-O-, cyclobutyl, cyclobutyl-O-, cyclopentyl, cyclopentyl-O-, tetrahydrofuranyl, or tetrahydrofuran-O-; wherein the cyclopropyl, cyclobutyl, cyclopentyl, tetrahydrofuranyl are each independently substituted with halogen.

In one embodiment, R⁴² is halogen.

In one embodiment, R⁴² is C1-C3 alkyl.

In one embodiment, R⁴² is fluoro.

In one embodiment, R⁴² is methyl.

In one embodiment, Z is N.

In one embodiment, Z is C(R⁴³); wherein R⁴³ is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

In one embodiment, R⁴³ is cyclopropyl, cyclopropyl-O-, cyclobutyl, cyclobutyl-O-, cyclopentyl, cyclopentyl-O-, tetrahydrofuranyl, or tetrahydrofuran-O-; wherein the cyclopropyl, cyclobutyl, cyclopentyl, tetrahydrofuranyl are each independently substituted with halogen.

In one embodiment, R⁴³ is halogen.

In one embodiment, R⁴³ is C1-C3 alkyl.

In one embodiment, R⁴³ is fluoro.

In one embodiment, R⁴³ is methyl.

In one embodiment, R⁴⁴ is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen; the C1-C3 alkyl is methyl, ethyl or propyl; the C1-C3 alkoxy is methoxy, ethoxy or propoxy; the C3-C6 monocyclic cycloalkyl is cyclopropyl, cyclobutyl, cyclopentyl or cyclohexyl; the 3- to 6-membered monocyclic heterocyclyl is tetrahydrofuranyl, tetrahydropyrrolyl, piperidinyl or piperazinyl.

In one embodiment, X is N; W is C(F); Z is C(F) or C(H).

In one embodiment, X is N; W is C(F); Z is N.

In one embodiment, the compound is shown in Formula (a), formula (b), formula (c), or Formula (d):

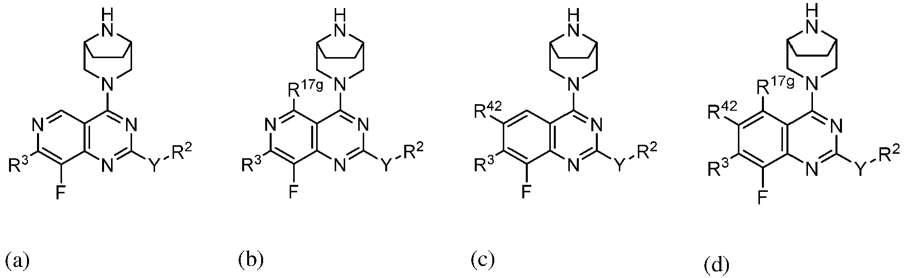

In each formula, each of R², R³, Y is as defined above; each R⁴² is independently hydrogen or halogen; each R^{17g} is independently halogen or C1-C4 alkyl.

In one embodiment, the compound is shown in Formula (a1), Formula (b1), Formula (c1), Formula (d1), Formula (a2), Formula (b2), Formula (c2), or Formula (d2):

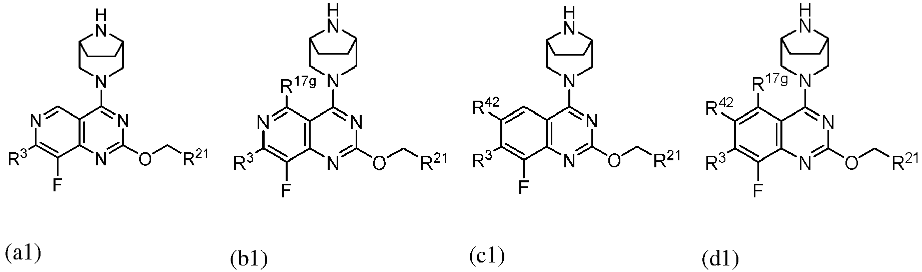

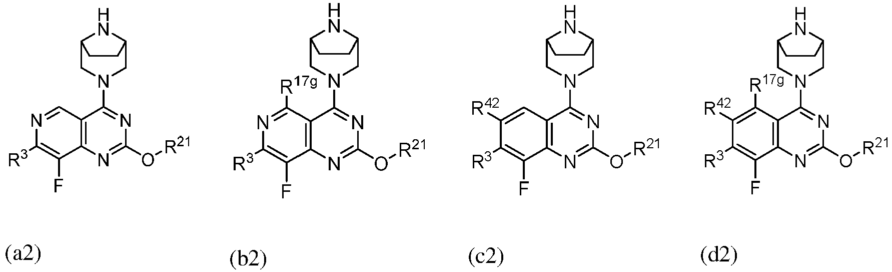

In each formula,
each R³ is as defined above; each R⁴² is independently hydrogen or halogen; each R^{17g} is independently halogen or C1-C4 alkyl;
each R²¹ is each independently 6- to 10-membered fused heterocyclyl, 7- to 11-membered spiroheterocyclyl, spirocyclic ring substituted 6- to 10-membered fused heterocyclyl, spirocyclic ring substituted 7- to 11-membered spiroheterocyclyl, fused ring substituted 6- to 10-membered fused heterocyclyl, fused ring substituted 7- to 11-membered spiroheterocyclyl, or spirocyclic and fused ring substituted 6- to 10-membered fused heterocyclyl;
in Formula (a1), Formula (c1), Formula (a2), Formula (c2), when each R²¹ is independently a 6- to 10-membered fused heterocyclyl or a 7- to 11-membered spiroheterocyclyl, two hydrogen atoms on the same carbon atom of any one or two carbon atoms of the 6- to 10-membered fused heterocyclyl or 7- to 11-membered spiroheterocyclyl must both be substituted with =CR^{2a}R^{2b} simultaneously; the 6- to 10-membered fused heterocyclyl or 7- to 11-membered spiroheterocyclyl is further optionally substituted with one or more R⁶;
in Formula (b1), Formula (d1), Formula (b2), Formula (d2), wherein, when each R²¹ is independently 6- to 10-membered fused heterocyclyl or 7- to 11-membered spiroheterocyclyl, two hydrogen atoms on the same carbon atom of any one or two carbon atoms of the 6- to 10-membered fused heterocyclyl or 7- to 11-membered spiroheterocyclyl are optionally simultaneously substituted with =CR^{2a}R^{2b}; the 6- to 10-membered fused heterocyclyl or 7- to 11-membered spiroheterocyclyl is further optionally substituted with one or more R⁶;
in Formula (a1), Formula (c1), Formula (a2), Formula (c2), when R²¹ is spirocyclic ring substituted 6- to 10-membered fused heterocyclyl and the spirocyclic ring substituted 6- to 10-membered fused heterocyclyl is 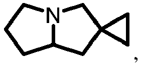 two hydrogen atoms on the same carbon atom of any one or two carbon atoms of the 6- to 10-membered fused heterocyclyl must both be substituted with =CR^{2a}R^{2b} simultaneously; the spirocyclic ring substituted 6- to 10-membered fused heterocyclyl is further optionally substituted with one or more R⁶;
in Formula (a1), Formula (c1), Formula (a2), and Formula (c2), when R²¹ is spirocyclic ring substituted 6- to 10-membered fused heterocyclyl and the spirocyclic ring substituted 6- to 10-membered fused heterocyclyl is not 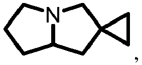 two hydrogen atoms on the same carbon atom of any one or two carbon atoms of the 6- to 10-membered fused heterocyclyl are optionally simultaneously substituted with =CR^{2a}R^{2b}; the spirocyclic ring substituted 6- to 10-membered fused heterocyclyl is further optionally substituted with one or more R⁶;
in Formula (b1), Formula (d1), Formula (b2), Formula (d2), when R²¹ is a spirocyclic ring substituted 6- to 10-membered fused heterocyclyl, a hydrogen atom on one or two carbon atoms of the spirocyclic ring substituted 6- to 10-membered fused heterocyclyl are optionally simultaneously substituted with =CR^{2a}R^{2b}; the spirocyclic ring substituted 6- to 10-membered fused heterocyclyl is further optionally substituted with one or more R⁶;
in Formula (a1), Formula (c1), Formula (a2), Formula (c2), Formula (b1), Formula (d1), Formula (b2), Formula (d2), when R²¹ is spirocyclic ring substituted 7- to 11-membered spiroheterocyclyl, two hydrogen atoms on the same carbon atom of any one or two carbon atoms of the spirocyclic ring substituted 7- to 11-membered spiroheterocyclyl are optionally simultaneously substituted with =CR^{2a}R^{2b}; the spirocyclic ring substituted 7- to 11-membered spiroheterocyclyl is further optionally substituted with one or more R⁶;
in Formula (a1), Formula (c1), Formula (a2), Formula (c2), Formula (b1), Formula (d1), Formula (b2), Formula (d2), when R²¹ is fused ring substituted 6- to 10-membered fused heterocyclyl, two hydrogen atoms on the same carbon atom of any one or two carbon atoms of the fused ring substituted 6- to 10-membered fused heterocyclyl are optionally simultaneously substituted with =CR^{2a}R^{2b}; the fused ring substituted 6- to 10-membered fused heterocyclyl is further optionally substituted with one or more R⁶;
in Formula (a1), Formula (c1), Formula (a2), Formula (c2), Formula (b1), Formula (d1), Formula (b2), Formula (d2), when R²¹ is fused ring substituted 7- to 11-membered spiroheterocyclyl, two hydrogen atoms on the same carbon atom of any one or two carbon atoms of the fused ring substituted 7- to 11-membered spiroheterocyclyl is optionally simultaneously substituted with =CR^{2a}R^{2b}; the fused ring substituted 7- to 11-membered spiroheterocyclyl is further optionally substituted with one or more R⁶;
in Formula (a1), Formula (c1), Formula (a2), Formula (c2), Formula (b1), Formula (d1), Formula (b2), Formula (d2), when R²¹ is spirocyclic and fused ring substituted 6- to 10-membered fused heterocyclyl, two hydrogen atoms on the same carbon atom of any one or two carbon atoms of the spirocyclic and fused ring substituted 6- to 10-membered fused heterocyclyl are optionally simultaneously substituted with =CR^{2a}R^{2b}; the spirocyclic and fused ring substituted 6- to 10-membered fused heterocyclyl is further optionally substituted with one or more R⁶.

In one embodiment, the 6- to 10-membered fused heterocyclyl is selected from the group consisting of:

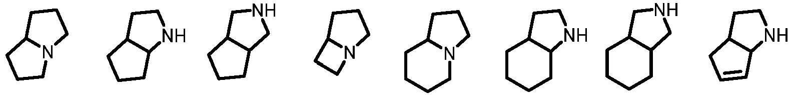

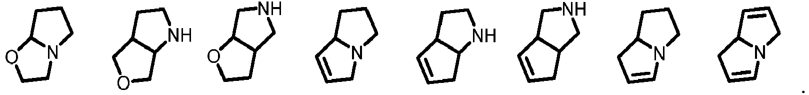

In one embodiment, the 6- to 10-membered fused heterocyclyl is selected from the group consisting of: 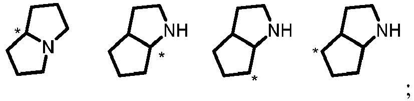 * labeled carbon atoms are carbon atoms attached to the rest of the molecule.

In one embodiment, the 7- to 11-membered spiroheterocyclyl is selected from the group consisting of:

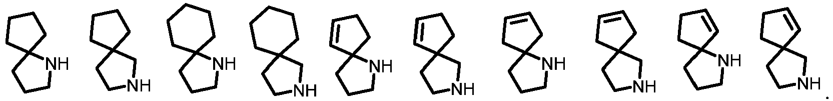

In one embodiment, the 7- to 11-membered spiroheterocyclyl is selected from the group consisting of: 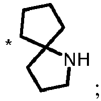 * labeled carbon atoms are carbon atoms attached to the rest of the molecule.

In one embodiment, the spirocyclic ring substituted 6- to 10-membered fused heterocyclyl is selected from the group consisting of:

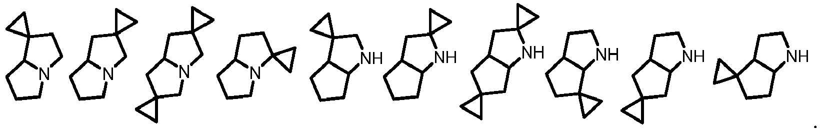

In one embodiment, the spirocyclic ring substituted 6- to 10-membered fused heterocyclyl is selected from the group consisting of: 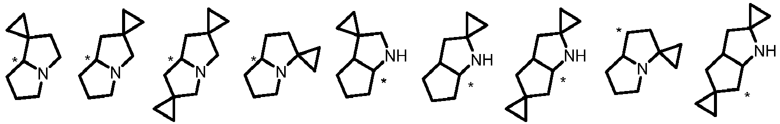 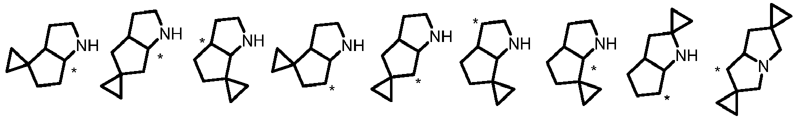 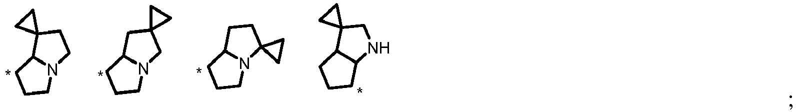 * labeled carbon atoms are carbon atoms attached to the rest of the molecule.

In one embodiment, the spirocyclic ring substituted 7- to 11-membered spiroheterocyclyl is selected from the group consisting of:

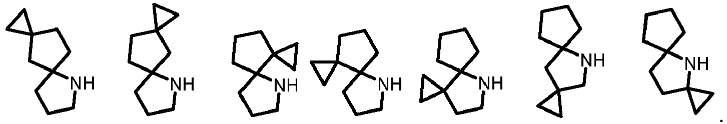

In one embodiment, the fused ring substituted 6- to 10-membered fused heterocyclyl is selected from the group consisting of:

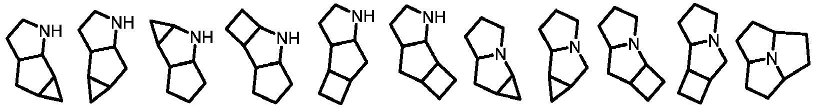

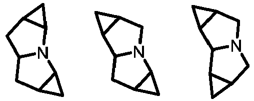

In one embodiment, the fused ring substituted 6- to 10-membered fused heterocyclyl is selected from the group consisting of: 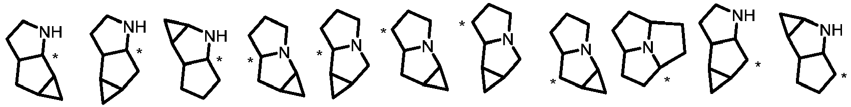 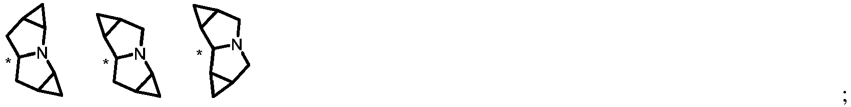 * labeled carbon atoms are carbon atoms attached to the rest of the molecule.

In one embodiment, the fused ring substituted 7- to 11-membered spiroheterocyclyl is selected from the group consisting of:

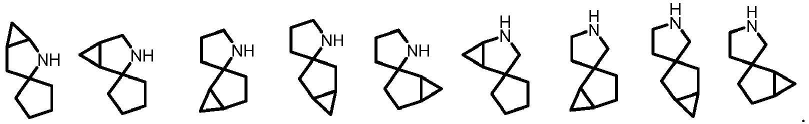

In one embodiment, the fused ring substituted 7- to 11-membered spiroheterocyclyl is selected from the group consisting of: 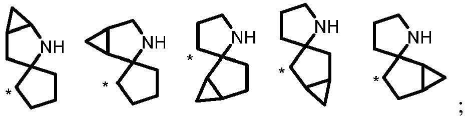 * labeled carbon atoms are carbon atoms attached to the rest of the molecule.

In one embodiment, the spirocyclic and fused ring substituted 6- to 10-membered fused heterocyclyl is selected from the group consisting of:

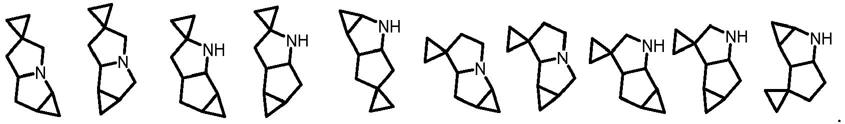

In one embodiment, the spirocyclic and fused ring substituted 6- to 10-membered fused heterocyclyl is selected from the group consisting of: 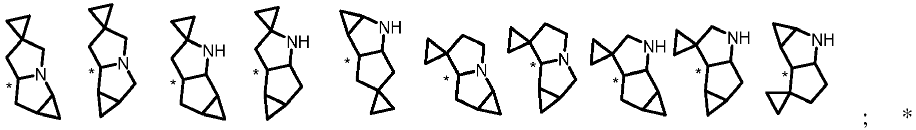 labeled carbon atoms are carbon atoms attached to the rest of the molecule.

In one embodiment, R¹ is selected from the group consisting of:

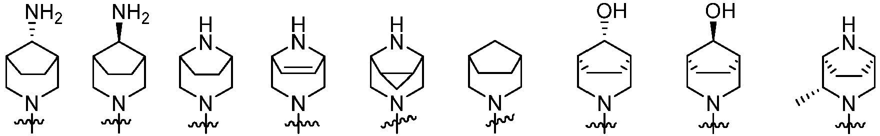

In one embodiment, Y is a bond.

In one embodiment, Y is O.

In one embodiment, Y is NR⁵.

In one embodiment, L is a bond, methylene or ethylene, or propylene.

In one embodiment, L is a bond.

In one embodiment, L is methylene.

In one embodiment, L is a bond, methylene or ethylene or propylene; wherein one or two hydrogen atoms on any one carbon atom of the methylene or ethylene or propylene group are each independently substituted with hydrogen, deuterium or C1-C3 alkyl; or two hydrogen atoms on any one carbon atom of the methylene or ethylene or propylene group are simultaneously substituted by -CH₂- or -CH₂CH₂- to form a cyclopropyl or cyclobutyl group.

In one embodiment, L is a bond, methylene or ethylene or propylene; wherein two hydrogen atoms on any one carbon atom of the methylene or ethylene or propylene group are each independently substituted with deuterium or C1-C3 alkyl; or two hydrogen atoms on any one carbon atom of the methylene or ethylene or propylene group are simultaneously substituted by -CH₂- or -CH₂CH₂- to form a cyclopropyl or cyclobutyl group.

In one embodiment, L is selected from the group consisting of: 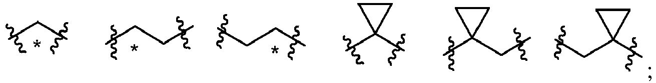 wherein, one or two hydrogen atoms on the * labeled carbon atom are each independently substituted with hydrogen, deuterium, or C1-C3 alkyl.

In one embodiment, R² is hydrogen, -N(R⁵)₂, 3 to 20 membered heterocyclyl, C1-C6 alkyl, -L-3 to 20 membered heterocyclyl, -L-C6-C14 aryl, -L-5 to 14 membered heteroaryl, -L-C3-C20 cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-C6-C14 aryl, -L-COOH or -L-C(O)OC1-C6 alkyl; wherein each of the 3- to 20-membered heterocyclyl, C6-C14 aryl of the -L-NR⁵C(O)-C6-C14 aryl, and the C3-C20 cycloalkyl independently may be optionally substituted with one or more R⁶; C6-C14 aryl in-L-C6-C14 aryl, 5-14 membered heteroaryl in-L-5-14 membered heteroaryl, each independently, may be optionally substituted with one or more R⁷; L, R⁵, R⁶, R⁷ are as defined above.

In one embodiment, R² is hydrogen, -N(R⁵)₂, 3- to 8-membered monocyclic heterocyclyl, 5- to 20-membered spiroheterocyclyl, 5- to 20-membered fused heterocyclyl, 5- to 20-membered bridged heterocyclyl, C1-C6 alkyl, -L-3- to 8-membered monocyclic heterocyclyl, -L-5- to 20-membered spiroheterocyclyl, -L-5- to 20-membered fused heterocyclyl, -L-5- to 20-membered bridged heterocyclyl, -L-phenyl, -L-naphthyl, -L-5- to 14-membered heteroaryl, -L-C3-C12 cycloalkyl, -L-C5-C20 spirocycloalkyl, C5-C20 fused cycloalkyl, -L-C5-C20 bridged cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-phenyl, -L-NR⁵C(O)-naphthyl, -L-COOH, or -L-C(O)OC1-C6 alkyl; wherein, the 3- to 8-membered monocyclic heterocyclyl, 5- to 20-membered spiroheterocyclyl, 5- to 20-membered fused heterocyclyl, 5- to 20-membered bridged heterocyclyl, C3-C12 cycloalkyl, C5-C20 spirocycloalkyl, C5-C20 fused cycloalkyl, C5-C20 bridged cycloalkyl, phenyl of the -L-NR⁵C(O)-phenyl, naphthyl of the -L-NR⁵C(O)-naphthyl each independently may be optionally substituted with one or more R⁶; phenyl in-L-phenyl, naphthyl in-L-naphthyl, 5- to 14-membered heteroaryl in-L-5- to 14-membered heteroaryl, each independently, may be optionally substituted with one or more R⁷; L and R⁵ are as defined above.

In one embodiment, R² is hydrogen or -N(R⁵)₂. Preferably, each R⁵ is independently hydrogen or C1-C3 alkyl; or one R⁵ is hydrogen and the other R⁵ is C1-C3 alkyl.

In one embodiment, R² is heterocyclyl. Preferably, the heterocyclyl is hexahydro-1H-pyrrolizinyl, hexahydro-3H-pyrrolizin-3-one, hexahydro-1H-pyrrolo[2,1-c][1,4]oxazinyl, octahydroindolizinyl, hexahydropyrrolizinyl 4 (1H)-oxide, azetidinyl, pyrrolidinyl, pyrrolidin-2-one, oxetanyl, piperidinyl, 1-azabicyclo[2.2.1]heptyl, morpholinyl, oxa-5-azabicyclo[2.2.1]hept -5-yl, thiopyranyl, 6-oxa-2-azaspiro[3.4]octyl, 7-oxa-2-azaspiro[3.5]nonyl, 2',3'-dihydrospiro[cyclopropane-1,1 '-indenyl], (2S)-1-azabicyclo[2.2.1]heptan-2-yl or tetrahydrofuranyl.

In one embodiment, R² is heterocyclyl or -L-heterocyclyl; wherein each the heterocyclyl is independently hexahydro-1H-pyrrolizinyl, hexahydro-3H-pyrrolizin-3-one, hexahydro-1H-pyrrolo[2,1-c][1,4]oxazinyl, octahydroindolizinyl, hexahydropyrrolizinyl 4 (1H)-oxide, azetidinyl, pyrrolidinyl, pyrrolidin-2-one, oxetanyl, piperidinyl, 1-azabicyclo[2.2.1]heptyl, morpholinyl, oxa-5-azabicyclo[2.2.1]hept-5-yl, thiopyranyl, 6-oxa-2-azaspiro[3.4]octyl, 7-oxa-2-azaspiro[3.5]nonyl, 2',3'-dihydrospiro[cyclopropane-1,1'-indenyl], (2S)-1-azabicyclo[2.2.1]heptan -2-yl or tetrahydrofuranyl; each of which is independently optionally substituted with one or more R⁶.

In one embodiment, R² is -L-heterocyclyl; wherein the heterocyclyl is hexahydro-1H-pyrrolizinyl. In one embodiment, R² is -L-heterocyclyl; wherein the heterocyclyl is hexahydro-1H-pyrrolizinyl substituted with one R⁶; wherein R⁶ is halogen, hydroxy, C1-C3 hydroxyalkyl, C1-C3 haloalkyl, C1-C3 alkyl, C1-C3 alkoxy, phenyl, pyrazolyl, or -CH₂OC(O)N(R⁵)₂; R⁵ is as defined above.

In one embodiment, the halogen is fluorine.

In one embodiment, the heterocyclyl is hexahydro-1H-pyrrolizinyl further substituted with two additional R⁶; the other two R⁶ are each independently C1-C3 alkyl.

In one embodiment, R² is -L-heterocyclyl; wherein the heterocyclyl is azetidinyl substituted with one R⁶; the R⁶ is C1-C3 alkyl.

In one embodiment, R² is -L-heterocyclyl; wherein the heterocyclyl is pyrrolidinyl substituted with one R⁶; R⁶ is hydroxyalkyl, haloalkyl, C1-C3 alkyl, alkoxy, aryl C1-C3 alkyl, -Q-phenyl, and - NHC(O)phenyl; wherein the aryl in aryl C1-C3 alkyl or the phenyl in-Q-phenyl or the phenyl in-NHC(O)phenyl are each independently optionally substituted with one or more R⁷.

In one embodiment, the phenyl in the -Q-phenyl or -NHC(O)phenyl is substituted with-SO₂F.

In one embodiment, R² is -L-heterocyclyl; wherein the heterocyclyl is pyrrolidinyl substituted with two R⁶ groups; wherein one R⁶ is C1-C3 alkyl and the other R⁶ is C1-C3 alkoxy or halogen.

In one embodiment, R² is -L-heterocyclyl; wherein the heterocyclyl is pyrrolidin -2-one substituted with one R⁶; wherein R⁶ is C1-C3 alkyl.

In one embodiment, R² is -L-heterocyclyl; wherein the heterocyclyl is piperidinyl substituted with one R⁶; wherein R⁶ is acetyl, (C1-C3 alkoxy) C1-C3 alkoxy or -C(O)CH₂Cl.

In one embodiment, R² is -L-heterocyclyl; wherein the heterocyclyl is morpholinyl or oxa-5-azabicyclo[2.2.1]hept-5-yl.

In one embodiment, R² is -L-heteroaryl; wherein the heteroaryl is optionally substituted with one or more R⁷; the heteroaryl is pyridinyl, pyrazolyl, imidazolyl, triazolyl, 4,5,6,7-tetrahydro-1H-indazolyl, benzimidazolyl, imidazo[1,2-a]pyridinyl or pyrimidinyl.

In one embodiment, R² is -L-heteroaryl; wherein the heteroaryl is optionally substituted with one R⁷; the heteroaryl is pyridyl; wherein R⁷ is halogen, C1-C4 alkyl, -N(R⁵)₂ or C1-C4 alkoxy.

In one embodiment, R² is -L-heteroaryl; wherein the heteroaryl is optionally substituted with one R⁷; the heteroaryl is pyrazolyl; wherein R⁷ is C1-C4 alkyl or -N(R⁵)₂.

In one embodiment, R² is -L-heteroaryl; wherein the heteroaryl is optionally substituted with one R⁷; the heteroaryl is imidazolyl; wherein R⁷ is C1-C4 alkyl, C1-C4 haloalkyl or C1-C4 hydroxyalkyl.

In one embodiment, R² is -L-heteroaryl; wherein the heteroaryl is optionally substituted with one R⁷; the heteroaryl is triazolyl; wherein R⁷ is C1-C4 alkyl.

In one embodiment, R² is -L-aryl; wherein the aryl is optionally substituted with one or more R⁷;
R⁷ is as defined above.

In one embodiment, R² is -L-cycloalkyl; wherein the cycloalkyl is optionally substituted with one or more R⁶.

In one embodiment, R² is -L-N(R⁵)₂; R⁵ is as defined above.

In one embodiment, R² is -L-N(R⁵)₂; L is ethylene; Each R⁵ is independently selected from C1-C3 alkyl; R⁵ is as defined above.

In one embodiment, R² is -L-N(R⁵)₂; wherein L is methylene, ethylene, or propylene; wherein one or two hydrogen atoms on any one carbon atom of the methylene, ethylene, or propylene groups are each independently substituted with hydrogen, deuterium or C1-C3 alkyl; or two hydrogen atoms on any one carbon atom of the methylene or ethylene or propylene group are simultaneously substituted by -CH₂- or -CH₂CH₂- to form a cyclopropyl or cyclobutyl group; each R⁵ is independently selected from C1-C3 alkyl.

In one embodiment, R² is -L-NC(=NH)-NH₂.

In one embodiment, R² is -L-C1-C6 haloalkyl.

In one embodiment, R² is -L-OR⁵; R⁵ is as defined above.

In one embodiment, R² is -L-(CH₂OR⁵)(CH₂)ₙOR⁵; R⁵ is as defined above.

In one embodiment, R² is -L-NR⁵C(O)-aryl; wherein each of the aryl groups may be optionally substituted with one or more R⁶; R⁵ and R⁶ are as defined above.

In one embodiment, the Y-R² is selected from the group consisting of: 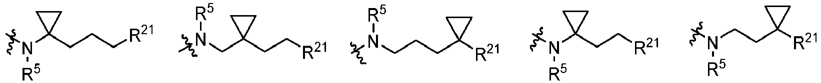 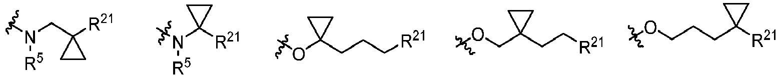 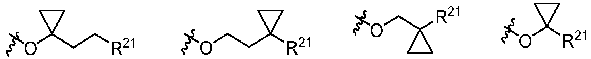 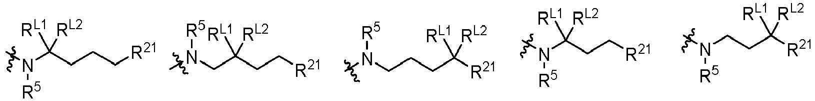 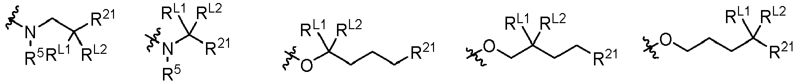 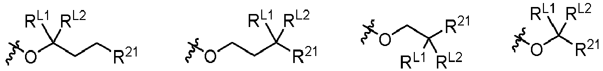 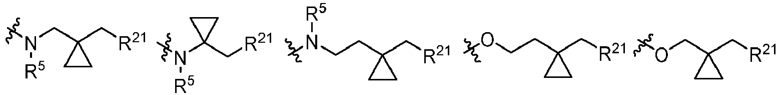 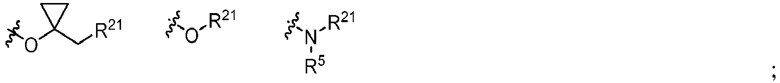
each R^{L1} is each independently hydrogen, deuterium, C1-C6 alkyl, or C1-C6 haloalkyl;
each R^{L2} is each independently hydrogen, deuterium, C1-C6 alkyl, or C1-C6 haloalkyl;
each R²¹ is independently heterocyclyl, aryl, heteroaryl, cycloalkyl, -N(R⁵)₂, -NHC(=NH)NH₂, - C(O)N(R⁵)₂, -C1-C6 haloalkyl, -OR⁵, -(CH₂OR⁵)(CH₂)ₙOR⁵, -NR⁵C(O)-aryl, -COOH, or - C(O)OC1-C6 alkyl; wherein each of the heterocyclyl, the aryl of -NR⁵C(O)-aryl, and the cycloalkyl may be optionally substituted with one or more R⁶; the aryl, each of the heteroaryl may be optionally substituted with one or more R⁷; n, R⁵, R⁶, R⁷ are as defined above.

In one embodiment, R²¹ is selected from the group consisting of:

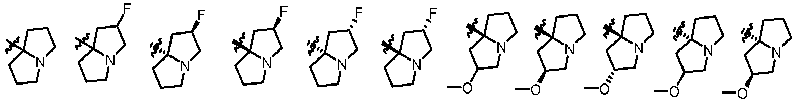

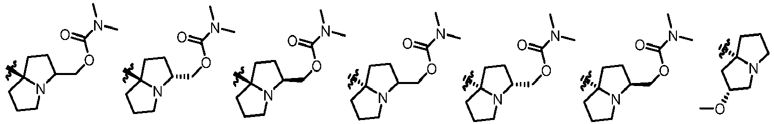

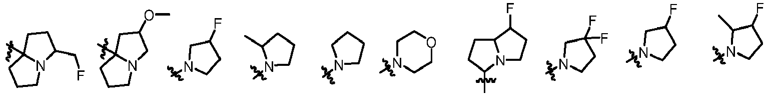

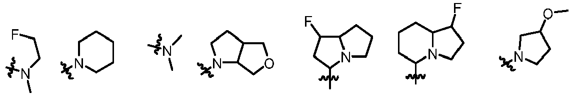

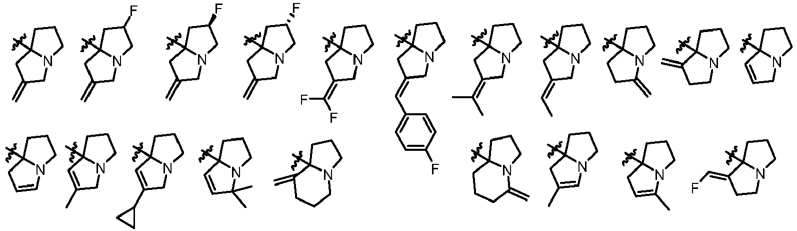

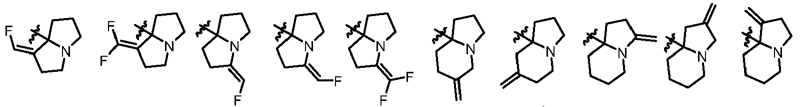

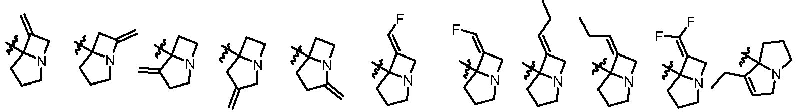

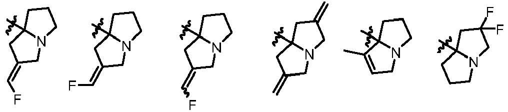

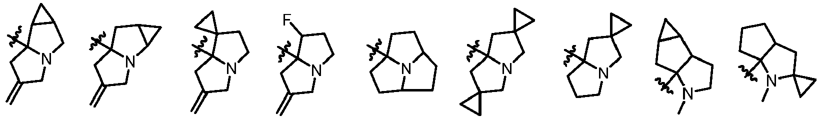

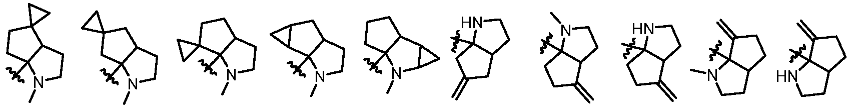

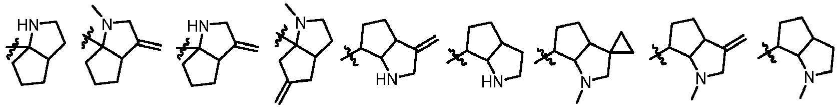

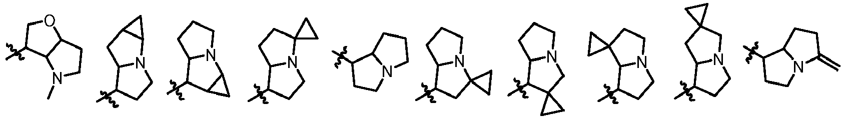

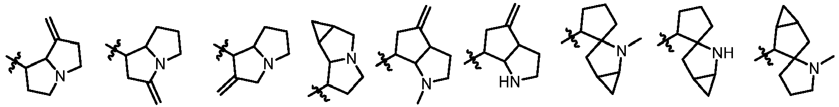

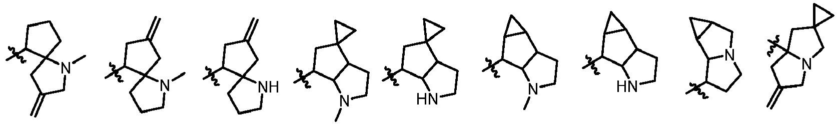

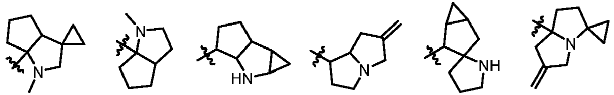

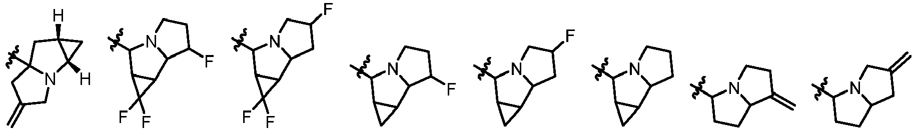

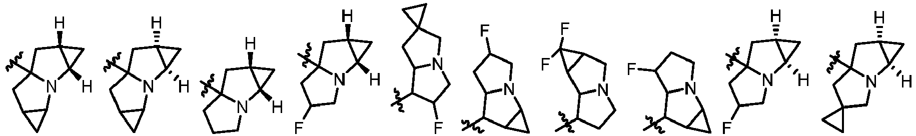

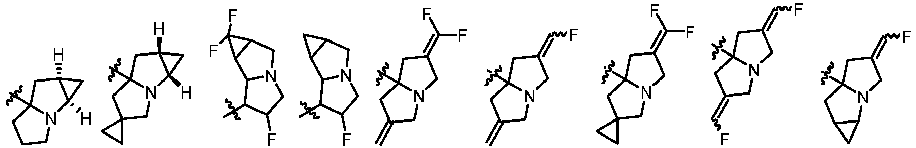

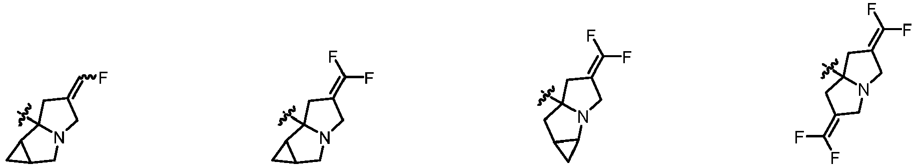

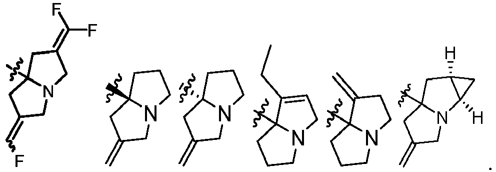

In one embodiment, R²¹ is hexahydro-1H-pyrrolizinyl.

In one embodiment, R²¹ is hexahydro-1H-pyrrolizinyl substituted with one R⁶; wherein R⁶ is halogen, hydroxy, C1-C3 hydroxyalkyl, C1-C3 haloalkyl, C1-C3 alkyl, C1-C3 alkoxy, phenyl, pyrazolyl, or -CH₂OC(O)N(R⁵)₂; each R⁵ is each independently hydrogen, C1-C3 alkyl, or C1-C3 haloalkyl; or two R⁵ are each independently taken together with the nitrogen atom to which they are attached to form a 3- to 6-membered nitrogen-containing heterocyclyl optionally substituted with one or two groups selected from halogen, methyl, trifluoromethyl, methoxy, trifluoromethoxy. In one embodiment, the Y-R² is selected from the group consisting of:

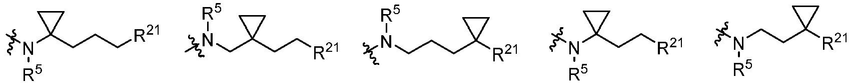

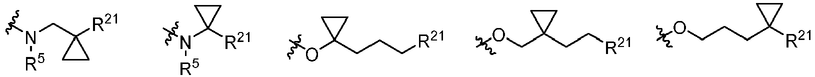

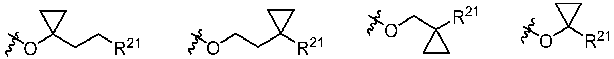

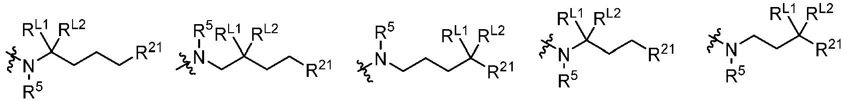

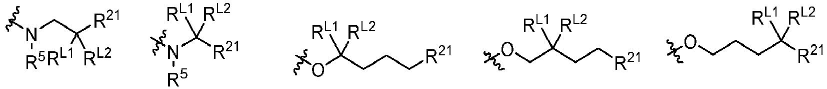

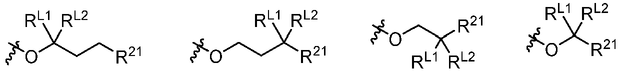

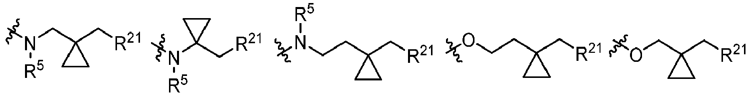

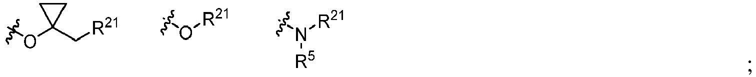

In each formula,
each R^{L1} is each independently hydrogen, deuterium, C1-C6 alkyl, or C1-C6 haloalkyl; each R^{L2} is each independently hydrogen, deuterium, C1-C6 alkyl, or C1-C6 haloalkyl; R⁵ is the same as before;
R²¹ is selected from the group consisting of:

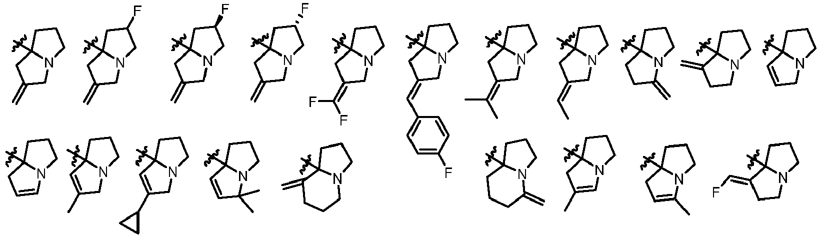

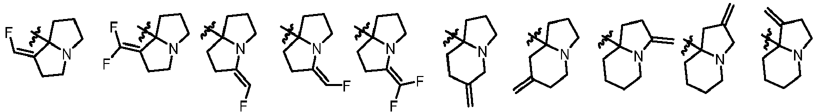

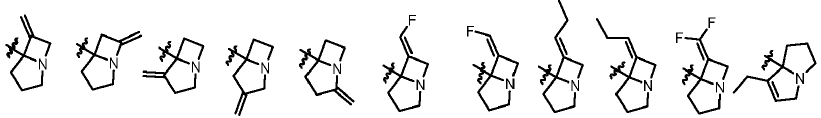

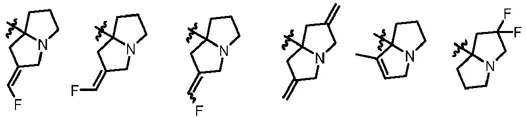

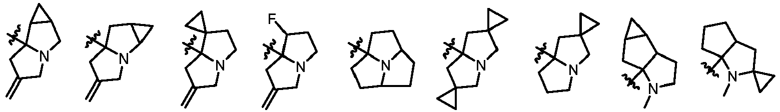

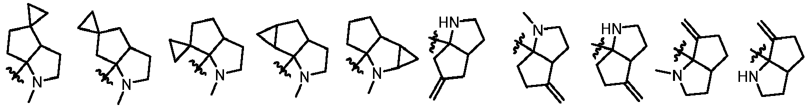

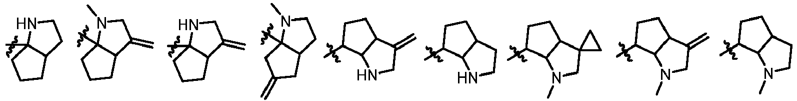

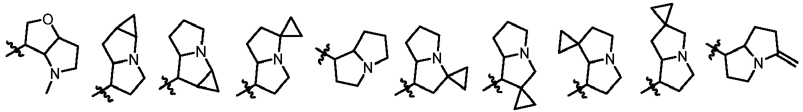

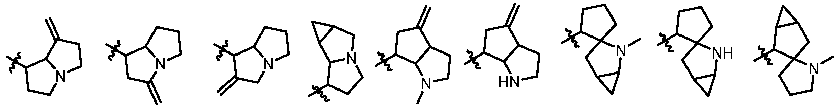

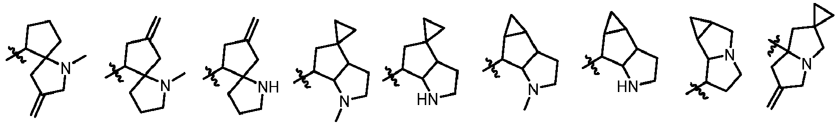

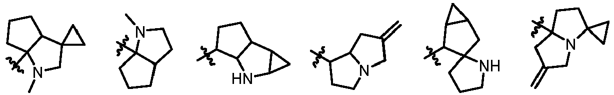

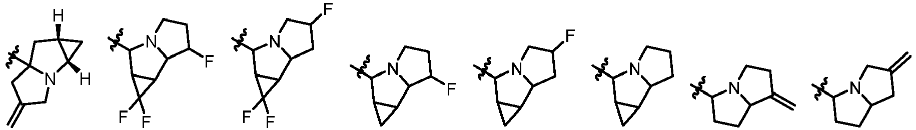

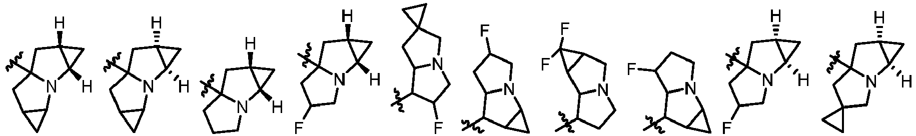

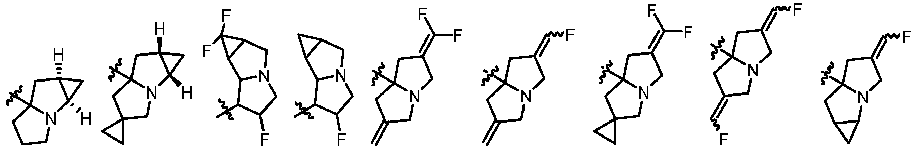

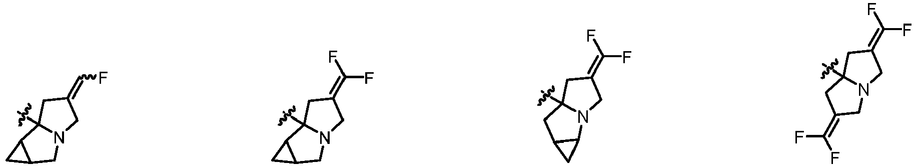

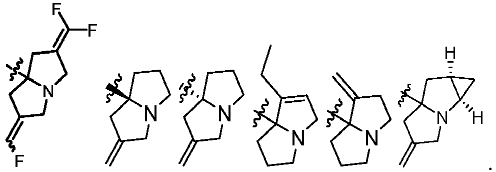

In one embodiment, R² is selected from the group consisting of: 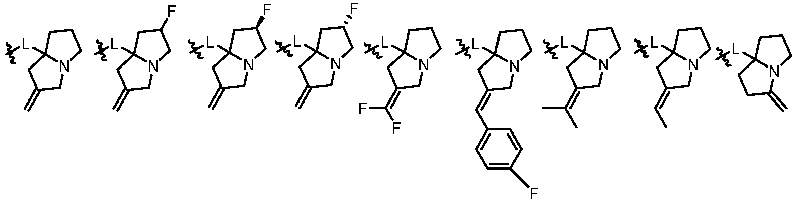 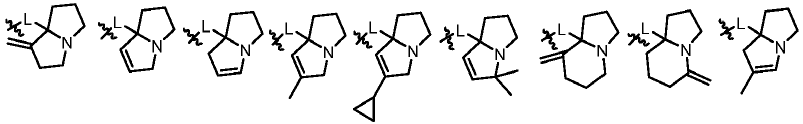 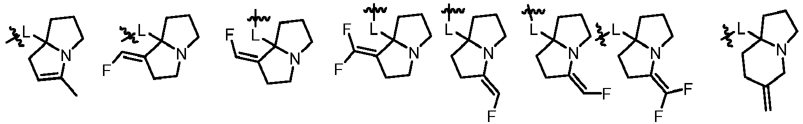 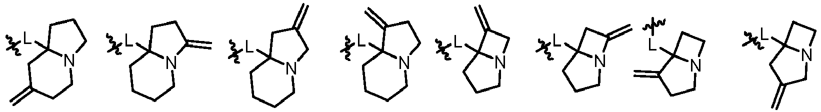 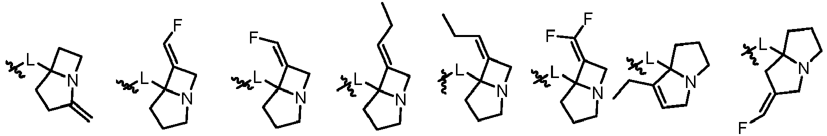 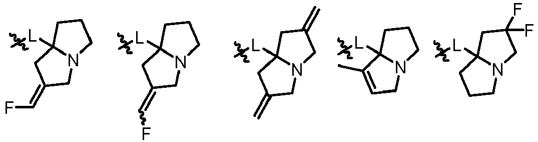 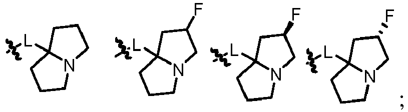 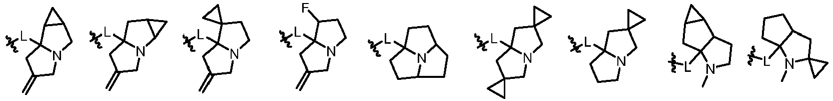 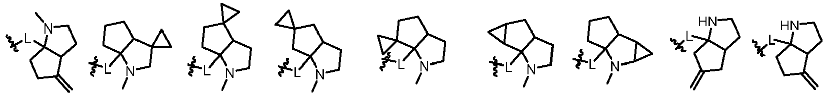 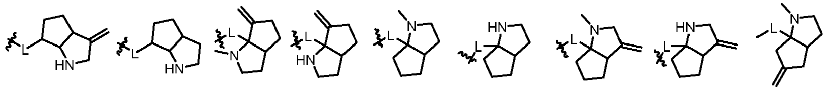 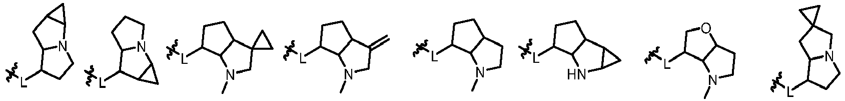 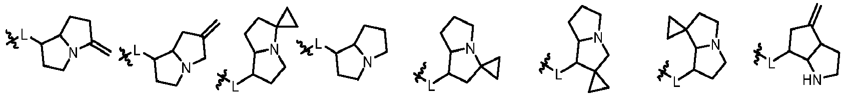 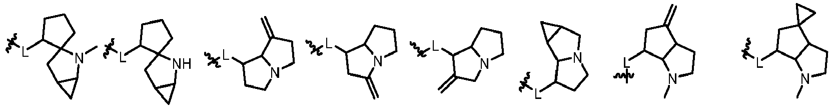 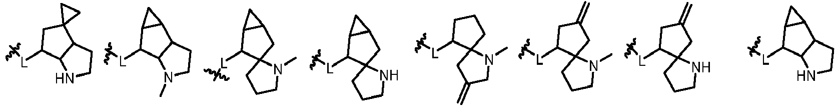 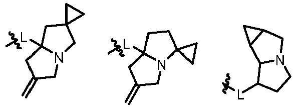 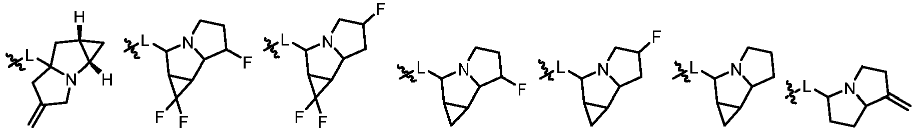 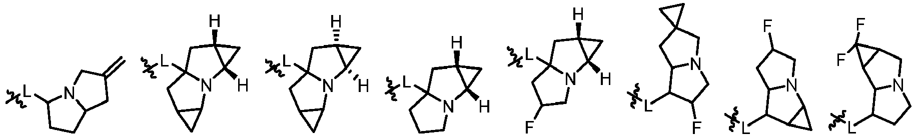 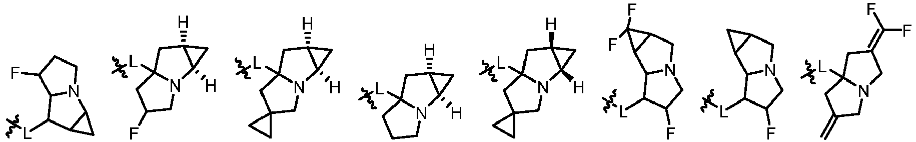 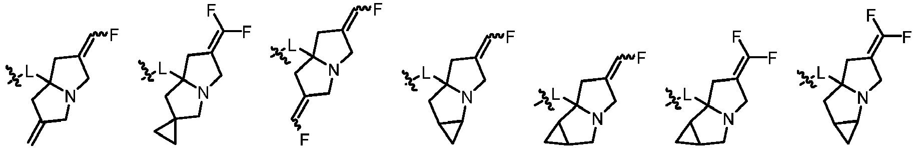 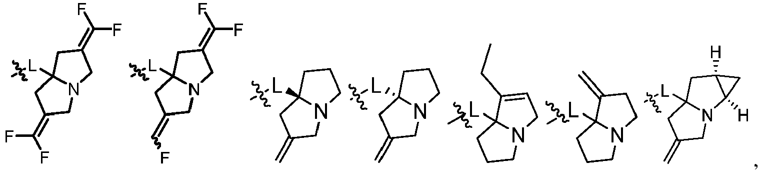 wherein L is as defined above.

In one embodiment, L is -CH₂-.

In one embodiment, L is a bond.

In one embodiment, R² is 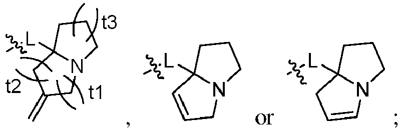 wherein t1, t2 are each independently 0, 1, 2, or 3; t3 is 0, 1, or 2; each of which is optionally substituted with one or more R⁶; or two hydrogen atoms on the same carbon atom on each of the above can be optionally substituted with-CH₂CH₂-, -CH₂OCH₂CH₂-, or -CH₂CH₂CH₂-.

In one embodiment, R² is selected from the group consisting of 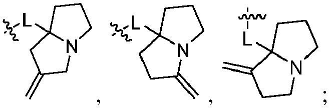 each of which is optionally substituted with one or more R⁶; or two hydrogen atoms on the same carbon atom on each of the above can be optionally substituted with-CH₂CH₂-, -CH₂OCH₂CH₂-, or -CH₂CH₂CH₂-.

In one embodiment, Y-R² is -O-R²¹, or -O-CH₂-R²¹; R²¹ is selected from the group consisting of:

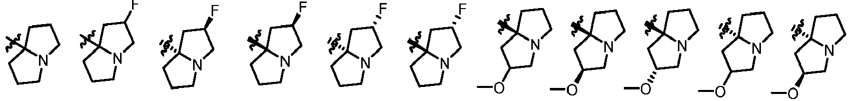

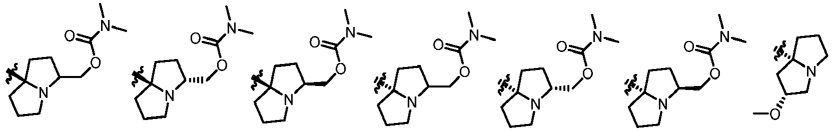

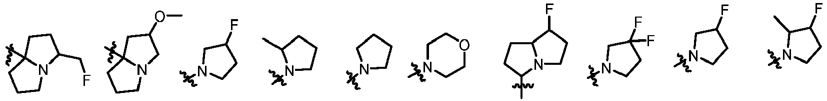

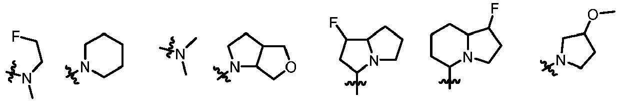

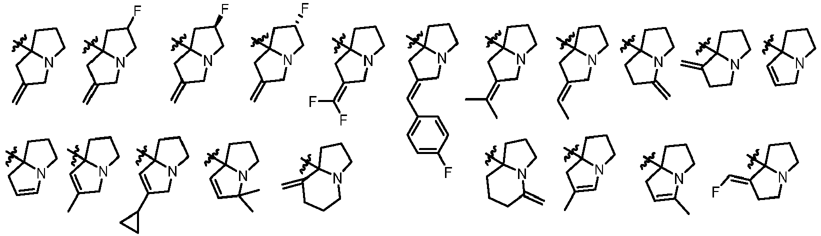

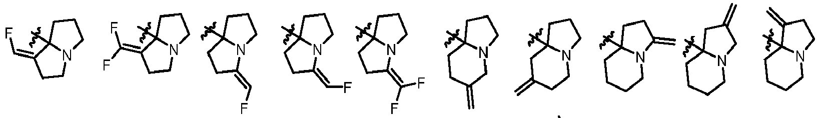

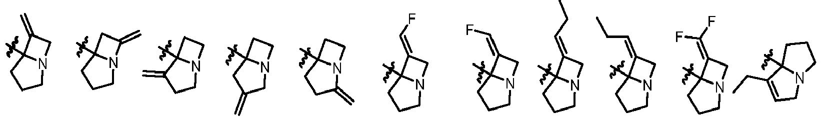

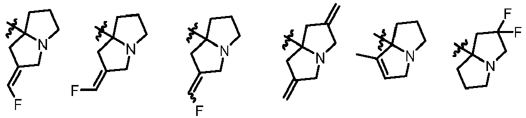

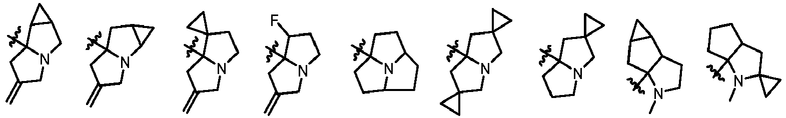

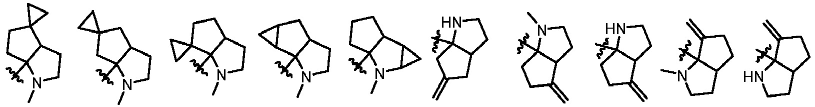

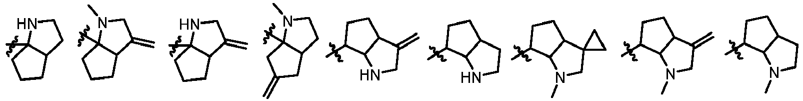

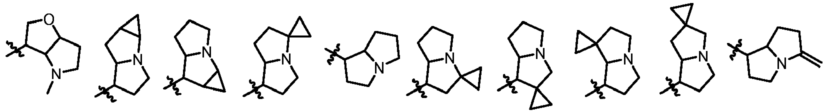

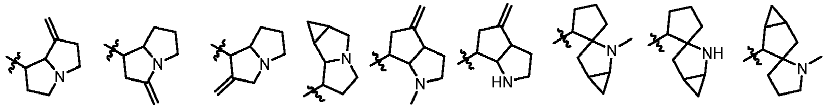

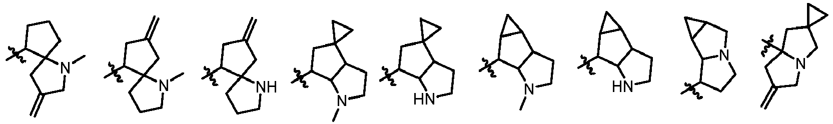

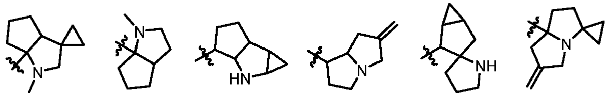

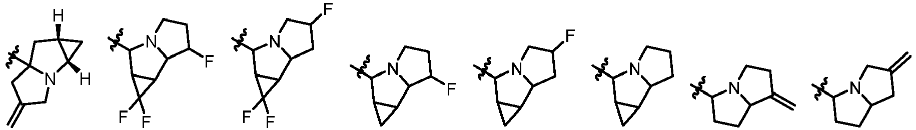

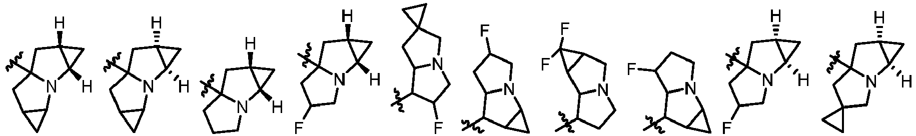

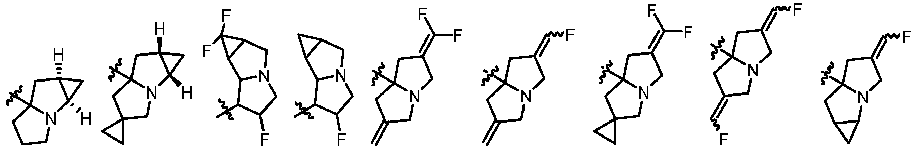

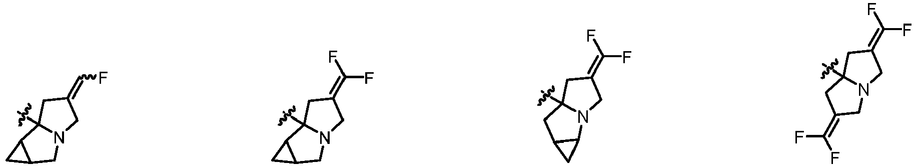

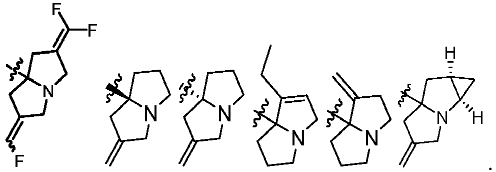

In one embodiment, R² is selected from the group consisting of:

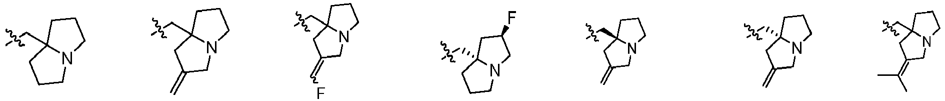

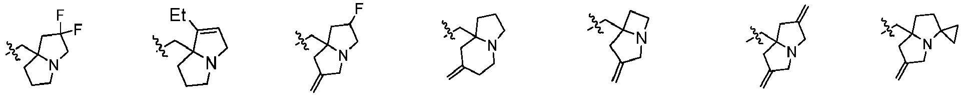

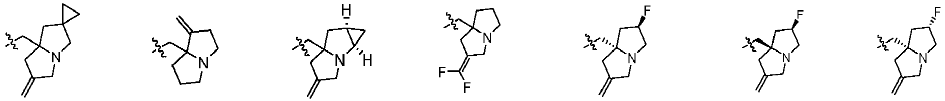

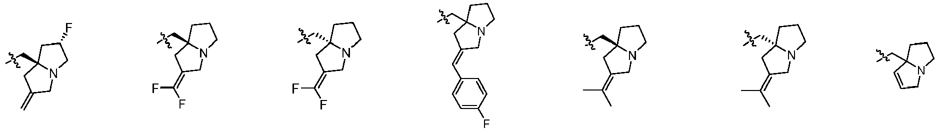

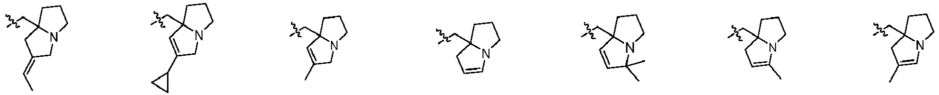

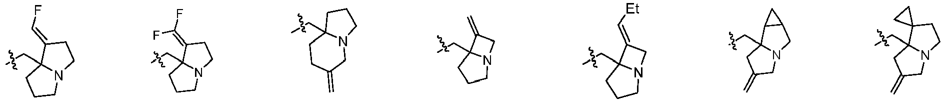

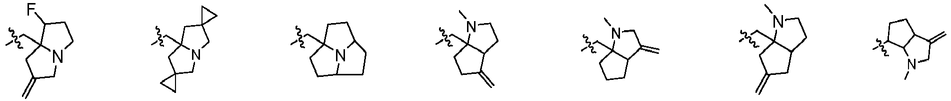

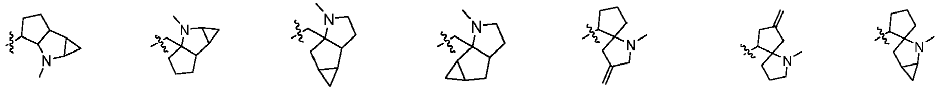

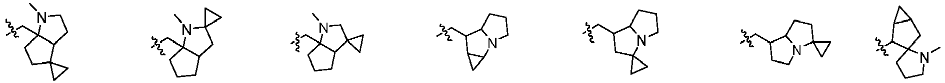

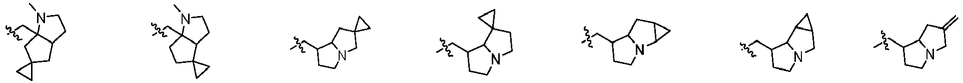

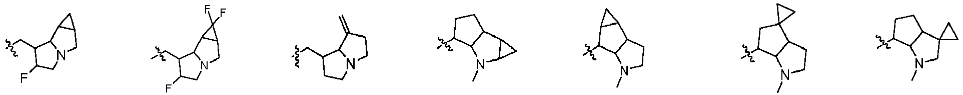

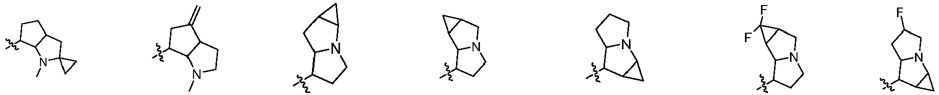

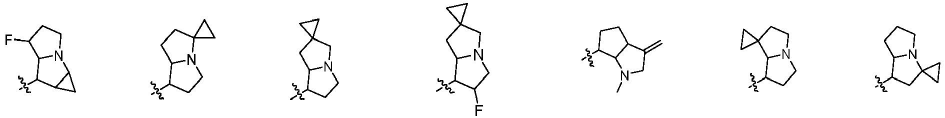

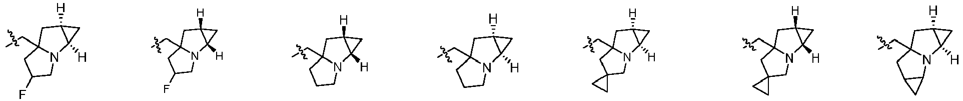

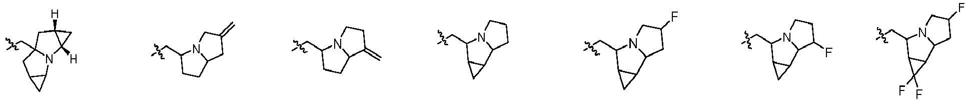

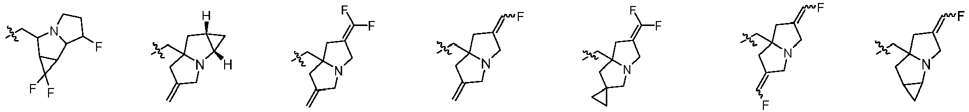

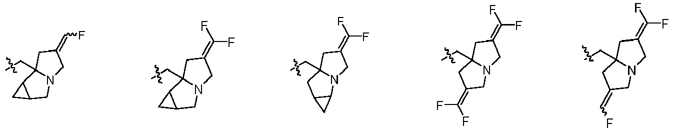

In one embodiment, R² is selected from the group consisting of:

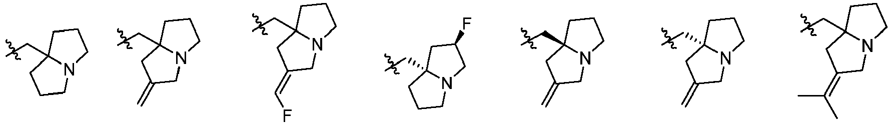

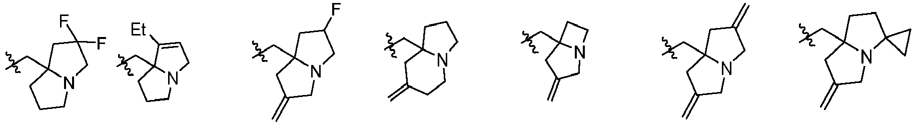

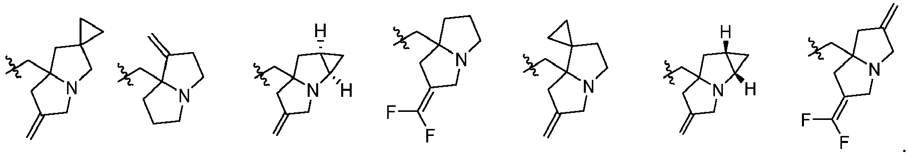

In one embodiment, R³ is aryl; wherein the aryl is optionally substituted with one or more R⁸.

In one embodiment, the aryl group is phenyl, naphthyl, 1,2,3,4-tetrahydronaphthalene or 2,3-dihydro-1H-indenyl; the phenyl, naphthyl, 1,2,3,4-tetrahydronaphthalene, 2,3-dihydro-1H-indenyl are each independently optionally substituted with one or more R⁸.

In one embodiment, R³ is naphthyl substituted with hydroxy.

In one embodiment, R³ is naphthyl substituted with halo.

In one embodiment, R³ is naphthyl substituted with fluoro, chloro, or bromo.

In one embodiment, R³ is naphthyl substituted with C1-C3.

In one embodiment, R³ is naphthyl substituted with methyl or ethyl.

In one embodiment, R³ is naphthyl substituted with C2-C4 alkenyl.

In one embodiment, R³ is naphthyl substituted with ethenyl or propenyl.

In one embodiment, R³ is naphthyl substituted with C2-C4 alkynyl.

In one embodiment, R³ is ethynyl or propynyl substituted naphthyl.

In one embodiment, R³ is naphthyl substituted with one or two, or three R⁸; wherein each R⁸ is independently halogen, hydroxy, cyano, C1-C3 alkyl, -S-C1-C3 alkyl, C2-C4 alkenyl, C2-C4 alkynyl, C2-C4 hydroxyalkynyl, C1-C3 hydroxyalkynyl, C1-C3 cyanoalkyl or triazolyl.

In one embodiment, R³ is naphthyl substituted with two or three R⁸; wherein each R⁸ is independently halogen, hydroxy, C1-C3 alkyl, or C2-C4 alkynyl.

In one embodiment, R³ is naphthyl substituted with two R⁸; wherein each R⁸ is independently hydroxy or ethynyl.

In one embodiment, R³ is naphthyl substituted with three R⁸; wherein each R⁸ is independently hydroxy, halogen, or ethynyl.

In one embodiment, R³ is phenyl substituted with one, two, or three R⁸; wherein each R⁸ is independently halogen, hydroxy, ethynyl, methyl, methyl-O-, methyl-S-, trifluoromethyl, trifluoromethyl-O-, amino, isopropyl, or cyclopropyl; The cyclopropyl is optionally substituted with halogen or methyl.

In one embodiment, R³ is naphthyl substituted with one, two, or three R⁸; wherein each R⁸ is independently halogen, hydroxy, ethynyl, methyl, methyl-O-, methyl-S-, trifluoromethyl, trifluoromethyl-O-, amino, isopropyl, or cyclopropyl; The cyclopropyl is optionally substituted with halogen or methyl.

In one embodiment, R³ is heteroaryl; wherein the heteroaryl is optionally substituted with one or more R⁸.

In one embodiment, R³ is isoquinolinyl, indazolyl, or benzo[d][1,3]dioxolane; wherein the isoquinolinyl, indazolyl, or benzo[d][1,3]dioxolane are each independently optionally substituted with one or more R⁸.

In one embodiment, R³ is pyridinyl substituted with one, two, or three R⁸; wherein each R⁸ is independently halogen, hydroxy, ethynyl, methyl, methyl-O-, methyl-S-, trifluoromethyl, trifluoromethyl-O-, amino, isopropyl, or cyclopropyl; The cyclopropyl is optionally substituted with halogen or methyl.

In one embodiment, R³ is isoquinolinyl, indazolyl, or benzo[d][1,3]dioxolane optionally substituted with one or more R⁸.

In one embodiment, R³ is isoquinolinyl substituted with one, two, or three R⁸; wherein each R⁸ is independently halogen, hydroxy, ethynyl, methyl, methyl-O-, methyl-S-, trifluoromethyl, trifluoromethyl-O-, amino, isopropyl, or cyclopropyl; The cyclopropyl is optionally substituted with halogen or methyl.

In one embodiment, R³ is indazolyl substituted with one, two, or three R⁸; wherein each R⁸ is independently C1-C3 alkyl.

In one embodiment, R³ is benzo[d][1,3]dioxolane substituted with two R⁸ groups; wherein each R⁸ is independently selected from halogen.

In one embodiment, R³ is phenyl, naphthyl, 1,2,3,4-tetrahydronaphthalene, 2,3-dihydro-1H-indenyl, isoquinolinyl, indazolyl, or benzo[d][1,3]dioxolane; wherein the phenyl, naphthyl, 1,2,3,4-tetrahydronaphthalene, 2,3,-dihydro-1H-indenyl, isoquinolinyl, indazolyl, or benzo[d][1,3]dioxolane are each independently optionally substituted with one, two, or three R⁸; wherein each R⁸ is independently selected from the group consisting of halogen, cyano, hydroxy, methyl, ethyl, cyclopropyl, halomethyl, ethynyl.

In one embodiment, R³ is selected from the group consisting of:

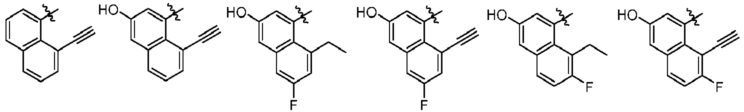

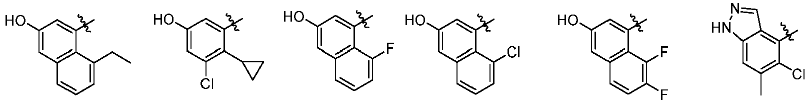

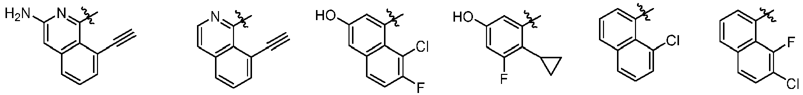

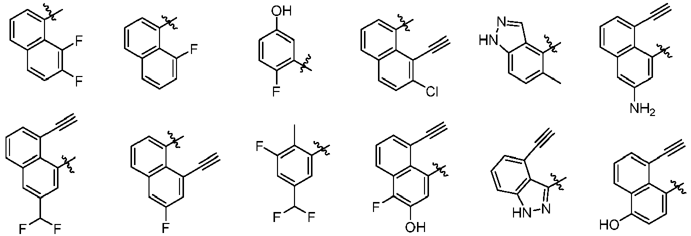

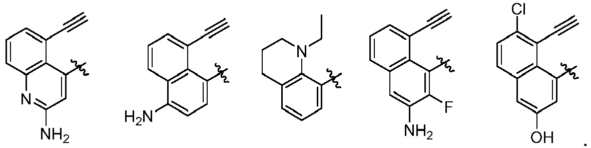

In one embodiment, R³ is selected from the group consisting of:

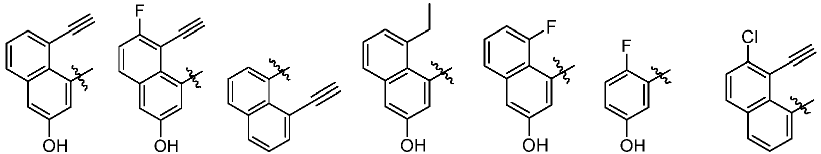

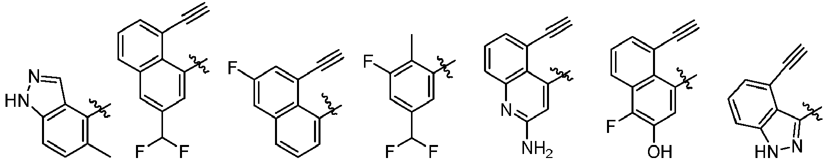

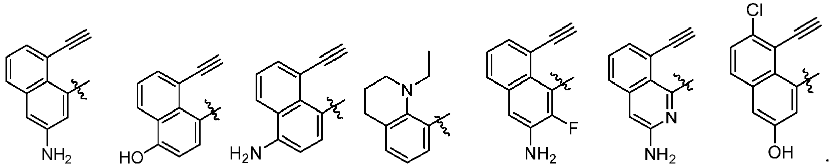

In one embodiment, R⁵ is hydrogen, C1-C3 alkyl, or C1-C3 cyanoalkyl.

In one embodiment, the C1-C6 alkyl is methyl, ethyl, isopropyl, or isobutyl.

In one embodiment, the compound of formula (AI) or the compound of formula (AII) is selected from Table (I):

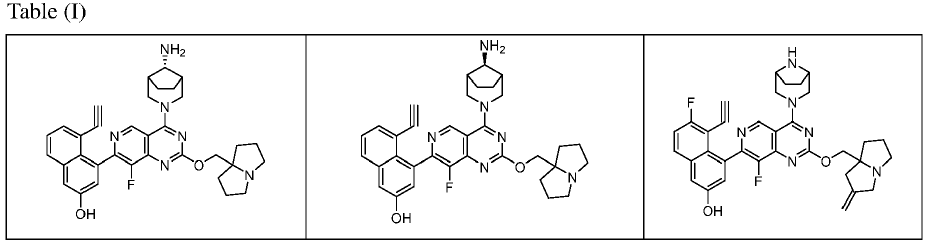

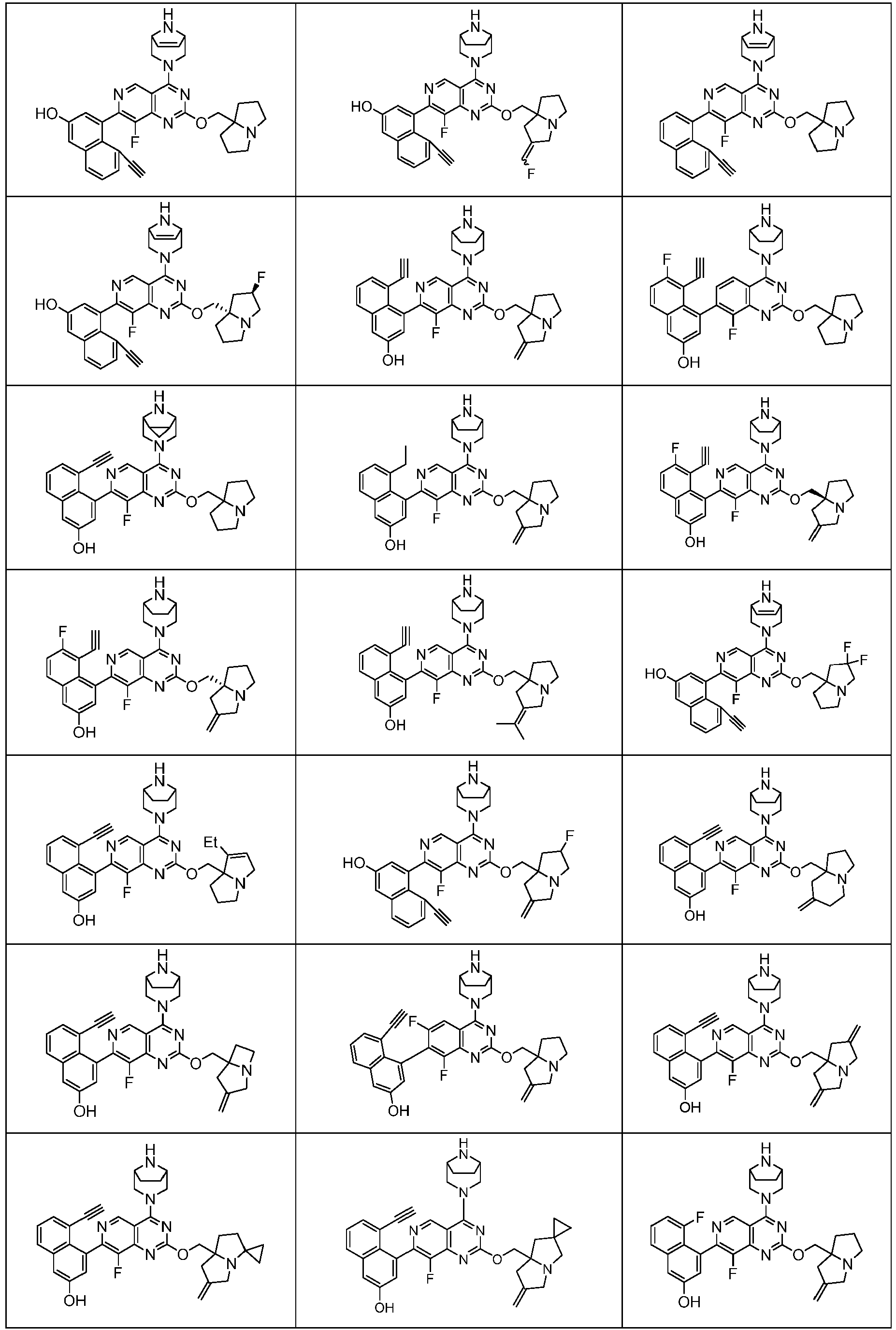

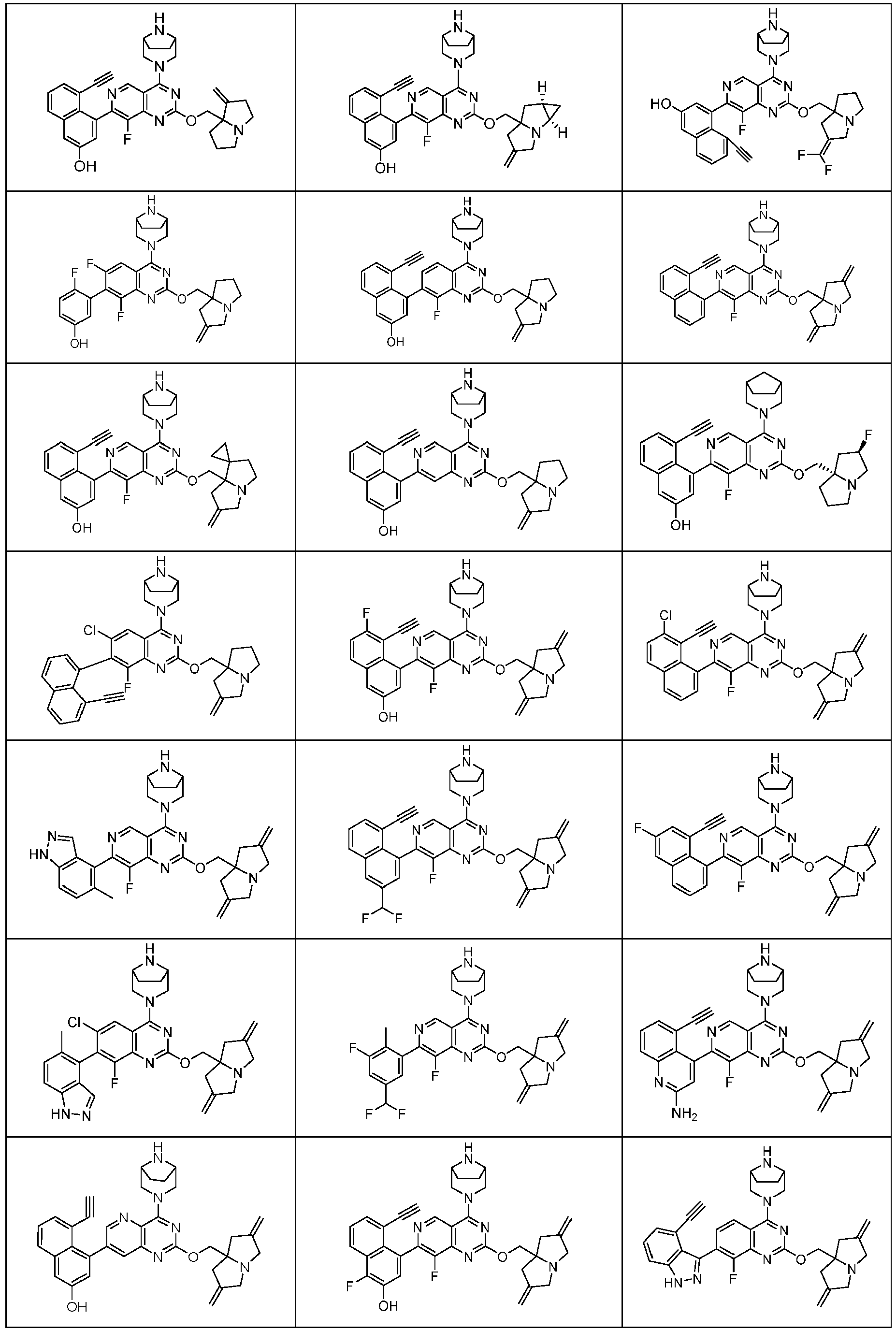

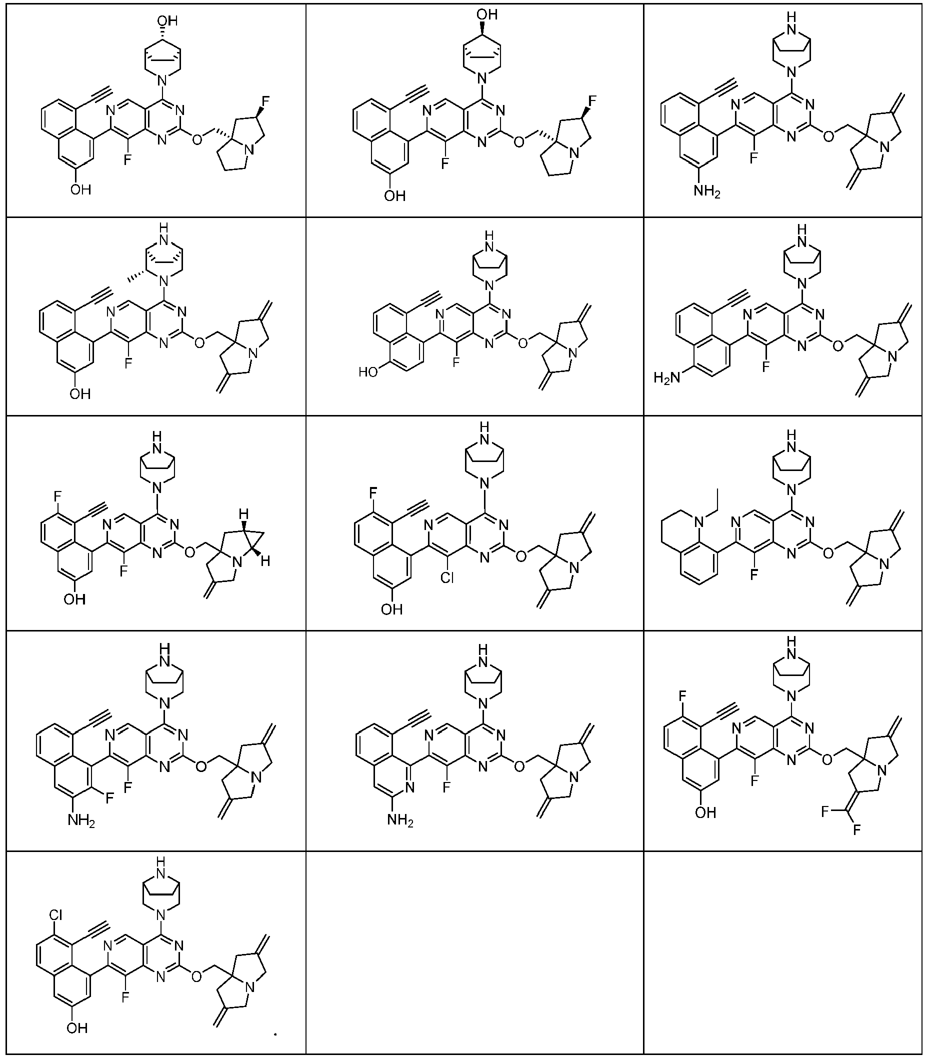

A second aspect of the invention provides a pharmaceutical composition including a compound of the above aspect or a pharmaceutically acceptable salt, a stereoisomer, or a solvate thereof; and a pharmaceutically acceptable carrier.

As used herein, the term "pharmaceutically acceptable carrier" refers to any formulation or carrier medium representative of carriers that are capable of delivering an effective amount of an active substance of the present disclosure without interfering with the biological activity of the active substance and without toxic side effects to the host or subject, including water, oils, vegetables and minerals, cream bases, lotion bases, ointment bases and the like. Such bases include suspending agents, viscosity enhancers, transdermal enhancers, and the like. Their formulations are well known to those skilled in the cosmetic or topical pharmaceutical arts.

In an embodiment of the invention, the pharmaceutical composition may be administered in any of the following ways: oral, inhalation spray, rectal, nasal, buccal, topical, parenteral, such as subcutaneous, intravenous, intramuscular, intraperitoneal, intrathecal, intraventricular, intrasternal and intracranial injection or infusion, or via an explanted reservoir. Oral, intraperitoneal, or intravenous administration is preferred. When administered orally, the compounds of the present disclosure may be formulated in any orally acceptable dosage form including, but not limited to, tablets, capsules, aqueous solutions, or aqueous suspensions. Carriers for tablets generally include lactose and corn starch, and lubricating agents such as magnesium stearate may be added. Diluents used in capsule formulations typically include lactose and dried corn starch. Aqueous suspension formulations are generally prepared by mixing the active ingredient with suitable emulsifying and suspending agents. If desired, certain sweetening, flavoring, or coloring agents may be added to the above oral dosage forms. In the case of topical application, and in particular in the treatment of conditions or organs readily accessible by topical application, such as neurological diseases of the eye, skin, or lower intestinal tract, the compounds of the present disclosure may take the form of various topical formulations depending on the condition or organ concerned. In the case of topical application to the eye, the compounds of the present disclosure may be formulated in the form of a micronized suspension or solution in a carrier such as isotonic, sterile saline of fixed pH, with or without a preservative such as benzylalkonium chloride. For ophthalmic use, the compounds may also be formulated in an ointment such as petrolatum. When applied topically to the skin, the compounds of the present disclosure can be formulated with a suitable ointment, lotion, or cream formulation in which the active ingredient is suspended or dissolved in one or more carriers. Carriers that may be used for ointment formulations include, but are not limited to mineral oil, liquid petrolatum, white petrolatum, propylene glycol, polyethylene oxide, polypropylene oxide, emulsifying wax, and water; carriers that may be used for lotions or creams include but are not limited to mineral oil, sorbitan monostearate, Tween 60, cetyl esters wax, cetearyl alcohol, 2-octyldodecanol, benzyl alcohol, and water. The compounds of the present disclosure can also be administered in the form of sterile injectable preparations, including sterile injectable aqueous or oleaginous suspensions or sterile injectable solutions. Among the acceptable vehicles and solvents that may be employed are water, ringer's solution, and isotonic sodium chloride solution. In addition, sterile, fixed oils may be employed as a solvent or suspending medium, such as mono- or diglycerides.

According to a further aspect of the present disclosure, there is provided the use of a compound according to the above aspect or a pharmaceutically acceptable salt, a stereoisomer or a solvate thereof, or a pharmaceutical composition according to the above aspect in the manufacture of a medicament for the prophylaxis and/or treatment of a disease or condition; the disease or condition is a KRAS G12D associated disease or disorder.

As used herein, "KRAS G12D" refers to a mutant form of a mammalian KRAS protein that contains an aspartic acid substituted for glycine at amino acid position 12. The assignment of amino acid codons and residue positions for human KRAS is based on the amino acid sequence identified by UniProtKB/Swiss-Prot P01116: Variantp.Gly12Asp.

As used herein, "KRAS G12D inhibitor" refers to a compound of the present disclosure. These compounds can negatively regulate or inhibit all or part of the enzymatic activity of KRAS G12D. As used herein, a "KRAS G12D associated disease or disorder" refers to a disease or disorder associated with, mediated by KRAS G12D, or having a KRAS G12D mutation. A non-limiting example of a KRAS G12D associated disease or disorder is KRAS G12D associated cancer. Another aspect of the invention provides a method of treating a KRAS G12D-associated cancer including the steps of: administering to a subject in need thereof a therapeutically effective amount of a compound according to the above aspect of the invention or a pharmaceutically acceptable salt, a stereoisomer, or a solvate thereof; or administering to a subject in need thereof a therapeutically effective amount of a pharmaceutical composition according to the above aspects of the invention. Herein, in one embodiment, the KRAS G12D-associated cancer includes but is not limited to, lung cancer, prostate cancer, breast cancer, brain cancer, skin cancer, cervical cancer, testicular cancer, and the like. In one embodiment, the KRAS G12D-associated cancer includes but is not limited to, astrocytoma, breast cancer, cervical cancer, colorectal cancer, endometrial cancer, esophageal cancer, gastric cancer, head and neck cancer, hepatocellular cancer, laryngeal cancer, lung cancer, oral cancer, ovarian cancer, prostate cancer, thyroid cancer, sarcomas, and the like. In one embodiment, the KRAS G12D-associated cancer includes, but is not limited to, cancers of cardiac sites such as sarcomas (angiosarcomas, fibrosarcomas, rhabdomyosarcomas, liposarcomas), myxomas, rhabdomyomas, fibromas, lipomas, teratomas, and the like; cancers of the lung, for example, bronchial carcinoma (squamous cell, undifferentiated small cell, undifferentiated large cell, adenocarcinoma), alveolar (bronchiolar) carcinoma, bronchial adenoma, sarcoma, lymphoma, chondroma hamartoma, mesothelioma; cancers of the gastrointestinal tract, for example, esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small intestine (adenocarcinoma, carcinoid tumors), kaposi's sarcoma, leiomyoma, hemangioma, lipoma, neurofibroma, fibroma), large intestine (adenocarcinoma, tubular adenoma, villous adenoma, hamartoma, leiomyoma); cancers of the genitourinary tract, for example, kidney (adenocarcinoma, wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratoma), choriocarcinoma, sarcoma, interstitial cell carcinoma, fibroma, fibroadenoma, adenomatoid tumors, lipoma); cancers of the liver, for example, liver cancer (hepatocellular carcinoma), cholangiocarcinoma, hepatoblastoma, angiosarcoma, hepatocellular adenoma, hemangioma; biliary: gallbladder cancer, ampulla cancer, bile duct cancer; cancers of bone sites, e.g. osteogenic sarcoma (osteosarcoma), fibrosarcoma, malignant fibrous histiocytoma, chondrosarcoma, ewing's sarcoma, malignant lymphoma (reticulum cell sarcoma), multiple myeloma, malignant giant cell tumor, chordoma, osteochondroma (osteochondral chondroma), benign chondroma, benign chondromatous osteoma, and giant cell tumor; cancers of the nervous system, for example, skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma, glioblastoma multiforme, glioblastoma, congenital tumors), spinal cord neurofibroma, meningioma, glioma, sarcoma); gynecological cancers, for example, uterus (endometrial carcinoma), cervix (cervical carcinoma, precancerous cervical dysplasia), ovaries (ovarian carcinoma (serous cystadenocarcinoma, mucinous cystadenocarcinoma, unclassified carcinoma), granulosa-thecal cell tumors, sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); hematologic cancers, e.g. blood (myeloid leukemia (acute) and chronic), acute lymphocytic leukemia, chronic lymphocytic leukemia, myeloproliferative diseases, multiple myeloma, myelodysplastic syndrome), hodgkin's disease, non-Hodgkin' s lymphoma (malignant lymphoma); cancers of the skin site, for example, malignant melanoma, basal cell carcinoma, squamous cell carcinoma, kaposi's sarcoma, moles dysplastic nevi, lipoma, hemangioma, dermatofibroma, keloids, psoriasis; and cancers of the adrenal sites, e.g. neuroblastoma; and the like.

In one embodiment, the KRAS G12D-associated cancer is lung cancer.

In one embodiment, the KRAS G12D-associated cancer is selected from the group consisting of non-small cell lung cancer, small cell lung cancer, colorectal cancer, rectal cancer, and pancreatic cancer.

Another aspect of the present disclosure provides a method for treating cancer in a subject in need thereof, the method including:
(a) determining that the cancer is associated with a KRAS G12D mutation (e.g. a KRAS G12D-associated cancer); and
(b) administering to the subject a therapeutically effective amount of a compound according to the above aspect of the invention, or a pharmaceutically acceptable salt, a stereoisomer, or a solvate thereof; or administering to the subject a therapeutically effective amount of a pharmaceutical composition according to the above aspects of the invention.

In one embodiment, the administering is accomplished by a route selected from the group consisting of parenteral, intraperitoneal, intradermal, intracardiac, intraventricular, intracranial, intracerebrospinal, intraluminal, intrasynovial, intrathecal, intramuscular injection, intravitreal injection, intravenous injection, intraarterial injection, oral, buccal, sublingual, transdermal, topical, intratracheal, rectal, subcutaneous, and topical administration.

Another aspect of the invention provides a method of inhibiting KRAS G12D activity in a cell including the steps of: contacting the cell with a compound according to the above aspect of the invention, or a pharmaceutically acceptable salt, a stereoisomer or a solvate thereof; or contacting the cell with a pharmaceutical composition according to the above aspect of the invention. Another aspect of the present disclosure provides the use of a compound according to the above aspect, or a pharmaceutically acceptable salt, a stereoisomer, or a solvate thereof, or a pharmaceutical composition according to the above aspect, in the preparation of a KRAS G12D inhibitor.

As used herein, the term "subject" refers to an animal, particularly a mammal. Human is preferred. As used herein, the term "effective amount" or "therapeutically effective amount" refers to a sufficient amount of a drug or pharmaceutical agent that is non- toxic but achieves the desired effect. In an embodiment of the present disclosure, the amount of a given drug in the treatment of a patient according to the present disclosure depends on several factors, such as the particular dosing regimen, the type of disease or disorder and its severity, the uniqueness (e.g. weight) of the subject or host in need of treatment, but can be routinely determined by methods known in the art according to the particular circumstances, including, for example, the particular drug that has been employed, the route of administration, the condition being treated, and the subject or host being treated. In general, for dosages employed in adult human therapy, dosages administered will typically be in the range of 0.02-5000mg/day, for example about 1-1500mg/day. The desired dose may conveniently be presented in one dose, as divided doses administered simultaneously (or over a short period), or at appropriate intervals, for example as two, three, four, or more divided doses per day. It will be understood by those skilled in the art that, although the above dosage ranges are given, the specific effective amount may be appropriately adjusted according to the patient's condition in conjunction with the physician's diagnosis.

As used herein, the term "pharmaceutically acceptable salt" refers to a salt of a compound of the present disclosure that is pharmaceutically acceptable and that possesses the pharmacological activity of the parent compound. Such salts include acid addition salts with inorganic acids such as nitric acid, phosphoric acid, carbonic acid, and the like or with organic acids; the organic acids such as propionic acid, hexanoic acid, cyclopentanoic acid, glycolic acid, pyruvic acid, gluconic acid, stearic acid, muconic acid and the like; or salts formed when an acidic proton present on the parent compound is substituted by a metal ion, such as an alkali metal ion or an alkaline earth metal ion; or a coordination compound formed with an organic base such as ethanolamine and the like. The pharmaceutically acceptable salts of the present disclosure can be synthesized from the parent compound which contains an acid group or base by conventional chemical methods. Generally, such salts are prepared by: prepared by reacting the free acid or base forms of these compounds with a stoichiometric amount of the appropriate base or acid in water or an organic solvent, or a mixture of the two. Generally, nonaqueous media like ether, ethyl acetate, ethanol, isopropanol, or acetonitrile are preferred. In addition to salt forms, the compounds provided herein exist in prodrug form. Prodrugs of the compounds described herein readily undergo chemical changes under physiological conditions to convert the compounds of the present disclosure. In addition, prodrugs can be converted to the compounds of the present disclosure by chemical or biochemical methods in an in vivo environment.

As used herein, the terms "solvent compound" and "solvate" refer to a material formed from the combination of a compound of the present disclosure and a pharmaceutically acceptable solvent. Pharmaceutically acceptable solvents include acetic acid and the like. Solvent compounds include stoichiometric amounts of solvent compounds and non-stoichiometric amounts of solvent compounds. Certain compounds of the present disclosure can exist in unsolvated forms as well as solvated forms. In general, the solvated forms are equivalent to unsolvated forms and are intended to be encompassed within the scope of the present disclosure.

As used herein, the term "stereoisomer" includes conformational isomers and configurational isomers, wherein configurational isomers primarily include cis-trans isomers and optical isomers. The compounds described herein may exist as stereoisomers and thus encompass all possible stereoisomeric forms, including but not limited to cis-trans isomers, tautomers, enantiomers, diastereomers, atropisomers, and the like, as well as any combination or any mixture of the aforementioned stereoisomers, such as meso-forms, racemates, equal mixtures of atropisomers, and the like. For example, a single enantiomer, a single diastereomer, or a mixture of the above, or a single atropisomer or a mixture thereof. When the compounds described herein contain olefinic double bonds, unless specified otherwise, they include cis-and trans-isomers, as well as any combination thereof. Atropisomers of the present disclosure are stereoisomers based on axial or planar chirality resulting from restricted intramolecular rotation. And as a drug, a stereoisomer having excellent activity is preferable. Each of the compounds of the formula of the present disclosure has an optical isomer an asymmetric carbon or the like, and if necessary, a single isomer can be obtained by a method known in the art, for example, crystallization or chiral chromatography or the like.

As used herein, the term "C1-C6 alkyl" refers to straight and branched aliphatic groups consisting of 1 to 6 carbon atoms. C1-C4 alkyl or C1-C3 alkyl is preferred. Examples of alkyl groups include but are not limited to, methyl, ethyl, propyl, isopropyl, butyl, isobutyl, sec-butyl, tert-butyl, pentyl, hexyl, and the like.

As used herein, the term "C1-C6 haloalkyl" refers to a C1-C6 alkyl in which one or more hydrogens are substituted with a halogen. It is used interchangeably with "halo C1-C6 alkyl", wherein the alkyl moiety is as defined above. Preferably given to C1-C3-haloalkyl or C1-C4-haloalkyl. Examples of haloalkyl include but are not limited to, trifluoromethyl, difluoromethyl, monofluoromethyl, and the like.

As used herein, the term "C1-C6 deuterated alkyl" refers to a C1-C6 alkyl group in which one or more hydrogens are substituted with deuterium atoms, which is used interchangeably with "deuterated C1-C6 alkyl", wherein the alkyl moiety is as defined above. Preferably C1-C3 deuteroalkyl or C1-C4 deuteroalkyl. Examples of deuterated alkyl groups include but are not limited to, deuterated methyl, deuterated ethyl, and the like.

As used herein, the term "C1-C6 alkoxy" refers to an-O-C1-C6 alkyl group. Preference is given to C1-C3 alkoxy or C1-C4 alkoxy, wherein the alkyl moiety is as defined above.

As used herein, the term "C1-C6 haloalkoxy" refers to an -O-C1-C6 haloalkyl group. Preferably C1-C3 haloalkoxy or C1-C4 haloalkoxy, which is used interchangeably with "halo C1-C6 alkoxy", wherein the haloalkyl moiety is defined above.

As used herein, the term "C1-C6 hydroxyalkyl" refers to a -C1-C6 alkyl-hydroxy group, which is used interchangeably with "hydroxy C1-C6 alkyl". C1-C3 hydroxyalkyl or C1-C4 hydroxyalkyl is preferred, wherein the alkyl moiety is as defined above.

As used herein, the term "C1-C6 cyanoalkyl" refers to a -C1-C6 alkyl-cyano group, which is used interchangeably with "cyano C1-C6 alkyl". Preferably C1-C3 cyanoalkyl or C1-C4 cyanoalkyl, wherein the alkyl moiety is as defined above.

As used herein, the term "C2-C6 alkynyl" refers to straight and branched aliphatic groups consisting of 2-6 carbon atoms having 1 or 2 carbon-carbon triple bonds. Preferably C2-C4 alkynyl or C2-C3 alkynyl. Examples of alkynyl groups include but are not limited to, ethynyl, propynyl, butynyl, and the like.

As used herein, the term "C2-C6 deuterated alkynyl" refers to a C2-C6 alkynyl in which one or more hydrogens are substituted with deuterium atoms, which is used interchangeably with "deuterated C2-C6 alkynyl", wherein the alkynyl moiety is as defined above. C2-C3 deuteroalkynyl or C2-C4 deuteroalkynyl is preferred. Examples of deuterated alkynyl groups include but are not limited to, deuterated ethynyl, deuterated propynyl, and the like.

As used herein, the term "C2-C6 alkenyl" refers to straight and branched aliphatic groups consisting of 2-6 carbon atoms having 1 or 2 carbon-carbon double bonds. C2-C4 alkenyl or C2-C3 alkenyl are preferred. Examples of alkenyl groups include but are not limited to, ethenyl, propenyl, butenyl, and the like.

As used herein, the term "C2-C6 deuteroalkenyl" refers to a C2-C6 alkenyl in which one or more hydrogens are substituted with deuterium atoms, which is used interchangeably with "deuterated C2-C6 alkenyl", wherein the alkenyl moiety is as defined above. Preferably C2-C3 deuteroalkenyl or C2-C4 deuteroalkenyl. Examples of deuterated alkenyl groups include but are not limited to, deuterated ethenyl, deuterated propenyl, and the like.

As used herein, the term "C2-C6 hydroxyalkynyl" refers to -C2-C6 alkynyl-hydroxy, which is used interchangeably with "hydroxy C2-C6 alkynyl", wherein alkynyl is as defined above. Preferably C2-C4 alkynyl or C2-C3 alkynyl.

As used herein, the term "C2-C6 hydroxyalkenyl" refers to -C2-C6 alkenyl-hydroxy, which is used interchangeably with "hydroxy C2-C6 alkenyl", wherein alkynyl is as defined above. C2-C4 alkenyl or C2-C3 alkenyl are preferred.

As used herein, the term "halogen" includes fluorine, chlorine, bromine, iodine.

As used herein, the term "hydroxy" may be represented by OH.

As used herein, the term "cyano" may be represented by CN.

As used herein, the term "aldehyde group" may be represented by C(O)H.

As used herein, the term "ester group" may be represented by C(O)₂ or C(O)O.

As used herein, the term "amido" may be represented by C(O)N.

As used herein, when a group is -CH₂CH₂-, it means that the group, together with the carbon atom to which it is attached, forms a cyclopropyl group. As used herein, when a group is -CH₂CH₂CH₂-, it means that the group, together with the carbon atom to which it is attached, forms a cyclobutyl group. And so on.

As used herein, when a group is -CH₂OCH₂CH₂-, it means that the group, together with the carbon atom to which it is attached, forms a tetrahydrofuran ring.

As used herein, the term "C1-C4 alkylene" refers to a C1-C4 alkyl group defined above, lacated between the other two parts of the molecule and used to connect them. Typical alkylene groups include but are not limited to, methylene, ethylene, proylene, and butylene.

As used herein, the terms "cycloalkyl" and "cycloalkyl ring" are used interchangeably to refer to saturated monocyclic or polycyclic cyclic hydrocarbon groups, including, for example, monocyclic cycloalkyls, spirocycloalkyls, fused cycloalkyls, and bridged cycloalkyls. In the present disclosure, the ring carbon atoms of the cycloalkyl group may be optionally substituted with 1, 2, or 3 oxo groups to form a cyclic ketone structure. For example, the term "C3-C20 cycloalkyl" refers to cycloalkyl groups having from 3 to 20 ring carbon atoms, including monocyclic cycloalkyl, spirocycloalkyl, fused cycloalkyl, and bridged cycloalkyl groups. Preferred are C3-C12 cycloalkyl (more preferably C3-C8 monocyclic cycloalkyl), C5-C20 spirocycloalkyl, C5-C20 fused cycloalkyl or C5-C20 bridged cycloalkyl.

The term "C3-C8 monocyclic cycloalkyl" refers to a saturated monocyclic cyclic hydrocarbon group having 3 to 8 ring carbon atoms. Preferred are C3-C6 monocyclic cycloalkyls or C4-C6 monocyclic cycloalkyls. More preferred are C3, C4, C5, or C6 monocyclic cycloalkyls. Specific examples of monocyclic cycloalkyls include but are not limited to, cyclopropyl, cyclobutyl, cyclopentyl, cyclohexyl, cycloheptyl, cyclooctyl, and the like.

As used herein, the terms "spirocycloalkyl" and "spirocycloalkyl ring" refer to polycyclic cyclic hydrocarbon groups formed by sharing one carbon atom (referred to as a spiro atom) between two or more monocyclic rings. Spirocycloalkyl groups are classified into mono-spirocycloalkyl, dispirocycloalkyl, and poly-spirocycloalkyl groups according to the number of spiro atoms shared from ring to ring. The term "C5-C20 spirocycloalkyl" refers to a polycyclic cyclic hydrocarbon radical having from 5 to 20 ring carbon atoms, wherein the monocyclic ring that shares the spiro atom is a C3-C8 monocyclic cycloalkyl ring. Preferred is C6-C14 spirocycloalkyl. More preferred is C6-C14 mono spirocycloalkyl. More preferred is C7-C11 spirocycloalkyl. More preferred is a 7-to 11-membered mono spirocycloalkyl. Most preferred are C7 (C4 monocyclic cycloalkyl ring/C4 monocyclic cycloalkyl ring), C8 (C4 monocyclic cycloalkyl ring/C5 monocyclic cycloalkyl ring), C9 (C4 monocyclic cycloalkyl ring/C6 monocyclic cycloalkyl ring, C5 monocyclic cycloalkyl ring/C5 monocyclic cycloalkyl ring), C10 (C5 monocyclic cycloalkyl ring/C6 monocyclic cycloalkyl ring) or C11 (C6 monocyclic cycloalkyl ring/C6 monocyclic cycloalkyl ring) monocyclic spirocycloalkyl. Specific examples of spirocycloalkyl groups include, but are not limited to:

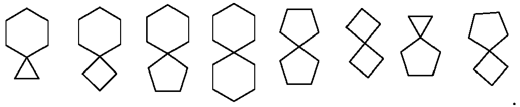

These spirocycloalkyl groups may be attached to the remainder of the molecule through any one of the ring atoms.

As used herein, the terms "fused cycloalkyl" and "fused cycloalkyl ring" refer to two or more monocyclic cyclic hydrocarbon groups formed by sharing an adjacent pair of carbon atoms. Bicyclic, tricyclic, tetracyclic, or polycyclic fused cycloalkyl groups can be classified according to the number of rings formed. The term "C5-C20 fused cycloalkyl" refers to a polycyclic cyclic hydrocarbon radical having from 5 to 20 ring carbon atoms, wherein the monocyclic ring sharing an adjacent pair of carbon atoms is a C3-C8 monocyclic cycloalkyl ring. C6-C14 fused cycloalkyl is preferred. More preferred are C6-C14 doubly fused cycloalkyl groups. More preferred are C7-C10 fused cycloalkyl groups. More preferred are C7-C10 doubly fused cycloalkyl groups. Most preferred are C8 (C5 monocyclic cycloalkyl ring fused to a C5 monocyclic cycloalkyl ring), C9 (C5 monocyclic cycloalkyl ring fused to a C6 monocyclic cycloalkyl ring), or C10 (C6 monocyclic cycloalkyl ring fused to a C6 monocyclic cycloalkyl ring) double fused cycloalkyl. Specific examples of fused cycloalkyl groups include, but are not limited to:

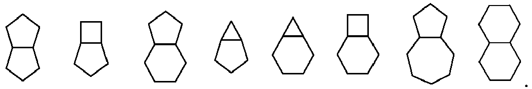

These fused cycloalkyl groups may be attached to the remainder of the molecule through any one of the ring atoms.

As used herein, the terms "bridged cycloalkyl" and "bridged cycloalkyl ring" refer to a polycyclic cyclic hydrocarbon group formed between two or more monocyclic rings by sharing two carbon atoms that are not directly connected. Bicyclic, tricyclic, tetracyclic, or polycyclic bridged cycloalkyl groups can be classified according to the number of rings formed. The term "C5-C20 bridged cycloalkyl" refers to a polycyclic cyclic hydrocarbon group having from 5 to 20 ring carbon atoms in which any two rings share two carbon atoms that are not directly connected. Preferred is C6-C14 bridged cycloalkyl. More preferred is C7-C10 bridged cycloalkyl. Specific examples of bridged cycloalkyl groups include, but are not limited to:

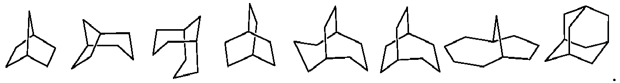

These bridged cycloalkyl groups may be attached to the rest of the molecule through any one of the ring atoms.

For example, examples of cycloalkyl groups herein include but are not limited to, cyclopropyl, cyclobutyl, cyclopentyl, cyclopentenyl, cyclohexyl, cyclohexenyl, cycloheptyl, cyclooctyl, bicyclo[1.1.1]pentyl.

As used herein, the term "cycloalkoxy" refers to an -O-cycloalkyl group, wherein cycloalkyl is as previously defined.

As used herein, the terms "heterocyclyl" and "heterocyclyl ring" are used interchangeably and refer to saturated or partially unsaturated monocyclic or polycyclic cyclic hydrocarbon groups, including, for example, monocyclic heterocyclyl, spiroheterocyclyl, fused heterocyclyl, and bridged heterocyclyl. Ring carbon atoms of the heterocyclyl described herein may be optionally substituted with 1, 2, or 3 oxo groups to form a cyclic ketone, cyclic lactone, or cyclic lactam structure. For example, the term "3- to 20-membered heterocyclyl" refers to a saturated or partially unsaturated monocyclic or polycyclic cyclic hydrocarbon radical having 3 to 20 ring atoms, of which one or more (preferably 1, 2, 3, or 4) ring atoms are heteroatoms selected from nitrogen, oxygen or S(=O)ₘ' (where m' is an integer from 0 to 2), but excluding the ring moieties -O-O-, -O-S- or -S-S-, the remaining ring atoms being carbon, wherein when the ring atom is a nitrogen atom, it may be substituted or unsubstituted (i.e. N or NR, R being hydrogen or a further substituent as already defined herein). The 3- to 20-membered heterocyclyl described in the present disclosure includes a monocyclic heterocyclyl (e.g. a 3- to 8-membered monocyclic heterocyclyl), a 5- to 20-membered spiro heterocyclyl, a 5- to 20-membered fused heterocyclyl and a 5- to 20-membered bridged heterocyclyl.

As used herein, the terms "3- to 8-membered monocyclic heterocyclyl" and "3- to 8-membered monocyclic heterocyclyl ring" refer to a saturated or partially unsaturated monocyclic cyclic hydrocarbon group having 3 to 8 ring atoms of which 1, 2 or 3 are heteroatoms selected from nitrogen, oxygen or S(=O)ₘ', wherein m' is an integer from 0 to 2. Preferred are 3- to 6-membered monocyclic heterocyclyl having 3 to 6 ring atoms of which 1 or 2 are heteroatoms. More preferred are 4 to 6-membered monocyclic heterocyclyl having 4 to 6 ring atoms of which 1 or 2 are heteroatoms. More preferred are 5- or 6-membered monocyclic heterocyclyl having 5 or 6 ring atoms of which 1 or 2 are heteroatoms. When the heteroatom is a nitrogen atom, the nitrogen atom may be substituted or unsubstituted (i.e. N or NR, R being hydrogen or a further substituent as already defined herein). When the heteroatom is a sulfur atom, the sulfur atom may be optionally oxidized (i.e. S(=O)ₘ', m' being an integer from 0 to 2). The ring carbon atoms of the monocyclic heterocyclyl can be optionally substituted with 1, 2, or 3 oxo groups to form a cyclic ketone, cyclic lactone, or cyclic lactam structure. Specific examples of monocyclic heterocyclyl include, but are not limited to, aziridine, oxirane, azetidine, azetidin -2-one, oxetane, oxetan -2-one, oxazolidine, pyrrolidin -2-one, pyrrolidin -2,5-dione, 1,3-dioxolane, dihydrofuran -2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrolidine, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholin-1,1-dioxide, tetrahydropyran, 1,2-dihydroazetidine, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridine-2,6-(1H, 3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine and the like. As used herein, the term "3- to 8-membered monocyclic nitrogen-containing heterocyclyl" refers to a saturated or partially unsaturated monocyclic cyclic hydrocarbon group having 3 to 8 ring atoms containing at least 1 nitrogen atom; wherein the group is optionally attached to the rest of the molecule through the nitrogen atom; and the group optionally also comprises another one or two heteroatom ring members selected from nitrogen, oxygen or S(=O)ₘ'(wherein m' is an integer from 0 to 2). Preferably 3- to 6-membered monocyclic nitrogen-containing heterocyclyl (i.e. 3- to 6-membered nitrogen-containing heterocyclyl) refers to a saturated or partially unsaturated monocyclic cyclic hydrocarbon radical having 3 to 6 ring atoms containing at least 1 nitrogen atom; wherein the group is optionally attached to the rest of the molecule through the nitrogen atom; and the group optionally also contains another one or two heteroatom ring members selected from nitrogen, oxygen or S(=O)ₘ' (wherein m' is an integer from 0 to 2). Specific examples of monocyclic nitrogen-containing heterocyclyl include, but are not limited to, aziridine, azetidine, oxetan -2-one, pyrrolidin -2-one, pyrrolidin -2,5-dione, piperidin-2-one, piperidin-2,6-dione, imidazolidine, tetrahydropyrrolidine, oxazolidin-2-one, imidazolidin-2-one, piperidine, piperazine, piperazin-2-one, 1,2-dihydroazetidine, 2,5-dihydro-1H-pyrrole, 2,3-dihydro-1H-pyrrole, 1,2,3,4-tetrahydropyridine, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, oxazol-2(3H)-one, 1,3-dihydro-2H-imidazol-2-one, 3,6-dihydropyridin-2(1H)-one, pyridine-2,6-(1H, 3H)-dione, 3,4-dihydro-2H-1,3-oxazine, 3,6-dihydro-2H-1,3-oxazine, 1,2,3,4-tetrahydropyrimidine and the like.

As used herein, the terms "spiroheterocyclyl" and "spiroheterocyclyl ring" refer to polycyclic heterocyclyl formed by sharing one carbon atom (referred to as a spiro atom) between two or more saturated or partially unsaturated monocyclic rings, wherein one or more (e.g. 1, 2, or 3) ring atoms are heteroatoms selected from nitrogen, oxygen, or S(=O)ₘ' (where m' is an integer from 0 to 2) and the remaining ring atoms are carbon. When the heteroatom is a nitrogen atom, the nitrogen atom may be substituted or unsubstituted (i.e. N or NR, R being hydrogen or a further substituent as already defined herein). Every single ring may contain one or more double bonds, but none of the rings have a completely conjugated pi-electron system. Spiroheterocyclyls are classified as mono-, bi-, or poly-spiroheterocyclyls depending on the number of spiro atoms shared between the rings. The term "5- to 20-membered spiroheterocyclyl" refers to a spiroheterocyclyl having 5 to 20 ring atoms wherein one of the monocyclic rings that share a spiro atom is a 3- to 8-membered monocyclic heterocyclyl ring and the other monocyclic ring is a 3- to 8-membered monocyclic heterocyclyl ring or a 3- to 8-membered monocyclic cycloalkyl ring. Preference is given to 6- to 14-membered spiroheterocyclyl having 6 to 14 ring atoms of which 1 or 2 are heteroatoms. More preferred are 7 to 11 membered spiroheterocyclyl having 7 to 11 ring atoms of which 1 or 2 are heteroatoms. More preferred are 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring) or 11-membered (6-membered monocyclic heterocyclyl ring/6-membered monocyclic heterocyclyl ring) monospiroheterocyclyl. Specific examples of spiroheterocyclyl include, but are not limited to:

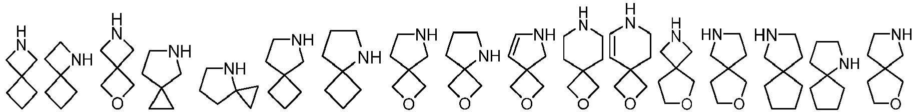

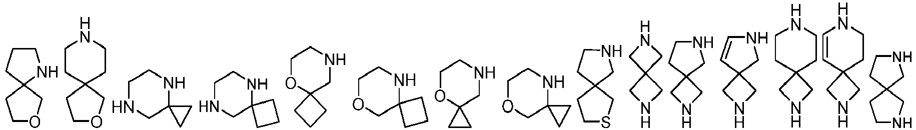

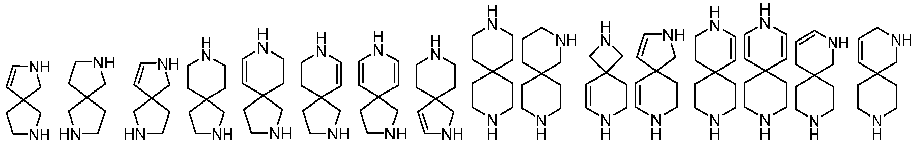

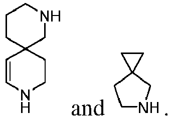

These spiroheterocyclyl may be attached to the remainder of the molecule through any suitable ring atom.

As used herein, the terms "fused heterocyclyl" and "fused heterocyclyl ring" refer to a polycyclic heterocyclyl in which two or more saturated or partially unsaturated monocyclic rings are formed by sharing an adjacent pair of ring atoms, in which one or more (e.g. 1, 2, or 3) ring atoms are heteroatoms selected from nitrogen, oxygen, or S(=O)ₘ' (where m' is an integer from 0 to 2), the remaining ring atoms being carbon. When the heteroatom is a nitrogen atom, the nitrogen atom may be substituted or unsubstituted (i.e. N or NR, R being hydrogen or a further substituent as already defined herein). Every single ring may contain one or more double bonds, but none of the rings have a completely conjugated pi-electron system. The shared adjacent pair of ring atoms may be C-C or N-C. Bicyclic, tricyclic, tetracyclic, or polycyclic fused heterocyclyl can be classified according to the number of constituent rings. The term "5- to 20-membered fused heterocyclyl" refers to fused heterocyclyl having from 5 to 20 ring atoms, wherein the single ring that shares an adjacent pair of ring atoms is a 3- to 8-membered monocyclic heterocyclyl ring. Preferred are 6- to 14-membered fused heterocyclyl having 6 to 14 ring atoms of which 1 or 2 are heteroatoms. More preferred are 6 to 10-membered fused heterocyclyl having 6 to 10 ring atoms of which 1 or 2 are heteroatoms. More preferred are 8 to 10-membered fused heterocyclyl having 8 to 10 ring atoms of which 1 or 2 are heteroatoms. More preferred are 8-membered (a 5-membered monocyclic heterocyclyl ring fused to a 5-membered monocyclic heterocyclyl ring), 9-membered (a 5-membered monocyclic heterocyclyl ring fused to a 6-membered monocyclic heterocyclyl ring) or 10-membered (a 6-membered monocyclic heterocyclyl ring fused to a 6-membered monocyclic heterocyclyl ring) bicyclic fused heterocyclyl. Specific examples of fused heterocyclyl include, but are not limited to:

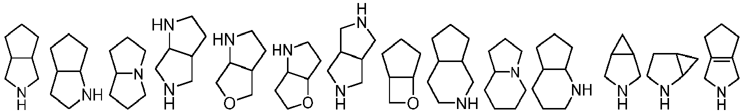

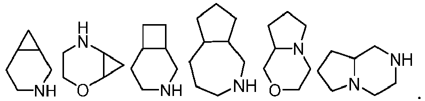

These fused heterocyclyl groups may be attached to the remainder of the molecule through any suitable ring atom.

As used herein, the terms "bridged heterocyclyl" and "bridged heterocyclyl ring" refer to a polycyclic heterocyclyl in which two or more saturated or partially unsaturated monocyclic rings are formed by sharing two ring atoms that are not directly connected, wherein one or more (e.g. 1, 2, or 3) ring atoms are heteroatoms selected from nitrogen, oxygen, or S(=O)ₘ' (where m' is an integer from 0 to 2) and the remaining ring atoms are carbon. Bicyclic, tricyclic, tetracyclic, or polycyclic bridged cycloalkyl groups can be classified according to the number of rings formed. The term "5- to 20-membered bridged heterocyclyl" refers to a saturated or partially unsaturated polycyclic heterocyclyl having from 5 to 20 ring atoms in which any two rings share two ring atoms that are not directly connected, each monocyclic ring may contain one or more double bonds, but no ring has a completely conjugated pi-electron system. Preferred are 6 to 14-membered bridged heterocyclyl. More preferred are 7 to 10-membered bridged heterocyclyl. Specific examples of bridged heterocyclyl include, but are not limited to:

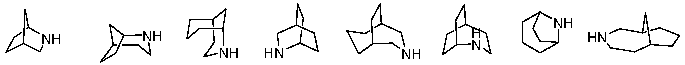

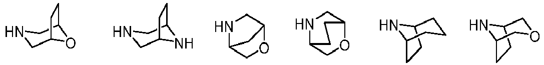

These bridged heterocyclyl groups may be attached to the rest of the molecule through any suitable ring atom.

In the present disclosure, each of the above heterocyclyl groups may be optionally substituted, and when substituted, the substituent is preferably one or more of the substituent groups described in the present application.

Specifically, specific examples of heterocyclyl described herein include but are not limited to, epoxy, azetidinyl, tetrahydrofuranyl, tetrahydropyranyl, pyrrolidinyl, pyrrolidonyl, piperidinyl, piperazinyl, imidazolyl, imidazopyridinyl, thiazolyl, oxazolidinyl, oxazolidinedionyl, decahydroquinolinyl, piperidonyl, morphinyl, azabicyclohexyl, azabicycloheptyl, azabicyclooctyl, azabicyclononyl, azabicyclodecyl, azaspiroheptyl, azaspirooctyl, azaspirononyl, azaspirodecyl, tetrahydrospiro[cyclopropane-1,2'-pyrrolizinyl], hexahydro-1H-pyrrolizinyl, hexahydro-1H-pyrrolo[2,1-c][1,4]oxazinyl, octahydroindolizinyl, oxaspiroheptyl, oxaspirooctyl, oxaspirononyl, oxaspirodecyl, diazaspirononyl, oxabicyclohexyl, oxabicycloheptyl, oxabicyclooctyl, hexahydroalloxazinyl 4(1H)-oxide.

As used herein, the term "aryl" refers to an all-carbon monocyclic, all-carbon non-fused polycyclic (rings are linked by a covalent bond, non-fused), or all-carbon fused polycyclic (i.e. rings that share an adjacent pair of carbon atoms) group in which at least one ring is aromatic, i.e. has a conjugated pi-electron system. For example, the term "C6-C14 aryl" refers to an aryl group having from 6 to 14 ring atoms. Preferred is C6-C10 aryl. The C6-C14 aryl group in the present disclosure includes a monocyclic aryl group, a non-fused polycyclic aryl group, and an aromatic fused polycyclic group, wherein examples of the monocyclic aryl group include a phenyl group, and examples of the non-fused polycyclic aryl group include a biphenyl group and the like.

In the present disclosure, when the C6-C14 aryl group is an aromatic fused polycyclic group, the aromatic fused polycyclic group may be a polycyclic group formed by fusing a single aryl ring with one or more single aryl rings, non-limiting examples of which include naphthyl, anthryl, and the like.

In some embodiments of the invention, when the C6-C14 aryl group is an aromatic fused polycyclic group, the aromatic fused polycyclic group may also be a polycyclic group formed by fusing a single aryl ring (e.g. phenyl) with one or more non-aromatic rings, wherein the ring attached to the parent structure is either an aromatic ring or a non-aromatic ring. The non-aromatic ring includes but is not limited to, a 3- to 6-membered monocyclic heterocyclyl ring (preferably a 5- or 6-membered monocyclic heterocyclyl ring, ring carbon atoms of which may be substituted with 1 to 2 oxo groups to form a cyclic lactam or cyclic lactone structure), a 3- to 6-membered monocyclic cycloalkyl ring (preferably a 5- or 6-membered monocyclic cycloalkyl ring, the ring carbon atoms of which may be substituted with 1 or 2 oxo groups to form a cyclic ketone structure). Polycyclic groups in which a single aryl ring is fused to one or more non-aromatic rings as described above may be attached to other groups or the parent structure via a nitrogen or carbon atom, the ring to which the parent structure is attached being a single aryl ring or a non-aromatic ring.

In the present disclosure, the above-mentioned kinds of aryl groups may be substituted or unsubstituted, and when substituted, the substituent is preferably one or more of the substituent groups described in the present application.

As used herein, the term "aryl" refers to a C6-C14 aromatic radical including 1 to 3 aromatic rings. It may be optionally substituted with one or more R6 or one or more R7. In one embodiment, an aryl group is a C6-C10 aryl group. Examples of aryl groups include but are not limited to, phenyl, naphthyl, anthracenyl, fluorenyl, and dihydrobenzofuranyl. "Aryl" also refers to a bicyclic or tricyclic ring system wherein one or both rings in the aryl group, respectively, may be saturated or partially saturated. If the aryl group contains two saturated rings, the two saturated rings may be fused ring systems or spiro ring systems. Examples of aryl groups including two saturated rings (and being a spiro ring system) include the following:

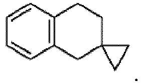

As used herein, the term "aryl C1-C6 alkyl" or "aralkyl" is intended to include one aryl group covalently linked to one alkyl group, wherein aryl is as defined above and alkyl is as defined above, and any or all of aryl, or alkyl may be independently optionally substituted or unsubstituted. An example of an aralkyl group is (C6-C10) aryl (C1-C6) alkyl-. Specific examples include but are not limited to, benzyl, phenethyl, and naphthylmethyl. An example of a substituted aryl C1-C6 alkyl is where the alkyl is substituted with hydroxyalkyl.

The term "heteroaryl", as used herein, refers to a monocyclic or fused polycyclic (i.e. sharing adjacent pairs of ring atoms, which may be C-C or N-C) group wherein the nitrogen and sulfur atoms may optionally be oxidized and the nitrogen atom may optionally be quaternized, with ring atoms being substituted with at least one heteroatom independently selected from nitrogen, oxygen, or sulfur. The heteroaryl group has 6, 10, or 14 π electrons shared, with at least one ring in the group being aromatic. For example, the term "5 to 14 membered heteroaryl" refers to a heteroaryl group having 5 to 14 ring atoms of which 1, 2, 3, or 4 are heteroatoms selected from nitrogen, oxygen, or S(=O)ₘ', wherein m' is an integer from 0 to 2. Preferred are 5 to 10-membered heteroaryl groups having 5 to 10 ring atoms of which 1, 2, 3, or 4 are heteroatoms. A 5- to 14-membered heteroaryl in the present disclosure can be a monocyclic heteroaryl (e.g. a 5- or 6-membered monocyclic heteroaryl), a fused bicyclic heteroaryl (e.g. an 8- to 10-membered bicyclic heteroaryl), or a fused tricyclic heteroaryl.

As used herein, the term "5- or 6-membered monocyclic heteroaryl" refers to a monocyclic heteroaryl having 5 or 6 ring atoms, wherein 1, 2, or 3 ring atoms are heteroatoms selected from nitrogen, oxygen, or S(=O)ₘ', wherein m' is an integer from 0 to 2. Specific examples of monocyclic heteroaryl groups include but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, and the like.

As used herein, the term "8- to 10-membered bicyclic heteroaryl" refers to a fused bicyclic heteroaryl having from 8 to 10 ring atoms of which 1, 2, 3, 4, or 5 ring atoms are heteroatoms selected from nitrogen, oxygen, or S(=O)ₘ' (wherein m' is an integer from 0 to 2). The fused bicyclic heteroaryl can be either a bicyclic group (preferably a 9- or 10-membered bicyclic heteroaryl ring) formed by fusing a single aryl ring (e.g. phenyl) to a monocyclic heteroaryl ring (preferably a 5- or 6-membered monocyclic heteroaryl ring), or a bicyclic group (preferably a 5- or 6-membered monocyclic heteroaryl ring) formed by fusing a monocyclic heteroaryl ring (preferably a 5- or 6-membered monocyclic heteroaryl ring).

Any two ring atoms, including C-C, N-C, N-N, attached to the monocyclic heteroaryl ring described above can be fused to a cycloalkyl, heterocyclyl, aryl, or heteroaryl, such as a monocyclic cycloalkyl, monocyclic heterocyclyl, monocyclic aryl, 5- or 6-membered monocyclic heteroaryl ring, as defined herein, to form a fused polycyclic ring. The two ring atoms attached to the monocyclic heteroaryl ring forming a fused ring with other rings are preferably C-C, including, but not limited to, the following forms:

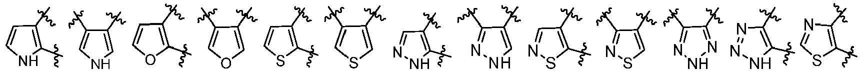

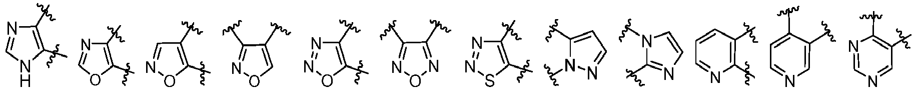

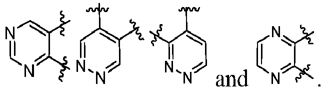

The above groups are attached to the rest of the molecule through the ring atom marked " 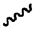 ".

Non-limiting examples of 8- to 10-membered bicyclic heteroaryls include benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine and the like.

Specific examples of bicyclic heteroaryl groups include, but are not limited to: 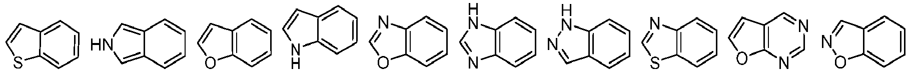 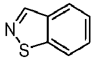 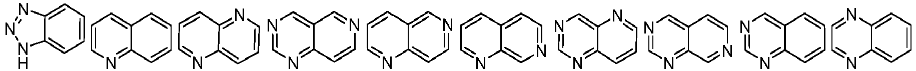 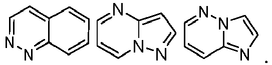 These groups may be attached to the remainder of the molecule through any suitable ring atom. The ring attached to the parent structure can be a monocyclic heteroaryl ring or a phenyl ring.

In some embodiments of the invention, the fused bicyclic heteroaryl or fused tricyclic heteroaryl may be a polycyclic group formed by fusing a monocyclic heteroaryl ring (preferably a 5- or 6-membered monocyclic heteroaryl ring) with one or more non-aromatic rings, wherein the ring attached to the parent structure is a monocyclic heteroaryl ring or a non-aromatic ring. The non-aromatic ring includes but is not limited to, a 3- to 6-membered monocyclic heterocyclyl ring (preferably a 5- or 6-membered monocyclic heterocyclyl ring, the ring carbon atoms of which may be substituted with 1 to 2 oxo groups to form a cyclic lactam or cyclic lactone structure), a 3- to 6-membered monocyclic cycloalkyl ring (preferably a 5- or 6-membered monocyclic cycloalkyl ring, the ring carbon atoms of which may be substituted with 1 or 2 oxo groups to form a cyclic ketone structure), and the like. Polycyclic groups formed by fusing the above monocyclic heteroaryl rings with one or more non-aromatic rings may be attached to other groups or the parent structure via a nitrogen or carbon atom, the ring to which the parent structure is attached being a monocyclic heteroaryl ring or a non-aromatic ring.

In the present disclosure, each of the above-mentioned types of heteroaryl groups may be substituted or unsubstituted, and when substituted, the substituent is preferably one or more of the substituent groups described in the present application.

As used herein, the term "acetyl" refers to -C(O)CH₃.

As used herein, "two hydrogen atoms on the same carbon atom are simultaneously substituted with =CR^{2a}R^{2b}" as described herein refers to when two hydrogen atoms on the carbon atom (e.g. C₁) are substituted, C₁ and CR^{2a}R^{2b} form C₁=CR^{2a}R^{2b} through a double bond, non-C1, and CR^{2a}R^{2b} forms C₁(-CR^{2a}R^{2b})₂ through two single bonds.

In the present disclosure, in any group, if 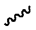 is labeled on a chemical bond (not the chemical bond itself), it means that the chemical bond labeled by that group is attached to the rest of the molecule. In any group or compound structural formula, if a bond to a double bond is drawn as 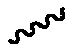 , it means that the group or compound can be a mixture of cis and trans isomers.

### DETAILED DESCRIPTION OF THE INVENTION

The compounds of the present disclosure can be prepared by a variety of synthetic methods well known to those skilled in the art, including the specific embodiments set forth below, embodiments formed in combination with other chemical synthetic methods, and equivalents thereof well known to those skilled in the art. Preferred embodiments include, but are not limited to, examples of the present disclosure.

It can also be prepared according to the following method. Preparing compound I-2 from compound I-1; further preparing compound I-3 from compound I-2; further preparing compound I-4 from compound I-3; then preparing compound AI from compound I-4;

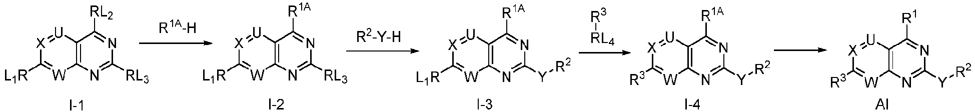

In each formula, RL₁ is 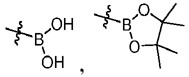 or halogen; RL₂ and RL₃ are each independently halogen or hydroxy; R^{1A} is R¹ protected with an amino protecting group (e.g. Boc, etc.); RL₄ is 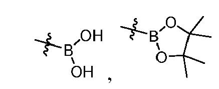 or halogen; other groups are as defined above. Wherein, when RL₁ is halogen, RL₄ is 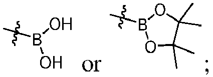 when RL₁ is 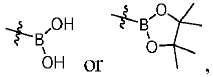 RL₄ is halogen.

The present disclosure will now be described in more detail by way of examples without implying any disadvantageous limitation thereof. While the invention has been described in detail and with reference to specific embodiments thereof, it will be apparent to one skilled in the art that various changes and modifications can be made therein without departing from the spirit and scope of the invention. Where specific conditions are not specified in the examples, they are carried out according to conventional conditions or conditions suggested by the manufacturer. The reagents or instruments used are conventional products that can be obtained commercially without indicating the manufacturer.

Preparative HPLC used in the following examples, unless otherwise specified, adopts the following conditions:
preparative HPLC (alkaline method): column type: Waters XBridge C18, 190*250 mm, 5 µm; mobile phase system: A: 0.1% ammonium bicarbonate aqueous solution; B: preparative grade acetonitrile; flow rate: 15 ml/min; B%=20%-100%; column temperature: room temperature. Preparative HPLC (formic acid method): column type: Waters XBridge C18, 190*250 mm, 5 um; mobile phase system: A: 0.1% formic acid; B: preparative grade acetonitrile; flow rate: 15 ml/min; B%=20%-100%; column temperature: room temperature.

Preparative HPLC (trifluoroacetic acid method): column type: Waters XBridge C18, 190*250 mm, 5 um; mobile phase system: A: 0.1% trifluoroacetic acid; B: preparative grade acetonitrile; flow rate: 15 ml/min; B%=20%-100%; column temperature: room temperature.

### Preparation Example 1: Synthesis of tert-butyl (1R,2S,4R,5S)-7,9-diazatricyclo[3.3.1.0^{2,4}]nonane-9-carboxylate

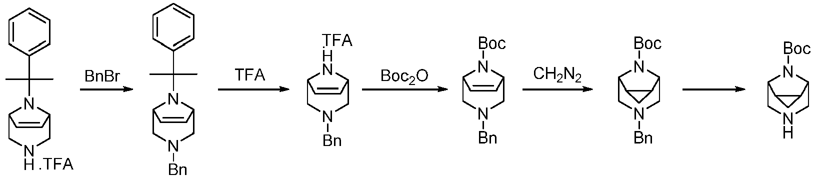

Step 1: the trifluoroacetate of (1R,5S)-8-(2-phenylpropan-2-yl)-3,8-diazabicyclo[3.2.1]oct-6-ene (976 mg, 3.0 mmol) was dissolved in DMF (6.0 mL), and benzyl bromide (616 mg, 3.6 mmol) and potassium carbonate (1.24 g, 9.0 mmol) were added successively. The mixture was reacted for 8 h at room temperature, filtered, washed with ethyl acetate, washed with appropriate amount of water, dried over anhydrous sodium sulfate, rotary dried, and purified by column chromatography (ethyl acetate/petroleum ether = 0-40%) to give (1R,5S)-3-benzyl-8-(2-phenylpropan-2-yl)-3,8-diazabicyclo[3.2.1]oct-6-ene (812 mg, yield: 85%). ES-API: [M+H]⁺=319.1.

Step 2: (1R,5S)-3-benzyl-8-(2-phenylpropan-2-yl)-3,8-diazabicyclo[3.2.1]oct-6-ene (812 mg, 2.55 mmol) was dissolved in trifluoroacetic acid (10.0 mL), reacted at 50°C for 2 h, and the reaction solution was concentrated to give the crude trifluoroacetate of (1R,SS)-3-benzyl-3,8-diazabicyclo[3.2.1]oct-6-ene (758 mg, yield: 100%). ES-API: [M+H]⁺=201.1.

Step 3: the trifluoroacetate of (1R,5S)-3-benzyl-3,8-diazabicyclo[3.2.1]oct-6-ene (758 mg, 2.55 mmol) was dissolved in dichloromethane (15.0 mL). Di-tert-butyl dicarbonate (660 mg, 3.0 mmol) and triethylamine (387 mg, 3.82 mmol) were added. The mixture was stirred at room temperature for 8 h. The reaction solution was concentrated and purified by column chromatography (ethyl acetate/petroleum ether = 0-30%) to give (1R,SS)-tert-butyl 3-benzyl-3,8-diazabicyclo[3.2.1]oct-6-ene-8-carboxylate (689 mg, yield: 90.0%). ES-API: [M+H]⁺=301.2.

Step 4: N,4-dimethyl-N-nitrobenzenesulfonamide (1.5 g, 7.0 mmol) was dissolved in diethyl ether (18.0 mL) and added slowly to a mixture solution of KOH (1.60 g, 28 mmol) aqueous solution (6.0 ml) and 2-(2-ethoxyethoxy) ethanol (6.0 mL) in an ice bath. After stirring at room temperature for 5 h, the mixture was allowed to stand and the resulting diazomethane solution in diethyl ether was slowly added dropwise to (1R,5S)-tert-butyl-3-benzyl-3,8-diazabicyclo[3.2.1]oct-6-ene-8-carboxylate (689 mg, 2.30 mmol) and palladium acetate (140 mg) in anhydrous dichloromethane (10.0 mL) under nitrogen in an ice bath, warmed to room temperature and stirred for 16 h, concentrated and purified by column chromatography (ethyl acetate/petroleum ether = 0-25%) to give tert-butyl (1R,2S,4R,5S)-7-benzyl-7,9-diazatricyclo[3.3.1.0^{2,4}]nonane-9-carboxylate (250 mg, yield: 34.6%). ES-API: [M+H]⁺=315.1.

Step 5: tert-butyl (1R,2S,4R,5S)-7-benzyl-7.9-diazatricyclo[3.3.1.0^{2,4}]nonane-9-carboxylate (250 mg, 0.80 mmol) was dissolved in methanol (8.0 mL) and 10% palladium on carbon (25 mg) was added. The system reacted at room temperature under hydrogen conditions for 2 h, filtered and concentrated to give tert-butyl (1R,2S,4R,5S)-7.9-diazatricyclo[3.3.1.0^{2,4}]nonane-9-carboxylate (170 mg, yield: 95.0%). ES-API: [M+H]⁺=225.1.

### Example 1: Synthesis of two isomers of 4-(4-((8-amino-3-azabicyclo[3.2.1]octan-3-yl)-8-fluoro-2-(((tetrahydro-1H-pyrrolizin 7a(5H)-yl)methoxy) pyrido[4,3-d]pyrimidin-7-yl)-5-ethynylnaphthalen-2-ol

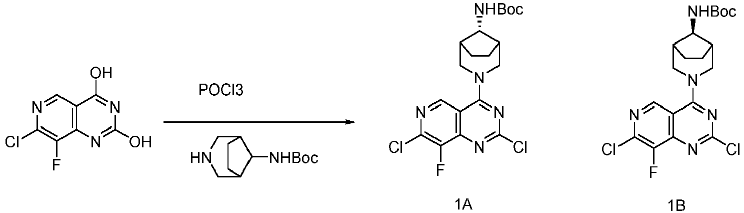

Step 1: 7-chloro-8-fluoropyrido[4,3-d]pyrimidine-2,4-diol (550 mg, 2.56 mmol) was added into the reactor, then phosphorus oxychloride (1.50 mL) and diisopropyl ethyl amine (1.5 g) were successively added into the reactor. The system reacted at 100°C for 2 h. After cooling to room temperature, phosphorus oxychloride was suspended and removed under reduced pressure. Then diisopropyl ethyl amine (1.5 g) and tert-butyl (3-azabicyclo[3.2.1]octan-8-yl) carbamate (578 mg, 2.56 mmol) were added, and the system reacted at -40°C for 0.5 h. Water (20 mL) was added to the reaction solution, which was extracted with dichloromethane, dried over anhydrous sodium sulfate, rotary dried, and purified by column (50-100% ethyl acetate/petroleum ether) to give two isomeric compounds. One isomer structure was arbitrarily assigned as Compound 1A (260 mg, yield 23%), LC-MS retention time of 2.170 min. ESI: [M+H]⁺=442.3; and the other isomer structure was arbitrarily assigned as compound 1B (264 mg, yield 23%), LC-MS retention time 2.141 min. ES-API: [M+H]⁺=442.3.

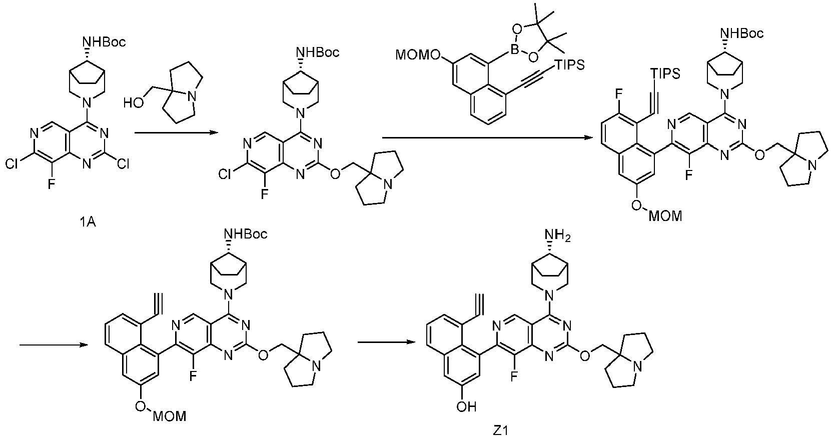

Step 2: compound 1A (260 mg, 0.59 mmol) was dissolved in 1,4 dioxane (3 mL), (tetrahydro-1H-pyrrolizin-7a(5H)-yl) methanol (166 mg, 1.18 mmol) and diisopropylethyl amine (228 mg, 1.77 mmol) were added and the mixture was allowed to react under argon protection at 80°C for 8 h. Water (20 mL) was added to the reaction solution, which was extracted with dichloromethane, dried over anhydrous sodium sulfate, rotary dried, and purified by column (50-100% ethyl acetate/petroleum ether) to give tert-butyl (3-(7-chloro-8-fluoro-2-((tetrahydro-1H-pyrrolizin-7a(5H)-yl)methoxy) pyrido[4,3-d]pyrimidin-4-yl)-3-azabicyclo[3.2.1]octan-8-yl) carbamate (125 mg, yield: 39%). ESI: [M+H]⁺=547.3.

Step 3: the obtained tert-butyl (3-(7-chloro-8-fluoro-2-((tetrahydro-1H-pyrrolizin-7a(5H)-yl)methoxy) pyrido[4,3-d]pyrimidin-4-yl)-3-azabicyclo[3.2.1]octan-8-yl) carbamate (120 mg, 0.22 mmol) was dissolved in dioxane/water (2 mL/0.2 mL), to which ((6-(methoxymethoxy)-8-(4,4,5,5-tetramethyl-1,3,2-dioxaborinan-2-yl)naphthalen-1-yl)ethynyl)triisopropylsilane (163 mg, 0.33 mmol), cesium carbonate (215 mg, 0.66 mmol) and 1,1'-bis(diphenylphosphino)ferrocene]dichloropalladium (16 mg, 0.022 mmol) was added. The system reacted at 100°C for 2 h. The reaction solution was extracted with ethyl acetate and water, the organic phase was dried, evaporated to dryness, and purified by preparative thin layer chromatography (dichloromethane/methanol = 10/1) to give the product tert-butyl (3-(8-fluoro-7-(3-(methoxymethoxy)-8-((triisopropylsilyl)ethynyl)naphthalen-1-yl)-2-((tetrahydro-1H-pyrrolidone-7a(5H)-yl)methoxy)pyrido[4,3-d]pyrimidin-4-yl)-3-azabicyclo[3.2.1]octan-8-yl) carbamate (70 mg, yield: 36%). ESI: [M+H]⁺=879.5.

Step 4: tert-butyl (3-(8-fluoro-7-(3-(methoxymethoxy)-8-((triisopropylsilyl)ethynyl)naphthalen-1-yl)-2-((tetrahydro-1H-pyrrolidone-7a(5H)-yl)methoxy)pyrido[4,3-d]pyrimidin-4-yl)-3-azabicyclo[3.2.1]octan-8-yl) carbamate (70 mg, 0.08 mmol) was dissolved in N, N-dimethylformamide (1 mL), to which cesium fluoride (122 mg, 0.8 mmol) was added. The mixture was allowed to react at room temperature for 2 h. The reaction solution was extracted with ethyl acetate and water, the organic phase was dried and evaporated to dryness to give the crude product tert-butyl (3-(7-(8-ethynyl-3-(methoxymethoxy)naphthalen-1-yl)-8-fluoro-2-((tetrahydro-1H-pyrrolizin 7a(5H)-yl)methoxy)pyrido[4,3-d]pyrimidin-4-yl)-3-azabicyclo[3.2.1]octan-8-yl) carbamate (60 mg). ESI: [M+H]⁺=723.3.

Step 5: the crude product tert-butyl (3-(7-(8-ethynyl-3-(methoxymethoxy)naphthalen-1-yl)-8-fluoro-2-((tetrahydro-1H-pyrrolizin 7a(5H)-yl)methoxy)pyridinyl[4,3-d]pyrimidin-4-yl)-3-azabicyclo[3.2.1]octan-8-yl) carbamate (60 mg) was dissolved in acetonitrile (0.5 mL), 4 M hydrochloric acid/dioxane solution (1 mL) was added in an ice bath. The mixture reacted for 0.5 h in an ice bath. The reaction solution was rotary dried and reverse phase preparative HPLC (trifluoroacetic acid method) was performed to give 4-(4-((8-amino-3-azabicyclo[3.2.1]octan-3-yl)-8-fluoro-2-(((tetrahydro-1H-pyrrolizin-7a(5H)-yl)methoxy)pyrido[4,3-d]pyrimidin-7-yl)-5-ethynylnaphthalen-2-ol (Z1, trifluoroacetate, 15 mg, yield over two steps: 33%). ESI: [M+H]⁺=579.3. ¹H NMR (500 MHz, CD₃OD) δ 9.12 (s, 1H), 7.84 (d, *J* = 8.2 Hz, 1H), 7.52 (d, *J* = 7.1 Hz, 1H), 7.44-7.38 (m, 1H), 7.35 (d, *J* = 2.5 Hz, 1H), 7.17 (d, *J* = 2.5 Hz, 1H), 4.78-4.65 (m, 4H), 3.91 (dd, *J =* 13.5, 7.1 Hz, 2H), 3.74-3.65 (m, 2H), 3.54 (t, *J =* 4.5 Hz, 1H), 3.28 (d, *J =* 6.3 Hz, 2H), 2.99 (s, 1H), 2.57 (d, *J =* 2.1 Hz, 2H), 2.38-2.29 (m, 2H), 2.25-2.09 (m, 6H), 2.00-1.92 (m, 2H), 1.75 (d, *J =* 9.2 Hz, 2H).

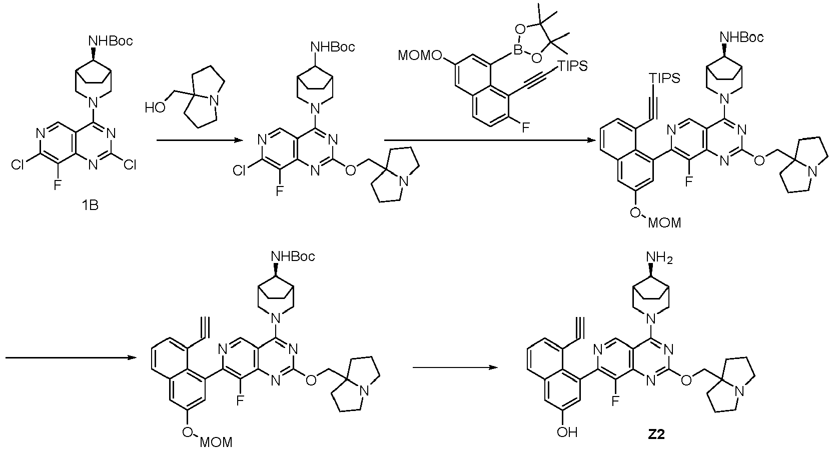

Step 6: compound 1B (260 mg, 0.59 mmol) was dissolved in 1,4 dioxane (3 mL), (tetrahydro-1H-pyrrolizin-7a(5H)-yl) methanol (166 mg, 1.18 mmol) and diisopropylethyl amine (228 mg, 1.77 mmol) were added and the mixture was allowed to react under argon protection at 80°C for 8 h. Water (20 mL) was added to the reaction solution, which was extracted with dichloromethane, dried over anhydrous sodium sulfate, rotary dried, and purified by column (50-100% ethyl acetate/petroleum ether) to give tert-butyl (3-(7-chloro-8-fluoro-2-((tetrahydro-1H-pyrrolizin-7a(5H)-yl)methoxy)pyrido][4,3-d]pyrimidin-4-yl)-3-azabicyclo[3.2.1]octan-8-yl) carbamate (135 mg, yield: 42%). ESI: [M+H]⁺=547.3.

Step 7: the obtained compound tert-butyl (3-(7-chloro-8-fluoro-2-((tetrahydro-1H-pyrrolizin-7a(5H)-yl)methoxy)pyridio][4,3-d]pyrimidin-4-yl)-3-azabicyclo[3.2.1]octan-8-yl) carbamate (130 mg, 0.24 mmol) was dissolved in dioxane/water (2 mL/0.2 mL), to which ((6-(methoxymethoxy)-8-(4,4,5,5-tetramethyl-1,3,2-dioxaborinan-2-yl)naphthalen-1-yl)ethynyl)triisopropylsilane (178 mg, 0.36 mmol), cesium carbonate (235 mg, 0.72 mmol) and 1,1'-bis(diphenylphosphino)ferrocene]dichloropalladium (17 mg, 0.024 mmol) were added. The mixture reacted at 100°C for 2 h. The reaction solution was extracted with ethyl acetate and water, the organic phase was dried, evaporated to dryness, and purified by preparative thin layer chromatography (dichloromethane/methanol = 10/1) to give the product tert-butyl (3-(8-fluoro-7-(3-(methoxymethoxy)-8-((triisopropylsilyl)ethynyl)naphthalen-1-yl)-2-((tetrahydro-1H-pyrrolidone-7a(5H)-yl)methoxy)pyrido[4,3-d]pyrimidin-4-yl)-3-azabicyclo[3.2.1]octan-8-yl) carbamate (55 mg, yield: 26%). ESI: [M+H]⁺=879.5.

Step 8: tert-butyl (3-(8-fluoro-7-(3-(methoxymethoxy)-8-((triisopropylsilyl)ethynyl)naphthalen-1-yl)-2-((tetrahydro-1H-pyrrolidone-7a(5H)-yl)methoxy)pyrido[4,3-d]pyrimidin-4-yl)-3-azabicyclo[3.2.1]octan-8-yl) carbamate (55 mg, 0.06 mmol) was dissolved in N, N-dimethylformamide (1 mL), to which cesium fluoride (91 mg, 0.6 mmol) was added. The system reacted at room temperature for 2 h. The reaction solution was extracted with ethyl acetate and water, the organic phase was dried and evaporated to dryness to give the crude product tert-butyl (3-(7-(8-ethynyl-3-(methoxymethoxy)naphthalen-1-yl)-8-fluoro-2-((tetrahydro-1H-pyrrolizin 7a(5H)-yl)methoxy)pyrido[4,3-d]pyrimidin-4-yl)-3-azabicyclo[3.2.1]octan-8-yl) carbamate (50 mg). ESI: [M+H]⁺=723.3.

Step 9: the crude product tert-butyl (3-(7-(8-ethynyl-3-(methoxymethoxy)naphthalen-1-yl)-8-fluoro-2-((tetrahydro-1H-pyrrolizin 7a(5H)-yl)methoxy)pyridinyl[4,3-d]pyrimidin-4-yl)-3-azabicyclo[3.2.1]octan-8-yl) carbamate (50 mg) was dissolved in acetonitrile (0.5 mL), 4 M hydrochloric acid/dioxane solution (1 mL) was added in an ice bath and reacted for 0.5 h in an ice bath. The reaction solution was rotary dried and reverse phase preparative HPLC (trifluoroacetic acid method) was performed to give 4-(4-((8-amino-3-azabicyclo[3.2.1]octan-3-yl)-8-fluoro-2-(((tetrahydro-1H-pyrrolizin-7a(5H)-yl)methoxy)pyrido[4,3-d]pyrimidin-7-yl)-5-ethynylnaphthalen-2-ol (Z2, trifluoroacetate, 15 mg, yield over two steps: 42%). ESI: [M+H]⁺=579.3. ¹H NMR (500 MHz, CD₃OD) δ 9.07 (s, 1H), 7.83 (d, *J* = 8.3 Hz, 1H), 7.51 (d, *J* = 7.1 Hz, 1H), 7.44-7.37 (m, 1H), 7.35 (d, *J* = 2.5 Hz, 1H), 7.17 (d, *J* = 2.5 Hz, 1H), 4.82 (d, *J* = 2.1 Hz, 2H), 4.71-4.62 (m, 2H), 3.72 (ddd, *J* = 19.3, 18.4, 8.7 Hz, 5H), 3.30-3.24 (m, 2H), 3.00 (s, 1H), 2.62 (d, *J* = 2.5 Hz, 2H), 2.39-2.28 (m, 2H), 2.26-2.06 (m, 6H), 1.99-1.92 (m, 2H), 1.80 (d, *J* = 9.5 Hz, 2H).

### Example 2: Synthesis of 4-(4-((1R,5S)-3,8-diazabicyclo[3.2.1]octan-3-yl)-8-fluoro-2-(((2-methylenetetrahydro-1H-pyrrolidone-7a(5H)-yl)methoxy)pyrido[4,3-d]pyrimidin-7-yl)-5-ethynyl-6-fluoronaphthalen-2-ol (Z3)

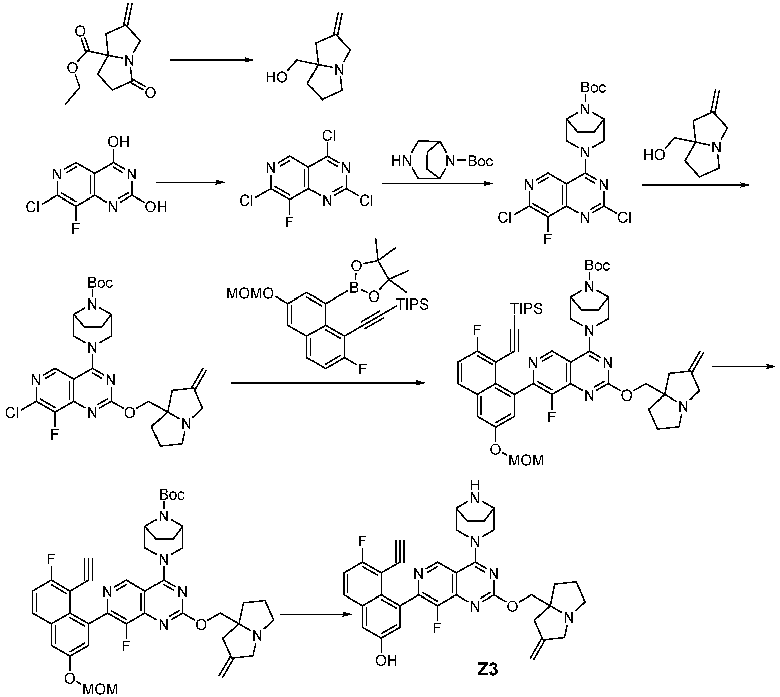

Step 1: lithium aluminum hydride (6.6 mL, 1 M, 6.60 mmol) was added dropwise to a solution of ethyl 2-methylene-5-oxotetrahydro-1H-pyrrolizine-7a(5H)-carboxylate (1.0 g, 3.317 mmol) in dry tetrahydrofuran (10.0 mL) under an ice-water bath condition, and the reaction was refluxed under nitrogen protection for 3 h. Upon completion of the reaction, the system was cooled to 0°C and quenched by dropwise addition of ice water (0.5 mL), dried over anhydrous sodium sulfate, filtered and the filtrate was rotary dried under reduced pressure to give the crude product (2-methylenetetrahydro-1H-pyrrolizin-7a(5H)-yl) methanol (560 mg, crude). ES-API: [M+H]⁺=154.1. Step 2: 7-chloro-8-fluoropyrido[4,3-d]pyrimidine-2,4-diol (500 mg, 2.32 mmol) was added in toluene (5.0 mL), then phosphorus oxychloride (1.07 g, 6.977 mmol) was added in the reactor followed by diisopropylethyl amine (0.904 g, 6.977 mmol). The system reacted at 110°C for 2 h, cooled to room temperature and the solvent was rotary dried under reduced pressure to give crude 2,4,7-trichloro-8-fluoropyrido[4,3-d]pyrimidine (602 mg, crude).

Step 3: diisopropylethyl amine (0.901 g, 6.975 mmol) and tert-butyl-3,8-azabicyclo[3.2.1]octane-8-carboxylate (542 mg, 2.557 mmol) was added to a solution of 2,4,7-trichloro-8-fluoropyrido[4,3-d]pyrimidine (602 mg, crude) in dichloromethane (15 mL), reacted at -40°C for 0.5 h. Upon completion of the reaction, water (20 mL) was added to the reaction solution, which was extracted with dichloromethane, dried over anhydrous sodium sulfate, rotary dried and purified by column (0-100% ethyl acetate/petroleum ether) to give compound (1R,5S)-tert-butyl-(2,7-dichloro-8-fluoropyrido[4,3-d]pyrimidin-4-yl-3,8-azabicyclo[3.2.1]octane)-8-carboxylate (500 mg, total yield over two steps: 50%). ES-API: [M+H]⁺=428.2.

Step 4: (1R,5S)-tert-butyl-(2,7-dichloro-8-fluoropyrido[4,3-d]pyrimidin-4-yl-3,8-azabicyclo[3.2.1]octane)-8-carboxylate (200 mg, 0.468 mmol) was dissolved in dioxane (2 mL) and (2-methylenetetrahydro-1H-pyrrolizin-7a(5H)-yl) methanol (200 mg, 1.307 mmol) and diisopropylethyl amine (200 mg, 1.55 mmol) were added and the mixture was allowed to react under argon protection at 120°C for 6 h. The reaction solution was cooled to room temperature, saturated brine (50 mL) was added to the reaction solution, and extracted twice with ethyl acetate (2 * 100 mL), combined with ethyl acetate phases, dried over anhydrous sodium sulfate, rotary dried under reduced pressure and the crude was purified by flash silica gel column (methanol/dichloromethane: 0-10%) to give the target product tert-butyl (1R,5S)-3-(7-chloro-8-fluoro-2-(((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (130mg, yield 51%). ES-API: [M+H]⁺=545.2. Step 5: tert-butyl (1R,5S)-3-(7-chloro-8-fluoro-2-(((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (130 mg, 0.2389 mmol) was dissolved in tetrahydrofuran/water (4 mL/0.5 mL) and ((2-fluoro-6-(methoxymethoxy)-8-(4,4,5,5-tetramethyl-1,3,2-dioxaborinan-2-yl)naphthalen-1-yl)ethynyl)triisopropylsilane (160 mg, 0.3125 mmol), cesium carbonate (232 mg, 0.7120 mmol) and tetrakis triphenylphosphine palladium (40 mg, 0.03463 mmol) were reacted at 115°C for 6 h after nitrogen gas replacement for four times. The reaction solution was cooled to room temperature, extracted with ethyl acetate (80 mL) and water (60 mL), the organic phase was dried over anhydrous sodium sulfate, filtered, the filtrate was rotary dried under reduced pressure and the crude was purified by flash silica gel column (methanol/dichloromethane: 0-10%) to give the product tert-butyl (1R,SS)-3-(8-fluoro-7-(7-fluoro-3-(methoxymethoxy))-8-((triisopropylsilyl)ethynyl)naphthalen-1-yl)-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (136 mg, yield: 64%). ES-API: [M/2+1]⁺=895.4.

Step 6: tert-butyl (1R,5S)-3-(8-fluoro-7-(7-fluoro-3-(methoxymethoxy))-8-((triisopropylsilyl)ethynyl)naphthalen-1-yl)-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (190 mg, 0.2125 mmol) was dissolved in N, N-dimethylformamide (10.0 mL). Cesium fluoride (323 mg, 2.125 mmol) was added, and the system reacted at room temperature for 2 h. Upon completion of the reaction, the reaction solution was extracted with ethyl acetate (80 mL) and water (60 mL), the organic phase was dried over anhydrous sodium sulfate, filtered and the filtrate was rotary dried under reduced pressure to give crude tert-butyl (1R,5S)-3-(7-(8-ethynyl-7-fluoro-3-(methoxymethoxy)naphthalen-1-yl)-8-fluoro-2-((2-methylenetetrahydro-1H-pyrrolizin)-7a(5H)-yl)methoxy)pyrido[4,3-d]pyrimidin-4-yl)-3,8-diazabicyclo[3.2.1]octane-8-carboxylate (200 mg, crude). ES-API: [M+1]⁺=739.3. The crude was used directly in the next reaction.

Step 7: tert-butyl (1R,5S)-3-(7-(8-ethynyl-7-fluoro-3-(methoxymethoxy)naphthalen-1-yl)-8-fluoro-2-((2-methylenetetrahydro-1H-pyrrolizin)-7a(5H)-yl)methoxy)pyrido[4,3-d]pyrimidin-4-yl)-3,8-diazabicyclo[3.2.1]octane-8-carboxylate (200 mg, crude) was dissolved in acetonitrile (10.0 mL), 4 M hydrochloric acid/dioxane solution (3.0 mL, 4 M, 12.0 mmol) was added in an ice bath, and the system reacted for 0.5 h in an ice bath. Upon completion of the reaction, the solvent was rotary dried under reduced pressure, dichloromethane (20 mL) was added, and triethylamine (3.0 mL) was added under an ice-water bath condition. After stirring for 10 min, dichloromethane (100 mL) and water (50 mL) were added and extracted once. The dichloromethane phase was dried over anhydrous sodium sulfate, filtered, and the filtrate was rotary dried under reduced pressure to give the crude, which was purified by preparative HPLC (formic acid method) to give the target compound 4-(4-((1R,5S)-3,8-diazabicyclo[3.2.1]octan-3-yl)-8-fluoro-2-(((2-methylenetetrahydro-1H-pyrrolizin-7a(5H)-yl)methoxy)pyrido[4,3-d]pyrimidin-7-yl)-5-ethynyl-6-fluoronaphthalen-2-ol (Z3, formate salt, 13.0 mg, total yield over two steps: 9.5%). ES-API: [M+1]⁺=595.3. ¹H NMR (500 MHz, CD₃OD) δ 9.08 (s, 1H), 8.47 (s, 2H), 7.87 (dd, *J* = 9.0, 5.5 Hz, 1H), 7.42-7.28 (m, 2H), 7.21 (d, *J* = 2.5 Hz, 1H), 5.22 (s, 2H), 4.75-4.68 (m, 2H), 4.66-4.55 (m, 2H), 4.23 (d, *J* = 14.0 Hz, 1H), 3.92 (s, 2H), 3.87-3.75 (m, 3H), 3.67-3.60 (m, 1H), 3.35 (s, 1H), 3.19-3.08 (m, 1H), 3.00 (d, *J* = 16.0 Hz, 1H), 2.73 (d, *J* = 16.0 Hz, 1H), 2.37-2.30 (m, 1H), 2.26-1.79 (m, 7H).

### Example 3: Synthesis of 4-(4-((1R,5S)-3,8-diazabicyclo[3.2.1]oct-6-en-3-yl)-8-fluoro-2-((tetrahydro-1H-pyrrolizin-7a(5H)-yl)methoxy)pyrido[4,3-d]pyrimidin-7-yl)-5-ethynylnaphthalen-2-ol (Z4)

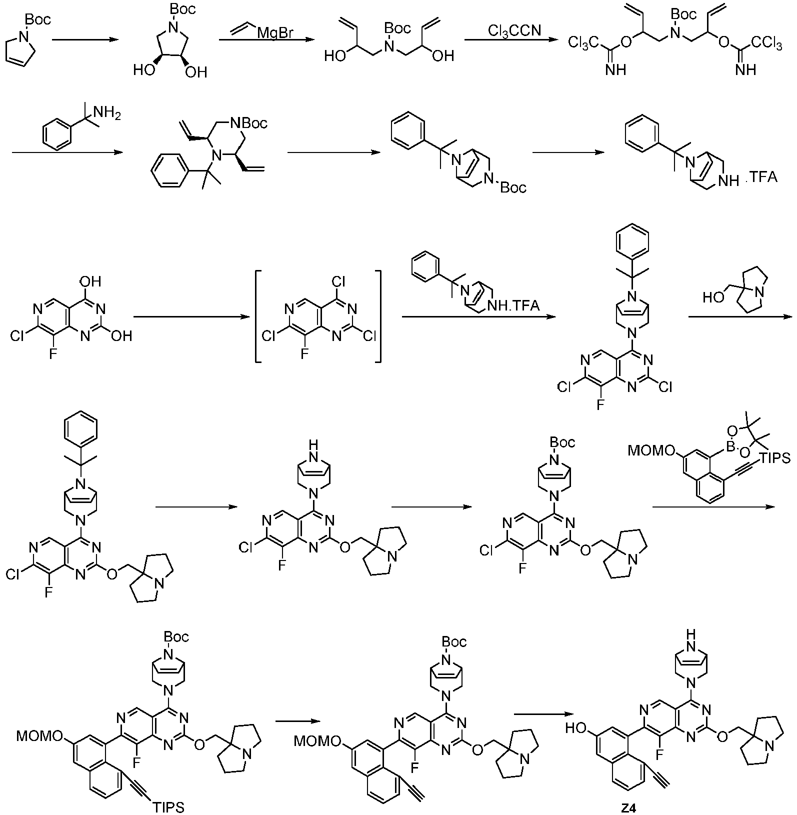

Step 1: tert-butyl 2,5-dihydro-1H-pyrrole-1-carboxylate (8.46 g, 50 mmol) was dissolved in acetone (100 mL), potassium osmate dihydrate (3.68 g, 10 mmol) and N-methylmorpholine-N-oxide (7.03 g, 60 mmol) were added successively. The mixture was allowed to react at room temperature for 24 h, filtered, washed with ethyl acetate, added with a proper amount of saturated sodium sulfite for washing, then dried by rotary drying and purified by column (0-90% ethyl acetate/petroleum ether) to give tert-butyl 3,4-dihydroxypyrrolidine-1-carboxylate (8.62 g, yield 85%). ES-API: [M-56]⁺=148.3.

Step 2: tert-butyl 3,4-dihydroxypyrrolidine-1-carboxylate (8.62 g, 42.46 mmol) was dissolved in 100 mL of dichloromethane, to which iodobenzene acetate (20.51 g, 163.69 mmol) slowly added in an ice bath, slowly warmed to room temperature and stirred for 1 h, after the completion of the reaction, cooled to -78°C, vinylmagnesium bromide (1.0 M solution in diethyl ether, 255 mmol, 255 mL) was slowly dropped in, then slowly warmed to room temperature and stirred for 16 h, add 1N HCl (150 mL) to quench the reaction, extracted with dichloromethane, dried over anhydrous sodium sulfate, concentrated and purified by column (0-80% ethyl acetate/petroleum ether) to give tert-butyl bis(2-hydroxybut-3-enyl) carbamate (8.19 g, yield 75%). ES-API: [M+Na]⁺=280.3.

Step 3: tert-butyl bis(2-hydroxybut-3-enyl) carbamate (8.19 g, 31.83 mmol) was dissolved in dichloromethane (100 mL), trichloroacetonitrile (27.57 g, 191 mmol) was added, DBU (4.06 g, 16.17 mmol) was slowly added in an ice bath, slowly warmed to room temperature and stirred for 18 h, concentrated and purified by column (0-30% ethyl acetate/petroleum ether) to give 1,1'-(tert-butoxycarbonyl azanediyl)bis(but-3-ene-2,1-diyl)bis(2,2,2-trichloroacetimidate) (8.5 g, yield 48.9%). ES-API: [M+Na]⁺=566.2.

Step 4: 1,1'-(tert-butoxycarbonyl azanediyl)bis(but-3-ene-2,1-diyl)bis(2,2,2-trichloroacetimidate) (8.5 g, 15.57 mmol) was dissolved in 1,2-dichloroethane (100 mL),[Ir(cod)Cl]₂ (628 mg, 0.93 mmol) was added, 2-phenylpropan-2-amine (2.53 g, 18.68 mmol) in 1,2-dichloroethane (10 mL) solution was slowly added in an ice bath. After stirring for 2 h at 0°C, the mixture was warmed to room temperature and stirred for 24 h, concentrated and purified by column (0-20% ethyl acetate/petroleum ether) to give tert-butyl 4-(2-phenylpropan-2-yl)-3,5-divinylpiperazine-1-carboxylate (4.16 g, yield 75.0%). ES-API: [M-116]⁺=239.3.

Step 5: tert-butyl 4-(2-phenylpropan-2-yl)-3,5-divinylpiperazine-1-carboxylate (4.16 g, 11.67 mmol) was dissolved in toluene (40 mL), to which the Grubbs 2^{nd} generation catalyst (495 mg, 0.58 mmol) was added. The mixture was refluxed the reaction at 120°C for 16 h, concentrated and purified by column (0-20% ethyl acetate/petroleum ether) to give tert-butyl 8-(2-phenylpropan-2-yl)-3,8-diazabicyclo[3.2.1]oct-6-ene-3-carboxylate (3.07 g, yield 80.0%). Characters: white solid. ES-API: [M+H]⁺=329.3.

Step 6: tert-butyl 8-(2-phenylpropan-2-yl)-3,8-diazabicyclo[3.2.1]oct-6-ene-3-carboxylate (3.07 g, 9.35 mmol) was dissolved in dichloromethane (30 mL), trifluoroacetic acid (5 mL) was added, reacted at room temperature for 2 h and concentrated to give the crude trifluoroacetate salt (3.04 g, yield 100%). ES-API: [M+H]⁺=229.2.

Steps 7 to 8: 7-chloro-8-fluoro[4,3-d]pyrimidine-2,4-diol (550 mg, 2.56 mmol) was added into a reactor, followed by phosphorus oxychloride (1.50 mL) and diisopropyl ethyl amine (1.5 g), and allowed to react at 100°C for 2 h, cooled to room temperature, and the phosphorus oxychloride was removed under reduced pressure, followed by adding diisopropyl ethyl amine (1.5 g), 8-(2-phenylpropan-2-yl)-3,8-diazabicyclo[3.2.1]oct-6-ene trifluoroacetate (833 mg, 2.56 mmol), and the system reacted at -40°C for 0.5 h. Water (20 mL) was added to the reaction solution, which was extracted with dichloromethane, dried over anhydrous sodium sulfate, rotary dried, and purified by column (0-50% ethyl acetate/petroleum ether) to give 2,7-dichloro-8-fluoro-4-((1R,SS)-8-(2-phenylpropan-2-yl)-3,8-diazabicyclo[3.2.1]oct-6-en-3-yl)pyrido[4,3-d]pyrimidine (220 mg, yield 19.3%). ES-API: [M+H]⁺=444.3.

Step 9: the compound 2,7-dichloro-8-fluoro-4-((1R,5S)-8-(2-phenylpropan-2-yl)-3,8-diazabicyclo[3.2.1]oct-6-en-3-yl)pyrido[4,3-d]pyrimidine (220 mg, 0.49 mmol) was dissolved in 1,4-dioxane (3 mL), (tetrahydro-1H-pyrrolizin-7a(5H)-yl) methanol (138 mg, 0.98 mmol) and diisopropylethyl amine (190 mg, 1.47 mmol) were added and the mixture was allowed to react under argon protection at 80°C for 8 h. Water (20 mL) was added to the reaction solution, which was extracted with ethyl acetate, dried over anhydrous sodium sulfate, rotary dried and purified by column (0-5% methanol/dichloromethane) to give 7-chloro-8-fluoro-2-((tetrahydro-1H-pyrrolizin-7a(5H)-yl)methoxy)-4-((1R,5S)-8-(2-phenylpropan-2-yl)-3,8-diazabicyclo[3.2.1]oct-6-en-3-yl)pyrido[4,3-d]pyrimidine (120 mg, yield: 44.6%). ESI: [M+H]⁺=549.2.

Step 10: 7-chloro-8-fluoro-2-((tetrahydro-1H-pyrrolizin-7a(5H)-yl)methoxy)-4-((1R,5S)-8-(2-phenylpropan-2-yl)-3,8-diazabicyclo[3.2.1]oct-6-en-3-yl)pyrido[4,3-d]pyrimidine (120 mg, 0.22 mmol) was dissolved in trifluoroacetic acid (6 mL), and the system reacted at 40°C for 2 h. Upon completion of the reaction, the reaction mixture was directly concentrated to give the crude product 4-((1R,5S)-3,8-diazabicyclo[3.2.1]oct-6-en-3-yl)-7-chloro-8-fluoro-2-((tetrahydro-1H-pyrrolizin-7a(5H)-yl)methoxy)pyrido[4,3-d]pyrimidine trifluoroacetate (116 mg, yield: 100%) ESI: [M+H]⁺=431.1.

Step 11: 4-((1R,5S)-3,8-diazabicyclo[3.2.1]oct-6-en-3-yl)-7-chloro-8-fluoro-2-((tetrahydro-1H-pyrrolizin-7a(5H)-yl)methoxy)pyrido[4,3-d]pyrimidine trifluoroacetate (116 mg, 0.22 mmol) was dissolved in tetrahydrofuran (10 mL). Triethylamine (67 mg, 0.66 mmol), di-tert-butyl dicarbonate (57 mg, 0.26 mmol) were added, and the system reacted at room temperature for 1 h, rotary dried and column purified (0-5% methanol/dichloromethane) to give compound tert-butyl (1R,5S)-3-(7-chloro-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (110 mg, yield: 94%). ESI: [M+H]⁺=531.2.

Step 12: the compound tert-butyl (1R,5S)-3-(7-chloro-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (110 mg, 0.20 mmol) was dissolved in tetrahydrofuran/water (4 mL/0.4 mL), triisopropyl ((6-(methoxymethoxy)-8-(4,4,5,5-tetramethyl-1,3,2-dioxaborolan-2-yl)naphthalen-1-yl)ethynyl)silane (198 mg, 0.40 mmol), potassium phosphate (127 mg, 0.60 mmol) and CataCXium A Pd G3 ((di (1-adamantyl)butylphosphino)-2-(2'-amino-1,1'-biphenyl)palladium (II) mesylate) (22 mg, 0.04 mmol) were added. The mixture reacted at 60°C for 2 h. The reaction solution was extracted with ethyl acetate and water, the organic phase was dried, evaporated to dryness, and purified by preparative thin layer chromatography (dichloromethane/methanol = 10/1) to give the product tert-butyl 3-(8-fluoro-2-((tetrahydro-1H-pyrrolizin-7a(5H)-yl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]oct-6-ene-8-carboxylate (110 mg, yield: 63.9%). ESI: [M+H]⁺=863.5.

Step 13: tert-butyl (1R,SS)-3-(8-fluoro-7-(3-(methoxymethoxy)-8-((triisopropylsilyl)ethynyl)naphthalen-1-yl)-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (110 mg, 0.13 mmol) was dissolved in N, N-dimethylformamide (2 mL) and cesium fluoride (91 mg, 0.64 mmol)was added, and the system reacted at room temperature for 15 min, and reverse phase preparation (CF₃COOH) was performed to give the product tert-butyl (1R,5S)-3-(7-(8-ethynyl-3-(methoxymethoxy)naphthalen-1-yl)-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (60 mg, yield: 65.2%). ESI: [M+H]⁺=707.3.

Step 14: tert-butyl (1R,5S)-3-(7-(8-ethynyl-3-(methoxymethoxy)naphthalen-1-yl)-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (30 mg) was dissolved in acetonitrile (3 mL), 4 M hydrochloric acid/dioxane solution (1 mL) was added in an ice bath, reacted for 1 h in an ice bath, alkalinity was adjusted by adding saturated sodium bicarbonate, and the reaction solution was rotary dried. Reverse phase preparative HPLC (formic acid method) was performed on the crude to give 4-(4-((1R,SS)-3,8-diazabicyclo[3.2.1]oct-6-en-3-yl)-8-fluoro-2-((tetrahydro-1H-pyrrolizin-7a(5H)-yl)methoxy)pyrido[4,3-d]pyrimidin-7-yl)-5-ethynylnaphthalen-2-ol (Z4, formate salt, 4.4 mg, yield: 18.5%). ESI: [M+H]⁺=563.3. ¹H NMR (400 MHz, CD₃OD) δ 9.10 (s, 1H), 8.45 (s, 2H), 7.83 (d, *J* = 8.4 Hz, 1H), 7.51 (d, *J* = 7.2 Hz, 1H), 7.42-7.39 (m, 1H), 7.34 (d, *J* = 2.4 Hz, 1H), 7.15 (d, *J* = 2.4 Hz, 1H), 6.43-6.21 (m, 2H), 4.70-4.52 (m, 4H), 4.22 (d, *J* = 4.4 Hz, 2H), 3.93 (d, *J* = 12.4 Hz, 2H), 3.69-3.65 (m, 2H), 3.29-3.18 (m, 2H), 3.02 (s, 1H), 2.46-1.96 (m, 8H).

### Example 4: Synthesis of 4-(4-((1R,5S)-3,8-diazabicyclo[3.2.1]octan-3-yl)-8-fluoro-2-((2-(fluoromethylene)tetrahydro-1H-pyrrolizin-7a(5H)-yl)methoxy)pyrido[4,3-d]pyrimidin-7-yl)-5-ethynylnaphthalen-2-ol (Z5)

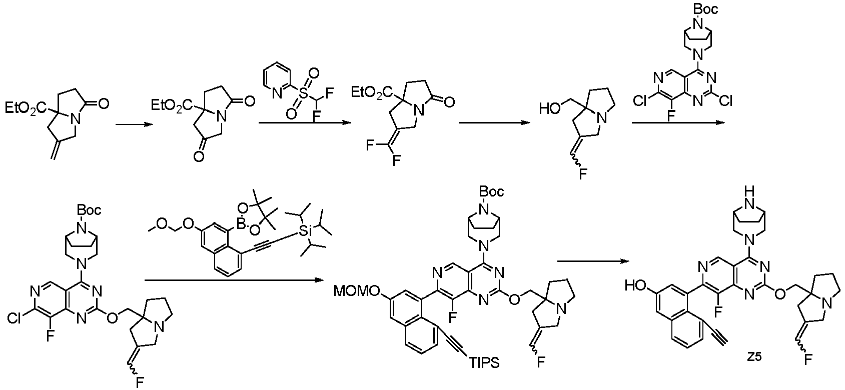

Step 1: a round bottom flask was charged with ethyl 2-methylene-5-oxotetrahydro-1H-pyrrolizine-7a(5H)-carboxylate (2 g, 9.57 mmol), potassium osmate dihydrate (176 mg, 0.48 mmol), water (40 mL) and tetrahydrofuran (20 mL) under nitrogen protection and cooled to 0°C. To the above mixture was added sodium periodate (6.14 g, 28.71 mmol) portionwise with stirring. The system was stirred at room temperature for 2 h. A saturated sodium sulfite aqueous solution (50 mL) was added to the reaction solution and extracted with dichloromethane/methanol (10/1) (50 mL x5). The organic phase was dried over anhydrous sodium sulfate and concentrated. The crude was purified by flash silica gel column (ethyl acetate/petroleum ether: 0-80%) to give ethyl 2,5-dioxotetrahydro-1H-pyrrolizine-7a(5H)-carboxylate (1.4 g, yield: 69%) as a brown oil. ES-API: [M+H]⁺=212.1.

Step 2: a round bottom flask was charged with ethyl 2,5-dioxotetrahydro-1H-pyrrolizine-7a(5H)-carboxylate (1g, 4.74 mmol), difluoromethyl (2-pyridyl)sulfone (1.65 g, 8.52 mmol) and N, N-dimethylformamide (24 mL). Cooled to -60°C under nitrogen protection. Potassium tert-butoxide in tetrahydrofuran (11.8 mL, 11.84 mmol) was slowly added dropwise to the above reaction solution with stirring over 30 min. The reaction was allowed to warm to room temperature and stirred at room temperature for 4 h. Saturated sodium bicarbonate aqueous solution (100 mL) was added to the reaction solution and extracted with ethyl acetate (50 mL x3). The organic phase was washed with saturated brine, dried over anhydrous sodium sulfate, and concentrated. The crude was purified by flash silica gel column (ethyl acetate/petroleum ether: 0-60%) to give ethyl 2-(difluoromethylene)-5-oxotetrahydro-1H-pyrrolizine-7a(5H)-carboxylate (130 mg, yield: 11.1%) as a yellow oil. ES-API: [M+H]⁺= 246.1.

Step 3: a reaction flask was charged with ethyl 2-(difluoromethylene)-5-oxotetrahydro-1H-pyrrolizine-7a(5H)-carboxylate (130 mg, 0.53 mmol) and tetrahydrofuran (5 mL) under nitrogen protection and cooled to 0°C. A 1 M solution of lithium tetrahydroaluminate in tetrahydrofuran (2.1 mL, 2.10 mmol) was added dropwise to the above reaction solution with stirring. The cold bath was removed and the reaction was stirred at 65°C for 2 h. Cooling to 0°C, the reaction was quenched with sodium sulfate decahydrate, and filtered, and the filter cake was washed with tetrahydrofuran. The filtrate was dried over anhydrous sodium sulfate and concentrated to give (2-(fluoromethylene)tetrahydro-1H-pyrrolizin-7a(5H)-yl) methanol (90 mg, crude) a yellow oil. ES-API: [M+H]⁺= 172.1.

Step 4: a reaction flask was charged with (2-(fluoromethylene)tetrahydro-1H-pyrrolizin-7a(5H)-yl) methanol (70 mg, crude), tert-butyl (1R,5S)-3-(2,7-dichloro-8-fluoropyrido[4,3-d]pyrimidin-4-yl)-3,8-diazabicyclo[3.2.1]octane-8-carboxylate (70 mg, 0.16 mmol), N, N-diisopropylethyl amine (62 mg, 0.48 mmol), dioxane (3 mL) and N, N-dimethylformamide (0.5 mL). The reaction was stirred at 110°C for 16 h. Water (30 mL) was added to the reaction solution and extracted with ethyl acetate (30 mL x3). The organic phase was washed with saturated brine, dried over anhydrous sodium sulfate, and concentrated. The crude was purified by flash silica gel column (methanol/dichloromethane = 0-6%) to give tert-butyl (1R,5S)-3-(7-chloro-8-fluoro-2-((2-(fluoromethylene)tetrahydro-1H-pyrrolizin-7a(5H)-yl)methoxy)pyrido[4,3-d]pyrimidin-4-yl)-3,8-diazabicyclo[3.2.1]octane-8-carboxylate (40 mg, yield: 44.4%). ES-API: [M+H]⁺= 563.3.

Step 5: a round bottom flask was charged with tert-butyl (1R,5S)-3-(7-chloro-8-fluoro-2-((2-(fluoromethylene)tetrahydro-1H-pyrrolizin-7a(5H)-yl)methoxy)pyrido[4,3-d]pyrimidin-4-yl)-3,8-diazabicyclo[3.2.1]octane-8-carboxylate (40mg, 0.071 mmol), triisopropyl ((6-(methoxymethoxy)-8-(4,4,5,5-tetramethyl-1,3,2-dioxaborolan-2-yl)naphthalen-1-yl)ethynyl)silane (70 mg, 0.14 mmol), cataCXiumA-Pd-G3 (5 mg, 0.0071 mmol), potassium phosphate (45 mg, 0.21 mmol), tetrahydrofuran (3 mL) and water (0.6 mL). The system was heated by stirring in an oil bath at 65°C under nitrogen protection for 16 h. Water (30 mL) was added to the reaction solution, which was extracted with ethyl acetate (30 mL x3). The organic phase dried over anhydrous sodium sulfate and concentrated. The crude was purified by flash silica gel column (methanol/dichloromethane = 0-6%) to give tert-butyl (1R,5S)-3-(8-fluoro-2-((2-(fluoromethylene)tetrahydro-1H-pyrrolizin-7a(5H)-yl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane -8-carboxylate (20 mg, yield: 31.5%) as a yellow solid. ES-API: [M+H]⁺= 895.5.

Step 6: a round bottom flask was charged with tert-butyl (1R,5S)-3-(8-fluoro-2-((2-(fluoromethylene)tetrahydro-1H-pyrrolizin-7a(5H)-yl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (20 mg, 0.022 mmol), cesium fluoride (33 mg, 0.22 mmol), and anhydrous N, N-dimethylformamide (2 mL). The reaction was stirred at room temperature for 1 hour. Water (30 mL) was added to the reaction solution, extracted with ethyl acetate (30 mL x3), and the organic phase was dried over anhydrous sodium sulfate and concentrated. The concentrate was dissolved in acetonitrile (1 mL). The solution was cooled to 0°C and a 4 M hydrogen chloride/dioxane solution (2 mL) was added. The reaction was stirred at 0°C for 1.5 h. The reaction solution was concentrated and the crude was purified by preparative HPLC (alkaline method) to give 4-(4-((1R,5S)-3,8-diazabicyclo[3.2.1]octan-3-yl)-8-fluoro-2-((2-(fluoromethylene)tetrahydro-1H-pyrrolizin-7a(5H)-yl)methoxy)pyrido[4,3-d]pyrimidin-7-yl)-5-ethynylnaphthalen-2-ol (Z5, 1.06 mg, yield: 8.1%) as a yellow solid. ES-API: [M+H]⁺=595.3.

### Example 5: Synthesis of 4-((1R,SS)-3,8-diazabicyclo[3.2.1]oct-6-en-3-yl)-7-(8-ethynylnaphthalen-1-yl)-8-tluoro-2-((tetrahydro-1H-pyrrolizin-7a(SH)-yl)methoxy)pyrido[4,3-d]pyrimidine (Z6)

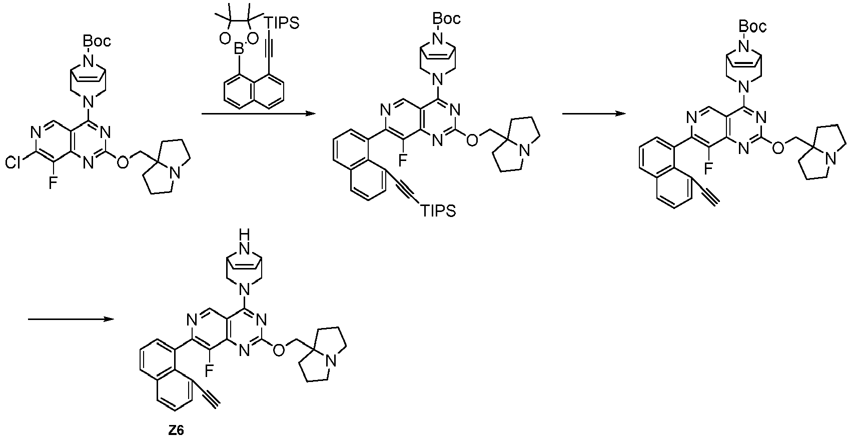

Step 1: the compound tert-butyl (1R,5S)-3-(7-chloro-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (110 mg, 0.20 mmol) was dissolved in tetrahydrofuran/water (4 mL/0.4 mL), triisopropyl ((8-(4,4,5,5-tetramethyl-1,3,2-dioxaborolan-2-yl)naphthalen-1-yl)ethynyl)silane (174 mg, 0.40mmol), potassium phosphate (127 mg, 0.60 mmol) and CataCXium A Pd G₃ (22 mg, 0.04 mmol) were reacted at 60°C for 2 h. The reaction solution was extracted with ethyl acetate (5 mL x2) and water (5 mL), the organic phase was dried over anhydrous sodium sulfate, evaporated to dryness and purified by preparative thin layer chromatography (dichloromethane/methanol = 10/1) to give the product tert-butyl (1R,5S)-3-(8-fluoro-2-((tetrahydro-1H-pyrrolizin-7a(5H)-yl)methoxy)-7-(8-((triisopropylsilyl)ethynyl)naphthalen-1-yl)pyrido[4,3-d]pyrimidin-4-yl)-3,8-diazabicyclo[3.2.1]oct-6-ene-8-carboxylate (100 mg, yield: 62.3%). ESI: [M+H]⁺=803.5.

Step 2: tert-butyl (1R,5S)-3-(8-fluoro-2-((tetrahydro-1H-pyrrolizin-7a(5H)-yl)methoxy)-7-(8-((triisopropylsilyl)ethynyl)naphthalen-1-yl)pyrido[4,3-d]pyrimidin-4-yl)-3,8-diazabicyclo[3.2.1]oct-6-ene-8-carboxylate (100 mg, 0.12 mmol) was dissolved in N, N-dimethylformamide (2 mL) and cesium fluoride (85 mg, 0.60 mmol) was added, and the system reacted at room temperature for 15 min, reverse phase preparation (CF₃COOH) was performed to give the product tert-butyl (1R,5S)-3-(7-(8-ethynylnaphthalen-1-yl)-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (65 mg, yield: 80.8%). ESI: [M+H]⁺=647.3.

Step 3: the compound (1R,5S)-tert-butyl 3-(7-(8-ethynylnaphthalen-1-yl)-8-fluoro-2-((tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (65 mg, 0.10 mmol) was dissolved in acetonitrile (3 mL), 4 M hydrochloric acid/dioxane solution (1 mL) was added in an ice bath, reacted for 1 h in an ice bath, alkalinity was adjusted by adding saturated sodium bicarbonate, and the reaction solution was rotary dried. Reverse phase preparative HPLC (formic acid method) was performed to give 4-((1R,5S)-3,8-diazabicyclo[3.2.1]oct-6-en-3-yl)-7-(8-ethynylnaphthalen-1-yl)-8-fluoro-2-((tetrahydro-1H-pyrrolizin-7a(5H)-yl)methoxy)pyrido[4,3-d]pyrimidine (Z6, formate salt, 9.9 mg, yield: 18.1%). ESI: [M+H]⁺=547.2. ¹H NMR (400 MHz, CD₃OD) δ 9.10 (s, 1H), 8.12 (d, *J* = 8.1 Hz, 1H), 8.07 (d, *J* = 8.1 Hz, 1H), 7.75 (d, *J* = 6.9 Hz, 1H), 7.68 (t, *J* = 7.6 Hz, 1H), 7.61-7.49 (m, 2H), 6.36 (d, *J* = 7.4 Hz, 2H), 4.73 (s, 2H), 4.67-4.57 (m, 2H), 4.39 (s, 2H), 4.03 (s, 2H), 3.68 (d, *J* = 4.3 Hz, 2H), 3.28-3.20 (m, 2H), 3.12 (s, 1H), 2.31 (s, 2H), 2.25-2.03 (m, 6H).

### Example 6: Synthesis of 4-(4-((1R,5S)-3,8-diazabicyclo[3.2.1]oct-6-en-3-yl)-8-fluoro-2-(((2R,7aS)-2-fluorotetrahydro-1H-pyrrolizin-7a(5H)-yl)methoxy)pyrido[4,3-d]pyrimidin-7-yl)-5-ethynylnaphthalen-2-ol (Z7)

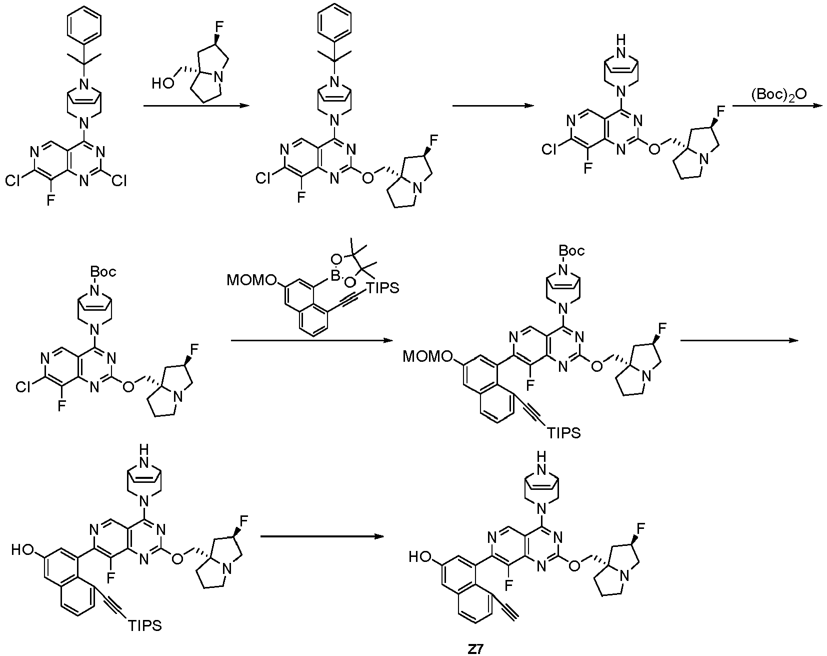

Step 1: the compound 2,7-dichloro-8-fluoro-4-((1R,5S)-8-(2-phenylpropan-2-yl)-3,8-diazabicyclo[3.2.1]oct-6-en-3-yl)pyrido[4,3-d]pyrimidine (150 mg, 0.34 mmol) was dissolved in 1,4 dioxane (3 mL). ((2R,7aS)-2-fluorotetrahydro-1H-pyrrolizin-7a(5H)-yl) methanol (108 mg, 0.68 mmol) and diisopropylethyl amine (131 mg, 1.02 mmol) were added, and the system reacted under argon protection at 80°C for 8 h. Water (20 mL) was added to the reaction solution, which was extracted with ethyl acetate and water (10 mL x3), dried over anhydrous sodium sulfate, rotary dried, and purified by column chromatography (methanol/dichloromethane = 0-5%) to give 7-chloro-8-fluoro-2-(((2R,7aS)-2-fluorotetrahydro-1H-pyrrolizin-7a(SH)-yl)methoxy)-4-((1R,SS)-8-(2-phenylpropan-2-yl)-3,8-diazabicyclo[3.2.1]oct-6-en-3-yl)pyrido[4,3-d]pyrimidine (110 mg, yield: 57.4%). ESI: [M+H]⁺=567.3.

Step 2: 7-chloro-8-fluoro-2-(((2R,7aS)-2-fluorotetrahydro-1H-pyrrolizin-7a(SH)-yl)methoxy)-4-((1R,5S)-8-(2-phenylpropan-2-yl)-3,8-diazabicyclo[3.2.1]oct-6-en-3-yl)pyrido[4,3-d]pyrimidine (110 mg, 0.19 mmol) was dissolved in trifluoroacetic acid (4 mL), and the system reacted at 40°C for 2 h. Upon completion of the reaction, the reaction solution was concentrated to give the crude product 4-((1R,5S)-3,8-diazabicyclo[3.2.1]oct-6-en-3-yl)-7-chloro-8-fluoro-2-(((2R,7aS)-2-fluorotetrahydro-1H-pyrrolizin-7a(5H)-yl)methoxy)pyrido[4,3-d]pyrimidine trifluoroacetate (105 mg, yield: 100%). ESI: [M+H]⁺=448.1.

Step 3: 4-((1R,5S)-3,8-diazabicyclo[3.2.1]oct-6-en-3-yl)-7-chloro-8-fluoro-2-(((2R,7aS)-2-fluorotetrahydro-1H-pyrrolizin-7a(5H)-yl)methoxy)pyrido[4,3-d]pyrimidine trifluoroacetate (105mg, 0.19 mmol) was dissolved in tetrahydrofuran (10 mL) and triethylamine (67 mg, 0.57 mmol), di-tert-butyl dicarbonate (50 mg, 0.23 mmol) was added and the mixture was reacted at room temperature for 1 h, rotary dried and purified by column chromatography (methanol/dichloromethane = 0-5%) to give compound tert-butyl (1R,5S)-3-(7-chloro-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (110 mg, yield: 94%). ESI: [M+H]⁺=549.3.

Step 4: the compound tert-butyl (1R,5S)-3-(7-chloro-8-fluoro-2-(((2R,7aS)-2-tetrahydro-1H-pyrrolizin-7a(5H)-yl)methoxy)pyrido[4,3-d]pyrimidin-4-yl)-3,8-diazabicyclo[3.2.1]oct-6-ene-8-carboxylate (110 mg, 0.20 mmol) was dissolved in tetrahydrofuran/water (4 mL/0.4 mL). Triisopropyl ((6-(methoxymethoxy)-8-(4,4,5,5-tetramethyl-1,3,2-dioxaborolan-2-yl)naphthalen-1-yl)ethynyl)silane (198mg, 0.40 mmol), potassium phosphate (127 mg, 0.60 mmol) and CataCXium A Pd G₃ (22 mg, 0.04 mmol) were added, and the system reacted at 60°C for 2 h. The reaction solution was extracted with ethyl acetate and water (10 mL x3), and the organic phase was dried over anhydrous sodium sulfate and rotary dried, purified by preparative thin layer chromatography (dichloromethane/methanol = 10/1) to give the product tert-butyl (1R,5S)-3-(8-fluoro-2-(((2R,7aS)-2-fluorotetrahydro-1H-pyrrolizin-7a(5H)-yl)methoxy)-7-(3-(methoxymethoxy)-8-((triisopropylsilyl)ethynyl)naphthalen-1-yl)pyrido[4,3-d]pyrimidin-4-yl)-3.8-diazabicyclo[3.2.1]oct-6-ene-8-carboxylate (150 mg, yield: 85.2%). ESI: [M+H]⁺=881.4.

Step 5: the compound tert-butyl (1R,SS)-3-(8-fluoro-2-(((2R,7aS)-2-fluorotetrahydro-1H-pyrrolizin-7a(5H)-yl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]oct-6-ene-8-carboxylate (75 mg, 0.085 mmol) was dissolved in acetonitrile (3 mL). A 4 M solution of hydrochloric acid/dioxane solution (1 mL) was added in an ice bath, and the system reacted for 1 h in an ice bath, alkalinity was adjusted by adding saturated sodium bicarbonate, extracted with ethyl acetate and water (5 mL x2), the organic phase was dried over anhydrous sodium sulfate and rotary dried to give 4-(4-((1R,5S)-3,8-diazabicyclo[3.2.1]oct-6-en-3-yl)-8-fluoro-2-(((2R,7aS)-2-fluorotetrahydro-1H-pyrrolizin-7a(5H)-yl)methoxy)pyrido[4,3-d]pyrimidin-7-yl)-5-((triisopropylsilyl)ethynyl)naphthalen-2-ol (80 mg, crude), the next reaction was carried on without purification. ESI: [M+H]⁺=737.3.

Step 6: crude 4-(4-((1R,5S)-3,8-diazabicyclo[3.2.1]oct-6-en-3-yl)-8-fluoro-2-(((2R,7aS)-2-fluorotetrahydro-1H-pyrrolizin-7a(5H)-yl)methoxy)pyrido[4,3-d]pyrimidin-7-yl)-5-((triisopropylsilyl)ethynyl)naphthalen-2-ol (80mg) was dissolved in N, N-dimethylformamide (2 mL). Cesium fluoride (91 mg, 0.64 mmol) was added, and the system reacted at room temperature for 15 min. Filtered and purified by preparative HPLC (formic acid method) to give 4-(4-((1R,5S)-3,8-diazabicyclo[3.2.1]oct-6-en-3-yl)-8-fluoro-2-(((2R,7aS)-2-fluorotetrahydro-1H-pyrrolizin-7a(5H)-yl)methoxy)pyrido[4,3-d]pyrimidin-7-yl)-5-ethynylnaphthalen-2-ol (Z7, formate salt, 12 mg, yield: 24%). ESI: [M+H]⁺=581.2. ¹H NMR (400 MHz, CD₃OD) δ 9.08 (s, 1H), 8.36 (s, 1H), 7.83 (d, J = 7.7 Hz, 1H), 7.50 (s, 1H), 7.37 (d, J = 24.6 Hz, 2H), 7.14 (s, 1H), 6.36 (s, 2H), 5.51 (d, J = 52.0 Hz, 1H), 4.70 (s, 2H), 4.56 (s, 2H), 4.32 (s, 2H), 4.06-3.58 (m, 5H), 3.01 (s, 1H), 2.56 (d, J = 40.7 Hz, 2H), 2.23 (t, J = 48.1 Hz, 5H).

### Example 7: Synthesis of 4-(4-((1R,5S)-3,8-diazabicyclo[3.2.1]octan-3-yl)-8-fluoro-2-((2-methylenetetrahydro-1H-pyrrolizin-7a(5H)-yl)methoxy)pyrido[4,3-d]pyrimidin-7-yl)-5-ethynylnaphthalen-2-ol (Z8)

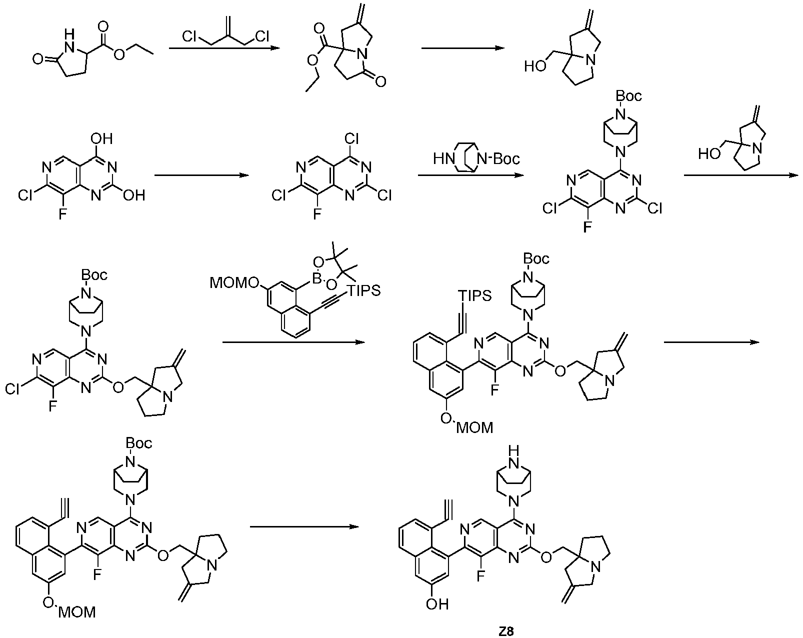

Step 1: ethyl 5-oxopyrrolidine-2-carboxylate (10 g, 63.69 mmol) and 3-chloro-2-(chloromethyl)prop -1-ene (12.64 g, 101.9 mmol) were dissolved in tetrahydrofuran (50 mL), 1 M LiHMDS (127 mL) was added at -40°C, the mixture was warmed to room temperature and reacted for 16 h, quenched with ammonium chloride solution (50 mL), extracted with ethyl acetate (50 ml x1), the organic phase was washed with brine, dried over anhydrous sodium sulfate and concentrated, which was purified by column chromatography (petroleum ether/ethyl acetate = 1/1) to give the product ethyl 2-methylene-5-oxotetrahydro-1H-pyrrolizine-7a(5H)-carboxylate (4.2 g, yield: 31.56%). ES-API: [M+H]⁺= 210.1.

Step 2: lithium tetrahydroaluminium (1.52 g, 40.15 mmol) was dissolved in tetrahydrofuran (20 mL), cooled to 0°C, a solution of ethyl 2-methylene-5-oxotetrahydro-1H-pyrrolizine-7a(5H)-carboxylate (4.2 g, 20.07 mmol) in tetrahydrofuran (20 mL) was added dropwise thereto. The mixture was heated to 70°C and reacted for 3 h. Water (1.5 mL) and 15% sodium hydroxide aqueous solution (1.5 mL) were added to the reaction solution. The mixture was quenched with water (4.5 mL) and filtered, and concentrated to give the crude product (2-methylenetetrahydro-1H-pyrrolizin-7a(5H)-yl)-methanol (2.5 g, yield: 81.29%). ES-API: [M+H]⁺= 154.1.

Step 3: 7-chloro-8-fluoro[4,3-d]pyrimidine-2,4-diol (1 g, 4.65 mmol) was dissolved in toluene (10 mL), phosphorus oxychloride (3.56 g, 23.19 mmol) was added followed by diisopropylethyl amine (3 g, 23.19 mmol), allowed to react at 110°C for 2 h, cooled to room temperature, and the phosphorus oxychloride was rotary dried to remove under reduced pressure to obtain crude 2,4,7-trichloro-8-fluoropyrido[4,3-d]pyrimidine (2 g, yield: 100%).

Step 4: 2,4,7-trichloro-8-fluoropyrido[4,3-d]pyrimidine (2 g, crude) was dissolved in dichloromethane (20 mL), diisopropyl ethyl amine (1.8g, 13.95 mmol) was added, tert-butyl-3,8-diazabicyclo[3.2.1]octane-8-carboxylate (986 mg, 4.65 mmol) was added at -40°C, and the system reacted for 0.5 h at -40°C. Water (20 mL) was added to the reaction solution, which was extracted with dichloromethane (20 mL x1), dried over anhydrous sodium sulfate, rotary dried, and purified by column chromatography (ethyl acetate/petroleum ether = 5/1) to give tert-butyl (1R,5S)-3-(2,7-dichloro-8-fluoropyrido[4,3-d]pyrimidin-4-yl)-3,8-diazabicyclo[3.2.1]octane-8-carboxylate (600 mg, yield 30.3%). ES-API: [M+H]⁺=428.1.

Step 5: tert-butyl (1R,SS)-3-(2,7-dichloro-8-fluoropyrido[4,3-d]pyrimidin-4-yl)-3,8-diazabicyclo[3.2.1]octane-8-carboxylate (600mg, 1.4 mmol) was dissolved in 1,4 dioxane (6 mL). (2-methylenetetrahydro-1H-pyrrolizin-7a(5H)-yl)-methanol (429 mg, 2.8 mmol) and diisopropylethyl amine (543 mg, 4.2 mmol) were added, and the system reacted under argon protection at 100°C for 16 h. Water (20 mL) was added to the reaction solution, which was extracted with ethyl acetate (10 mL x1), dried over anhydrous sodium sulfate, rotary dried and purified by column chromatography (ethyl acetate/petroleum ether = 1/1) to give tert-butyl (1R,5S)-3-(7-chloro-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (400 mg, yield 52.39%). ES-API: [M+H]⁺=545.2

Step 6: tert-butyl (1R,5S)-3-(7-chloro-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (100 mg, 0.018 mmol) was dissolved in tetrahydrofuran/water (1 mL/0.2 mL). ((6-(methoxymethoxy)-8-(4,4,5,5-tetramethyl-1,3,2-dioxaborinan-2-yl)naphthalen-1-yl)ethynyl)triisopropylsilane (18.2 mg, 0.036 mmol), potassium phosphate (11.6 mg, 0.055 mmol) and (docosyl-n-butylphosphino) (2'-amino-1,1'-biphenyl-2-yl)palladium (II) methanesulfonate dichloromethane complex (2 mg, 0.002 mmol) were added, and the system reacted at 60°C for 2 h. Water (5 mL) was added to the reaction solution, which was extracted with ethyl acetate (5 mL x1), the organic phase was dried over anhydrous sodium sulfate and rotary dried, and purified by preparative thin layer chromatography (dichloromethane/methanol = 10/1) to give the product tert-butyl (1R,5S)-3-(8-fluoro-7-(3-(methoxymethoxy)-8-((triisopropylsilyl)ethynyl)naphthalen-1-yl)-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (40 mg, yield: 24.8%). ES-API: [M+1]+=877.5.

Step 7: tert-butyl (1R,5S)-3-(8-fluoro-7-(3-(methoxymethoxy)-8-((triisopropylsilyl)ethynyl)naphthalen-1-yl)-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (40mg, 0.046 mmol) was dissolved in N, N-dimethylformamide (1 mL). Cesium fluoride (69.3 mg, 0.456 mmol) and one drop of water were added, and the system reacted at room temperature for 16 h. Water (5 mL) was added to the reaction solution, which was extracted with ethyl acetate (5 mL x1) and the organic phase was dried over anhydrous sodium sulfate and rotary dried to give the crude product tert-butyl (1R,5S)-3-(7-(8-ethynyl-3-(methoxymethoxy)naphthalen-1-yl)-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (60 mg, crude). ES-API: [M+1]+=721.3.

Step 8: tert-butyl (1R,5S)-3-(7-(8-ethynyl-3-(methoxymethoxy)naphthalen-1-yl)-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (60 mg, 0.044 mmol) was dissolved in acetonitrile (0.5 mL), 4 M hydrochloric acid/dioxane solution (0.5 mL) was added in an ice bath, and the system reacted for 0.5 h in an ice bath. The reaction solution was rotary dried and purified by thin layer chromatography (dichloromethane/methanol = 5/1) to give the product 4-(4-((1R,5S)-3,8-diazabicyclo[3.2.1]octan-3-yl)-8-fluoro-2-((2-methylenetetrahydro-1H-pyrrolizin-7a(5H)-yl)methoxy)pyrido[4,3-d]pyrimidin-7-yl)-5-ethynylnaphthalen-2-ol (Z8, 4.4 mg, yield: 17.2%). ES-API: [M+1]⁺=577.2. ¹H NMR (400 MHz, CDCl₃) δ 8.89 (s, 1H), 7.63-7.61(d, 1H), 7.46-7.40 (t,1H), 7.25 (s,1H), 6.98 (s,2H), 4.92(s,2H), 4.54-4.51 (m, 1H), 4.41-4.38 (m, 1H), 4.21 (s,2H), 3.79-3.75 (m, 1H), 3.57-3.50 (m, 3H), 3.49-3.47 (m, 1H),3.25-3.21 (m, 2H), 2.80-2.77(m, 1H), 2.69-2.67(m, 1H), 2.48-2.46(m, 1H), 2.41-2.36(m, 2H), 2.36-2.15(m, 3H), 2.11-1.91(m, 2H), 1.70-1.67(m, 2H), 1.60-1.57(m, 2H).

### Example 8: Synthesis of 4-(4-((1R,5S)-3,8-diazabicyclo[3.2.1]octan-3-yl)-8-fluoro-2-((2-(propan-ylidene) tetrahydro-1H-pyrrolizin-7a(5H)-yl)methoxy)pyrido[4,3-d]pyrimidin-7-yl)-5-ethynylnaphthalen-2-ol (Z14)

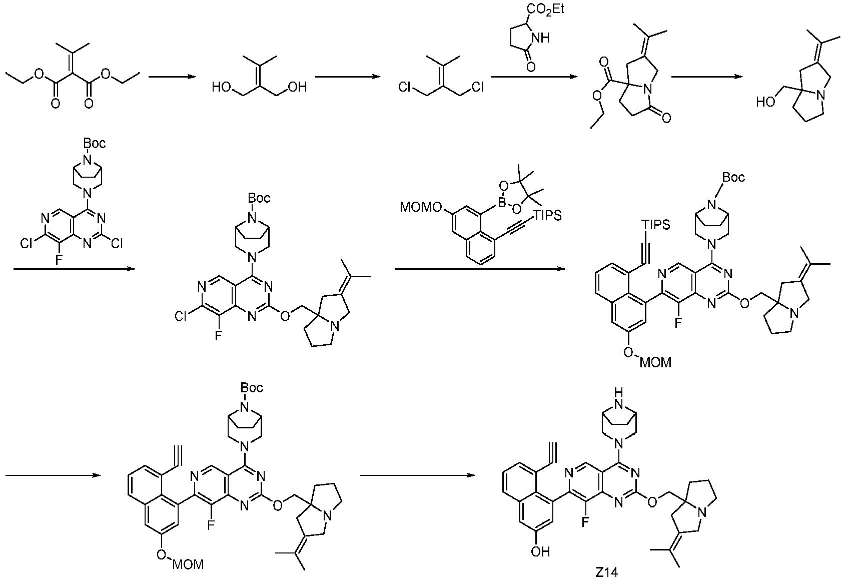

Step 1: a solution of diethyl 2-(propan-2-ylidene) malonate (3.0 g, 15.0 mmol) in dichloromethane (20 mL) was added dropwise to a solution of diisobutylaluminum hydride (50.0 mL, 50.0 mmol, 1 M in Toluene) at -78°C. After the addition was complete, the reaction was slowly warmed to 0°C and reacted for 1 h. Upon completion of the reaction, water (4 mL) and sodium hydroxide aqueous solution (8.0 mL, 2 M, 16.0 mmol) were added to the system successively, dried over anhydrous sodium sulfate, filtered, and the filtrate was rotary dried under reduced pressure. The crude was purified by flash silica gel column (ethyl acetate/petroleum ether = 0-100%) to obtain 2-(propan-2-ylidene) propane-1,3-diol (450 mg, yield: 26.5%). ES-API: [M+H]⁺=117.1.

Step 2: triethylamine (2.5 mL, 17.97 mmol) and methanesulfonyl chloride (1.50 g, 13.09 mmol) was successively added to a solution of 2-(propan-2-ylidene) propane-1,3-diol (500 mg, 4.310 mmol) in dichloromethane (10.0 mL) at 0°C. After the addition, the reaction was slowly warmed to room temperature and reacted under a nitrogen atmosphere for 3 h. Upon completion of the reaction, saturated sodium bicarbonate solution (50 mL) was added to the system and extracted with dichloromethane (80 mL X2). The organic phase was combined, dried over anhydrous sodium sulfate, and filtered, and the filtrate was rotary dried under reduced pressure. The crude was purified by flash silica gel column (ethyl acetate/petroleum ether = 0-10%) to obtain the target compound 1-chloro-2-(chloromethyl)-3-methylbut-2-ene (249.0 mg, yield: 38%). ES-API:
[M+H]⁺=153.0.

Step 3: to dry tetrahydrofuran (10.0 mL) was added ethyl 5-oxopyrrolidine-2-carboxylate (300 mg, 1.910 mmol) and 1-chloro-2-(chloromethyl)-3-methylbut-2-ene (400 mg, 2.631 mmol) at -40°C under nitrogen atmosphere and lithium bis trimethyl silylamide solution (5.0 mL, 5.0 mmol, 1 M in THF) was added dropwise to the above reaction. The reaction was slowly warmed to room temperature and stirred for 20 h. The reaction was cooled to 0-5°C, poured into hydrochloric acid aqueous solution (2.0 mL, 1 M, 2.0 mmol), and the pH was adjusted to 7 with hydrochloric acid aqueous solution (2 M). After extraction with ethyl acetate (50 mL X2), the organic phase was combined, dried over anhydrous sodium sulfate, and filtered and the filtrate was rotary dried under reduced pressure. The crude was purified by flash silica gel column (ethyl acetate/petroleum ether = 0-50%) to give ethyl 5-oxo-2-(propan-2-ylidene) tetrahydro-1H-pyrrolizine-7a(5H)-carboxylate (57 mg, yield: 12.6%). ES-API: [M+H]⁺=238.1.

Step 4: lithium aluminum hydride solution (20 mL, 20.0 mmol, 1 M in THF) was addedd ropwise to a solution of ethyl 2-(propan-2-ylidene)-5-oxotetrahydro-1H-pyrrolizine 7a(5H)-carboxylate (300 mg, 1.265 mmol) in dry tetrahydrofuran (10.0 mL) under an ice-water bath condition, and the reaction was refluxed under nitrogen protection for 3 h. Upon completion of the reaction, it was cooled to 0°C, quenched by dropwise addition of ice water (0.5 mL), dried over anhydrous sodium sulfate, filtered and the filtrate was rotary dried under reduced pressure to give (2-(propan-2-ylidene) tetrahydro-1H-pyrrolizin-7a(5H)-yl) methanol (180 mg, yield: 78%). ES-API:
[M+H]⁺=182.2.

Step 5: tert-butyl (1R,SS)-3-(2,7-dichloro-8-fluoropyrido[4,3-d]pyrimidin-4-yl)-3,8-diazabicyclo[3.2.1]octane-8-carboxylate (300 mg, 0.7025 mmol) was dissolved in dioxane (10 mL). (2-(propan-2-ylidene) tetrahydro-1H-pyrrolizin-7a(5H)-yl) methanol (180 mg, 0.9944 mmol) and diisopropylethyl amine (908 mg, 7.025 mmol) were added, and the system reacted under argon protection at 110°C for 6 h. The reaction solution was cooled to room temperature, saturated brine (50 mL) was added, and extracted with ethyl acetate (100 mL X2). The organic phase was combined, dried over anhydrous sodium sulfate, and rotary dried under reduced pressure. The crude was purified by flash silica gel column (methanol/dichloromethane: 0-10%) to give tert-butyl (1R,5S)-3-(7-chloro-8-fluoro-2-((2-(propan-2-ylidene) tetrahydro-1H-pyrrolizin-7a(5H)-yl)methoxy)pyrido[4,3-d]pyrimidin-4-yl)-3,8-diazabicyclo[3.2.1]octane-8-carboxylate (250 mg, yield: 62%). ES-API: [M+H]⁺=573.3.

Step 6: tert-butyl (1R,5S)-3-(7-chloro-8-fluoro-2-((2-(propan-2-ylidene) tetrahydro-1H-pyrrolizin-7a(5H)-yl)methoxy)pyrido[4,3-d]pyrimidin-4-yl)-3,8-diazabicyclo[3.2.1]octane-8-carboxylate (250 mg, 0.4370 mmol) was dissolved in tetrahydrofuran/water (10 mL/2.0 mL) and triisopropyl ((6-(methoxymethoxy)-8-(4,4,5,5-tetramethyl-1,3,2-dioxaboran-2-yl)naphthalen-1-yl)ethynyl)silane (430mg, 0.8704 mmol), potassium phosphate (370 mg, 1.745 mmol) and[n-butyldi (1-adamantyl)phosphino](2-amino-1,1'-biphenyl-2-yl)palladium (ll) methanesulfonate (60.0 mg, 0.08241 mmol) were displaced with nitrogen four times and allowed to react at 70°C for 2 h. The reaction solution was cooled to room temperature, extracted with ethyl acetate (80 mL) and water (60 mL), the organic phase was dried over anhydrous sodium sulfate, filtered, the filtrate was rotary dried under reduced pressure, and the crude was purified by flash silica gel column (methanol/dichloromethane: 0-10%) to give tert-butyl(1R,5S)-3-(8-fluoro-7-(3-(methoxymethoxy))-8-((triisopropylsilyl)ethynyl)naphthalen-1-yl)-2-((2-(propan-2-ylidene)tetrahydro-1H-pyrrolizin-7a(5H)-yl)methoxy)pyrido[4,3-d]pyrimidin-4-yl)-3,8-diazabicyclo[3.2.1]octane-8-carboxylate (260 mg, yield: 65.8%). ES-API: [M+1]⁺=905.5.

Step 7: tert-butyl (1R,5S)-3-(8-fluoro-7-(3-(methoxymethoxy))-8-((triisopropylsilyl)ethynyl)naphthalen-1-yl)-2-((2-(propan-2-ylidene)tetrahydro-1H-pyrrolizin-7a(5H)-yl)methoxy)pyrido[4,3-d]pyrimidin-4-yl)-3,8-diazabicyclo[3.2.1]octane-8-carboxylate (260 mg, 0.2876 mmol) was dissolved in N, N-dimethylformamide (10.0 mL). Cesium fluoride (437.0 mg, 2.876 mmol) was added, and the system reacted at room temperature for 2 h. Upon completion of the reaction, it was extracted with ethyl acetate (80 mL) and water (60 mL), the organic phase was dried over anhydrous sodium sulfate, filtered and the filtrate was rotary dried under reduced pressure to give tert-butyl (1R,5S)-3-(7-(8-ethynyl-3-(methoxymethoxy)naphthalen-1-yl)-8-fluoro-2-((2-(propan-2-ylidene) tetrahydro-1H-pyrrolizin-7a(5H)-yl)methoxy)pyrido[4,3-d]pyrimidin-4-yl)-3,8-diazabicyclo[3.2.1]octane-8-carboxylate (280 mg, crude), which was used directly in the next reaction. ES-API: [M+1]⁺=749.3.

Step 8: tert-butyl (1R,5S)-3-(7-(8-ethynyl-3-(methoxymethoxy)naphthalen-1-yl)-8-fluoro-2-((2-(propan-2-ylidene)tetrahydro-1H-pyrrolizin-7a(5H)-yl)methoxy)pyrido[4,3-d]pyrimidin-4-yl)-3,8-diazabicyclo[3.2.1]octane-8-carboxylate (280 mg, crude) was dissolved in acetonitrile (10.0 mL), 4 M hydrochloric acid/dioxane solution (3.0 mL) was added in an ice bath, and the system reacted for 0.5 h in an ice bath. Upon completion of the reaction, the solvent was rotary dried under reduced pressure, dichloromethane (20 mL) was added and triethylamine (3.0 mL) was added in an ice bath. Stirred for 10 min, extracted with dichloromethane (100 mL) and water (50 mL). The organic phase was dried over anhydrous sodium sulfate, and filtered, and the filtrate was rotary dried under reduced pressure. The crude was prepared by high performance liquid chromatography to give 4-(4-((1R,5S)-3,8-diazabicyclo[3.2.1]octan-3-yl)-8-fluoro-2-((2-(propan-2-ylidene)tetrahydro-1H-pyrrolizin-7a(5H)-yl)methoxy)pyrido[4,3-d]pyrimidin-7-yl)-5-ethynylnaphthalen-2-ol (Z14, formate salt, 48.0 mg, total yield over 2 steps: 27%). ES-API: [M+1]⁺=605.3. ¹H NMR (500 MHz, CD₃OD) δ 9.09 (s, 1H), 8.28 (s, 1H), 7.83 (d, *J* = 7.9 Hz, 1H), 7.51 (d, *J* = 6.3 Hz, 1H), 7.43-7.37 (m, 1H), 7.35 (d, *J* = 2.4 Hz, 1H), 7.16 (d, *J* = 1.9 Hz, 1H), 4.82 (s, 2H), 4.75 (dt, *J* = 18.5, 8.5 Hz, 2H), 4.34 (d, *J* = 14.7 Hz, 1H), 4.24 (s, 2H), 4.00 (dd, *J* = 24.3, 10.3 Hz, 3H), 3.79 (dt, *J* = 12.5, 6.4 Hz, 1H), 3.26 (dd, *J* = 13.0, 6.0 Hz, 1H), 3.07 (d, *J* = 6.8 Hz, 1H), 2.96 (d, *J* = 16.5 Hz, 1H), 2.81 (d, *J* = 16.1 Hz, 1H), 2.45-2.34 (m, 1H), 2.30-2.05 (m, 8H), 1.74 (d, *J* = 8.5 Hz, 6H).

### Example 9: Synthesis of 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)-5-ethynyl-6-fluoronaphthalen-2-ol (Z9)

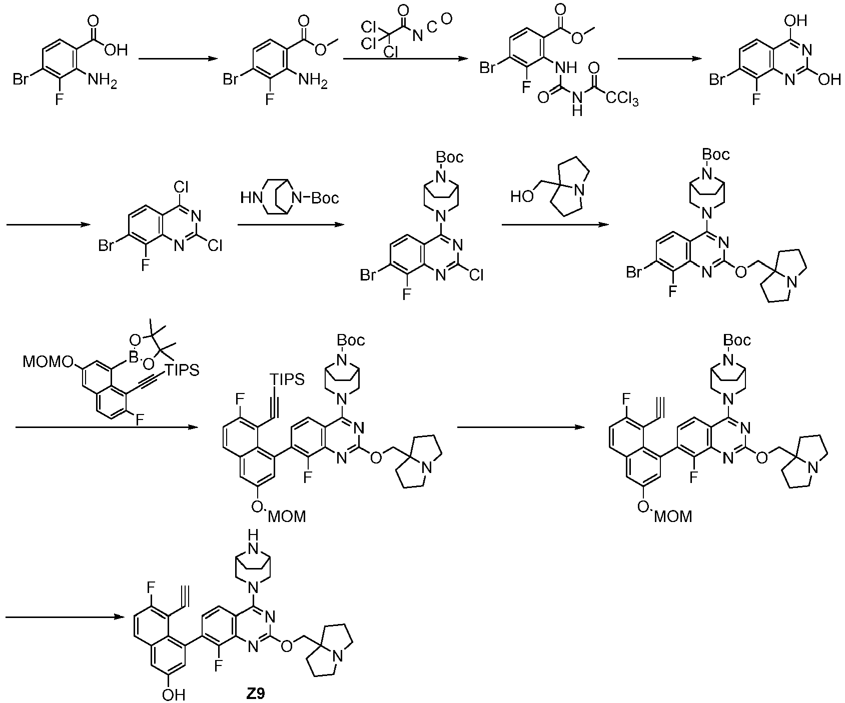

Step 1: 2-amino-4-bromo-3-fluorobenzoic acid (0.4g, 1.72 mmol) was dissolved in methanol (4 mL), thionyl chloride (4 mL) was added at 0°C, and the system reacted at 80°C for 6 h. The reaction solution was concentrated, and the crude was dissolved in ethyl acetate (4 mL). The organic phase was washed with sodium bicarbonate, brine, dried over anhydrous sulfuric acid, and concentrated to give the product methyl 2-amino-4-bromo-3-fluorobenzoate (0.4 g, yield: 100%). ES-API: [M+H]⁺= 248.1.

Step 2: methyl 2-amino-4-bromo-3-fluorobenzoate (0.4 g, 1.62 mmol) was dissolved in tetrahydrofuran (4 mL), 2,2,2-trichloroacetyl isocyanate (0.46 g, 2.42 mmol) was added, and the system reacted at room temperature for 0.5 h. The reaction solution was concentrated and triturated with petroleum ether/ethyl acetate (10/1, 5 mL) to give the product methyl 4-bromo-3-fluoro-2-(3-(2,2,2-trichloroacetyl)ureido) benzoate (0.6 g, yield: 85.5%) ES-API: [M+H]⁺= 434.9. Step 3: methyl 4-bromo-3-fluoro-2-(3-(2,2,2-trichloroacetyl)ureido) benzoate (0.6g, 1.38 mmol) was dissolved in methanol (6 mL), 7 M ammonia in methanol (3 mL) was added, and the system reacted at room temperature for 1 h. The reaction solution was concentrated and triturated with petroleum ether/ethyl acetate (10/1, 5 mL) to give the product 2,4-dihydroxy-7-bromo-8-fluoro-quinazoline (0.4 g, yield: 100%). ES-API: [M+H]⁺= 219.1

Step 4: 2,4-dihydroxy-7-bromo-8-fluoro-quinazoline (0.4 g, 1.55 mmol) was dissolved in phosphorus oxychloride (5 mL) and allowed to react at 110°C for 16 h. After cooling to room temperature, the phosphorus oxychloride was rotary dried to remove under reduced pressure to give crude 2,4-dichloro-7-bromo-8-fluoro-quinazoline (0.6 g, yield 100%).

Step 5: 2,4-dichloro-7-bromo-8-fluoro-quinazoline (0.6 g, crude) was dissolved in dichloromethane, diisopropyl ethyl amine (0.6g, 4.65 mmol) was added, tert-butyl-3,8-diazabicyclo[3.2.1]octane-8-carboxylate (0.33 g, 1.55 mmol) was added at -40°C, and the system reacted for 0.5 h at -40°C. Water (20 mL) was added to the reaction solution, which was extracted with dichloromethane (20 mL), dried over anhydrous sodium sulfate, rotary dried, and purified by column chromatography (ethyl acetate/petroleum ether = 5/1) to give tert-butyl (1R,5S)-3-(7-bromo-2-chloro-8-fluoro-quinazolin-4-yl)-3,8-diazabicyclo[3.2.1]octane-8-carboxylate (220 mg, yield: 30.3%). ES-API: [M+H]⁺=471.1.

Step 6: tert-butyl (1R,5S)-3-(7-bromo-2-chloro-8-fluoro-quinazolin-4-yl)-3,8-diazabicyclo[3.2.1]octane-8-carboxylate (150 mg, 0.318 mmol) was dissolved in 1,4 dioxane (0.5 mL). (Tetrahydro-1H-pyrrolizin-7a(5H)-yl) methanol (897 mg, 6.36 mmol) and diisopropylethyl amine (123 mg, 0.954 mmol) were added, and the system reacted at 120°C under microwave condition for 5 h. Water (20 mL) was added to the reaction solution, which was extracted with ethyl acetate (20 mL), dried over anhydrous sodium sulfate, rotary dried, and purified by column chromatography (ethyl acetate/petroleum ether = 1/1) to give tert-butyl (1R,5S)-3-(7-bromo-8-fluoro-2-(((tetrahydro-1H-pyrrolizin-7a(5H)-yl)methoxy)quinazolin-4-yl)-3,8-diazabicyclo[3.2.1]octane-8-carboxylate (120 mg, yield: 49%). ES-API: [M+H]⁺=576.2.

Step 7: tert-butyl (1R,5S)-3-(7-bromo-8-fluoro-2-(((tetrahydro-1H-pyrrolizin-7a(5H)-yl)methoxy)quinazolin-4-yl)-3,8-diazabicyclo[3.2.1]octane-8-carboxylate (70mg, 0.122 mmol) was dissolved in toluene/tetrahydrofuran/water (0.5 mL/10.5 mL/0.25 mL) and ((2-fluoro-6-(methoxymethoxy)-8-(4,4,5,5-tetramethyl-1,3,2-dioxaborinan-2-yl)naphthalen-1-yl)ethynyl)triisopropylsilane (125 mg, 0.244 mmol), sodium carbonate (39 mg, 0.366 mmol) and tetrakis triphenylphosphine palladium (14 mg, 0.0122 mmol) were reacted under microwave condition at 90°C for 2 h. Ethyl acetate (5 mL) and water (5 mL) were added to the reaction solution, and the liquid separation was performed. The organic phase was dried over anhydrous sodium sulfate, rotary dried, and purified by preparative thin layer chromatography (dichloromethane/methanol = 10/1) to give the product tert-butyl (1R,5S)-3-(8-fluoro-7-(7-fluoro-3-(methoxymethoxy)-8-((triisopropylsilyl)ethynyl)naphthalen-1-yl)-2-((tetrahydro-1H-pyrrolizin-7a(5H)-yl)methoxy)quinazolin-4-yl)-3.8-diazabicyclo[3.2.1]octane-8-carboxylate (30 mg, yield: 24.8%). ES-API: [M+1]⁺=882.5.

Step 8: tert-butyl (1R,5S)-3-(8-fluoro-7-(7-fluoro-3-(methoxymethoxy)-8-((triisopropylsilyl)ethynyl)naphthalen-1-yl)-2-((tetrahydro-1H-pyrrolizin-7a(5H)-yl)methoxy)quinazolin-4-yl)-3,8-diazabicyclo[3.2.1]octane-8-carboxylate (30 mg, 0.034 mmol) was dissolved in N, N-dimethylformamide (0.5 mL). Cesium fluoride (52 mg, 0.34 mmol) and a drop of water were added, and the system reacted at room temperature for 16 h. Ethyl acetate (5 mL) and water (5 mL) were added to the reaction solution, the liquid separation was performed, the organic phase was dried over anhydrous sodium sulfate and rotary dried to give the crude product tert-butyl (1R,5S)-3-(7-(8-ethynyl-7-fluoro-3-(methoxymethoxy)naphthalen-1-yl)-8-fluoro-2-((tetrahydro-1H-pyrrolizin-7a(5H)-yl)methoxy)quinazolin-4-yl)-3,8-diazabicyclo[3.2.1]octane-8-carboxylate (60 mg, yield: 100%). ES-API: [M+1]⁺=725.3.

Step 9: tert-butyl (1R,5S)-3-(7-(8-ethynyl-7-fluoro-3-(methoxymethoxy)naphthalen-1-yl)-8-fluoro-2-((tetrahydro-1H-pyrrolizin-7a(5H)-yl)methoxy)quinazolin-4-yl)-3,8-diazabicyclo[3.2.1]octane-8-carboxylate (60 mg, crude, 0.0414 mmol) was dissolved in acetonitrile (0.5 mL), 4 M hydrochloric acid/dioxane solution (0.5 mL) was added in an ice bath, and the system reacted for 0.5 h in an ice bath. The reaction solution was rotary dried and purified by thin layer chromatography (dichloromethane/methanol = 5/1) to give the product 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)-5-ethynyl-6-fluoronaphthalen-2-ol (Z9, 4.9 mg, yield: 20.4%). ES-API: [M+1]⁺=582.2. ¹H NMR (400 MHz, DMSO-d6) δ 10.19 (s, 1H), 7.93-7.91(m, 1H), 7.77-7.76(d,1H), 7.34-7.30 (m,1H), 7.25 (s,1H), 7.07-7.05(d,1H), 4.38-4.28 (m, 4H), 3.99-3.97 (m, 2H), 3.82 (s,1H), 3.68-3.65 (m, 2H), 2.67-2.63 (m, 1H), 2.57-2.54 (m, 1H), 2.49-2.46 (m, 3H), 2.31-2.29(m, 1H), 2.04-1.95(m, 4H), 1.88-1.79(m, 8H).

### Example 10: Synthesis of 5-ethynyl-4-(8-fluoro-2-((tetrahydro-1H-pyrrolizin-7a(5H)-yl)methoxy)-4-((1R,2S,4R,5S)-7,9-diazatricyclo[3.3.1.0^{2,4}]nonan-7-yl)pyrido[4,3-d]pyrimidin-7-yl)naphthalen-2-ol (Z10)

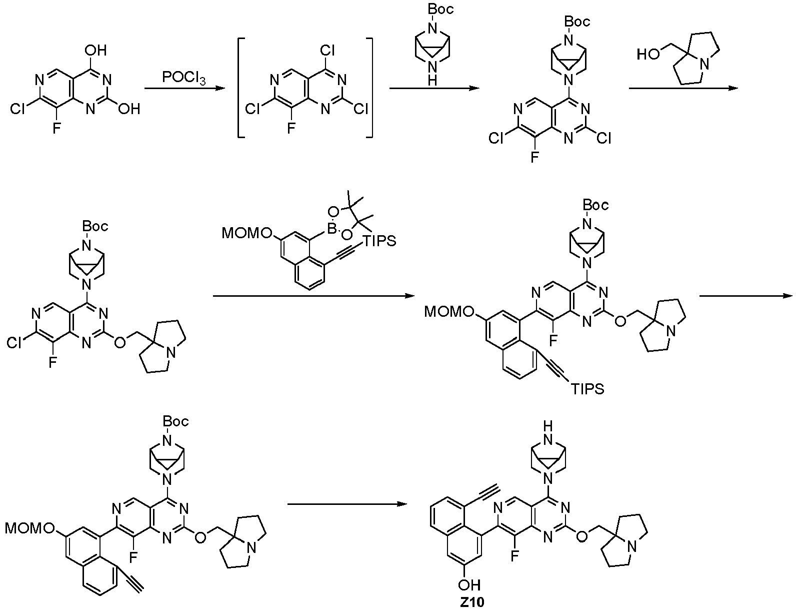

Step 1: 7-chloro-8-fluoro[4,3-d]pyrimidine-2,4-diol (163 mg, 0.76 mmol) was added into a reactor, followed by phosphorus oxychloride (1.50 mL) and diisopropyl ethyl amine (1.5 g), and allowed to react at 100°C for 2 h. After cooling to room temperature, the phosphorus oxychloride was rotary dried to remove under reduced pressure, followed by adding diisopropyl ethyl amine (1.5 g) and tert-butyl (1R,2S,4R,5S)-7,9-diazatricyclo[3.3.1.0^{2,4}]nonane-9-carboxylate (170 mg, 0.76 mmol), and allowed to react at -40°C for 0.5 h. Water (20 mL) was added to the reaction solution, which was extracted with dichloromethane, dried over anhydrous sodium sulfate, rotary dried, and purified by column chromatography (ethyl acetate/petroleum ether = 0-50%) to give tert-butyl (1R,2S,4R,SS)-7-(2,7-dichloro-8-fluoropyrido[4,3-d]pyrimidin-4-yl)-7,9-diazatricyclo[3.3.1.0^{2,4}]nonane-9-carboxylate (165 mg, 49.5% yield). ES-API: [M+H]⁺=440.3. Step 2: the compound tert-butyl (1R,2S,4R,SS)-7-(2,7-dichloro-8-fluoropyrido[4,3-d]pyrimidin-4-yl)-7,9-diazatricyclo[3.3.1.0^{2,4}]nonane-9-carboxylate (165 mg, 0.37 mmol) was dissolved in 1,4 dioxane (3 mL). (Tetrahydro-1H-pyrrolizin-7a(5H)-yl) methanol (104 mg, 0.74 mmol) and diisopropylethyl amine (143 mg, 1.11 mmol) were added, and the system reacted under argon protection at 80°C for 8 h. Water (20 mL) was added to the reaction solution, which was extracted with ethyl acetate, dried over anhydrous sodium sulfate, rotary dried and purified by column chromatography (methanol/dichloromethane = 0-5%) to give tert-butyl (1R,2S,4R,5S)-7-(7-chloro-8-fluoro-2-((tetrahydro-1H-pyrrolizin-7a(5H)-yl)methoxy)pyrido[4,3-d]pyrimidin-4-yl)-7,9-diazatricyclo[3.3.1.0^{2,4}]nonane-9-carboxylate (120 mg, yield: 59.6%). ESI: [M+H]⁺=546.2. Step 3: the compound tert-butyl (1R,2S,4R,5S)-7-(7-chloro-8-fluoro-2-((tetrahydro-1H-pyrrolizin-7a(5H)-yl)methoxy)pyrido[4,3-d]pyrimidin-4-yl)-7,9-diazatricyclo[3.3.1.0^{2,4}]nonane-9-carboxylate (120 mg, 0.22 mmol) was dissolved in tetrahydrofuran/water (4 mL/0.4 mL). Triisopropyl ((6-(methoxymethoxy)-8-(4,4,5,5-tetramethyl-1,3,2-dioxaborolan-2-yl)naphthalen-1-yl)ethynyl)silane (198 mg, 0.40 mmol), potassium phosphate (127 mg, 0.60 mmol) and CataCXium A Pd G₃ (22 mg, 0.04 mmol) were added, and the system reacted at 60°C for 2 h. The reaction solution was extracted with ethyl acetate and water, the organic phase was dried over anhydrous sodium sulfate and rotary dried, and purified by preparative thin layer chromatography (dichloromethane/methanol = 10/1) to give the product tert-butyl (1R,2S,4R,5S)-7-(8-fluoro-7-(3-(methoxymethoxy)-8-((triisopropylsilyl)ethynyl)naphthalen-1-yl)-2-((tetrahydro-1H-pyrrolizin-7a(5H)-yl)methoxy)pyrido[4,3-d]pyrimidin-4-yl)-7,9-diazatricyclo[3.3.1.0^{2,4}]nonane-9-carboxylate (110 mg, yield: 57.0%). ESI: [M+H]⁺=877.5.

Step 4: tert-butyl (1R,2S,4R,5S)-7-(8-fluoro-7-(3-(methoxymethoxy)-8-((triisopropylsilyl)ethynyl)naphthalen-1-yl)-2-((tetrahydro-1H-pyrrolizin-7a(5H)-yl)methoxy)pyrido[4,3-d]pyrimidin-4-yl)-7,9-diazatricyclo[3.3.1.0^{2,4}]nonane-9-carboxylate (110 mg, 0.12 mmol) was dissolved in N, N-dimethylformamide (2 mL), cesium fluoride (91 mg, 0.64 mmol) was added, and the system reacted at room temperature for 15 min and reverse phase preparation (CF₃COOH) was performed to give the product tert-butyl (1R,2S,4R,5S)-7-(7-(8-ethynyl-3-(methoxymethoxy)naphthalen-1-yl)-8-fluoro-2-((tetrahydro-1H-pyrrolizin-7a(5H)-yl)methoxy)pyrido[4,3-d]pyrimidin-4-yl)-7,9-diazatricyclo[3.3.1.0^{2,4}]nonane-9-carboxylate (50mg, yield: 55.5%). ESI: [M+H]⁺=721.3.

Step 5: the compound tert-butyl (1R,2S,4R,5S)-7-(7-(8-ethynyl-3-(methoxymethoxy)naphthalen-1-yl)-8-fluoro-2-((tetrahydro-1H-pyrrolizin-7a(5H)-yl)methoxy)pyrido[4,3-d]pyrimidin-4-yl)-7,9-diazatricyclo[3.3.1.0^{2,4}]nonane-9-carboxylate (50 mg, 0.07 mmol) was dissolved in acetonitrile (3 mL), 4 M hydrochloric acid/dioxane solution (1 mL) was added in an ice bath and the reaction was carried out for 1 h in an ice bath. After alkalinity was adjusted by adding saturated sodium bicarbonate, the reaction solution was rotary dried and reverse phase preparative HPLC (formic acid method) was performed to give 5-ethynyl-4-(8-fluoro-2-((tetrahydro-1H-pyrrolizin-7a(5H)-yl)methoxy)-4-((1R, 2S, 4R, 5S)-7,9-diazatricyclo[3.3.1.0^{2,4}]nonan-7-yl)pyrido[4,3-d]pyrimidin-7-yl)naphthalen-2-ol (Z10, formate salt, 9.7 mg, yield: 24.1%). ESI: [M+H]⁺=577.3. ¹H NMR (400 MHz, CD₃OD) δ 9.13 (s, 1H), 8.46 (s, 1H), 7.83 (d, *J* = 8.2 Hz, 1H), 7.51 (d, *J* = 6.5 Hz, 1H), 7.44-7.37 (m, 1H), 7.34 (d, *J* = 2.4 Hz, 1H), 7.16 (d, *J* = 2.5 Hz, 1H), 4.73 (s, 2H), 4.64 (t, *J* = 9.5 Hz, 2H), 3.79 (d, *J* = 11.9 Hz, 2H), 3.68 (dd, *J* = 11.5, 6.4 Hz, 2H), 3.46 (s, 2H), 3.27-3.20 (m, 1H), 3.05 (s, 1H), 2.32 (dt, *J* = 12.3, 6.1 Hz, 2H), 2.27-2.02 (m, 6H), 1.37 (dd, *J* = 7.2, 3.6 Hz, 2H), 0.44 (dd, *J* = 14.9, 8.0 Hz, 2H).

### Example 11: Synthesis of 4-(4-((1R,SS)-3,8-diazabicyclo[3.2.1]octan-3-yl)-8-tluoro-2-((2-methylenetetrahydro-1H-pyrrolizin-7a(5H)-yl)methoxy)pyrido[4,3-d]pyrimidin-7-yl)-5-ethylnaphthalen-2-ol (Z11)

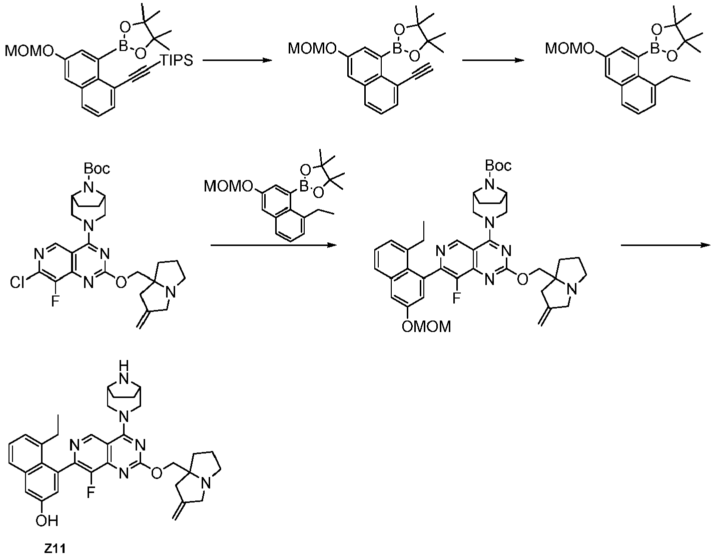

Step 1: cesium fluoride (3.07 g, 20.20 mmol) was added to a solution of triisopropyl ((6-(methoxymethoxy)-8-(4,4,5,5-tetramethyl-1,3,2-dioxaborolan-2-yl)naphthalen-1-yl)ethynyl)silane (1.0g, 2.02 mmol) in N, N-dimethylformamide (6.0 mL). The reaction was carried out under nitrogen protection at room temperature for 2 h. Upon completion of the reaction, the reaction solution was extracted with ethyl acetate (100 mL) and saturated brine (60 mL x5), the organic phase was dried over anhydrous sodium sulfate, filtered and the filtrate was rotary dried under reduced pressure to give 2-(8-ethynyl-3-(methoxymethoxy)naphthalen-1-yl)-4,4,5,5-tetramethyl-1,3,2-dioxaborolane (1.1 g, crude).

Step 2: palladium on carbon (500 mg) with a mass fraction of 10% was added to a solution of 2-(8-ethynyl-3-(methoxymethoxy)naphthalen-1-yl)-4,4,5,5-tetramethyl-1,3,2-dioxaborolane (1.1 g, crude) in methanol (30 mL) and the system was allowed to react at room temperature under hydrogen atmosphere for 0.5 h. Upon completion of the reaction, it was filtered and rotary dried under reduced pressure to give 2-(8-ethyl-3-(methoxymethoxy)naphthalen-1-yl)-4,4,5,5-tetramethyl-1,3,2-dioxaborolane (0.7 g, total yield over two steps: 98%). ES-API: [M+1]⁺=343.3. Step 3: tert-butyl (1R,5S)-3-(7-chloro-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (180 mg, 0.3308 mmol) was dissolved in tetrahydrofuran/water (10 mL/2 mL) and 2-(8-ethyl-3-(methoxymethoxy)naphthalen-1-yl)-4,4,5,5-tetramethyl-1,3,2-dioxoborane (340 mg, 0.9941 mmol), potassium phosphate (350 mg, 1.651 mmol) and[n-butyldi (1-adamantyl)phosphine](2-amino-1,1'-biphenyl-2-yl)palladium (II) methanesulfonate (40 mg, 0.055 mmol) were displaced with nitrogen for four times, and the system reacted at 75°C for 3 h. The reaction solution was cooled to room temperature, extracted with ethyl acetate (80 mL) and water (60 mL), the organic phase was dried over anhydrous sodium sulfate, filtered, and the filtrate was rotary dried under reduced pressure. The crude was purified by flash silica gel column (methanol/dichloromethane = 0-10%) to give the product tert-butyl (1R,5S)-3-(7-(8-ethyl-3-(methoxymethoxy)naphthalen-1-yl)-8-fluoro-2-((2-2-methylenetetrahydro-1H-pyrrolizin-7a(5H)-yl)methoxy)pyrido[4,3-d]pyrimidin-4-yl)-3.8-diazabicyclo[3.2.1]octane-8-carboxylate (Z11, 130 mg, yield: 54%). ES-API: [M/2+1]⁺=725.3.

Step 4: tert-butyl (1R,5S)-3-(7-(8-ethyl-3-(methoxymethoxy)naphthalen-1-yl)-8-fluoro-2-((2-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (130 mg, 0.1795 mmol) was dissolved in acetonitrile (4.0 mL), 4 M hydrochloric acid/dioxane solution (2.0 mL, 8.0 mmol) was added in an ice bath, and the system reacted for 0.5 h in an ice bath. Upon completion of the reaction, the solvent was rotary dried under reduced pressure, dichloromethane (20 mL) was added, and triethylamine (3.0 mL) was added under an ice-water bath condition. Stirred for 10 min, extracted with dichloromethane (100 mL x1) and water (50 mL x1). The organic phase was dried over anhydrous sodium sulfate, filtered and the filtrate was rotary dried under reduced pressure. The crude was purified by preparative HPLC (trifluoroacetic acid method) to give the target compound 4-(4-((1R,5S)-3,8-diazabicyclo[3.2.1]octan-3-yl)-8-fluoro-2-((2-methylenetetrahydro-1H-pyrrolizin-7a(5H)-yl)methoxy)pyrido[4,3-d]pyrimidin-7-yl)-5-ethylnaphthalen-2-ol (Z11, trifluoroacetate, 27.0 mg, 26% yield). ES-API: [M+1]⁺=581.3. ¹H NMR (500 MHz, CD₃OD)δ 9.13 (s, 1H), 7.64 (d, *J* = 8.0 Hz, 1H), 7.41-7.35 (m, 1H), 7.30 (d, *J* = 2.6 Hz, 1H), 7.17 (d, *J* = 6.9 Hz, 1H), 7.00 (d, *J* = 2.6 Hz, 1H), 5.31 (d, *J* = 7.1 Hz, 2H), 4.93 (d, *J* = 14.0 Hz, 1H), 4.83 (d, *J* = 13.8 Hz, 1H), 4.74 (p, *J* = 12.3 Hz, 2H), 4.38 (d, *J* = 14.5 Hz, 1H), 4.27 (d, *J* = 17.3 Hz, 2H), 4.01 (d, *J* = 13.8 Hz, 1H), 3.93 (dd, *J* = 14.1, 5.2 Hz, 2H), 3.85-3.75 (m, 1H), 3.26 (dd, *J* = 11.7, 7.0 Hz, 1H), 3.08 (dd, *J* = 16.0, 6.5 Hz, 1H), 2.82 (d, *J* = 16.3 Hz, 1H), 2.47-2.02 (m, 11H), 0.89 (t, *J* = 7.4 Hz, 3H).

### Example 12: Synthesis of 4-(4-((1R,5S)-3,8-diazabicyclo[3.2.1]octan-3-yl)-8-fluoro-2-(((S)-2-methylenetetrahydro-1H-pyrrolizin-7a(5H)-yl)methoxy)pyrido[4,3-d]pyrimidin-7-yl)-5-ethynyl-6-fluoronaphthalen-2-ol (Z12)

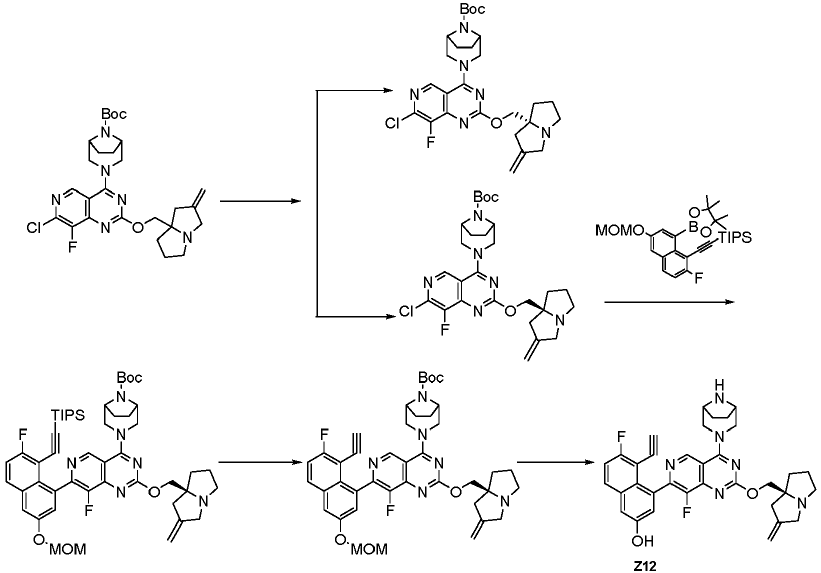

Step 1: chiral preparative resolution on (tert-butyl (1R,5S)-3-(7-chloro-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane -8-carboxylate (160 mg, 0.2941 mmol) was performed (Column: IC, 30*250 mm*10 um; mobile phase: CO2: EtOH(0.2% DEA); flow rate: 3 ml/min; temperature: room temperature) to give two isomeric compounds; one isomeric structure was arbitrarily assigned tert-butyl (1R,5S)-3-(7-chloro-8-fluoro-2-(((S)-2-methylenetetrahydro-1H-pyrrolizin-7a(5H)-yl)methoxy)pyridine)[4,3-d]pyrimidin-4-yl)-3,8-diazabicyclo[3.2.1]octane -8-carboxylate (33 mg, peak 1, retention time 10.61 min, yield: 20%), light brown solid; the other isomer was arbitrarily assigned tert-butyl (1R,5S)-3-(7-chloro-8-fluoro-2-(((R)-2-methylenetetrahydro-1H-pyrrolizin-7a(5H)-yl)methoxy)pyridine)[4,3-d]pyrimidin-4-yl)-3,8-diazabicyclo[3.2.1]octane -8-carboxylate (33 mg, peak 2, retention time 11.57 min, yield: 20%), light brown solid.

Step 2: tert-butyl (1R,5S)-3-(7-chloro-8-fluoro-2-(((S)-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (retention time 10.61 min, 33 mg, 0.06066 mmol) was dissolved in tetrahydrofuran/water (2 mL/0.5 mL), ((2-fluoro-6-(methoxymethoxy)-8-(4,4,5,5-tetramethyl-1,3,2-dioxaborolan-2-yl)naphthalen-1-yl)ethynyl)triisopropylsilane (60 mg, 0.1172 mmol), potassium phosphate (51 mg, 0.2358 mmol) and[n-butyldi (1-adamantyl)phosphine methanesulfonate](2-amino-1,1'-biphenyl-2-yl)palladium (II) (6.0 mg, 0.00824 mmol) were added and displaced with nitrogen for 4 times, and the system reacted for 2 h at 70°C. The reaction solution was cooled to room temperature, extracted with ethyl acetate (80 mL) and water (60 mL), the organic phase was dried over anhydrous sodium sulfate, filtered, the filtrate was rotary dried under reduced pressure, and the crude was purified by flash silica gel column (methanol/dichloromethane = 0-10%) to give the product tert-butyl (1R,SS)-3-(8-fluoro-7-(7-fluoro-3-(methoxymethoxy))-8-((triisopropylsilyl)ethynyl)naphthalen-1-yl)-2-(((S)-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (61 mg, crude). ES-API: [M/2+1]⁺=895.4.

Step 3: tert-butyl (1R,SS)-3-(8-fluoro-7-(7-fluoro-3-(methoxymethoxy))-8-((triisopropylsilyl)ethynyl)naphthalen-1-yl)-2-(((S)-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (61 mg, crude) was dissolved in N, N-dimethylformamide (1.0 mL). Cesium fluoride (92 mg, 0.6066 mmol) was added, and the system reacted at room temperature for 2 h. Upon completion of the reaction, the reaction solution was extracted with ethyl acetate (80 mL) and water (60 mL), the organic phase was dried over anhydrous sodium sulfate, filtered and the filtrate was rotary dried under reduced pressure to give crude tert-butyl (1R,5S)-3-(7-(8-ethynyl-7-fluoro-3-(methoxymethoxy)naphthalen-1-yl)-8-fluoro-2-(((S)-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (71 mg, crude). ES-API: [M+1]⁺=739.3. The crude was used directly in the next reaction.

Step 4: tert-butyl (1R,5S)-3-(7-(8-ethynyl-7-fluoro-3-(methoxymethoxy)naphthalen-1-yl)-8-fluoro-2-(((S)-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (71 mg, crude) was dissolved in acetonitrile (3.0 mL), 4 M hydrochloric acid/dioxane solution (2.0 mL, 8.0 mmol) was added in an ice bath, and the system reacted for 0.5 h in an ice bath. Upon completion of the reaction, the solvent was rotary dried under reduced pressure, and dichloromethane (20 mL) was added. Triethylamine (3.0 mL) was added under an ice-water bath condition condition. Stirred for 10 min, extracted with dichloromethane (100 mL x1) and water (50 mL x1). The organic phase was dried over anhydrous sodium sulfate, and filtered, and the filtrate was rotary dried under reduced pressure. The crude was purified by preparative HPLC (formic acid method) to give the target compound 4-(4-((1R,5S)-3,8-diazabicyclo[3.2.1]octan-3-yl)-8-fluoro-2-(((S)-2-methylenetetrahydro-1H-pyrrolizin-7a(5H)-yl)methoxy)pyrido[4,3-d]pyrimidin-7-yl)-5-ethynyl-6-fluoronaphthalen-2-ol (Z12, formate salt, 6.05 mg, total yield over three steps: 13%). ES-API: [M+1]⁺=595.3.

### Example 13: Synthesis of 4-(4-((1R,5S)-3,8-diazabicyclo[3.2.1]octan-3-yl)-8-fluoro-2-(((R)-2-methylenetetrahydro-1H-pyrrolizin-7a(5H)-yl)methoxy)pyrido[4,3-d]pyrimidin-7-yl)-5-ethynyl-6-fluoronaphthalen-2-ol (Z13)

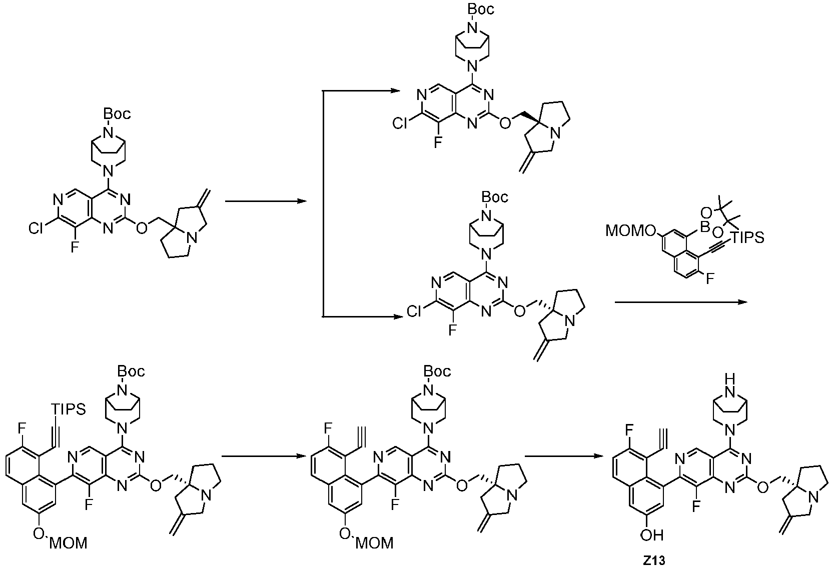

Step 1: chiral preparative resolution on tert-butyl (1R,5S)-3-(7-chloro-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane -8-carboxylate (160 mg, 0.2941 mmol) was performed (Column: IC, 30*250 mm*10 um; mobile phase: CO2: EtOH(0.2% DEA); flow rate: 3 ml/min; temperature: room temperature) to give two isomeric compounds; one isomeric structure was arbitrarily assigned tert-butyl (1R,5S)-3-(7-chloro-8-fluoro-2-(((S)-2-methylenetetrahydro-1H-pyrrolizin-7a(5H)-yl)methoxy)pyridine)[4,3-d]pyrimidin-4-yl)-3,8-diazabicyclo[3.2.1]octane -8-carboxylate (33 mg, peak 1, retention time 10.61 min, yield: 20%), light brown solid; the other isomer was arbitrarily assigned tert-butyl (1R,SS)-3-(7-chloro-8-fluoro-2-(((R)-2-methylenetetrahydro-1H-pyrrolizin-7a(5H)-yl)methoxy)pyridine)[4,3-d]pyrimidin-4-yl)-3,8-diazabicyclo[3.2.1]octane -8-carboxylate (33 mg, peak 2, retention time 11.57 min, yield: 20%), light brown solid.

Step 2: tert-butyl (1R,SS)-3-(7-chloro-8-fluoro-2-(((R)-2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (retention time 11.57 min, 33mg, 0.06066 mmol) was dissolved in tetrahydrofuran/water (2 mL/0.5 mL) and ((2-fluoro-6-(methoxymethoxy)-8-(4,4,5,5-tetramethyl-1,3,2-dioxaborolan-2-yl)naphthalen-1-yl)ethynyl)triisopropylsilane (60 mg, 0.1172 mmol), potassium phosphate (51 mg, 0.2358 mmol) and[n-butyldi (1-adamantyl)phosphine methanesulfonate](2-amino-1,1'-biphenyl-2-yl)palladium (II) (6.0 mg, 0.00824 mmol) were displaced with nitrogen 4 times, and the system reacted for 2 h at 70°C. The reaction solution was cooled to room temperature, extracted with ethyl acetate (80 mL) and water (60 mL), the organic phase was dried over anhydrous sodium sulfate, filtered, the filtrate was rotary dried under reduced pressure, and the crude was purified by flash silica gel column (methanol/dichloromethane: 0-10%) to give the product tert-butyl (1R,5S)-3-(8-fluoro-7-(7-fluoro-3-(methoxymethoxy))-8-((triisopropylsilyl)ethynyl)naphthalen-1-yl)-2-(((R)-2-methylenetetrahydro-1H-pyrrolizin-7a(5H)-yl)methoxy)pyridyl[4,3-d]pyrimidin-4-yl)-3,8-diazabicyclo[3.2.1]octane-8-carboxylate (62 mg, crude). ES-API: [M/2+1]⁺=895.4.

Step 3: tert-butyl (1R,SS)-3-(8-fluoro-7-(7-fluoro-3-(methoxymethoxy))-8-((triisopropylsilyl)ethynyl)naphthalen-1-yl)-2-(((R)-2-methylenetetrahydro-1H-pyrrolizin-7a(5H)-yl)methoxy)pyridyl[4,3-d]pyrimidin-4-yl)-3,8-diazabicyclo[3.2.1]octane-8-carboxylate (62 mg, crude) was dissolved in N, N-dimethylformamide (1.0 mL). Cesium fluoride (92 mg, 0.6066 mmol) was added, and the system reacted at room temperature for 2 h. Upon completion of the reaction, it was extracted with ethyl acetate (80 mL) and water (60 mL), the organic phase was dried over anhydrous sodium sulfate, filtered, and the filtrate was rotary dried under reduced pressure to give crude tert-butyl (1R,5S)-3-(7-(8-ethynyl-7-fluoro-3-(methoxymethoxy)naphthalen-1-yl)-8-fluoro-2-(((R)-2-methylenetetrahydro-1H-pyrrolizin)-7a(5H)-yl)methoxy)pyrido[4,3-d]pyrimidin-4-yl)-3,8-diazabicyclo[3.2.1]octane-8-carboxylate (72 mg, crude). ES-API: [M+1]⁺=739.3. The crude was used directly in the next reaction.

Step 4: tert-butyl (1R,5S)-3-(7-(8-ethynyl-7-fluoro-3-(methoxymethoxy)naphthalen-1-yl)-8-fluoro-2-(((R)-2-methylenetetrahydro-1H-pyrrolizin)-7a(SH)-yl)methoxy)pyrido[4,3-d]pyrimidin-4-yl)-3,8-diazabicyclo[3.2.1]octane-8-carboxylate (71 mg, crude) was dissolved in acetonitrile (3.0mL), 4 M hydrochloric acid/dioxane solution (2.0 mL, 8.0 mmol) was added in an ice bath, and the system reacted for 0.5 h in an ice bath. Upon completion of the reaction, the solvent was rotary dried under reduced pressure, dichloromethane (20 mL) was added, and triethylamine (3.0 mL) was added in an ice bath. Stirred for 10 min, extracted with dichloromethane (100 mL x1) and water (50 mL x1). The organic phase was dried over anhydrous sodium sulfate, and filtered, and the filtrate was rotary dried under reduced pressure. The crude was purified by preparative HPLC (formic acid method) to give the target compound 4-(4-((1R,5S)-3,8-diazabicyclo[3.2.1]octan-3-yl)-8-fluoro-2-(((R)-2-methylenetetrahydro-1H-pyrrolizin-7a(5H)-yl)methoxy)pyrido[4,3-d]pyrimidin-7-yl)-5-ethynyl-6-fluoronaphthalen-2-ol (Z13, formate salt, 35 mg, total yield over three steps: 12%). ES-API: [M+1]⁺=595.3.

### Example 14: Synthesis of 4-(4-((1R,5S)-3,8-diazabicyclo[3.2.1]oct-6-en-3-yl)-2-((2,2-difluorotetrahydro-1H-pyrrolizin-7a(5H)-yl)methoxy)-8-fluoropyrido[4,3-d]pyrimidin-7-yl)-5-ethynylnaphthalen-2-ol (Z15)

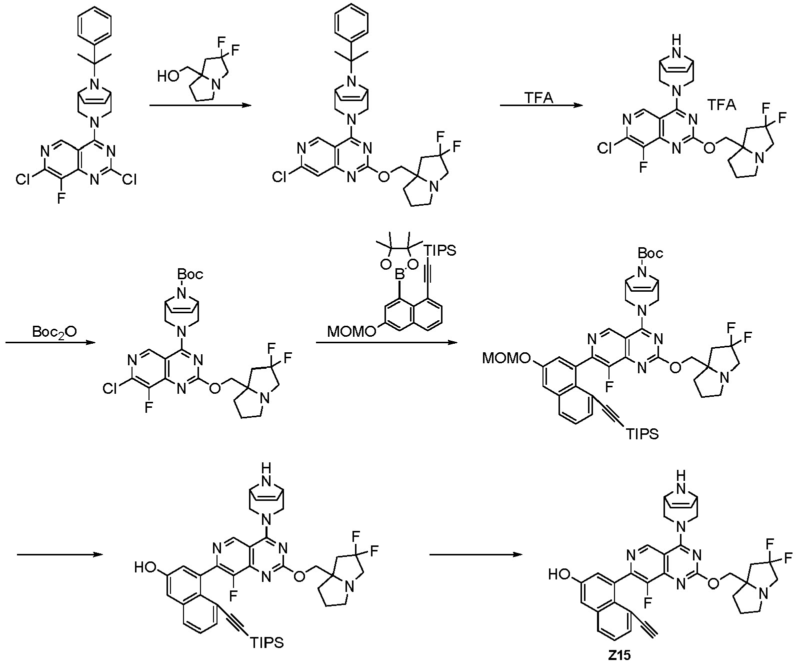

Step 1: the compound 2,7-dichloro-8-fluoro-4-((1R,5S)-8-(2-phenylpropan-2-yl)-3,8-diazabicyclo[3.2.1]oct-6-en-3-yl)pyrido[4,3-d]pyrimidine (150 mg, 0.34 mmol) was dissolved in dry tetrahydrofuran (3 mL), (2,2-difluorotetrahydro-1H-pyrrolizin-7a(5H)-yl) methanol (120 mg, 0.68 mmol) and sodium hydride (41 mg, 1.02 mmol, 60%) were added and the reaction was carried out under argon protection in an ice bath for 2 h. The reaction solution was quenched with saturated ammonium chloride (20 mL), extracted with ethyl acetate (40 ml X2), dried over anhydrous sodium sulfate, rotary dried, and purified by column chromatography (0-10% methanol/dichloromethane) to give 7-chloro-2-((2,2-difluorotetrahydro-1H-pyrrolizin-7a(5H)-yl)methoxy)-4-((1R,5S)-8-(2-phenylpropan-2-yl)-3,8-diazabicyclo[3.2.1]oct-6-en-3-yl)pyrido[4,3-d]pyrimidine (115 mg, yield: 59.9%). ESI: [M+H]⁺=567.3.

Step 2: 7-chloro-2-((2,2-difluorotetrahydro-1H-pyrrolizin-7a(5H)-yl)methoxy)-4-((1R,5S)-8-(2-phenylpropan-2-yl)-3,8-diazabicyclo[3.2.1]oct-6-en-3-yl)pyrido[4,3-d]pyrimidine (115 mg, 0.20 mmol) was dissolved in trifluoroacetic acid (6 mL) and allowed to react at 40°C for 2 h. Upon completion of the reaction, it was concentrated to give 4-((1R,5S)-3.8-diazabicyclo[3.2.1]oct-6-en-3-yl)-7-chloro-2-((2,2-difluorotetrahydro-1H-pyrrolizin-7a(SH)-yl)methoxy)-8-fluoropyrido[4,3-d]pyrimidine trifluoroacetate (112 mg, yield: 100%). ESI: [M+H]⁺=467.1.

Step 3: 4-((1R,5S)-3,8-diazabicyclo[3.2.1]oct-6-en-3-yl)-7-chloro-2-((2,2-difluorotetrahydro-1H-pyrrolizin-7a(5H)-yl)methoxy)-8-fluoropyrido[4,3-d]pyrimidine trifluoroacetate (112 mg, 0.20 mmol) was dissolved in tetrahydrofuran (10 mL), triethylamine (67 mg, 0.57 mmol) and di-tert-butyl dicarbonate (50 mg, 0.23 mmol) were added, allowed to react at room temperature for 1 h, rotary dried, and purified by column chromatography (0-10% methanol/dichloromethane) to give compound (1R,5S)-tert-butyl-3-(7-chloro-2-((2,2-difluorotetrahydro-1H-pyrrolizin-7a(5H)-yl)methoxy)-8-fluoropyrido[4,3-d]pyrimidin-4-yl)-3,8-diazabicyclo[3.2.1]oct-6-ene-8-carboxylate (110 mg, yield: 97.3%). ESI: [M+H]⁺=567.3.

Step 4: the compound (1R,5S)-tert-butyl-3-(7-chloro-2-((2,2-difluorotetrahydro-1H-pyrrolizin-7a(5H)-yl)methoxy)-8-fluoropyrido[4,3-d]pyrimidin-4-yl)-3,8-diazabicyclo[3.2.1]oct-6-ene-8-carboxylate (110 mg, 0.19 mmol) was dissolved in tetrahydrofuran/water (4 mL/0.4 mL). Triisopropyl ((6-(methoxymethoxy)-8-(4,4,5,5-tetramethyl-1,3,2-dioxaborolan-2-yl)naphthalen-1-yl)ethynyl)silane (198 mg, 0.40 mmol), potassium phosphate (127 mg, 0.60 mmol) and CataCXium A Pd G₃ (22 mg, 0.04 mmol) were added, and the system reacted at 60°C for 2 h. The reaction solution was concentrated and evaporated to dryness, and the crude was purified by preparative thin layer chromatography (dichloromethane/methanol = 10/1) to give (1R,5S)-tert-butyl 3-(2-((2,2-difluorotetrahydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]oct-6-ene-8-carboxylate (130 mg, yield: 74.7%). ESI: [M+H]⁺=899.2.

Step 5: (1R,5S)-tert-butyl 3-(2-((2,2-difluorotetrahydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]oct-6-ene-8-carboxylate (75 mg, 0.083 mmol) was dissolved in acetonitrile (3 mL), sodium bicarbonateand 4 M hydrochloric acid/dioxane solution (1 mL) was added in an ice bath. The reaction was carried out in an ice bath for 1 h, alkalinity was adjusted by adding saturated sodium hydrogen carbonate (pH = 8.0) and extracted with ethyl acetate (50 ml X2). The organic phase was rotary dried anhydrous sodium sulfate and dried to give 4-(4-((1R,5S)-3,8-diazabicyclo[3.2.1]oct-6-en-3-yl)-2-((2,2-difluorotetrahydro-1H-pyrrolizin-7a(5H)-yl)methoxy)-8-fluoropyrido[4,3-d]pyrimidin-7-yl)-5-((triisopropylsilyl)ethynyl)naphthalen-2-ol (80 mg, crude). The next reaction was carried on without purification. ESI: [M+H]⁺=755.3.

Step 6: 4-(4-((1R,5S)-3,8-diazabicyclo[3.2.1]oct-6-en-3-yl)-2-((2,2-difluorotetrahydro-1H-pyrrolizin-7a(5H)-yl)methoxy)-8-fluoropyrido[4,3-d]pyrimidin-7-yl)-5-((triisopropylsilyl)ethynyl)naphthalen-2-ol (80mg, crude) was dissolved in N, N-dimethylformamide (2 mL). Cesium fluoride (91 mg, 0.64 mmol) was added, and the system reacted at room temperature for 15 min. Filtered and high performance liquid phase preparation (0.05% formic acid/acetonitrile 0-30%, 10 min, 30%, 5 min) was performed to give 4-(4-((1R,5S)-3,8-diazabicyclo[3.2.1]oct-6-en-3-yl)-2-((2,2-difluorotetrahydro-1H-pyrrolizin-7a(5H)-yl)methoxy)-8-fluoropyrido[4,3-d]pyrimidin-7-yl)-5-ethynylnaphthalen-2-ol (Z15, formate salt, 18.5 mg, yield: 32.3%). ESI: [M+H]⁺=599.3. ¹H NMR (400 MHz, CD₃OD) δ 9.02 (s, 1H), 7.81 (d, *J* = 7.5 Hz, 1H), 7.50 (dd, *J* = 7.1, 1.1 Hz, 1H), 7.42-7.37 (m, 1H), 7.32 (d, *J* = 2.5 Hz, 1H), 7.14 (d, *J* = 2.5 Hz, 1H), 6.40-6.26 (m, 2H), 4.63 (d, *J* = 16.2 Hz, 2H), 4.28-4.22(m, 4H), 3.96-3.86 (m, 2H), 3.50-3.40 (m, 1H), 3.23-3.11 (m, 2H), 3.03 (d, *J* = 4.1 Hz, 1H), 2.91-2.79 (m, 1H), 2.58 (dd, *J* = 27.8, 14.0 Hz, 1H), 2.33 (dd, *J* = 27.4, 13.2 Hz, 1H), 2.15 (s, 1H), 2.07-1.81 (m, 3H).

### Example 15: Synthesis of 4-(4-((1R,5S)-3,8-diazabicyclo[3.2.1]octan-3-yl)-2-((7-ethyl-2,3-dihydro-1H-pyrrolizin-7a(5H)-yl)methoxy)-8-fluoropyrido[4,3-d]pyrimidin-7-yl)-5-ethynylnaphthalen-2-ol (Z16)

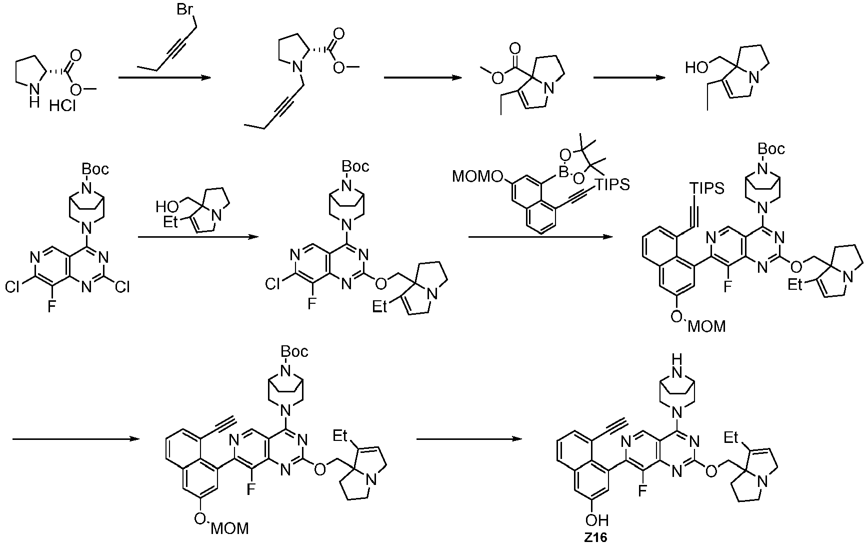

Step 1: D-proline methyl ester hydrochloride (2.76 g, 16.7 mmol) was dissolved in dichloromethane (25 mL) and triethylamine (5.07 g, 50 mmol) was added dropwise at 0°C followed by 1-bromo-2-pentyne (2.45 g, 16.7 mmol) and stirred at room temperature for 24 h. Water was added to the reaction solution, which was extracted with (100 mL X3), the organic phase was dried over anhydrous sodium sulfate and rotary dried, and purified by preparative thin layer chromatography (dichloromethane/methanol = 1/10) to give pent-2-yn-1-yl-D-proline methyl ester (710 mg, yield: 22%). ESI: [M+H]⁺=196.1.

Step 2: pent-2-yn-1-yl-D-proline methyl ester (710 mg, 3.64 mmol) was dissolved in anhydrous toluene (5 mL), the reaction solution was cooled to 0°C, KHMDS solution (1.82 mL, 1 M in THF) was slowly added dropwise and stirred at room temperature for 12 h. Water was added to the reaction solution, which was extracted with dichloromethane (30 mL X3), and the organic phase was dried over anhydrous sodium sulfate and rotary dried, and purified by preparative thin layer chromatography (dichloromethane/methanol = 1/10) to give methyl 7-ethyl-2,3-dihydro-1H-pyrrolizine-7a(5H)-carboxylate (650 mg, yield: 91.4%). ESI: [M+H]⁺=196.1.

Step 3: the compound 7-ethyl-2,3-dihydro-1H-pyrrolizine-7a(5H)-carboxylic acid methyl ester (650 mg, 3.33 mmol) was dissolved in tetrahydrofuran (4 mL), cooled to 0°C, and lithium aluminum tetrahydride in tetrahydrofuran (3.1 mL, 1 M) was added to the above solution under nitrogen atmosphere. The mixture was warmed to 60°C and reacted for 1 h. The reaction solution was cooled to 0°C, and quenched by the addition of water (0.16 mL), 15% sodium hydroxide aqueous solution (0.16 mL), and water (0.48 mL). Tetrahydrofuran (20 mL) was added, filtered and the filtrate was concentrated to give (7-ethyl-2,3-dihydro-1H-pyrrolizin-7a(5H)-yl) methanol (400 mg, 2.4 mmol, yield: 72%) as a colorless transparent liquid. ES-API: [M+H]⁺= 168.1.

Step 4: tert-butyl (1R,SS)-3-(2,7-dichloro-8-fluoropyrido[4,3-d]pyrimidin-4-yl)-3,8-diazabicyclo[3.2.1]octane-8-carboxylate (650 mg, 1.5 mmol) was dissolved in dioxane (10 mL). (7-ethyl-2,3-dihydro-1H-pyrrolizin-7a(5H)-yl) methanol (400 mg, 2.4 mmol) and diisopropylethyl amine (970 mg, 7.5 mmol) were added, and the system reacted under argon protection at 120°C for 12 h. The reaction solution was cooled to room temperature, saturated brine (50 mL) was added, and extracted with ethyl acetate (100 mL X2). The organic phase was combined, dried over anhydrous sodium sulfate, and rotary dried under reduced pressure. The crude was purified by flash silica gel column (methanol/dichloromethane: 0-20%) to give tert-butyl (1R,5S)-3-(7-chloro-2-((7-ethyl-2,3-dihydro-1H-pyrrolizin-7a(5H)-yl)methoxy)-8-fluoropyrido[4,3-d]pyrimidin-4-yl)-3,8-diazabicyclo[3.2.1]octane-8-carboxylate (390 mg, yield: 46.5%) as a pale yellow liquid. ES-API: [M+H]⁺=559.2.

Step 5: the mixture of tert-butyl (1R,5S)-3-(7-chloro-2-((7-ethyl-2,3-dihydro-1H-pyrrolizin-7a(5H)-yl)methoxy)-8-fluoropyrido[4,3-d]pyrimidin-4-yl)-3,8-diazabicyclo[3.2.1]octane-8-carboxylate (145 mg, 0.26 mmol), triisopropyl ((6-(methoxymethoxy)-8-(4,4,5,5-tetramethyl-1,3,2-dioxaborolan-2-yl)naphthalen-1-yl)ethynyl)silane (258 mg, 0.52 mmol),[n-butylbis(1-adamantyl)phosphine methanesulfonate](2-amino-1,1'-biphenyl-2-yl)palladium (II) (28 mg, 0.039 mmol), potassium phosphate (165 mg, 0.78 mmol), tetrahydrofuran (5 mL) and water (1 mL) was stirred at 70°C for 6 h under nitrogen atmosphere. Water (10 mL) was added to the reaction solution, which was extracted with ethyl acetate (10 mL X3). The organic phase was washed with saturated brine (20 mL), dried over anhydrous sodium sulfate, concentrated and the crude was purified by flash silica gel column (methanol/dichloromethane: 0-8%) to give tert-butyl (1R,5S)-3-(2-((7-ethyl-2,3-dihydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (230 mg, 0.26 mmol, yield: 99.6%) as a yellow oily liquid. ES-API: [M+H]⁺= 891.5.

Step 6: a mixed solution of tert-butyl (1R,5S)-3-(2-((7-ethyl-2,3-dihydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (230 mg, 0.26 mmol), cesium fluoride (395 mg, 2.6 mmol) and N, N-dimethylformamide (5 mL) was stirred at room temperature for 2 h. Water (20 mL) was added to the reaction solution, which was extracted with ethyl acetate (20 mL X2). The organic phase was washed with saturated brine (20 mL X3), dried over anhydrous sodium sulfate and concentrated to give tert-butyl (1R,5S)-3-(2-((7-ethyl-2,3-dihydro-1H-pyrrolizin-7a(5H)-yl)methoxy)-7-(8-ethynyl-3-(methoxymethoxy)naphthalen-1-yl)-8-fluoropyrido[4,3-d]pyrimidin-4-yl)-3,8-diazabicyclo[3.2.1]octane-8-carboxylate (184 mg, 0.25 mmol, yield: 97%) as a yellow oily liquid. ES-API: [M+H]⁺= 735.3.

Step 7: tert-butyl (1R,5S)-3-(2-((7-ethyl-2,3-dihydro-1H-pyrrolizin-7a(5H)-yl)methoxy)-7-(8-ethynyl-3-(methoxymethoxy)naphthalen-1-yl)-8-fluoropyrido[4,3-d]pyrimidin-4-yl)-3,8-diazabicyclo[3.2.1]octane-8-carboxylate (184 mg, 0.25 mmol) was dissolved in acetonitrile (2 mL) and cooled to 0°C. 4 M hydrogen chloride/dioxane solution (1 mL) was added to the above solution and stirred at 0°C for 2 h. The reaction solution was concentrated, dissolved in dichloromethane (3 mL), alkalinity was adjusted with triethylamine (pH = 8), concentrated, and the crude was prepared by high performance liquid chromatography (column type: Waters XBridge C18, 190*250 mm, 5 um; mobile phase system: A: 0.1% formic acid solution; B: preparative grade acetonitrile; flow rate: 15 ml/min; B%=20%-100%; column temperature: room temperature) to give 4-(4-((1R,5S)-3,8-diazabicyclo[3.2.1]octan-3-yl)-2-((7-ethyl-2,3-dihydro-1H-pyrrolizin-7a(5H)-yl)methoxy)-8-fluoropyrido[4,3-d]pyrimidin-7-yl)-5-ethynylnaphthalen-2-ol (Z16, formate salt, 3.88 mg, yield: 2.3%). ES-API: [M+H]⁺= 591.3.

### Example 16: Synthesis of 4-(4-((1R,5S)-3,8-diazabicyclo[3.2.1]octan-3-yl)-8-fluoro-2-((2-fluoro-6-methylenetetrahydro-1H-pyrrolizine7a(5H)-yl)methoxy)pyrido[4,3-d]pyrimidin-7-yl)-5-ethynylnaphthalen-2-ol (Z17)

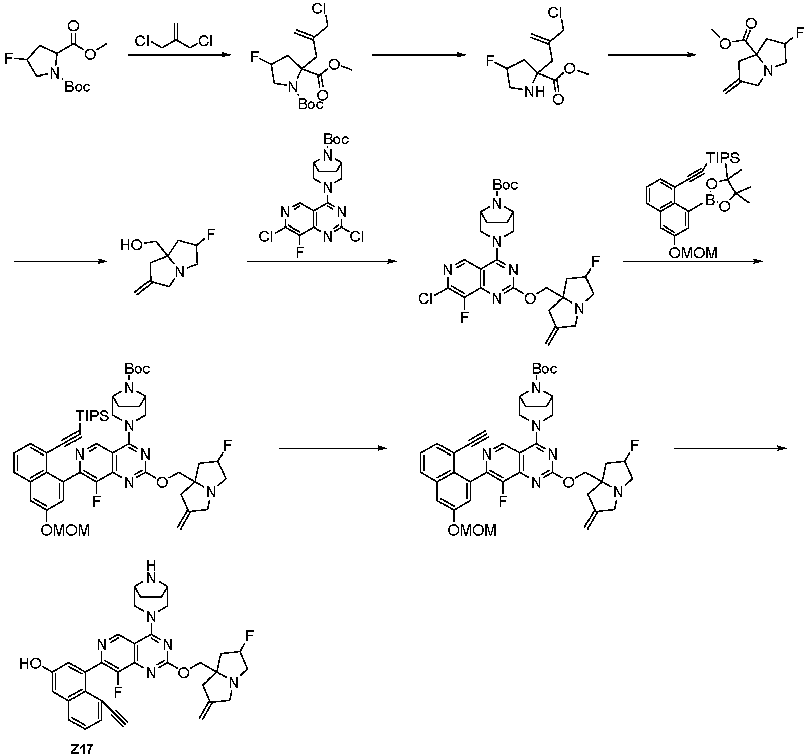

Step 1: methyl N-Boc-trans-4-fluoro-L-prolinate (1 g, 4.044 mmol) was dissolved in anhydrous tetrahydrofuran (15 mL) under nitrogen protection and the mixture was placed in a dry ice-acetone bath. A solution of lithium bis-trimethylsilylamide (6.1 mL, 6.066 mmol, 1 M in THF) was slowly added dropwise to the above solution and stirred at this temperature for 1 h. A solution of 3-chloro-2-chloromethyl propene (2.53 g, 20.222 mmol) in anhydrous tetrahydrofuran (10 mL) was added slowly via syringe to the above solution and the reaction solution was stirred at -78°C for an additional hour. The reaction was determined to be complete by LCMS, the reaction was quenched with saturated ammonium chloride solution (30 mL), extracted with ethyl acetate (20 mL X3), the organic phase was washed with saturated brine (15 mL X3), dried over anhydrous sodium sulfate, filtered and concentrated. The crude was purified by flash silica column to give 1-(tert-butyl)2-methyl 2-(2-(chloromethyl)allyl)-4-fluoropyrrolidine-1,2-dicarboxylate (1354 mg, yield: 100%). ES-API: [M-55]⁺= 280.1.

Step 2: 4 M hydrochloric acid/dioxane solution (10 mL) was added slowly to a solution of 1-(tert-butyl)2-methyl 2-(2-(chloromethyl)allyl)-4-fluoropyrrolidine-1,2-dicarboxylate (1354 mg, 4.044 mmol) in acetonitrile (6 mL) at room temperature and the reaction solution was stirred at room temperature for 0.5 h. The reaction was determined to be complete by LCMS and the reaction solution was concentrated to give methyl 2-(2-(chloromethyl)allyl)-4-fluoropyrrolidine-2-carboxylate (1.2 g, crude). ES-API: [M+H]⁺= 236.2.

Step 3: sodium bicarbonate (1.93 g, 22.979 mmol) and potassium iodide (76 mg, 0.460 mmol) were added to a solution of methyl 2-(2-(chloromethyl)allyl)-4-fluoropyrrolidine-2-carboxylate (1.08 g, 4.596 mmol) in acetonitrile (60 mL) at room temperature and stirred at room temperature for 1.5 h. The reaction was determined to be complete by LCMS, filtered, concentrated and the crude was purified by flash silica gel column to give methyl 2-fluoro-6-methylenetetrahydro-1H-pyrrolizine-7a(5H)-carboxylate (879 mg, yield: 96%). ES-API: [M+H]⁺= 200.1.

Step 4: methyl 2-fluoro-6-methylenetetrahydro-1H-pyrrolizine-7a(SH)-carboxylate (573 mg, 2.879 mmol) was dissolved in 10 mL of anhydrous tetrahydrofuran under nitrogen protection and the mixture was placed under an ice-water bath condition. A solution of lithium tetrahydroaluminate (5.8 mL, 5.76 mmol, 1 M in THF) was slowly added dropwise to the above solution and stirred at this temperature for 0.5 h. The reaction was determined to be complete by LCMS and quenched by the addition of sodium sulfate decahydrate (438 mg), stirred at room temperature for 0.5 h, filtered and the filtrate was concentrated under reduced pressure to give (2-fluoro-6-methyltetrahydro-1H-pyrrolizin-7a(5H)-yl) methanol (370 mg, yield: 75%). ES-API: [M+H]⁺= 172.1.

Step 5: sodium hydride (161 mg, 4.035 mmol) was added portion-wise to a solution of (2-fluoro-6-methyltetrahydro-1H-pyrrolizin-7a(5H)-yl) methanol (230 mg, 1.345 mmol) in tetrahydrofuran (10 mL) under an ice-water bath condition, the mixture was stirred at 0°C for 1 hour, a solution of tert-butyl (1R,SS)-3-(2,7-dichloro-8-fluoropyrido[4,3-d]pyrimidin-4-yl)-3,8-diazabicyclo[3.2.1]octane-8-carboxylate (574 mg, 2.018 mmol) in tetrahydrofuran (10 mL) was slowly added dropwise via syringe to the above solution, and stirring was continued at this temperature for 3 h. The reaction was determined to be complete by LCMS, the reaction was quenched with saturated ammonium chloride solution (30 mL), extracted with ethyl acetate (20 mL X3), the organic phase was washed with saturated brine (15 mL X3), dried over anhydrous sodium sulfate, filtered and concentrated. The crude was purified by flash silica gel column to give tert-butyl (1R,5S)-3-(7-chloro-8-fluoro-2-((2-fluoro-6-methylenetetrahydro-1H-pyrrolizin-7a(5H)-yl)methoxy)pyrido[4,3-d]pyrimidin-4-yl)-3.8-diazabicyclo[3.2.1]octane-8-carboxylate (734 mg, yield: 97%). ES-API: [M+H]⁺= 563.2. Step 6: under nitrogen protection, tert-butyl (1R,5S)-3-(7-chloro-8-fluoro-2-((2-fluoro-6-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (350 mg, 0.623 mmol), triisopropyl ((6-(methoxymethoxy)-8-(4,4,5,5-tetramethyl-1,3,2-dioxaborinan-2-yl)naphthalen-1-yl)ethynyl)silane (613 mg, 1.246 mmol), potassium phosphate (397 mg, 1.869 mmol) and[n-butyldi (1-adamantyl)phosphino](2-amino-1,1'-biphenyl-2-yl)palladium (II) methanesulfonate (68 mg, 0.093 mmol) were dissolved in tetrahydrofuran (10 mL) and water (2 mL) and heated to 65°C with stirring for 3 h. The reaction was determined to be complete by LCMS, concentrated and the crude was purified by flash silica gel column to give tert-butyl (1R,5S)-3-(8-fluoro-2-((2-fluoro-6-methylenetetrahydro-1H-pyrrolizin-7a(5H)-yl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (472 mg, yield: 85%). ES-API: [M+H]⁺= 895.4.

Step 7: cesium fluoride (272 mg, 1.790 mmol) was added to the N, N-dimethylformamide (5 mL) of tert-butyl (1R,5S)-3-(8-fluoro-2-((2-fluoro-6-methylenetetrahydro-1H-pyrrolizin-7a(SH)-yl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (160 mg, 0.179 mmol) and stirred at room temperature for 2 h. The reaction was determined to be complete by LCMS and ethyl acetate (20 mL), water (15 mL X2), and saturated brine (15 mL X3) were added. The mixture was dried over anhydrous sodium sulfate, filtered and concentrated to give tert-butyl (1R,5S)-3-(7-(8-ethynyl-3-(methoxymethoxy)naphthalen-1-yl)-8-fluoro-2-((2-fluoro-6-methylenetetrahydro-1H-pyrrolizin-7a(5H)-yl)methoxy)pyrido[4,3-d]pyrimidin-4-yl)-3.8-diazabicyclo[3.2.1]octane-8-carboxylate (132 mg, yield: 99%). ES-API: [M+H]⁺= 739.3.

Step 8: 4 M hydrochloric acid/dioxane solution (5 mL) was added slowly to a solution of tert-butyl (1R,5S)-3-(7-(8-ethynyl-3-(methoxymethoxy)naphthalen-1-yl)-8-fluoro-2-((2-fluoro-6-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (132 mg, 0.179 mmol) in acetonitrile (3 mL) under an ice-water bath condition. The reaction mixture was stirred at 0°C for 0.5 h. The reaction was determined to be complete by LCMS, the reaction solution was concentrated to dryness and the crude was prepared by high performance liquid chromatography (chromatographic column: Welch Xtimate, 21.2*150 mm, 5 um; mobile phase A: 5 mmol/L NH₄HCO₃ aqueous solution, mobile phase B: ACN; flow rate: 15 ml/min; chromatographic conditions: 15 ml-25-75-13 min; column temperature: room temprature) to give 4-(4-((1R,5S)-3,8-diazabicyclo[3.2.1]octan-3-yl)-8-fluoro-2-((2-fluoro-6-methylenetetrahydro-1H-pyrrolizine 7a(5H)-yl)methoxy)pyrido[4,3-d]pyrimidin-7-yl)-5-ethynylnaphthalen-2-ol (Z17, 15 mg, yield: 14%) as a pale yellow solid. ES-API: [M+H]⁺= 595.3.

### Example 17: Synthesis of 4-(4-((1R,5S)-3,8-diazabicyclo[3.2.1]octan-3-yl)-8-fluoro-2-((7-methylenehexahydroindolin-8a(1H)-yl)methoxy)pyrido[4,3-d]pyrimidin-7-yl)-5-ethynylnaphthalen-2-ol (Z18)

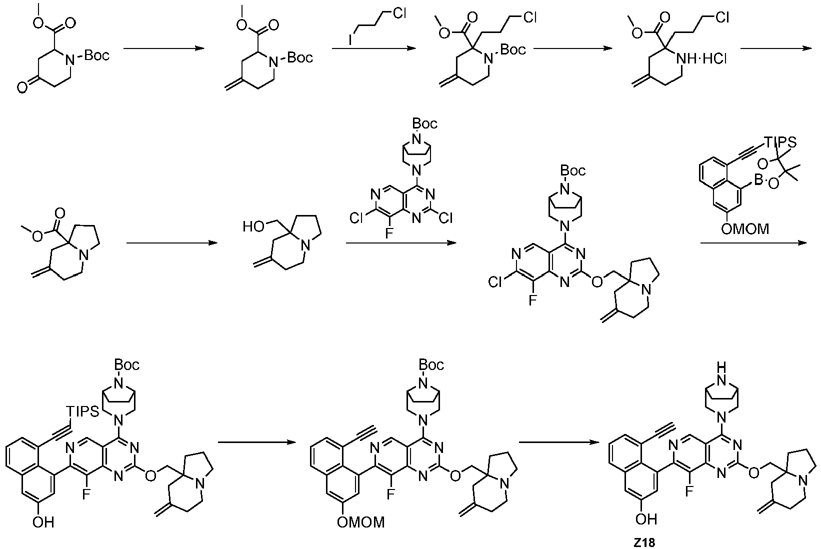

Step 1: methyl triphenylphosphonium bromide (10.41 g, 29.15 mmol) was added to tetrahydrofuran (200 mL) and cooled to 0°C. Solid potassium tert-butoxide (3.27 g, 29.15 mmol) was added to the above mixture at 0°C under a nitrogen atmosphere. After the addition was completed, the reaction was warmed to room temperature and stirred for 1 h. 1-(tert-butyl)2-methyl 4-oxopiperidine-1,2-dicarboxylate (3 g, 11.66 mmol) was dissolved in tetrahydrofuran (15 mL) and slowly added dropwise to the above reaction solution under nitrogen atmosphere. After the addition was complete, the reaction was stirred for an additional 3 h. Saturated ammonium chloride (100 mL) was added to the reaction solution and extracted with ethyl acetate (100 mL X3). The organic phase was washed with saturated brine (200 mL), dried over anhydrous sodium sulfate, and concentrated and the crude was purified by flash silica gel column (ethyl acetate/petroleum ether: 2-15%) to give 1-(tert-butyl)2-methyl 4-methylenepiperidine-1,2-dicarboxylate (2.5g, 9.27 mmol, yield: 84%) as a colorless transparent liquid. ES-API: [M-C₄H₈+H]⁺=156.1.

Step 2: 1-(tert-butyl)2-methyl 4-methylenepiperidine-1,2-dicarboxylate (1.0 g, 3.92 mmol) was dissolved in tetrahydrofuran (30 mL) and cooled to -50°C. A solution of lithium diisopropylamide in tetrahydrofuran was slowly added dropwise to the above solution (2.94 mL, 5.88 mmol, 2 M) under nitrogen protection, keeping the internal temperature at -50°C. After the addition was complete, the reaction was stirred at -50°C for an additional hour. After 1-chloro-3-iodopropane (1.60 g, 7.83 mmol) dissolving in tetrahydrofuran (5 mL), the above reaction solution was slowly added dropwise while keeping the internal temperature at -50°C. After the addition was complete, the reaction was slowly warmed to room temperature and stirred for 17 h. Saturated ammonium chloride (30 mL) was added to the reaction solution, which was extracted with ethyl acetate (30 mL X3). The organic phase was washed with saturated brine (30 mL), dried over anhydrous sodium sulfate, concentrated and the crude was purified by flash silica gel column (ethyl acetate/petroleum ether: 0-9%) to give 1-(tert-butyl)2-methyl 2-(3-chloropropyl)-4-methylenepiperidine-1,2-dicarboxylate (1.0 g, 3.01 mmol, yield: 77%) as a colorless transparent oily liquid. ES-API: [M-Boc+H]⁺=232.1.

Step 3: 1-(tert-butyl)2-methyl 2-(3-chloropropyl)-4-methylenepiperidine-1,2-dicarboxylate (1.0 g, 3.01 mmol) was dissolved in 4 M hydrogen chloride/dioxane solution (15 mL) and the reaction was stirred at room temperature for 2 h. Upon completion of the reaction, the reaction solution was concentrated to give methyl 2-(3-chloropropyl)-4-methylenepiperidine-2-carboxylate hydrochloride (808 mg, 3.01 mmol, yield: 100%) as a white solid. ES-API: [M+H]⁺= 232.1.

Step 4: a mixed solution of methyl 2-(3-chloropropyl)-4-methylenepiperidine-2-carboxylate hydrochloride (808 mg, 3.01 mmol), potassium carbonate (1205 mg, 9.04 mmol), potassium iodide (50 mg, 0.301 mmol) and acetonitrile (20 mL) was heated to 80°C and stirred for 17 s. The reaction solution was filtered, washed with ethyl acetate (20 mL), and concentrated and the crude was purified by flash silica gel column (methanol/dichloromethane: 0-8%) to give methyl 7-methylenehexahydroindolizine-8a(1H)-carboxylate (500 mg, 2.56 mmol, yield: 85%) as a colorless transparent liquid. ES-API: [M+H]⁺= 196.1.

Step 5: methyl 7-methylenehexahydroindolizine-8a(1H)-carboxylate (500 mg, 2.56 mmol) was dissolved in tetrahydrofuran (10 mL) and cooled to 0°C. A solution of lithium aluminum tetrahydride in tetrahydrofuran (5.12 mL, 5.12 mmol, 1 M) was added to the above solution under a nitrogen atmosphere. The reaction was stirred at 0°C for 1 h. Water (0.20 mL) was added to quench the reaction and 15% sodium hydroxide aqueous solution (0.20 mL) and water (0.60 mL) were added. Tetrahydrofuran (20 mL) was added, filtered, and the filtrate was concentrated to give (7-methylenehexahydroindolin-8a(1H)-yl) methanol (400 mg, 2.39 mmol, yield: 93%) as a colorless transparent liquid. ES-API: [M+H]⁺= 168.1.

Step 6: (7-methylenehexahydroindolin-8a(1H)-yl) methanol (150 mg, 0.897 mmol) was dissolved in tetrahydrofuran (5 mL) and cooled to 0°C. Sodium hydride (72 mg, 1.97 mmol, 60%) was added to the above solution at 0°C under nitrogen atmosphere. The reaction was stirred at 0°C for 1 h. Tert-butyl (1R,SS)-3-(2,7-dichloro-8-fluoropyrido[4,3-d]pyrimidin-4-yl)-3,8-diazabicyclo[3.2.1]octane-8-carboxylate (270 mg, 0.628 mmol) was dissolved in tetrahydrofuran (2 mL) and added slowly to the above reaction solution and stirring was continued at 0°C for 1 h. Saturated ammonium chloride (10 mL) was added to the reaction solution and extracted with ethyl acetate (10 mL X3). The organic phase was washed with saturated brine (20 mL), dried over anhydrous sodium sulfate, concentrated and the crude was purified by flash silica gel column (methanol/dichloromethane: 0-7%) to give tert-butyl (1R,5S)-3-(7-chloro-8-fluoro-2-((7-methylenehexahydroindolin-8a(1H)-yl)methoxy)pyrido[4,3-d]pyrimidin-4-yl)-3,8-diazabicyclo[3.2.1]octane-8-carboxylate (250 mg, 0.447 mmol, yield: 71%) as a pale yellow solid. ES-API: [M+H]⁺= 559.3.

Step 7: a mixture of tert-butyl (1R,5S)-3-(7-chloro-8-fluoro-2-((7-methylenehexahydroindolin-8a(1H)-yl)methoxy)pyrido[4,3-d]pyrimidin-4-yl)-3,8-diazabicyclo[3.2.1]octane-8-carboxylate (250 mg, 0.447 mmol), triisopropyl ((6-(methoxymethoxy)-8-(4,4,5,5-tetramethyl-1,3,2-dioxaborolan-2-yl)naphthalen-1-yl)ethynyl)silane (442 mg, 0.897 mmol),[n-butyldi (1-adamantyl)phosphine methanesulfonate](2-amino-1,1'-biphenyl-2-yl)palladium (ll) (49 mg, 0.067 mmol), potassium phosphate (285 mg, 1.34 mmol), tetrahydrofuran (10 mL) and water (2 mL) was stirred at 70°C for 6 h. Water (20 mL) was added to the reaction solution, which was extracted with ethyl acetate (20 mL X3). The organic phase was washed with saturated brine (40 mL), dried over anhydrous sodium sulfate, concentrated and the crude was purified by flash silica gel column (methanol/dichloromethane: 0-10%) to give tert-butyl (1R,5S)-3-(8-fluoro-7-(3-(methoxymethoxy)-8-((triisopropylsilyl)ethynyl)naphthalen-1-yl)-2-((7-methylenehexahydroindolin-8a(1H)-yl)methoxy)pyrido[4,3-d]pyrimidin-4-yl)-3,8-diazabicyclo[3.2.1]octane-8-carboxylate (280 mg, 0.315 mmol, yield: 70.5%) as a pale yellow solid. ES-API: [M+H]⁺= 891.2.

Step 8: a mixed solution of tert-butyl (1R,5S)-3-(8-fluoro-7-(3-(methoxymethoxy)-8-((triisopropylsilyl)ethynyl)naphthalen-1-yl)-2-((7-methylenehexahydroindolin-8a(1H)-yl)methoxy)pyrido[4,3-d]pyrimidin-4-yl)-3,8-diazabicyclo[3.2.1]octane-8-carboxylate (280 mg, 0.314 mmol), cesium fluoride (447 mg, 3.14 mmol) and N, N-dimethylformamide (5 mL) was stirred at room temperature for 2 h. Water (40 mL) was added to the reaction solution, which was extracted with ethyl acetate (20 mL X2). The organic phase was washed with saturated brine (30 mL X3), dried over anhydrous sodium sulfate and concentrated to give tert-butyl (1R,5S)-3-(7-(8-ethynyl-3-(methoxymethoxy)naphthalen-1-yl)-8-fluoro-2-((7-methylenehexahydroindolin-8a(1H)-yl)methoxy)pyrido[4,3-d]pyrimidin-4-yl)-3,8-diazabicyclo[3.2.1]octane-8-carboxylate (230 mg, 0.314 mmol, yield: 100%) as a yellow oily liquid. ES-API: [M+H]⁺= 735.2.

Step 9: tert-butyl (1R,5S)-3-(7-(8-ethynyl-3-(methoxymethoxy)naphthalen-1-yl)-8-fluoro-2-((7-methylenehexahydroindolin-8a(1H)-yl)methoxy)pyrido[4,3-d]pyrimidin-4-yl)-3,8-diazabicyclo[3.2.1]octane-8-carboxylate (50 mg, 0.068 mmol) was dissolved in acetonitrile (2 mL) and cooled to 0°C. 4 M hydrogen chloride/dioxane solution (1 mL) and the reaction was stirred at 0°C for 2 h. The reaction solution was concentrated, dissolved in dichloromethane (3 mL) was added to the above solution, alkalinity was adjusted with triethylamine (pH = 8), concentrated, and the crude was prepared by high performance liquid chromatography (column type: Waters XBridge C18, 190*250 mm, 5 um; mobile phase system: A: 0.1% aqueous ammonia solution; B: preparative grade acetonitrile; flow rate: 15 ml/min; B%=20%-100%; column temperature: room temperature) to give 4-(4-((1R,5S)-3,8-diazabicyclo[3.2.1]octan-3-yl)-8-fluoro-2-((7-methylenehexahydroindolin-8a(1H)-yl)methoxy)pyrido[4,3-d]pyrimidin-7-yl)-5-ethynylnaphthalen-2-ol (Z18, 1.7 mg, 0.0029 mmol, yield: 4%). ES-API: [M+H]⁺= 591.3.

### Example 18: Synthesis of 4-(4-((1R,5S)-3,8-diazabicyclo[3.2.1]octan-3-yl)-8-fluoro-2-((3-methylene-1-azabicyclo[3.2.0]heptan-5-yl)methoxy)pyrido[4,3-d]pyrimidin-7-yl)-5-ethynylnaphthalen-2-ol (Z19)

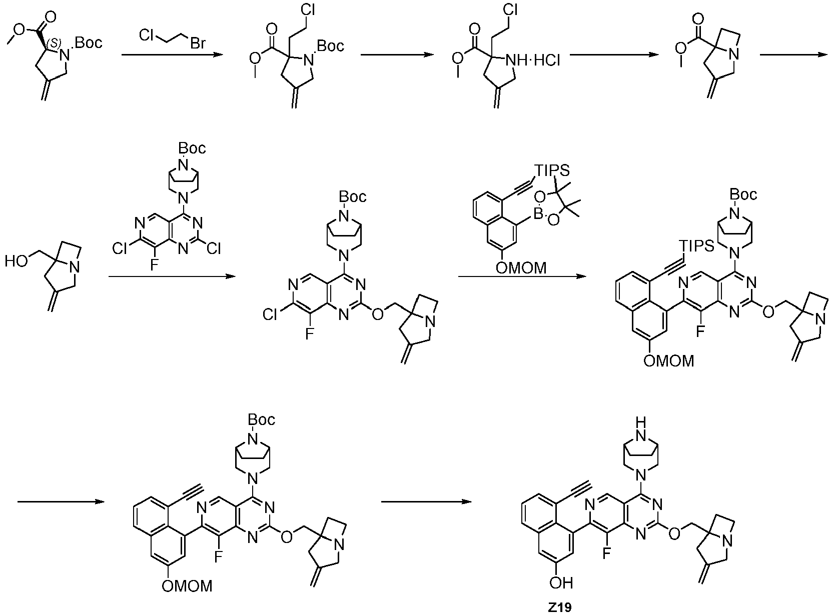

Step 1: 1-(tert-butyl)2-methyl (S)-4-methylenepyrrolidine-1,2-dicarboxylate (5.0 g, 20.72 mmol) was dissolved in tetrahydrofuran (100 mL) and cooled to -50°C. A solution of lithium bis(trimethylsilyl)amide in tetrahydrofuran (31.08 mL, 31.08 mmol, 1 M) was slowly added dropwise to the above solution under nitrogen protection, keeping the internal temperature at - 50°C. After the addition was complete, the reaction was stirred at -50°C for an additional hour. After 1-bromo-2-chloroethane (5.94 g, 41.44 mmol) dissolving in tetrahydrofuran (50 mL), the above reaction solution was slowly added dropwise while keeping the internal temperature at - 50°C. After the addition was complete, the reaction was slowly warmed to room temperature and stirred for 17 h. Saturated ammonium chloride (100 mL) was added to the reaction solution and extracted with ethyl acetate (100 mL X3). The organic phase was washed with saturated brine (300 mL), dried over anhydrous sodium sulfate, and concentrated and the crude was purified by flash silica gel column (ethyl acetate/petroleum ether: 2-18%) to give 1-(tert-butyl)2-methyl 2-(2-chloroethyl)-4-methylenepyrrolidine-1,2-dicarboxylate (1.1 g, 3.62 mmol, yield: 17.5%) as a colorless transparent oily liquid. ES-API: [M-Boc+H]⁺=204.1.

Step 2: 1-(tert-butyl)2-methyl 2-(2-chloroethyl)-4-methylenepyrrolidine-1,2-dicarboxylate (1.1 g, 3.62 mmol) was dissolved in 4 M hydrogen chloride/dioxane solution (20 mL) and stirred at room temperature for 2 h. Upon completion of the reaction, the reaction solution was concentrated to give methyl 2-(2-chloroethyl)-4-methylenepyrrolidine-2-carboxylate hydrochloride (870 mg, 3.62 mmol, yield: 100%) as a white solid. ES-API: [M+H]⁺= 204.1.

Step 3: a mixed solution of methyl 2-(2-chloroethyl)-4-methylenepyrrolidine-2-carboxylate hydrochloride (870 mg, 3.62 mmol), sodium bicarbonate (913 mg, 10.87 mmol), potassium iodide (60 mg, 0.36 mmol) and acetonitrile (10 mL) were heated to 90°C and stirred for 17 h. The reaction solution was filtered and washed with ethyl acetate (20 mL). The filtrate was concentrated, and the crude was purified by flash silica gel column (methanol/dichloromethane: 0-5%) to give methyl 3-methylene-1-azabicyclo[3.2.0]heptane-5-carboxylate (350 mg, 2.09 mmol, yield: 58%) as a colorless transparent liquid. ES-API: [M+H]⁺= 168.1.

Step 4: methyl 3-methylene-1-azabicyclo[3.2.0]heptane-5-carboxylate (350 mg, 2.09 mmol) was dissolved in tetrahydrofuran (4 mL) and cooled to 0°C. Lithium aluminum tetrahydride solution (4.19 mL, 4.19 mmol, 1 M in THF) was added to the above solution under a nitrogen atmosphere. The reaction was stirred at 0°C for 1 h. Water (0.16 mL) was added to quench the reaction and 15% sodium hydroxide aqueous solution (0.16 mL) and water (0.48 mL) were added. Tetrahydrofuran (20 mL) was added, filtered and the filtrate was concentrated to give (3-methylene-1-azabicyclo[3.2.0]heptan-5-yl) methanol (200 mg, 1.44 mmol, yield: 69%), colorless transparent liquid. ES-API: [M+H]⁺= 140.0.

Step 5: (3-methylene-1-azabicyclo[3.2.0]heptan-5-yl) methanol (200 mg, 1.44 mmol) was dissolved in tetrahydrofuran (6 mL) and cooled to 0°C. Sodium hydride (172 mg, 4.31 mmol, 60%) was added to the above solution at 0°C under a nitrogen atmosphere. The reaction was stirred at 0°C for 1 h. Tert-butyl (1R,5S)-3-(2,7-dichloro-8-fluoropyrido[4,3-d]pyrimidin-4-yl)-3,8-diazabicyclo[3.2.1]octane-8-carboxylate (492 mg, 1.15 mmol) was dissolved in tetrahydrofuran (2 mL) and added slowly to the above reaction solution and stirring was continued at 0°C for 1 h. Saturated ammonium chloride (10 mL) was added to the reaction solution and extracted with ethyl acetate (10 mL X3). The organic phase was washed with saturated brine (20 mL), dried over anhydrous sodium sulfate, and concentrated and the crude was purified by flash silica gel column (methanol/dichloromethane: 0-7%) to give tert-butyl (1R,5S)-3-(7-chloro-8-fluoro-2-((3-methylene-1-azabicyclo[3.2.0]heptan-5-yl)methoxy)pyrido[4,3-d]pyrimidin-4-yl)-3,8-diazabicyclo[3.2.1]octane-8-carboxylate (180 mg, 0.339 mmol, yield: 29.5%) as a pale yellow solid. ES-API: [M+H]⁺= 531.1.

Step 6: a mixture of tert-butyl (1R,5S)-3-(7-chloro-8-fluoro-2-((3-methylene-1-azabicyclo[3.2.0]heptan-5-yl)methoxy)pyrido[4,3-d]pyrimidin-4-yl)-3,8-diazabicyclo[3.2.1]octane-8-carboxylate (90 mg, 0.170 mmol), triisopropyl ((6-(methoxymethoxy)-8-(4,4,5,5-tetramethyl-1,3,2-dioxaborolan-2-yl)naphthalen-1-yl)ethynyl)silane (168 mg, 0.339 mmol),[n-butyldi (1-adamantyl)phosphine methanesulfonate](2-amino-1,1'-biphenyl-2-yl)palladium (II) (19 mg, 0.025 mmol), potassium phosphate (108 mg, 0.508 mmol), tetrahydrofuran (5 mL) and water (1 mL) was stirred at 70°C for 6 h. Water (10 mL) was added to the reaction solution, which was extracted with ethyl acetate (10 mL X3). The organic phase was washed with saturated brine (20 mL), dried over anhydrous sodium sulfate, concentrated and the crude was purified by flash silica gel column (methanol/dichloromethane: 0-8%) to give tert-butyl (1R,SS)-3-(8-fluoro-7-(3-(methoxymethoxy)-8-((triisopropylsilyl)ethynyl)naphthalen-1-yl)-2-((3-methylene-1-azabicyclo[3.2.0]heptan-5-yl)methoxy)pyrido[4,3-d]pyrimidin-4-yl)-3,8-diazabicyclo[3.2.1]octane-8-carboxylate (120 mg, 0.139 mmol, yield: 82%) as a pale yellow solid. ES-API: [M+H]⁺= 863.3.

Step 7: a mixed solution of tert-butyl (1R,SS)-3-(8-fluoro-7-(3-(methoxymethoxy)-8-((triisopropylsilyl)ethynyl)naphthalen-1-yl)-2-((3-methylene-1-azabicyclo[3.2.0]heptan-5-yl)methoxy)pyrido[4,3-d]pyrimidin-4-yl)-3,8-diazabicyclo[3.2.1]octane-8-carboxylate (120 mg, 0.139 mmol), cesium fluoride (211 mg, 1.39 mmol) and N, N-dimethylformamide (3 mL) was stirred at room temperature for 2 h. Water (20 mL) was added to the reaction solution, which was extracted with ethyl acetate (20 mL X2). The organic phase was washed with saturated brine (20 mL X3), dried over anhydrous sodium sulfate and concentrated to give tert-butyl (1R,5S)-3-(7-(8-ethynyl-3-(methoxymethoxy)naphthalen-1 -yl)-8-fluoro-2-((3-methylene-1 - azabicyclo[3.2.0]heptan-5-yl)methoxy)pyrido[4,3-d]pyrimidin-4-yl)-3,8-diazabicyclo[3.2.1]octane-8-carboxylate (98 mg, 0.139 mmol, yield: 100%) as a yellow oil liquid. ES-API: [M+H]⁺= 707.3.

Step 8: tert-butyl (1R,5S)-3-(7-(8-ethynyl-3-(methoxymethoxy)naphthalen-1-yl)-8-fluoro-2-((3-methylene-1-azabicyclo[3.2.0]heptan-5-yl)methoxy)pyrido[4,3-d]pyrimidin-4-yl)-3,8-diazabicyclo[3.2.1]octane-8-carboxylate (98 mg, 0.139 mmol) was dissolved in acetonitrile (2 mL) and cooled to 0°C. 4 M hydrogen chloride/dioxane solution (1 mL) was added to the above solution and stirred at 0°C for 2 h. The reaction solution was concentrated, dissolved in dichloromethane (3 mL), alkalinity was adjusted with triethylamine (pH = 8), concentrated, and the crude was prepared by high performance liquid chromatography (column type: Waters XBridge C18, 190*250 mm, 5 um; mobile phase system: A: 0.1% aqueous ammonia solution; B: preparative grade acetonitrile; flow rate: 15 ml/min; B%=20%-100%; column temperature: room temperature) to give 4-(4-((1R,5S)-3,8-diazabicyclo[3.2.1]octan-3-yl)-8-fluoro-2-((3-methylene-1-azabicyclo[3.2.0]heptan-5-yl)methoxy)pyrido[4,3-d]pyrimidin-7-yl)-5-ethynylnaphthalen-2-ol (Z19, 20.63 mg, 0.037 mmol, yield: 26%). ES-API: [M+H]⁺= 563.3.

### Example 19: Synthesis of 4-(4-(1R,5S)-3,8-diazabicyclo[3.2.1]octan-3-yl)-6,8-difluoro-2-(2-methylenetetrahydro-1H-pyrrolizin-7a(5H)-yl)methoxy)quinazolin-7-yl)-5-ethynylnaphthalen-2-ol (Z20)

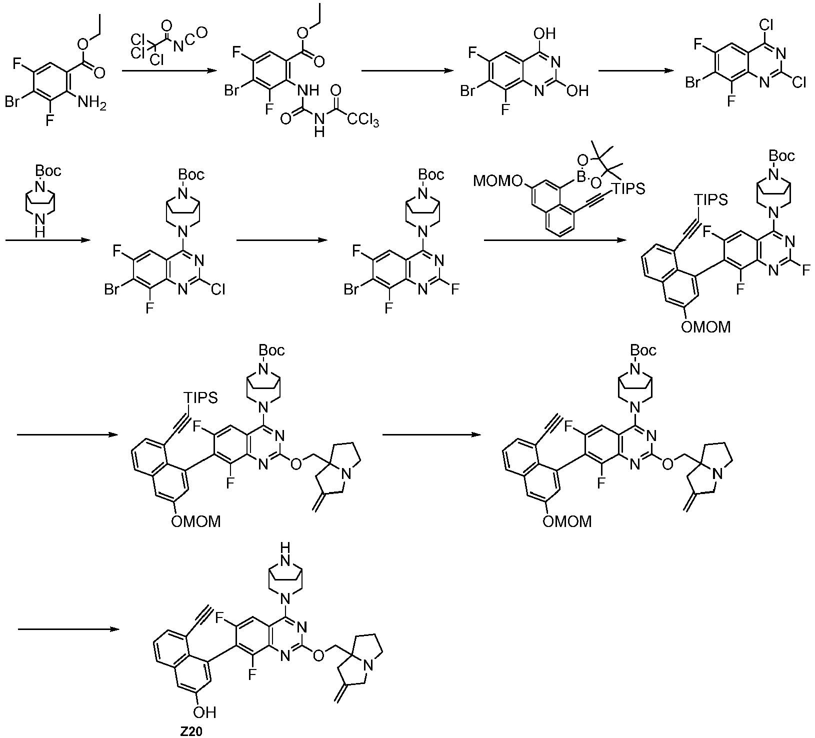

Step 1: 2,2,2-trichloroacetyl isocyanate (2.17 g, 11.60 mmol) was added dropwise to a solution of ethyl 2-amino-4-bromo-3,5-difluorobenzoate (2.70 g, 9.677 mmol) in dry tetrahydrofuran (30.0 mL) under an ice-water bath condition and allowed to react under nitrogen protection at 0°C for 0.5 h. Upon completion of the reaction, the solvent was rotary dried under reduced pressure to give ethyl 4-bromo-3,5-difluoro-2-(3-(2,2,2-trichloroacetyl)ureido) benzoate (2.90 g, crude). ES-API: [M+H]⁺=466.9.

Step 2: ethyl 4-bromo-3,5-difluoro-2-(3-(2,2,2-trichloroacetyl)ureido) benzoate (2.90 g, crude) was added to a solution of methanol (48.0 mL), ammonia (48.0 mL) with a mass fraction of 17% was slowly added dropwise at room temperature. The reaction was carried out for 12 h at room temperature, filtered, washed with methanol (5.0 mL), and rotary dried under reduced pressure to give 7-bromo-6,8-difluoroquinazoline-2,4-diol (2.70 g, crude). ES-API: [M+H]⁺=277.0/279.0. Step 3: phosphorus oxychloride (2.0 g, 13.0 mmol) followed by diisopropylethyl amine (1.68 g, 13.0 mmol) was added to a solution of 7-bromo-6,8-difluoroquinazoline-2,4-diol (1.15 g, crude) in toluene (15 mL) under an ice-water bath condition and allowed to react at 120°C for 2 h. Upon completion of the reaction, the solvent was rotary dried under reduced pressure. Water (20 mL) was added to the reaction solution, which was extracted with dichloromethane (80 mL X2). The organic phase was dried over anhydrous sodium sulfate, filtered, and rotary dried under reduced pressure to give 7-bromo-2,4-dichloro-6,8-difluoroquinazoline (1.5 g, crude).

Step 4: 7-bromo-2,4-dichloro-6,8-difluoroquinazoline (1.5 g, crude) was dissolved in dichloromethane (20.0 mL). Diisopropylethyl amine (1.68 g, 13.0 mmol) was added. After stirring for 5 min, tert-butyl (1R,SS)-3,8-diazabicyclo[3.2.1]octane-8-carboxylate (1.10 g, 5.20 mmol) was added, and the system reacted at room temperature for 5 h. Upon completion of the reaction, saturated brine (50 mL) was added to the reaction solution, which was extracted with ethyl acetate (100 mL X2). The organic phase was combined, dried over anhydrous sodium sulfate, and rotary dried under reduced pressure. The crude was purified by flash silica gel column (ethyl acetate/petroleum ether: 0-50%) to give the target product (1R,5S)-tert-butyl 3-(7-bromo-2-chloro-6,8-difluoroquinazolin-4-yl)-3,8-diazabicyclo[3.2.1]octane-8-carboxylate (406 mg, yield over 4 steps: 20%). ES-API: [M+H]⁺=489.0.

Step 5: under nitrogen protection, tert-butyl (1R,5S)-3-(7-bromo-2-chloro-6,8-difluoroquinazolin-4-yl)-3,8-diazabicyclo[3.2.1]octane-8-carboxylate (345.0 mg, 0.7070 mmol) was dissolved in dimethylsulfoxide (5.0 mL). Potassium fluoride (2.05 g, 35.35 mmol) was added, and the system reacted under nitrogen at 120°C for 8 h. Upon completion of the reaction, the reaction solution was cooled to room temperature, extracted with ethyl acetate (100 mL), washed with saturated brine (100 mL X3), the organic phase was dried over anhydrous sodium sulfate, rotary dried under reduced pressure, and the crude was purified by flash silica gel column (ethyl acetate/petroleum ether: 0-50%) to give tert-butyl (1R,5S)-3-(7-bromo-2,6,8-trifluoroquinazolin-4-yl)-3,8-diazabicyclo[3.2.1]octane-8-carboxylate (120 mg, yield: 35%). ES-API: [M+H]⁺=473.0/475.0. Step 6: tert-butyl (1R,5S)-3-(7-bromo-2,6,8-trifluoroquinazolin-4-yl)-3,8-diazabicyclo[3.2.1]octane-8-carboxylate (120 mg, 0.2536 mmol) was dissolved in tetrahydrofuran/water (2 mL/0.5 mL) and triisopropyl ((6-(methoxymethoxy)-8-(4,4,5,5-tetramethyl-1,3,2-dioxaborolan-2-yl)naphthalen-1-yl)ethynyl)silane (793 mg, 1.606 mmol), potassium phosphate (340mg, 1.606 mmol) and[n-butyldi (1-adamantyl)phosphino](2-amino-1,1'-biphenyl-2-yl)palladium (II) methanesulfonate (45.0 mg, 0.06025 mmol) were added, displaced with nitrogen for 4 times and allowed to react under microwave condition at 80°C for 1 h. The reaction solution was cooled to room temperature, and extracted with ethyl acetate (80 mL) and water (60 mL), the organic phase was dried over anhydrous sodium sulfate, filtered, and rotary dried. The crude was purified by flash silica gel column (ethyl acetate/petroleum ether: 0-70%) to give tert-butyl (1R,5S)-3-(2,6,8-trifluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate (100 mg, crude). ES-API: [M/2+1]⁺=761.3.

Step 7: sodium hydride (21 mg, 0.526 mmol) was added to a solution of (2-methylenetetrahydro-1H-pyrrolizin-7a(5H)-yl) methanol (50 mg, 0.3947 mmol) in tetrahydrofuran (3.0 mL) under an ice-water bath condition, and the system reacted at room temperature for 0.5 h. Tert-butyl (1R,5S)-3-(2,6,8-trifluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate (100 mg, crude) was added to the above reaction solution, and the system reacted at room temperature for 1-2 h. Upon completion of the reaction, ice water (50 mL) was added to the reaction solution and extracted with dichloromethane (50 mL X2). The organic phase was combined, dried over anhydrous sodium sulfate, and filtered, and the filtrate was rotary dried. The crude was purified by flash silica gel column (methanol/dichloromethane: 0-10%) to give tert-butyl (1R,5S)-3-(6,8-difluoro-7-(3-(methoxymethoxy)-8-((triisopropylsilyl)ethynyl)naphthalen-1-yl)-2-((2-methylenetetrahydro-1H-pyrrolizin-7a(5H)-yl)methoxy)quinazolin-4-yl)-3,8-diazabicyclo[3.2.1]octane-8-carboxylate (62 mg, total yield over 2 steps: 27.4%). ES-API: [M/2+1]⁺=894.4.

Step 8: tert-butyl (1R,5S)-3-(6,8-difluoro-7-(3-(methoxymethoxy)-8-((triisopropylsilyl)ethynyl)naphthalen-1-yl)-2-((2-methylenetetrahydro-1H-pyrrolizin-7a(5H)-yl)methoxy)quinazolin-4-yl)-3,8-diazabicyclo[3.2.1]octane-8-carboxylate (62 mg, 0.067) was dissolved in N, N-dimethylformamide (1.0 mL). Cesium fluoride (92 mg, 0.6066 mmol) was added, and the system reacted at room temperature for 2 h. Upon completion of the reaction, it was extracted with ethyl acetate (80 mL) and water (60 mL), and the organic phase was dried over anhydrous sodium sulfate, filtered, and rotary dried to give tert-butyl (1R,5S)-3-(7-(8-ethynyl-3-(methoxymethoxy)naphthalen-1-yl)-6,8-difluoro-2-((2-methylenetetrahydro-1H-pyrrolizin-7a(5H)-yl)methoxy)quinazolin-4-yl)-3,8-diazabicyclo[3.2.1]octane-8-carboxylate (92 mg, crude). ES-API: [M+1]⁺=738.3, directly used for the next step reaction.

Step 9: tert-butyl (1R,5S)-3-(7-(8-ethynyl-3-(methoxymethoxy)naphthalen-1-yl)-6,8-difluoro-2-((2-methylenetetrahydro-1H-pyrrolizin-7a(5H)-yl)methoxy)quinazolin-4-yl)-3,8-diazabicyclo[3.2.1]octane-8-carboxylate (92 mg, crude) was dissolved in acetonitrile (3.0 mL), 4 M hydrochloric acid/dioxane solution (2.0 mL) was added in an ice bath, and the system reacted for 0.5 h in an ice bath. Upon completion of the reaction, it was rotary dried under reduced pressure, dichloromethane (20 mL) was added, and triethylamine (3.0 mL) was added in an ice bath. Stirred for 10 min, extracted with dichloromethane (100 mL x1) and water (50 mL x1). The organic phase was dried over anhydrous sodium sulfate, filtered, and rotary dried, and the crude was prepared by high performance liquid chromatography to give 4-(4-(1R,5S)-3,8-diazabicyclo[3.2.1]octan-3-yl)-6,8-difluoro-2-(2-methylenetetrahydro-1H-pyrrolizin-7a(5H)-yl)methoxy)quinazolin-7-yl)-5-ethynylnaphthalen-2-ol (Z20, 13.0 mg, total yield over 2 steps: 32%). ES-API: [M+1]⁺=594.3.

### Example 20: Synthesis of 4-(4-((1R,5S)-3,8-diazabicyclo[3.2.1]octan-3-yl-2-((2,6-dimethylenetetrahydro-1H-pyrrolizin-7a(5H)-yl)methoxy)-8-fluoropyrido[4,3-d]pyrimidin-7-yl)-5-ethynylnaphthalen-2-ol (Z21)

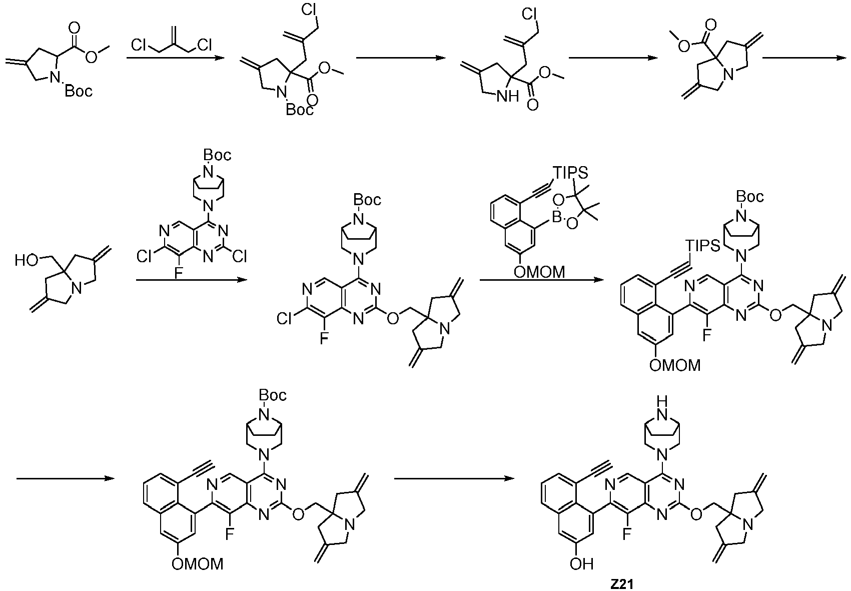

Step 1: methyl N-Boc-4-methylene-L-prolinate (1 g, 4.145 mmol) was dissolved in anhydrous tetrahydrofuran (15 mL) under nitrogen protection and the mixture was placed in a dry ice-acetone bath. Lithium bistrimethylsilylamide (6.3 mL, 6.218 mmol, 1 M in THF) was slowly added dropwise to the above solution, stirred at this temperature for 1 h, a solution of 3-chloro-2-chloromethyl-propene (2.59 g, 20.723 mmol) in anhydrous tetrahydrofuran (10 mL) was slowly added via syringe to the above solution, and the reaction solution was stirred at -78°C for another 1 h. The reaction was determined to be complete by LCMS, quenched with saturated ammonium chloride solution (30 mL), extracted with ethyl acetate (20 mL X3), washed with saturated brine (15 mL X3), dried over anhydrous sodium sulfate, filtered and concentrated. The crude was purified by flash silica gel column (petroleum ether: ethyl acetate = 0-25%) to give 1-(tert-butyl)2-methyl 2-(2-(chloromethyl)allyl)-4-methylenepyrrolidine-1,2-dicarboxylate (863 mg, yield: 63%). ES-API: [M-55]⁺= 274.1.

Step 2: a 4 M hydrochloric acid/dioxane solution (8 mL) was slowly added to a solution of 1-(tert-butyl)2-methyl 2-(2-(chloromethyl)allyl)-4-methylenepyrrolidine-1,2-dicarboxylate (863 mg, 2.623 mmol) in acetonitrile (4 mL) at room temperature and stirred at room temperature for 0.5 h. The reaction was determined to be complete by LCMS and the reaction solution was concentrated under reduced pressure to give methyl 2-(2-(chloromethyl)allyl)-4-methylenepyrrolidine-2-carboxylate (980 mg, crude). ES-API: [M+H]⁺= 230.1.

Step 3: sodium bicarbonate (1.583 g, 18.843 mmol) and potassium iodide (63 mg, 0.377 mmol) were added to a solution of methyl 2-(2-(chloromethyl)allyl)-4-methylenepyrrolidine-2-carboxylate (863 mg, 3.769 mmol) in acetonitrile (50 mL) at room temperature and stirred at room temperature for 1.5 h. The reaction was determined to be complete by LCMS, filtered, washed with acetonitrile, and concentrated under reduced pressure, and the crude was purified by flash silica gel column (petroleum ether: ethyl acetate = 0-30%) to give methyl 2,6-dimethylenetetrahydro-1H-pyrrolizine-7a(5H)-carboxylate (382 mg, yield: 53%). ES-API: [M+H]⁺= 194.2.

Step 4: methyl 2,6-dimethylenetetrahydro-1H-pyrrolizine-7a(5H)-carboxylate (382 mg, 1.98 mmol) was dissolved in anhydrous tetrahydrofuran (10 mL) under nitrogen protection and the mixture was placed under an ice-water bath condition. A solution of lithium tetrahydroaluminate (4 mL, 3.96 mmol, 1 M in THF) was slowly added dropwise to the above solution and stirred at this temperature for 0.5 h. The reaction was determined to be complete by LCMS and quenched by the addition of sodium sulfate decahydrate (304 mg) to the reaction solution, stirred at room temperature for 0.5 h, filtered, and concentrated to give (2,6-dimethylenetetrahydro-1H-pyrrolizin-7a(5H)-yl) methanol (303 mg, yield: 92%). ES-API: [M+H]⁺= 166.1.

Step 5: sodium hydride (110 mg, 2.727 mmol) was added portionwise to a solution of ((2,6-dimethylenetetrahydro-1H-pyrrolizin-7a(5H)-yl) methanol (150 mg, 0.909 mmol) in tetrahydrofuran (10 mL) under an ice-water bath condition, and stirred at 0°C for 1 h, a solution of tert-butyl (1R,5S)-3-(2,7-dichloro-8-fluoropyrido[4,3-d]pyrimidin-4-yl)-3,8-diazabicyclo[3.2.1]octane-8-carboxylate (582 mg, 1.364 mmol) in tetrahydrofuran (10 mL) was slowly added dropwise via syringe to the above solution and the reaction solution was stirred at this temperature for an additional 3 h. The reaction was determined to be complete by LCMS, quenched with saturated ammonium chloride solution (30 mL), extracted with ethyl acetate (20 mL X3), the organic phase was washed with saturated brine (15 mL X3), dried over anhydrous sodium sulfate, filtered and concentrated. The crude was purified by flash silica gel column (dichloromethane: methanol = 0-7%) to give tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (334 mg, yield: 66%). ES-API: [M+H]⁺= 557.2.

Step 6: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (200 mg, 0.360 mmol), triisopropyl ((6-(methoxymethoxy)-8-(4,4,5,5-tetramethyl-1,3,2-dioxoboran-2-yl)naphthalen-1-yl)ethynyl)silane (354 mg, 0.720 mmol), potassium phosphate (229 mg, 1.080 mmol) and [n-butyldi(1-adamantyl)phosphino](2-amino-1,1'-biphenyl-2-yl)palladium (II) methanesulfonate (39 mg, 0.054 mmol) were dissolved in tetrahydrofuran (10 mL) and water (2 mL) and heated to 65°C with stirring for 3 h. The reaction was determined to be complete by LCMS, the reaction solution was concentrated under reduced pressure and the crude was purified by flash silica gel column (dichloromethane: methanol = 0-15%) to give tert-butyl (1R,5S)-3-(2-((2,6-dimethylenetetrahydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (248 mg, yield: 78%). ES-API: [M+H]⁺= 889.5.

Step 7: cesium fluoride (425 mg, 2.793 mmol) was added to A solution of tert-butyl (1R,5S)-3-(2-((2,6-dimethylenetetrahydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (248 mg, 0.279 mmol) in the N, N-dimethylformamide (10 mL) and stirred at room temperature for 2 h. The reaction was determined to be complete by LCMS and washed with ethyl acetate (20 mL), water (15 mL X2) and saturated brine (15 mL X3), dried over anhydrous sodium sulfate, filtered and concentrated to give tert-butyl (1R,5S)-3-(2-((2,6-dimethylenetetrahydro-1H-pyrrolizin-7a(5H)-yl)methoxy)-7-(8-ethynyl-3-(methoxymethoxy)naphthalen-1-yl)-8-fluoropyrido[4,3-d]pyrimidin-4-yl)-3,8-diazabicyclo[3.2.1]octane-8-carboxylate (204 mg, yield: 99%). ES-API: [M+H]⁺= 733.3.

Step 8: a 4 M hydrochloric acid/dioxane solution (5mL) was added slowly to a solution of tert-butyl (1R,5S)-3-(2-((2,6-dimethylenetetrahydro-1H-pyrrolizin-7a(5H)-yl)methoxy)-7-(8-ethynyl-3-(methoxymethoxy)naphthalen-1-yl)-8-fluoropyrido[4,3-d]pyrimidin-4-yl)-3,8-diazabicyclo[3.2.1]octane-8-carboxylate (204 mg, 0.279 mmol) in acetonitrile (5 mL) under an ice-water bath condition and stirred at 0°C for 0.5 h. The reaction was determined to be complete by LCMS, the reaction solution was concentrated to dryness under reduced pressure, and the crude was prepared by high performance liquid chromatography (chromatographic column: Welch Xtimate, 21.2*150 mm, 5 um; mobile phase A: 5 mmol/L NH₄HCO₃ aqueous solution, mobile phase B: ACN; flow rate: 15 ml/min; chromatographic conditions: 15 ml-35-85-13 min; column temperature: room temperature) to give 4-(4-((1R,5S)-3,8-diazabicyclo[3.2.1]octan-3-yl-2-((2,6-dimethylenetetrahydro-1H-pyrrolizin-7a(5H)-yl)methoxy)-8-fluoropyrido[4,3-d]pyrimidin-7-yl)-5-ethynylnaphthalen-2-ol (Z21, 48 mg, yield: 29%). ES-API: [M+H]⁺= 589.2. ¹H NMR (500 MHz, CD₃OD) δ 8.99 (s, 1 H), 7.81 (dd, J1= 1.0 Hz, J2= 8.5 Hz, 1 H), 7.50 (dd, J1= 1.0 Hz, J2= 7.0 Hz, 1 H), 7.40-7.37 (m, 1 H), 7.32 (d, J= 2.5 Hz, 1 H), 7.15 (d, J= 2.5 Hz, 1 H), 5.03-5.00 (m, 4 H), 4.64-4.55 (m, 2 H), 4.34-4.30 (m, 2 H), 3.78-3.65 (m, 6 H), 2.81-2.77 (m, 2 H), 2.58 (d, J= 16.5 Hz, 2H), 2.05-1.75 (m, 7 H).

### Example 21: Synthesis of 4-(4-((1R,5S)-3,8-diazabicyclo[3.2.1]oct-3-yl)-8-fluoro-2-((6'-methylenetetrahydrospiro[cyclopropane-1,3'-pyrrolizin]-7a'(5'H)-yl)methoxy)pyrido[4,3-d]pyrimidin-7-yl)-5-ethynylnaphthalen-2-ol (Z22)

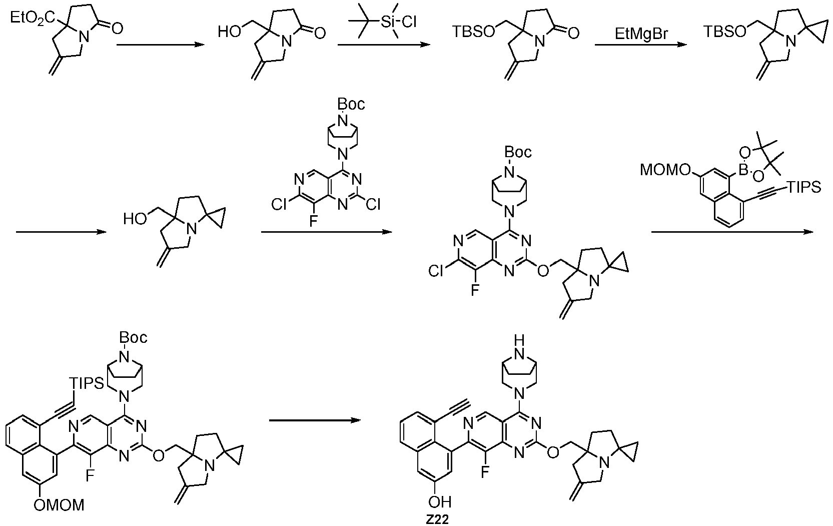

Step 1: under nitrogen protection, a round bottom flask was charged with ethyl 2-methylene-5-oxotetrahydro-1H-pyrrolizine-7a(5H)-carboxylate (1.5 g, 7.17 mmol), tetrahydrofuran (15 mL). After cooling to 0°C, a solution of lithium borohydride (9 mL, 18 mmol, 2 M in THF) was added dropwise to the above solution. The addition was complete and stirred at 0°C for 30 min. Stirring at room temperature for an additional 20 min. After cooling to 0°C, 2 M hydrochloric acid (10 mL) was added dropwise thereto until no bubbling occurred. To this was added 2 M sodium hydroxide aqueous solution (30 mL) until the solution was clear. Extracted with dichloromethane/isopropanol (3:1, 50 mL X3). The organic phase was dried over anhydrous sodium sulfate and concentrated to give 7a-(hydroxymethyl)-6-methylenehexahydro-3H-pyrrolizin-3-one (1.05 g, yield: 87%) as a colorless oil. ES-API: [M+H]⁺= 168.1.

Step 2: a round bottom flask was charged with sodium hydride (595 mg, 14.87 mmol) and tetrahydrofuran (20 mL). Cooled to 0°C under nitrogen protection. A solution of 7a-(hydroxymethyl)-6-methylenehexahydro-3H-pyrrolizin-3-one (1 g, 5.95 mmol) in tetrahydrofuran (2 mL) was added dropwise to the above reaction solution with stirring. After stirring at 0°C for 10 min, t-butyl chlorodimethylsilane (1.3 g, 8.93 mmol) was added to the above reaction solution and stirred at room temperature for 1 h. A saturated ammonium chloride aqueous solution (50 mL) was added to the reaction solution and extracted with ethyl acetate (50 mL X3). The organic phase was washed with saturated brine, dried over anhydrous sodium sulfate, and concentrated and the crude was purified by flash silica gel column (dichloromethane/petroleum ether: 0-100%) to give 7a-(((tert-butyldimethylsilyl)oxy)methyl)-6-methylenehexahydro-3H-pyrrolizin-3-one (750 mg, yield: 44%) as a colorless oil. ES-API: [M+H]⁺= 282.3.

Step 3: a reaction flask was charged with titanium tetraisopropoxide (1.3g, 4.62 mmol) and tetrahydrofuran (10 mL) under nitrogen protection and cooled to -70°C. Ethylmagnesium bromide solution (9.24 mL, 9.24 mmol, 1 M in THF) was added dropwise to the above reaction solution with stirring. Stirred at -70°C for 5 min. A solution of 7a-(((tert-butyldimethylsilyl)oxy)methyl)-6-methylenehexahydro-3H-pyrrolizin-3-one (650 mg, 2.31 mmol) in tetrahydrofuran (3 mL) was added dropwise to the above reaction solution and after stirring for 20 min the ice bath was removed. Stirred at room temperature for 2 h. A saturated sodium bicarbonate aqueous solution (50 mL) was added to the reaction solution and extracted with ethyl acetate (50 mL X3). The organic phase was washed with saturated brine, dried over anhydrous sodium sulfate, and concentrated and the crude was purified by flash silica gel column (ethyl acetate/petroleum ether: 0-50%) to give 7a'-(((tert-butyldimethylsilyl)oxy)methyl)-6'-methylenehexahydrospiro[cyclopropane-1,3'-pyrrolizine](200 mg, yield: 29%) as a yellow oil. ES-API: [M+H]⁺= 294.3.

Step 4: a reaction flask was charged with 7a'-(((tert-butyldimethylsilyl)oxy)methyl)-6'-methylenehexahydrospiro[cyclopropane-1,3'-pyrrolizine](200 mg, 0.68 mmol), methanol (1 mL) and 4 M hydrogen chloride/dioxane solution (3 mL). The reaction was stirred at room temperature for 1 hour. The reaction solution was concentrated to give (6'-methylenetetrahydrospiro[cyclopropane-1,3'-pyrrolizin]-7a'(5'H)-yl) methanol (122 mg, crude), which was used directly in the next step. ES-API: [M+H]⁺= 180.2.

Step 5: a round bottom flask was charged with sodium hydride (37 mg, 0.92 mmol) and tetrahydrofuran (3 mL). Cooled to 0°C under nitrogen protection. A solution of (6'-methylenetetrahydrospiro[cyclopropane-1,3'-pyrrolizin]-7a'(5'H)-yl) methanol (83 mg, crude) in tetrahydrofuran (1 mL) was added dropwise to the above reaction solution with stirring. Stirred at room temperature for 10 min. After cooling to 0°C, tert-butyl (1R,5S)-3-(2,7-dichloro-8-fluoropyrido[4,3-d]pyrimidin-4-yl)-3,8-diazabicyclo[3.2.1]octane-8-carboxylate (100 mg, 0.23 mmol) was added to the above reaction solution and stirred at room temperature for 1 h. The reaction was stirred in a 40°C oil bath for 30 min. Cooling to 0°C, the saturated sodium bicarbonate aqueous solution (50 mL) was added to the reaction solution, which was extracted with ethyl acetate (50 mL X3). The organic phase was washed with saturated brine, dried over anhydrous sodium sulfate, concentrated and the crude was purified by flash silica (ethyl acetate/petroleum ether: 0-50%) to give tert-butyl (1R,5S)-3-(7-chloro-8-fluoro-2-((6'-methylenetetrahydrospiro[cyclopropane-1,3'-pyrrolizin]-7a'(5'H)-yl)methoxy)pyrido[4,3-d]pyrimidin-4-yl)-3,8-diazabicyclo[3.2.1]octane-8-carboxylate (30 mg, yield: 22%) as a yellow solid. ES-API: [M+H]⁺= 571.3.

Step 6: a round bottom flask was charged with tert-butyl (1R,5S)-3-(7-chloro-8-fluoro-2-((6'-methylenetetrahydrospiro[cyclopropane-1,3'-pyrrolizin]-7a'(5'H)-yl)methoxy)pyrido[4,3-d]pyrimidin-4-yl)-3,8-diazabicyclo[3.2.1]octane-8-carboxylate (30 mg, 0.052 mmol), triisopropyl ((6-(methoxymethoxy)-8-(4,4,5,5-tetramethyl-1,3,2-dioxoboran-2-yl)naphthalen-1-yl)ethynyl)silane (65 mg, 0.13 mmol), cataCXiumA-Pd-G3 (6 mg, 0.008 mmol), potassium phosphate (33 mg, 0.16 mmol), tetrahydrofuran (3 mL) and water (0.6 mL). It was heated and stirred at 75°C in an oil bath under nitrogen protection for 3 h. Water (30 mL) was added into the reaction solution, extracted with ethyl acetate (30 mL X3), the organic phase was dried over anhydrous sodium sulfate, concentrated and the crude was purified by flash silica gel column (ethyl acetate/petroleum ether: 0-40%) to give tert-butyl (1R,5S)-3-(8-fluoro-7-(3-(methoxymethoxy)-8-((triisopropylsilyl)ethynyl)naphthalen-1-yl)-2-((6'-methylenetetrahydrospiro[cyclopropane-1,3'-pyrrolizin]-7a'(5'H)-yl)methoxy)pyrido[4,3-d]pyrimidin-4-yl)-3,8-diazabicyclo[3.2.1]octane-8-carboxylate (20 mg, yield: 42%) as a yellow solid. ES-API: [M+H]⁺= 903.4.

Step 7: a round bottom flask was charged with tert-butyl (1R,5S)-3-(8-fluoro-7-(3-(methoxymethoxy)-8-((triisopropylsilyl)ethynyl)naphthalen-1-yl)-2-((6'-methylenetetrahydrospiro[cyclopropane-1,3'-pyrrolizin]-7a'(5'H)-yl)methoxy)pyrido[4,3-d]pyrimidin-4-yl)-3,8-diazabicyclo[3.2.1]octane-8-carboxylate (20 mg, 0.022 mmol), cesium fluoride (17 mg, 0.11 mmol) and anhydrous N, N-dimethylformamide (1.5 mL). The reaction was stirred at room temperature for 1 hour. Water (50 mL) was added to the reaction solution, extracted with ethyl acetate (20 mL X2), and the organic phase was dried over anhydrous sodium sulfate and concentrated. The concentrate was dissolved in acetonitrile (1 mL), cooled to 0°C, and 4 M hydrogen chloride/dioxane solution (1 mL) was added. Stirred at 0°C for 1.5 h. The reaction solution was concentrated and the crude was prepared by high performance liquid chromatography (alkaline method) to give 4-(4-((1R,5S)-3,8-diazabicyclo[3.2.1]octan-3-yl)-8-fluoro-2-((6'-methylenetetrahydrospiro[cyclopropane-1,3'-pyrrolizin]-7a'(5'H)-yl)methoxy)pyrido[4,3-d]pyrimidin-7-yl)-5-ethynylnaphthalen-2-ol (Z22, 0.77mg, yield: 6%) as a yellow solid. ES-API: [M+H]⁺=603.3.

### Example 22: Synthesis of 4-(4-((1R,5S)-3,8-diazabicyclo[3.2.1]octan-3-yl)-8-fluoro-2-((6'-methylenedihydro-1'H,3'H-spiro[cyclopropane-1,2'-pyrrolizin]-7a'(5'H)-yl)methoxy)pyrido[4,3-d]pyrimidin-7-yl)-5-ethynylnaphthalen-2-ol (Z23)

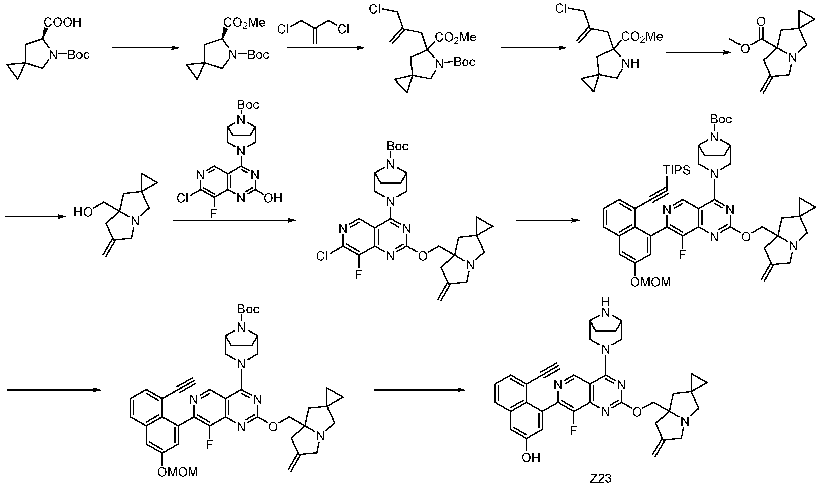

Step 1: potassium carbonate (8.6 g, 62.25 mmol) and iodomethane (3.8 mL, 62.25 mmol) were added to a solution of (S)-5-(tert-butoxycarbonyl)-5-azaspiro[2.4]heptane-6-carboxylic acid (10 g, 41.5 mmol) in N, N-dimethylformamide (50 mL) under an ice-water bath condition and stirred at room temperature for 1.5 h. The reaction was determined to be complete by LCMS, filtered, washed with ethyl acetate and the filtrate was concentrated under reduced pressure to give 5-(tert-butyl)6-methyl (S)-5-azaspiro[2.4]heptane-5,6-dicarboxylate (11.2g, yield: 71%). ES-API: [M-55]⁺= 200.1.

Step 2: 5-(tert-butyl)6-methyl (S)-5-azaspiro[2.4]heptane-5,6-dicarboxylate (5.0 g, 20 mmol) was dissolved in anhydrous tetrahydrofuran (50 mL) under nitrogen protection and the mixture was placed in a dry ice-acetone bath. Lithium bistrimethylsilylamide solution (30 mL, 30 mmol, 1 M in THF) was slowly added dropwise to the above solution, stirred at this temperature for 1 h, a solution of 3-chloro-2-chloromethyl-propene (12.4 g, 100 mmol) in anhydrous tetrahydrofuran (20 mL) was slowly added via syringe to the above solution, and the reaction solution was stirred at - 78°C for another 1 h. The reaction was determined to be complete by LCMS, quenched with saturated ammonium chloride solution (50 mL), extracted with ethyl acetate (50 mL X3), the organic phase was washed with saturated brine (50 mL X3), dried over anhydrous sodium sulfate, filtered and concentrated. The crude was purified by flash silica gel column (petroleum ether: ethyl acetate = 20:1) to give 5-(tert-butyl)6-methyl (R)-6-(2-(chloromethyl)allyl)-5-azaspiro[2.4]heptane-5,6-dicarboxylate (5.2 g, yield: 75%). ES-API: [M-55]⁺= 288.1.

Step 3: a 4 M solution of hydrochloric acid/dioxane solution (15 mL) was slowly added to a solution of 5-(tert-butyl)6-methyl (R)-6-(2-(chloromethyl)allyl)-5-azaspiro[2.4]heptane-5,6-dicarboxylate (5.2 g, 15 mmol) in acetonitrile (15 mL) at room temperature and stirred at room temperature for 0.5 h. The reaction was determined to be complete by LCMS and the reaction solution was concentrated under reduced pressure to give methyl (R)-6-(2-(chloromethyl)allyl)-5-azaspiro[2.4]heptane-6-carboxylate (3.65 g, yield: 100%). ES-API: [M+H]⁺= 244.1.

Step 4: sodium bicarbonate (6.3 g, 75 mmol) and potassium iodide (250 mg, 1.5 mmol) were added to a solution of methyl (R)-6-(2-(chloromethyl)allyl)-5-azaspiro[2.4]heptane-6-carboxylate (3.65g, 15 mmol) in acetonitrile (50 mL) at room temperature and stirred at room temperature for 1.5 h. The reaction was determined to be complete by LCMS, filtered, and washed with acetonitrile, and the filtrate was concentrated under reduced pressure. The crude was purified by flash silica gel column (petroleum ether: ethyl acetate = 15:1) gave methyl 6'-methylenedihydro-1'H,3'H-spiro[cyclopropane-1,2'-pyrrolizine]-7a'(5'H)-carboxylate (3.1 g, yield: 100%). ES-API: [M+H]⁺= 208.2.

Step 5: under nitrogen protection, methyl 6'-methylenedihydro-1'H,3'H-spiro[cyclopropane-1,2'-pyrrolizine]-7a'(5'H)-carboxylate (3.1 g, 15 mmol) was dissolved in anhydrous tetrahydrofuran (50 mL) under nitrogen atmosphere, the mixture was placed under an ice-water bath condition, lithium tetrahydroaluminate solution (30 mL, 30 mmol, 1 M in THF) was slowly added dropwise to the above solution, and stirred at this temperature for 0.5 h. The reaction was determined to be complete by LCMS and quenched by the addition of sodium sulfate decahydrate (3.2 g), stirred at room temperature for 0.5 h, filtered and the filtrate was concentrated under reduced pressure to give (6'-methylenedihydro-1'H,3'H-spiro[cyclopropane-1,2'-pyrrolizin]-7a'(5'H)-yl) methanol (2.7 g, yield: 100%). ES-API: [M+H]⁺= 180.3.

Step 6: a solution of (6'-methylenedihydro-1'H,3'H-spiro[cyclopropane-1,2'-pyrrolizin]-7a'(5'H)-yl) methanol (188mg, 2.1 mmol), tert-butyl (1R,5S)-3-(7-chloro-8-fluoro-2-hydroxypyrido[4,3-d]pyrimidin-4-yl)-3,8-diazabicyclo[3.2.1]octane-8-carboxylate (300 mg, 0.7 mmol) and N, N-diisopropylethyl amine (271 mg, 2.1 mmol) in dioxane (5 mL) was stirred at 110°C for 6 h. The reaction was determined to be complete by LCMS, quenched with saturated ammonium chloride solution (30 mL), extracted with ethyl acetate (20 mL X3), the organic phase was washed with saturated brine (15 mL X3), dried over anhydrous sodium sulfate, filtered and concentrated. The crude was purified by flash silica gel column (dichloromethane: methanol = 20:1) to give tert-butyl (1R,SS)-3-(7-chloro-8-fluoro-2-((6'-methylenedihydro-1'H,3'H-spiro[cyclopropane-1,2'-pyrrolizin]-7a'(5'H)-yl)methoxy)pyrido[4,3-d]pyrimidin-4-yl)-3,8-diazabicyclo[3.2.1]octane-8-carboxylate (268 mg, yield: 67%). ES-API: [M+H]⁺= 571.3.

Step 7: under nitrogen protection, tert-butyl (1R,5S)-3-(7-chloro-8-fluoro-2-((6'-methylenedihydro-1'H,3'H-spiro[cyclopropane-1,2'-pyrrolizin]-7a'(5'H)-yl)methoxy)pyrido[4,3-d]pyrimidin-4-yl)-3,8-diazabicyclo[3.2.1]octane-8-carboxylate (268 mg, 0.47 mmol), triisopropyl ((6-(methoxymethoxy)-8-(4,4,5,5-tetramethyl-1,3,2-dioxoboran-2-yl)naphthalen-1-yl)ethynyl)silane (462 mg, 0.94 mmol), potassium phosphate (300 mg, 1.41 mmol) and CataXiumAPdG3 (51 mg, 0.071 mmol) were dissolved in tetrahydrofuran (10 mL) and water (2 mL) and heated to 65°C with stirring for 3 h. The reaction was determined to be complete by LCMS, the reaction solution was concentrated under reduced pressure and the crude was purified by flash silica gel column (dichloromethane: methanol = 20:1) to give tert-butyl (1R,5S)-3-(8-fluoro-7-(3-(methoxymethoxy)-8-((triisopropylsilyl)ethynyl)naphthalen-1-yl)-2-((6'-methylenedihydro-1'H,3'H-spiro[cyclopropane-1,2'-pyrrolizin]-7a'(5'H)-yl)methoxy)pyrido[4,3-d]pyrimidin-4-yl)-3,8-diazabicyclo[3.2.1]octane-8 carboxylate (186 mg, yield: 44%). ES-API: [M+H]⁺= 903.4.

Step 8: cesium fluoride (319 mg, 2.1 mmol) was added to a solution of tert-butyl (1R,5S)-3-(8-fluoro-7-(3-(methoxymethoxy)-8-((triisopropylsilyl)ethynyl)naphthalen-1-yl)-2-((6'-methyldihydro-1'H,3'H-spiro[cyclopropane-1,2'-pyrrolizin]-7a'(5'H)-yl)methoxy)pyrido[4,3-d]pyrimidin-4-yl)-3,8-diazabicyclo[3.2.1]octane-8-carboxylate (186 mg, 0.21 mmol) in N, N-dimethylformamide (5 mL), stirred at room temperature for 2 h. The reaction was determined to be complete by LCMS, and water was added to the reaction solution. The obtained mixture was washed with ethyl acetate (20 mL), water (15 mL X2), saturated brine (15 mL X3), dried over anhydrous sodium sulfate, filtered and concentrated to give tert-butyl (1R,5S)-3-(7-(8-ethynyl-3-(methoxymethoxy)naphthalen-1-yl)-8-fluoro-2-((6'-methylenedihydro-1'H,3'H-spiro[cyclopropane-1,2'-pyrrolizin]-7a'(5'H)-yl)methoxy)pyrido[4,3-d]pyrimidin-4-yl)-3,8-diazabicyclo[3.2.1]octane-8-carboxylate (154 mg, yield: 100%). ES-API: [M+H]⁺= 747.3.

Step 9: a 4 M hydrochloric acid/dioxane solution (5mL) was slowly added to a solution of tert-butyl (1R,5S)-3-(7-(8-ethynyl-3-(methoxymethoxy)naphthalen-1-yl)-8-fluoro-2-((6'-methylenedihydro-1'H,3'H-spiro[cyclopropane-1,2'-pyrrolizin]-7a'(5'H)-yl)methoxy)pyrido[4,3-d]pyrimidin-4-yl)-3,8-diazabicyclo[3.2.1]octane-8-carboxylate (154 mg, 0.20 mmol) in acetonitrile (5 mL) under an ice-water bath condition, stirred at 0°C for 0.5 h. The reaction was determined to be complete by LCMS, the reaction solution was concentrated to dryness under reduced pressure and the crude was prepared by high performance liquid chromatography (chromatographic column: Welch Xtimate, 21.2*150 mm, 5 um; mobile phase A: 5 mmol/L NH₄HCO₃ aqueous solution, mobile phase B: ACN; flow rate: 15 ml/min; chromatographic conditions: 15 ml-35-85-13 min; column temperature: room temprature) to give 4-(4-((1R,5S)-3,8-diazabicyclo[3.2.1]octan-3-yl)-8-fluoro-2-((6'-methylenedihydro-1'H,3'H-spiro[cyclopropane-1,2'-pyrrolizin]-7a'(5'H)-yl)methoxy)pyrido[4,3-d]pyrimidin-7-yl)-5-ethynylnaphthalen-2-ol (Z23, 28 mg, yield: 23 %), pale yellow solid. ES-API: [M+H]+= 603.3. ¹H NMR (400 MHz, CD₃OD) δ 9.00 (s, 1H), 7.81 (d, *J* = 8.4 Hz, 1H), 7.50 (d, *J* = 6.8 Hz, 1H), 7.44-7.28 (m, 2H), 7.16 (d, *J* = 2.0 Hz, 1H), 5.02 (s, 2H), 4.69-4.53 (m, 2H), 4.50-4.40 (m, 1H), 4.39-4.27 (m, 1H), 3.80-3.66 (m, 5H), 3.48-3.42 (m, 1H), 3.02 (s, 1H), 2.96-2.93 (m, 1H), 2.90-2.80 (m, 2H), 2.61 (d, *J* = 15.2 Hz, 1H), 2.01 (d, *J* = 2.8 Hz, 2H), 1.93-1.75 (m, 4H), 1.36-1.26 (m, 1H), 0.71-0.51 (m, 4H).

### Example 23: Synthesis of 4-(4-((1R,5S)-3,8-diazabicyclo[3.2.1]octan-3-yl)-8-fluoro-2-((2-methylenetetrahydro-1H-pyrrolizin-7a(5H)-yl)methoxy)pyrido[4,3-d]pyrimidin-7-yl)-5-fluoronaphthalen-2-ol (Z24)

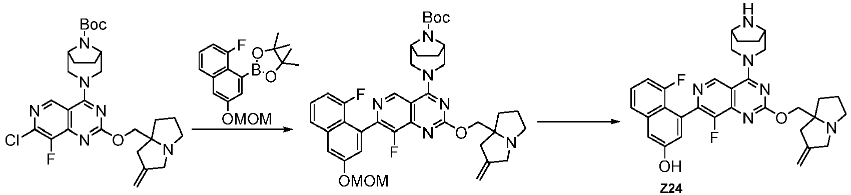

Step 1: the compound tert-butyl (1R,5S)-3-(7-chloro-8-fluoro-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (140 mg, 0.26 mmol) was dissolved in tetrahydrofuran/water (4 mL/0.4 mL). 2-(8-fluoro-3-(methoxymethoxy)naphthalen-1-yl)-4,4,5,5-tetramethyl-1,3,2-dioxaborane (130 mg, 0.39 mmol), potassium phosphate (110 mg, 0.52 mmol) and CataCXium A Pd G3 (42 mg, 30% w/w) were added, and the system reacted at 60°C for 1 h. The reaction solution was concentrated and purified by preparative thin layer chromatography (dichloromethane/methanol = 15/1) to give tert-butyl (1R,5S)-3-(8-fluoro-7-(8-fluoro-3-(methoxymethoxy)naphthalen-1-yl)-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (60 mg, yield: 32.4%). ESI: [M+H]⁺=715.2.

Step 2: the compound tert-butyl (1R,SS)-3-(8-fluoro-7-(8-fluoro-3-(methoxymethoxy)naphthalen-1-yl)-2-((2-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (60 mg, 0.084 mmol) was dissolved in acetonitrile (3 mL), 4 M hydrochloric acid/dioxane solution (1 mL) was added in an ice bath, and the system reacted for 1 h in an ice bath. Alkalinity was adjusted by saturated sodium bicarbonate to about (pH = 8.0) and high performance liquid phase preparation (0.05% formic acid/acetonitrile 0-30%, 10min, 30%, 5 min) was performed to give 4-(4-((1R,5S)-3,8-diazabicyclo[3.2.1]octan-3-yl)-8-fluoro-2-((2-methylenetetrahydro-1H-pyrrolizin-7a(5H)-yl)methoxy)pyrido[4,3-d]pyrimidin-7-yl)-5-fluoronaphthalen-2-ol (Z24, 16 mg, yield: 33.3%). ESI: [M+H]⁺=571.2. ¹H NMR (500 MHz, CD₃OD) δ 9.10 (s, 1H), 7.60 (d, *J* = 8.3 Hz, 1H), 7.39 (tt, *J* = 16.6, 8.3 Hz, 1H), 7.34 (t, *J* = 2.1 Hz, 1H), 7.17 (d, *J* = 2.3 Hz, 1H), 6.92 (dd, *J* = 13.1, 7.6 Hz, 1H), 5.23 (s, 2H), 4.74 (t, *J* = 11.9 Hz, 2H), 4.62 (q, *J* = 12.1 Hz, 2H), 4.24 (d, *J* = 14.4 Hz, 1H), 3.93 (s, 2H), 3.87-3.75 (m, 3H), 3.70-3.60 (m, 1H), 3.21-3.07 (m, 1H), 3.01 (d, *J* = 15.9 Hz, 1H), 2.73 (d, *J* = 16.0 Hz, 1H), 2.36-2.32 (m, 1H), 2.24-2.02 (m, 3H), 2.02-1.83 (m, 4H).

### Example 24: Synthesis of 4-(4-((1R,5S)-3,8-diazabicyclo[3.2.1]octan-3-yl)-8-fluoro-2-((1-methylenetetrahydro-1H-pyrrolizin-7a(5H)-yl)methoxy)pyrido[4,3-d]pyrimidin-7-yl)-5-ethynylnaphthalen-2-ol (Z25)

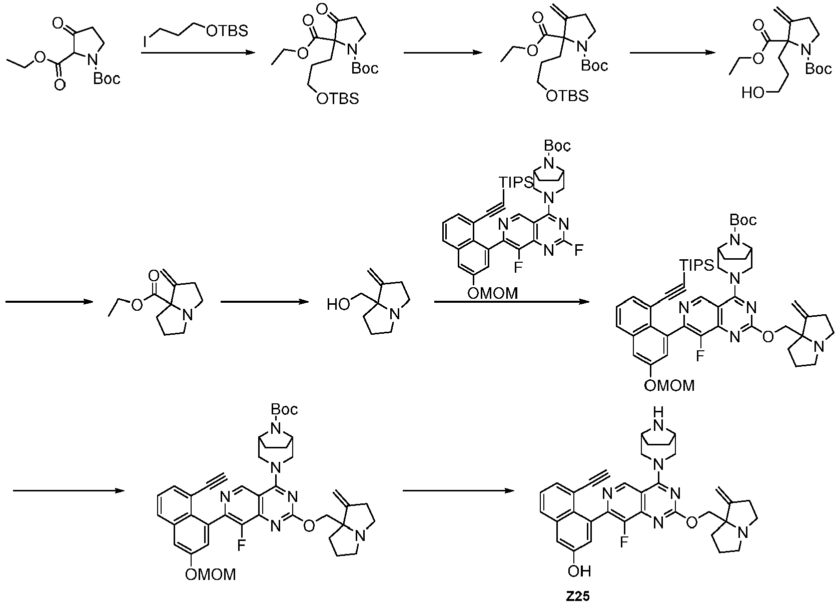

Step 1: a mixture of ethyl N-Boc-3-oxopyrrolidine-2-carboxylate (1.0 g, 3.89 mmol), tert-butyl (3-iodopropoxy) dimethylsilane (3.0 g, 9.99 mmol), potassium carbonate (1.61 g, 11.61 mmol) and N, N-dimethylformamide (40 mL) was stirred at room temperature under nitrogen protection overnight. Water (100 mL) was added to the reaction solution and extracted with ethyl acetate (50 mL X2). The mixture was washed with saturated brine (200 mL X3), dried over anhydrous sodium sulfate, and concentrated and the crude was purified by flash silica gel column (ethyl acetate/petroleum ether: 0-10%) to give 1-(tert-butyl)2-ethyl 2-(3-((tert-butyldimethylsilyl)oxy) propyl)-3-oxopyrrolidine-1,2-dicarboxylate (600 mg, 1.40 mmol, 36% yield) as a colorless transparent oily liquid. ES-API: [M-Boc+H]⁺=330.3.

Step 2: methyl triphenylphosphine bromide (2.49 g, 6.98 mmol) was added to tetrahydrofuran (25 mL) and cooled to 0°C. A solution of potassium tert-butoxide in tetrahydrofuran (5.59 mL, 5.59 mmol, 1 M) was added to the above mixture at 0°C under a nitrogen atmosphere. After the addition was complete, the reaction was warmed to room temperature and stirred for 0.5 h. 1-(tert-butyl)2-ethyl 2-(3-((tert-butyldimethylsilyl)oxy)propyl)-3-oxopyrrolidine-1,2-dicarboxylate (600 mg, 1.40 mmol) was dissolved in tetrahydrofuran (5 mL) and slowly added to the above reaction solution under nitrogen atmosphere. After the addition was complete, stirring was continued at 60°C for 5 h. Saturated ammonium chloride (30 mL) was added to the reaction solution and extracted with ethyl acetate (30 mL X3). The organic phase was washed with saturated brine (200 mL), dried over anhydrous sodium sulfate, and concentrated and the crude was purified by flash silica gel column (ethyl acetate/petroleum ether: 0%-8%) to give 1-(tert-butyl)2-ethyl 2-(3-((tertbutyldimethylsilyl)oxy)propyl)-3-methylenepyrrolidine-1,2-dicarboxylate (300 mg, 0.701 mmol, 50% yield) as a colorless transparent liquid. ES-API: [M-Boc+H]⁺=328.3.

Step 3: 1-(tert-butyl)2-ethyl 2-(3-((tert-butyldimethylsilyl)oxy)propyl)-3-methylenepyrrolidine-1,2-dicarboxylate (300 mg, 0.701 mmol) was dissolved in tetrahydrofuran (3 mL). A solution of tetra-n-butylammonium fluoride in tetrahydrofuran (1.40 mL, 1.40 mmol, 1 M) was added to the above solution at room temperature and stirred for 4 h. Add water (15 mL) to the above solution and extracted with ethyl acetate (15 mL X2). The organic phase was washed with saturated brine (20 mL), dried over anhydrous sodium sulfate, and concentrated and the crude was purified by flash silica gel column (ethyl acetate/petroleum ether: 0%-30%) to give 1-(tert-butyl)2-ethyl 2-(3-hydroxypropyl)-3-methylenepyrrolidine-1,2-dicarboxylate (180 mg, 0.574 mmol, yield 82%) as a colorless transparent liquid. ES-API: [M-Boc+H]⁺=214.1.

Step 4: 1-(tert-butyl) 2-ethyl 2-(3-hydroxypropyl)-3-methylenepyrrolidine-1,2-dicarboxylate (150 mg, 0.479 mmol) was dissolved in dichloromethane (5 mL) and cooled to 0°C. Thionyl chloride (569 mg, 4.79 mmol) was added to the above solution. After the addition was complete, it was warmed to room temperature and stirred for 17 h. The reaction solution was concentrated to give the crude hydrochloride salt. The crude hydrochloride salt was dissolved in dichloromethane/methanol (v/v = 10/1, 10 mL) and solid potassium carbonate (1 g, 7.24 mmol) was added and stirred for 1 h. Filtered, washed with ethyl acetate (20 mL), and the filtrate was concentrated to give ethyl 1-methylenetetrahydro-1H-pyrrolizine-7a(SH)-carboxylate (80 mg, 0.409 mmol, yield 86%) as a colorless transparent liquid. ES-API: [M+H]⁺= 196.1.

Step 5: ethyl 1-methylenetetrahydro-1H-pyrrolizine-7a(SH)-carboxylate (80 mg, 0.409 mmol, yield 86%) was dissolved in tetrahydrofuran (2 mL) and cooled to 0°C. Lithium aluminum tetrahydride solution (0.82 mL, 0.82 mmol, 1 M in THF) was added to the above solution under a nitrogen atmosphere. The reaction was stirred at 0°C for 1 h. Water (0.03 mL) was added to quench the reaction and 15% sodium hydroxide aqueous solution (0.03 mL) and water (0.09 mL) were added. Tetrahydrofuran (10 mL) was added and filtered. The filtrate was concentrated to give (1-methylenetetrahydro-1H-pyrrolizin-7a(5H)-yl) methanol (50 mg, 0.326 mmol, yield 79%) as a colorless transparent liquid. ES-API: [M+H]⁺= 154.1.

Step 6: a mixture of tert-butyl (1R,5S)-3-(2,8-difluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (100 mg, 0.134 mmol), (1-methylenetetrahydro-1H-pyrrolizin-7a(5H)-yl) methanol (41 mg, 0.269 mmol), potassium carbonate (37 mg, 0.269 mmol) and acetonitrile (3 mL) was stirred at 60°C for 1 h. Saturated ammonium chloride (10 mL) was added to the reaction solution and extracted with ethyl acetate (10 mL X3). The organic phase was washed with saturated brine (20 mL), dried over anhydrous sodium sulfate, concentrated and the crude was purified by flash silica gel column (methanol/dichloromethane: 0-6%) to give tert-butyl (1R,SS)-3-(8-fluoro-7-(3-(methoxymethoxy)-8-((triisopropylsilyl)ethynyl)naphthalen-1-yl)-2-((1-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (80 mg, 0.091 mmol, yield 68%) as a pale yellow solid. ES-API: [M+H]⁺= 877.5.

Step 7: a mixed solution of (1R,5S)-3-(8-fluoro-7-(3-(methoxymethoxy)-8-((triisopropylsilyl)ethynyl)naphthalen-1-yl)-2-((1-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (80 mg, 0.091 mmol), cesium fluoride (138 mg, 0.91 mmol) and N, N-dimethylformamide (2 mL) was stirred at room temperature for 2 h. Water (10 mL) was added to the reaction solution, which was extracted with ethyl acetate (20 mL X2). The organic phase was washed with saturated brine (15 mLX3), dried over anhydrous sodium sulfate and concentrated to give tert-butyl (1R,5S)-3-(7-(8-ethynyl-3-(methoxymethoxy)naphthalen-1-yl)-8-fluoro-2-((1-methylenetetrahydro-1H-pyrrolizin-7a(5H)-yl)methoxy)pyrido[4,3-d]pyrimidin-4-yl)-3,8-diazabicyclo[3.2.1]octane-8-carboxylate (65.74 mg, 0.092 mmol, yield: 100%) as a yellow oily liquid. ES-API: [M+H]⁺= 721.3.

Step 8: tert-butyl (1R,5S)-3-(7-(8-ethynyl-3-(methoxymethoxy)naphthalen-1-yl)-8-fluoro-2-((1-methylenetetrahydro-1H-pyrrolizin-7a(5H))-yl)methoxy)pyrido[4,3-d]pyrimidin-4-yl)-3,8-diazabicyclo[3.2.1]octane-8-carboxylate (65.74 mg, 0.092 mmol) was dissolved in acetonitrile (2 mL) and cooled to 0°C. 4 M hydrogen chloride/dioxane solution (1 mL) and stirred at 0°C for 2 h. The reaction solution was concentrated, dissolved in dichloromethane (3 mL), alkalinity was adjusted with triethylamine (pH value = 8), concentrated, and the crude was prepared by high performance liquid chromatography (column type: Waters XBridge C18, 190*250 mm, 5 um; mobile phase system: A: 0.1% trifluoroacetic acid aqueous solution; B: preparative grade acetonitrile; flow rate: 15 ml/min; B%=20%-100%; column temperature: room temperature) to give 4-(4-((1R,5S)-3,8-diazabicyclo[3.2.1]octan-3-yl)-8-fluoro-2-((1-methylenetetrahydro-1H-pyrrolizin-7a(5H)-yl)methoxy)pyrido[4,3-d]pyrimidin-7-yl)-5-ethynylnaphthalen-2-ol (Z25, bistrifluoroacetate, 7.7 mg, 0.0096 mmol, yield: 10%) as a brown solid. ES-API: [M+H]⁺= 577.1.

### Example 25: Synthesis of 4-(4-((1R,5S)-3,8-diazabicyclo[3.2.1]octan-3-yl)-8-fluoro-2-(((laR,6aR)-4-methylenehexahydrocyclopropa[b]pyrrolizin-5a(3H)-yl)methoxy)pyrido[4,3-d]pyrimidin-7-yl)-5-ethynylnaphthalen-2-ol (Z26)

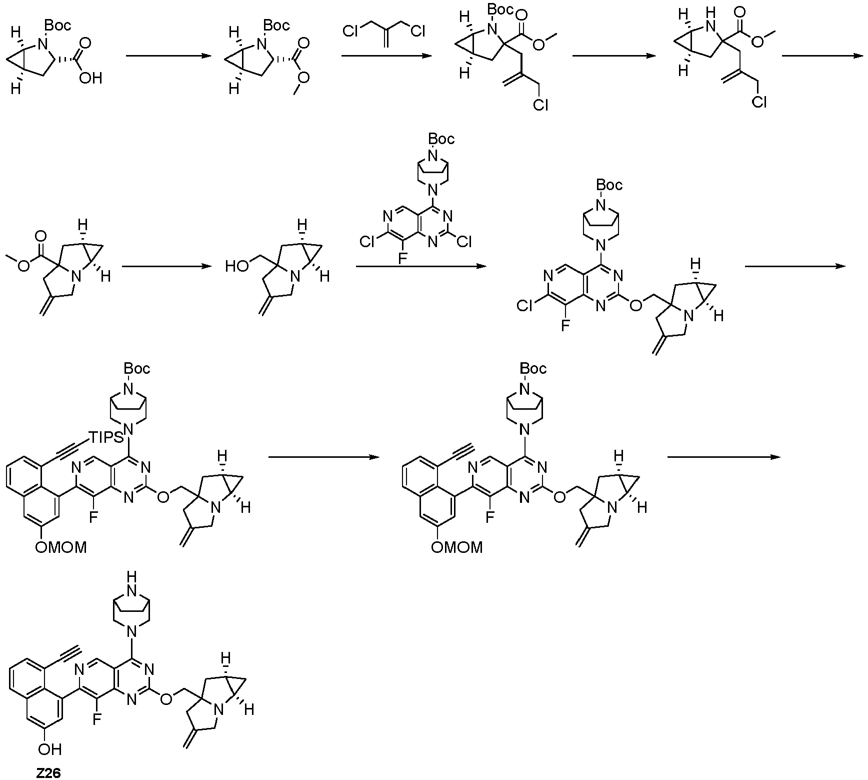

Step 1: potassium carbonate (1.7 g, 12.6 mmol) and iodomethane (1.79 g, 12.6 mmol) were added to a solution of (1R, 3S, 5R)-2-(tert-butoxycarbonyl)-2-azabicyclo[3.1.0]hexane -3-carboxylic acid (1.9 g, 8.4 mmol) in N, N-dimethylformamide (20 mL) under an ice-water bath condition, and the reaction mixture was stirred at room temperature for 1.5 h. The reaction was determined to be complete by LCMS, filtered, washed with ethyl acetate and the filtrate was concentrated under reduced pressure to give crude 2-(tert-butyl)3-methyl (1R, 3S, 5R)-2-azabicyclo[3.1.0]hexane - 2,3-dicarboxylate (2.04 g, yield: 100%). ES-API: [M-55]⁺= 186.1.

Step 2: 2-(tert-butyl)3-methyl (1R, 3S, 5R)-2-azabicyclo[3.1.0]hexane -2,3-dicarboxylate (2.04 g, 8.4 mmol) was dissolved in anhydrous tetrahydrofuran (50 mL) under nitrogen protection and the mixture was placed in a dry ice-acetone bath. Lithium bistrimethylsilylamide (13 mL, 13 mmol, 1 M in THF) was slowly added dropwise to the above solution, stirred at this temperature for 1 h, then a solution of 3-chloro-2-chloromethyl-propene (5.4 g, 43.5 mmol) in anhydrous tetrahydrofuran (20 mL) was slowly added via syringe to the above solution, and the reaction solution was stirred at -78°C for another 1 h. The reaction was determined to be complete by LCMS, quenched with saturated ammonium chloride solution (50 mL), extracted with ethyl acetate (50 mL X3), the organic phase was washed with saturated brine (50 mLX 3), dried over anhydrous sodium sulfate, filtered and concentrated. The crude was purified by flash silica gel column (petroleum ether: ethyl acetate = 15:1) to give 2-(tert-butyl)3-methyl (1R, 5R)-3-(2-(chloromethyl)allyl)-2-azabicyclo[3.1.0]hexane -2,3-dicarboxylate (2.7 g, yield: 97.6%). ES-API: [M-55]⁺= 274.1.

Step 3: 4 M hydrochloric acid-dioxane solution (15 mL) was slowly added to a solution of 2-(tert-butyl)3-methyl (1R, 5R)-3-(2-(chloromethyl)allyl)-2-azabicyclo[3.1.0]hexane -2,3-dicarboxylate (2.7 g, 8.2 mmol) in acetonitrile (10 mL) at room temperature. The reaction mixture was stirred at room temperature for 0.5 h. The reaction was determined to be complete by LCMS. The reaction solution was concentrated to dryness under reduced pressure to give methyl (1R,5R)-3-(2-(chloromethyl)allyl)-2-azabicyclo[3.1.0]hexane -3-carboxylate (1.88 g, yield: 100%). ES-API: [M+H]⁺= 230.2.

Step 4: sodium bicarbonate (4.8 g, 57 mmol) and potassium iodide (189 mg, 1.14 mmol) were added to a solution of methyl (1R, 5R)-3-(2-(chloromethyl)allyl)-2-azabicyclo[3.1.0]hexane -3-carboxylate (1.88 g, 8.2 mmol) in acetonitrile (20 mL) at room temperature and the reaction mixture was stirred at room temperature for 1.5 h. The reaction was detected by LCMS, filtered, the insoluble material was washed with acetonitrile, the filtrate was concentrated under reduced pressure, and the residue was purified by automated flash chromatography on silica gel (petroleum ether: ethyl acetate = 15:1) to give methyl (1aR,6aR)-4-methylenehexahydrocyclopropa[b]pyrrolizine-5a(3H)-carboxylate (1.41 g, Y: 89%). ES-API: [M+H]⁺= 194.2.

Step 5: under nitrogen protection, methyl (1aR,6aR)-4-methylene hexahydrocyclopropane[b]pyrrolizine-5a(3H)-carboxylate (1.41 g, 7.3 mmol) was dissolved in anhydrous tetrahydrofuran (20 mL) under nitrogen atmosphere, the mixture was placed under an ice-water bath condition, lithium tetrahydroaluminium (11 mL, 11 mmol, 1 M in THF) was slowly added dropwise to the above solution, and stirred at this temperature for 0.5 h. The reaction was determined to be complete by LCMS, quenched by adding sodium sulfate decahydrate (1.6 g), stirred at room temperature for 0.5 h, filtered, and the filtrate was concentrated under reduced pressure to give methyl (1aR,6aR)-4-methylenehexahydrocyclopropane[b]pyrrolizine-5a(3H)-methanol (1.2 g, yield: 100%). ES-API: [M+H]⁺= 166.3.

Step 6: a mixture of methyl (1aR,6aR)-4-methylenehexahydrocyclopropa[b]pyrrolidine-5a(3H)-methanol (231 mg, 1.4 mmol), tert-butyl (1R,5S)-3-(2,7-dichloro-8-fluoropyrido[4,3-d]pyrimidin-4-yl)-3,8-diazabicyclo[3.2.1]octane-8-carboxylate (300 mg, 0.7 mmol) and DIPEA (271 mg, 2.1 mmol) in dioxane (5 mL) was stirred at 1 10°C for 6 h. The reaction was determined to be complete by LCMS, quenched with saturated ammonium chloride solution (30 mL), extracted with ethyl acetate (20 mL X3), the organic phase was washed with saturated brine (15 mL X3), dried over anhydrous sodium sulfate, filtered and concentrated. The crude was purified by flash silica gel column (dichloromethane: methanol = 20:1) to give tert-butyl (1R,5S)-3-(7-chloro-8-fluoro-2-(((1aR,6aR)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (221 mg, yield: 57%). ES-API: [M+H]⁺= 557.2.

Step 7: under nitrogen protection, tert-butyl (1R,5S)-3-(7-chloro-8-fluoro-2-(((laR,6aR)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (221 mg, 0.40 mmol), triisopropyl ((6-(methoxymethoxy)-8-(4,4,5,5-tetramethyl-1,3,2-dioxoboran-2-yl)naphthalen-1-yl)ethynyl)silane (394 mg, 0.8 mmol), potassium phosphate (254mg, 1.2 mmol) and CataXiumAPdG3 (44 mg, 0.06 mmol) were dissolved in tetrahydrofuran (10 mL) and water (2 mL) and the reaction mixture was heated to 65°C and stirred for 3 h. The reaction was determined to be complete by LCMS, the reaction solution was concentrated under reduced pressure, and the crude was purified by flash silica gel column (dichloromethane: methanol = 15:1) to give tert-butyl (1R,5S)-3-(8-fluoro-7-(3-(methoxymethoxy)-8-((triisopropylsilyl)ethynyl)naphthalen-1-yl)-2-(((1 aR,6aR)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (331 mg, yield: 93%). ES-API: [M+H]⁺= 889.5.

Step 8: cesium fluoride (562 mg, 3.7 mmol) was added to tert-butyl (1R,5S)-3-(8-fluoro-7-(3-(methoxymethoxy)-8-((triisopropylsilyl)ethynyl)naphthalen-1-yl)-2-(((1aR,6aR)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (331 mg, 0.37 mmol) in N, N-dimethylformamide (5 mL), the reaction mixture was stirred at room temperature for 2 h. The reaction was determined to be complete by LCMS, and ethyl acetate (20 mL), water (15 mL X2), and saturated brine (15 mL X3) were added to the reaction solution. The obtained miture was dried over anhydrous sodium sulfate, filtered and concentrated to give tert-butyl (1R,5S)-3-(7-(8-ethynyl-3-(methoxymethoxy)naphthalen-1-yl)-8-fluoro-2-(((1aR,6aR)-4-methylenehexahydrocyclopropa[b]-pyrrolizin-5a(3H)-yl)methoxy)pyrido[4,3-d]pyrimidin -4-yl)-3.8-diazabicyclo[3.2.1]octane-8-carboxylate (271 mg, yield: 100%). ES-API: [M+H]⁺= 733.3.

Step 9: a 4 M hydrochloric acid-dioxane solution (5 mL) was slowly added to a solution of tert-butyl (1R,5S)-3-(7-(8-ethynyl-3-(methoxymethoxy)naphthalen-1-yl)-8-fluoro-2-(((1aR,6aR)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (271 mg, 0.37 mmol) in acetonitrile (5 mL) under an ice-water bath condition and the reaction mixture was stirred at 0°C for 0.5 h. The reaction was determined to be complete by LCMS, the reaction solution was concentrated to dryness under reduced pressure and the residue was prepared by high performance liquid chromatography (chromatographic column: Welch Xtimate, 21.2*150 mm, 5 um; mobile phase A: 5 mmol/L NH₄HCO₃ aqueous solution, mobile phase B: ACN; flow rate: 15 ml/min; chromatographic conditions: 15 ml-35-85-13 min; column temperature: room temprature) to give 4-(4-((1R,5S)-3,8-diazabicyclo[3.2.1]octan-3-yl)-8-fluoro-2-(((1aR,6aR)-4-methylenehexahydrocyclopropa[b]pyrrolizin-5a(3H)-yl)methoxy)pyrido[4,3-d]pyrimidin-7-yl)-5-ethynylnaphthalen-2-ol (Z26, 40 mg, yield: 18 %) as a pale yellow solid. ES-API: [M+H]⁺= 589.3. ¹H NMR (500 MHz, CD₃OD) δ 8.98 (s, 1H), 7.81 (d, *J* = 7.5 Hz, 1H), 7.50 (d, *J* = 7.5 Hz, 1H), 7.38 (d, *J* = 7.5 Hz, 1H), 7.32 (d, *J* = 2.5 Hz, 1H), 7.15 (d, *J* = 2.5 Hz, 1H), 4.97 (s, 2H), 4.62 (d, *J* = 12.5 Hz, 1H), 4.56 (d, *J* = 12.5 Hz, 1H), 4.24-4.12 (m, 2H), 3.80-3.78 (m, 1H), 3.75-3.65 (m, 4H), 3.61-3.56 (m, 1 H), 3.02 (d, *J* = 2.0 Hz, 1H), 2.79-2.74 (m, 1H), 2.67-2.65 (m, 1H), 2.64-2.61 (m, 1H), 2.21-2.19 (m, 1H), 2.04-2.01 (m, 2H), 1.90-1.84 (m, 2H), 1.81-1.76 (m, 2H), 1.70-1.58 (m, 1H), 1.28 (s, 1H), 0.76-0.74 (m, 1H), 0.52-0.49 (m, 1H).

### Example 26: Synthesis of 4-(4-((1R,5S)-3,8-diazabicyclo[3.2.1]octan-3-yl)-2-((2-(difluoromethylene) tetrahydro-1H-pyrrolizine 7a(5H)-yl)methoxy)-8-fluoropyrido[4,3-d]pyrimidin-7-yl)-5-ethynylnaphthalen-2-ol (Z27)

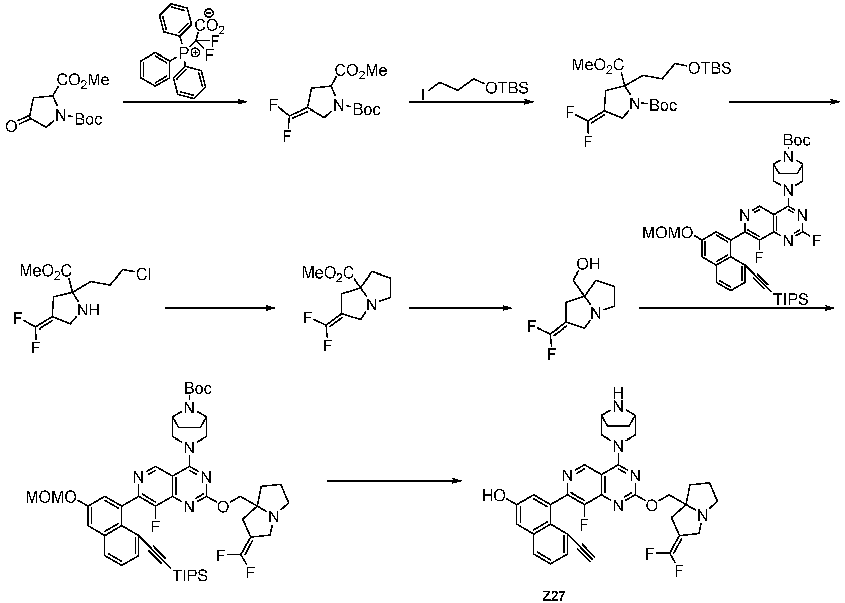

Step 1: a round bottom flask was charged with 1-(tert-butyl)2-methyl 4-oxopyrrolidine-1,2-dicarboxylic acid (2.6 g, 10.69 mmol), (triphenylphosphonium) difluoroacetic acid ylide (9.5 g, 26.75 mmol), and N, N-dimethylformamide (30 mL) under nitrogen protection and the system was stirred at 80°C for 6 h. To this was added water (100 mL) and extracted with ethyl acetate (50 mL X2). The organic phase was washed with saturated brine (50 mL X2), dried over anhydrous sodium sulfate, concentrated and the crude was purified by flash silica gel column (ethyl acetate/petroleum ether: 0-8%) to give 1-(tert-butyl)2-methyl 4-(difluoromethylene) pyrrolidine - 1,2-dicarboxylate (1.2 g, 40%) as a colorless oil. ES-API: [M+Na]⁺=300.1.

Step 2: a round bottom flask was charged with 1-(tert-butyl)2-methyl 4-(difluoromethylene) pyrrolidine-1,2-dicarboxylate (1.4 g, 5.05 mmol), tert-butyl (3-iodopropoxy) dimethylsilane (3.03 g, 10.11 mmol) and tetrahydrofuran (20 mL). It was cooled to -78°C under nitrogen protection. Potassium bis(trimethylsilyl)amide solution (10.11 mL, 10.11 mmol, 1 M in THF) was slowly added dropwise to the above reaction solution with stirring. The addition was complete; the reaction was stirred at -78°C for 30 min. The ice bath was removed and the reaction solution was poured into saturated ammonium chloride aqueous solution (100 mL) and extracted with ethyl acetate (50 mL X3). The organic phase was washed with saturated brine, dried over anhydrous sodium sulfate, and concentrated. The crude was purified by flash silica gel column (ethyl acetate/petroleum ether: 0-7%) to give 1-(tert-butyl) 2-methyl 2-(3-((tertbutyldimethylsilyl)oxy)propyl)-4-(difluoromethylene)pyrrolidine-1,2-dicarboxylate (900 mg, yield: 40%) as a colorless oil. ES-API: [M+Na]⁺=472.3.

Step 3: 1-(tert-butyl)2-methyl 2-(3-((tert-butyldimethylsilyl)oxy)propyl)-4-(difluoromethylene)pyrrolidine-1,2-dicarboxylate (200 mg, 0.44 mmol), dichloromethane (1 mL), 4 M hydrogen chloride/dioxane solution (0.6 mL) and thionyl chloride (3 mL) were added to a reaction flask successively under nitrogen protection. The reaction was stirred at 50°C for 4 h. The reaction solution was concentrated, dichloromethane (30 mL) was added and washed with saturated sodium bicarbonate aqueous solution (50 mL). The organic phase was dried over anhydrous sodium sulfate and concentrated to give methyl 2-(3-chloropropyl)-4-(difluoromethylene) pyrrolidine -2-carboxylate (111 mg, crude) as a yellow oil. ES-API: [M+H]⁺= 254.1.

Step 4: methyl 2-(3-chloropropyl)-4-(difluoromethylene)pyrrolidine-2-carboxylate (220 mg, crude), potassium carbonate (119 mg, 0.86 mmol), potassium iodide (143 mg, 0.86 mmol), and N, N-dimethylformamide (5 mL) were added to a reaction flask. The reaction was stirred at 85°C for 2 h. Water (50 mL) was added to the reaction solution, which was extracted with ethyl acetate (30 mL X3). The organic phase was washed with saturated brine, dried over anhydrous sodium sulfate, concentrated and the crude was purified by flash silica gel column (ethyl acetate/petroleum ether: 0-40%) to give methyl 2-(difluoromethylene) tetrahydro-1H-pyrrolizine-7a(5H)-carboxylate (120 mg, yield over 2 steps: 64%). ES-API: [M+H]⁺= 218.1.

Step 5: under nitrogen protection, a round bottom flask was charged with methyl 2-(difluoromethylene) tetrahydro-1H-pyrrolizine-7a(5H)-carboxylate (100 mg, 0.46 mmol), and tetrahydrofuran (15 mL). It was cooled to 0°C, and a solution of lithium borohydride (0.46 mL, 0.92 mmol, 2 M in THF) was added dropwise to the above solution. The addition was complete, the reaction was stirred at 0°C for 30 min. 4 M hydrochloric acid (1 mL) was added dropwise thereto and stirred at room temperature for 30 min. 2 M sodium hydroxide aqueous solution (5 mL) and water (20 mL) were added. It was extracted with dichloromethane/methanol (10:1, 30 mL X3). The organic phase was dried over anhydrous sodium sulfate and concentrated to give (2-(difluoromethylene) tetrahydro-1H-pyrrolizine 7a(5H)-yl) methanol (40 mg, yield: 46%) as a yellow oil. ES-API: [M+H]⁺= 190.1.

Step 6: a round bottom flask was charged with sodium hydride (35 mg, 0.87 mmol) and tetrahydrofuran (2 mL). Cooled to 0°C under nitrogen protection. To the above reaction solution was added dropwise a solution of (2-(difluoromethylene) tetrahydro-1H-pyrrolizine 7a(5H)-yl) methanol (40 mg, crude) in tetrahydrofuran (1 mL) with stirring. The reaction was stirred at room temperature for 30 min. After cooling to 0°C, tert-butyl (1R,5S)-3-(2,8-difluoro-7-(3-(methoxymethoxy)-8-((triisopropylsilyl)ethynyl)naphthalen-l-yl)pyrido[4,3-d]pyrimidin-4-yl)-3,8-diazabicyclo[3.2.1]octane-8-carboxylate (130 mg, 0.17 mmol) was added to the above reaction solution and stirred at room temperature for 1 h. After cooling to 0°C, saturated ammonium chloride aqueous solution (30 mL) was added to the reaction solution and extracted with ethyl acetate (20 mL X3). The organic phase was washed with saturated brine, dried over anhydrous sodium sulfate, concentrated and the crude was purified by flash silica gel column (ethyl acetate/petroleum ether: 0-50%) to give tert-butyl (1R,5S)-3-(2-((2-(difluoromethylene) tetrahydro-1H-pyrrolizine 7a(5H)-yl)methoxy -8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (100 mg, yield: 62%) as a yellow solid. ES-API: [M+H]⁺= 913.3.

Step 7: a round bottom flask was charged with tert-butyl (1R,5S)-3-(2-((2-(difluoromethylene) tetrahydro-1H-pyrrolizine 7a(5H)-yl)methoxy -8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (100 mg, 0.11 mmol), cesium fluoride (17 mg, 0.11 mmol), and anhydrous N, N-dimethylformamide (3 mL). The reaction was stirred at room temperature for 1 hour. Water (30 mL) was added to the reaction solution, which was extracted with ethyl product acetate (30 mL X3), and the organic phase was dried over anhydrous sodium sulfate and concentrated. The concentrate was dissolved in acetonitrile (2 mL), the solution was cooled to 0°C and 4 M hydrogen chloride/dioxane solution (0.53 mL, 2.12 mmol) was added. Stirred at 0°C for 0.5 h. The reaction solution was concentrated and the crude was prepared by high performance liquid chromatography (alkaline method) to give 4-(4-((1R,5S)-3,8-diazabicyclo[3.2.1]octan-3-yl)-2-((2-(difluoromethylene) tetrahydro-1H-pyrrolizine 7a(5H)-yl)methoxy)-8-fluoropyrido[4,3-d]pyrimidin-7-yl)-5-ethynylnaphthalen-2-ol (Z27, 19 mg, yield: 29%) as a yellow solid. ES-API: [M+H]⁺=613.3. ¹HNMR: (500MHz, CD₃OD) 8.99 (s, 1H), 7.82 (d, *J*=7.5 Hz, 1H), 7.82 (dd, *J*=7 Hz, 1.0 Hz, 1H), 7.40-7.37 (m, 1H), 7.32 (d, *J*=2.5 Hz, 1H), 7.15 (d, *J*=2.5 Hz, 1H), 4.64-4.56 (m, 2H), 4.37-4.33 (m, 1H), 4.28-4.24 (m, 1H), 3.80-3.77 (m, 1H), 3.73-3.65 (m, 4H), 3.43-3.41 (m, 1H), 3.16-3.11 (m, 1H), 3.01 (s, 1H), 2.81-2.77 (m, 1H), 2.72-2.67(m, 1H), 2.52-2.49 (m, 1H), 2.12-2.11 (m, 1H), 2.00-1.17 (m, 7H).

### Example 27: Synthesis of 3-(4-((1R,5S)-3,8-diazabicyclo[3.2.1]octan-3-yl)-6,8-difluoro-2-((2-methylenetetrahydro-1H-pyrrolizin-7a(5H)-yl)methoxy)quinazolin-7-yl)-4-fluorophenol (Z189)

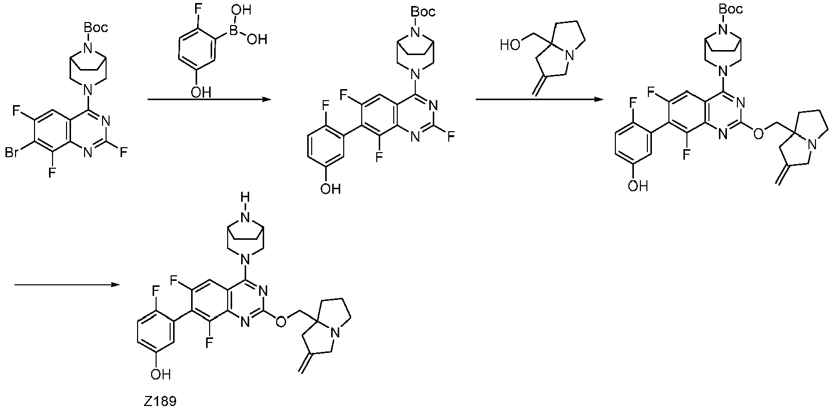

Step 1: tert-butyl (1R,5S)-3-(7-bromo-2,6,8-trifluoroquinazolin-4-yl)-3,8-diazabicyclo[3.2.1]octane-8-carboxylate (120 mg, 0.2536 mmol) was dissolved in dioxane/water (6 mL/1.0 mL), (2-fluoro-5-hydroxyphenyl)boronic acid (80 mg, 0.513 mmol), potassium phosphate (215 mg, 1.014 mmol), chloro (2-dicyclohexylphosphino-2', 6'-dimethoxy-1,1'-biphenyl)(2'-amino-1,1'-biphenyl-2-yl)palladium (II) (20.0 mg, 0.028 mmol) and 2-dicyclohexylphosphine-2', 6'-dimethoxybiphenyl (40.0 mg, 0.097 mmol) were added, replaced 4 times with nitrogen, and the system reacted under microwave condition at 110°C for 1 h. The reaction solution was cooled to room temperature, extracted with ethyl acetate (80 mL) and water (60 mL), the organic phase was dried over anhydrous sodium sulfate, filtered, the filtrate was rotary dried under reduced pressure, and the crude was purified by silica gel column chromatography (ethyl acetate/petroleum ether: 0-70%) to give the product tert-butyl (1R,5S)-3-(2,6,8-trifluoro-7-(2-fluoro-5-hydroxyphenyl)quinazolin-4-yl)-3,8-diazabicyclo[3.2.1]octane-8-carboxylate (180 mg, crude). ES-API: [M+1]⁺=505.1.

Step 2: sodium hydride (21 mg, 0.526 mmol) was added to a solution of (2-methylenetetrahydro-1H-pyrrolizin-7a(5H)-yl) methanol (50 mg, 0.3947 mmol) in tetrahydrofuran (3.0 mL) under an ice-water bath condition, and the system reacted at room temperature for 0.5 h. After 0.5 h, tert-butyl (1R,5S)-3-(2,6,8-trifluoro-7-(2-fluoro-5-hydroxyphenyl)quinazolin-4-yl)-3,8-diazabicyclo[3.2.1]octane-8-carboxylate (180 mg, crude) was added to the above reaction system. The system reacted at room temperature for 1-2 h. Upon completion of the reaction, ice water (50 mL) was added to the system, which was extracted with dichloromethane (50 mL X2), the dichloromethane phases were combined, dried over anhydrous sodium sulfate, filtered, the filtrate was rotary dried under reduced pressure, and the crude was purified by silica gel column chromatography (methanol/dichloromethane: 0-10%) to give tert-butyl (1R,5S)-3-(6,8-difluoro-7-(2-fluoro-5-hydroxyphenyl)-2-((2-methylenetetrahydro-1H-pyrrolizin-7a(5H)-yl)methoxy)quinazolin-4-yl)-3,8-diazabicyclo[3.2.1]octane-8-carboxylate (300 mg, crude). ES-API: [M+1]⁺=638.3.

Step 3: tert-butyl (1R,5S)-3-(6,8-difluoro-7-(2-fluoro-5-hydroxyphenyl)-2-((2-methylenetetrahydro-1H-pyrrolizin-7a(5H)-yl)methoxy)quinazolin-4-yl)-3,8-diazabicyclo[3.2.1]octane-8-carboxylate (300 mg, crude) was dissolved in acetonitrile (10.0 mL), 4 M hydrochloric acid/dioxane solution (2.0 mL, 8.0 mmol) was added in an ice bath, and the system reacted for 0.5 h in an ice bath. Upon completion of the reaction, the solvent was rotary dried under reduced pressure, dichloromethane (20 mL) was added, and triethylamine (3.0 mL) was added under an ice-water bath condition. After stirring for 10 min, dichloromethane (100 mL) and water (50 mL) were added for extraction. The dichloromethane phase was dried over anhydrous sodium sulfate, filtered and the filtrate was rotary dried under reduced pressure to give the crude. High performance liquid phase preparation (ammonium bicarbonate method) was performed to give the target compound 3-(4-((1R,5S)-3,8-diazabicyclo[3.2.1]octan-3-yl)-6,8-difluoro-2-((2-methylenetetrahydro-1H-pyrrolizin-7a(5H)-yl)methoxy)quinazolin-7-yl)-4-fluorophenol (Z189, 13.0 mg, total yield over 3 steps: 9.3%). ES-API: [M+1]⁺=538.3.

### Example 28: Synthesis of 4-(4-(1R,5S)-3,8-diazabicyclo[3.2.1]octan-3-yl)-8-fluoro-2-((2-methylenetetrahydro-1H-pyrrolizin-7a(5H)-yl)methoxy)quinazolin-7-yl)-5-ethynylnaphthalen-2-ol (Z99)

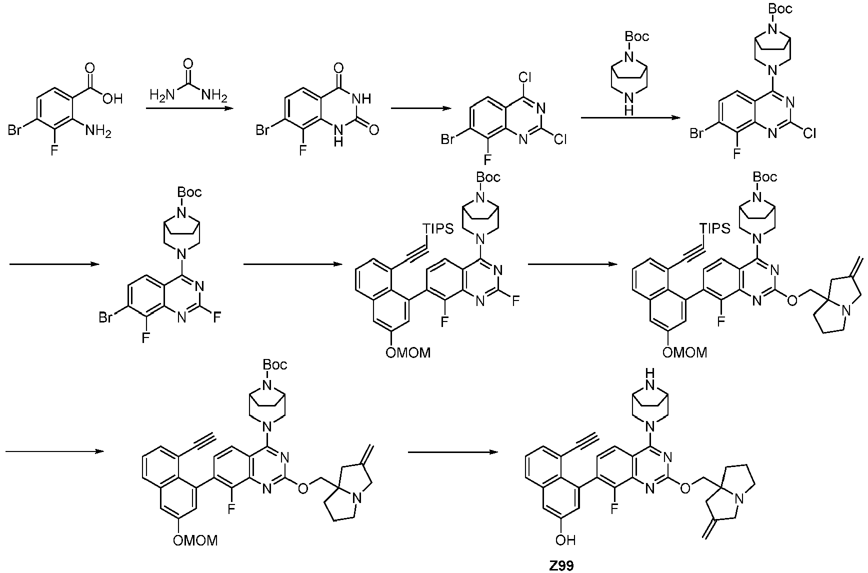

Step 1: a one-neck flask was charged with 2-amino-4-bromo-3-fluorobenzoic acid (3.0 g, 12.819 mmol) and urea (40.0 g, 666.0 mmol) and allowed to react under nitrogen protection at 200°C for 1 h. Upon completion of the reaction, the temperature was lowered to 80°C, water (100 mL) was added, and filtered, the filter cake was washed with water (60 mL X2), and the solvent was rotary dried to obtain the final product 7-bromo-8-fluoroquinazoline-2,4(1H,3H)-dione (3.3 g, yield: 99.38%). ES-API: [M+H]⁺=259.0/261.0.

Step 2: phosphorus oxychloride (2.0 g, 13.0 mmol) followed by diisopropylethyl amine (1.68 g, 13.0 mmol) was added to a solution of 7-bromo-8-fluoroquinazoline-2,4(1H,3H)-dione (0.50 g, 1.93 mmol) in toluene (15 mL), and the system reacted at 120°C for 2 h under an ice-water bath condition. Upon completion of the reaction, the solvent was rotary dried under reduced pressure, water (20 mL) was added to the reaction solution, which was extracted with dichloromethane (80 mL X2), the organic phase was dried over anhydrous sodium sulfate, filtered, and the filtrate was rotary dried under reduced pressure to give 7-bromo-2,4-dichloro-8-fluoroquinazoline (0.40 g, yield: 70%). ES-API: [M+H]⁺=294.9.

Step 3: the compound 7-bromo-2,4-dichloro-8-fluoroquinazoline (0.40 g, 1.352 mmol) was dissolved in dichloromethane (20.0 mL) and diisopropyl ethylamine (1.68 g, 13.0 mmol) was added. After stirring for 5 min, tert-butyl (1R,5S)-3,8-diazabicyclo[3.2.1]octane-8-carboxylate (400 mg, 1.884 mmol) was added, and the system reacted at room temperature for 5 h. After the completion of the reaction, saturated brine (50 mL) was added to the reaction solution, which was extracted with ethyl acetate (100 mL X2). Ethyl acetate phase was combined, dried over anhydrous sodium sulfate, rotary dried under reduced pressure, and the crude was purified by silica gel column chromatography (ethyl acetate/petroleum ether: 0-50%) to give the target product tert-butyl (1R,5S)-3-(7-bromo-2-chloro-8-fluoroquinazolin-4-yl)-3,8-diazabicyclo[3.2.1]octane-8-carboxylate (300 mg, yield: 47%). ES-API: [M+H]⁺=471.1/473.1.

Step 4: tert-butyl (1R,5S)-3-(7-bromo-2-chloro-8-fluoroquinazolin-4-yl)-3,8-diazabicyclo[3.2.1]octane-8-carboxylate (300.0 mg, 0.636 mmol) was dissolved in dimethylsulfoxide (5.0 mL), finally potassium fluoride (2.05 g, 35.35 mmol) was added, and the system reacted at 120°C for 8 h under nitrogen protection. Upon completion of the reaction, the reaction solution was cooled to room temperature, saturated ethyl acetate (100 mL) was added, and extracted with saturated brine (100 mL X3). The ethyl acetate phase was dried over anhydrous sodium sulfate. After rotary dried under reduced pressure, the crude was purified by silica gel column chromatography (ethyl acetate/petroleum ether: 0-50%) to give the target product (1R,5S)-tert-butyl 3-(7-bromo-2,8-difluoroquinazolin-4-yl)-3,8-diazabicyclo[3.2.1]octane-8-carboxylate (270 mg, yield: 82%). ES-API: [M+H]⁺=455.1/457.1.

Step 5: tert-butyl (1R,5S)-3-(7-bromo-2,8-difluoroquinazolin-4-yl)-3,8-diazabicyclo[3.2.1]octane-8-carboxylate (270 mg, 0.522 mmol) was dissolved in tetrahydrofuran/water (12 mL/2 mL) and triisopropyl ((6-(methoxymethoxy)-8-(4,4,5,5-tetramethyl-1,3,2-dioxaborolan-2-yl)naphthalen-1-yl)ethynyl)silane (600 mg, 1.213 mmol), potassium phosphate (600 mg, 2.830 mmol) and[n-butyldi (1-adamantyl)phosphino](2-amino-1,1'-biphenyl-2-yl)palladium (II) methanesulfonate (80.0 mg, 0.110 mmol), nitrogen were added and displaced with nitrogen for 4 times, reacted under microwave condition at 80°C for 1 h. The reaction solution was cooled to room temperature, extracted with ethyl acetate (80 mL) and water (60 mL), the organic phase was dried over anhydrous sodium sulfate, filtered, the filtrate was rotary dried under reduced pressure, and the crude was purified by silica gel column chromatography (ethyl acetate/petroleum ether: 0-70%) to give the product tert-butyl (1R,5S)-3-(2,8-difluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate (412 mg, crude). ES-API: [M+1]⁺=744.3.

Step 6: sodium hydride (50 mg, 1.25 mmol) was added to a solution of (2-methylenetetrahydro-1H-pyrrolizin-7a(5H)-yl) methanol (82 mg, 0.535 mmol) in tetrahydrofuran (3.0 mL) under an ice-water bath condition, and the system reacted at room temperature for 0.5 h. Tert-butyl (1R,5S)-3-(2,8-difluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate (200 mg, 0.269 mmol) was added to the above reaction system and allowed to react at room temperature for 1-2 h. Upon completion of the reaction, ice water (50 mL) was added to the system, which was extracted with dichloromethane (50 mL X2), the dichloromethane phases were combined, dried over anhydrous sodium sulfate, filtered, the filtrate was rotary dried under reduced pressure, and the crude was purified by silica gel column chromatography (methanol/dichloromethane: 0-10%) to give tert-butyl (1R,5S)-3-(8-fluoro-7-(3-(methoxymethoxy)-8-((triisopropylsilyl)ethynyl)naphthalen-1-yl)-2-((2-methylenetetrahydro-1H-pyrrolizin-7a(5H)-yl)methoxy)quinazolin-4-yl)-3,8-diazabicyclo[3.2.1]octane-8-carboxylate (300 mg, yield: 82.68%). ES-API: [M/2+1]⁺=876.4.

Step 7: tert-butyl (1R,5S)-3-(8-fluoro-7-(3-(methoxymethoxy)-8-((triisopropylsilyl)ethynyl)naphthalen-1-yl)-2-((2-methylenetetrahydro-1H-pyrrolizin-7a(5H)-yl)methoxy)quinazolin-4-yl)-3,8-diazabicyclo[3.2.1]octane-8-carboxylate (300 mg, 0.223 mmol) was dissolved in N, N-dimethylformamide (5.0 mL). Cesium fluoride (500 mg, 3.292 mmol) was added, and the system reacted at room temperature for 2 h. Upon completion of the reaction, the reaction solution was extracted with ethyl acetate (80 mL) and water (60 mL), the organic phase was dried over anhydrous sodium sulfate, filtered, and the filtrate was rotary dried under reduced pressure to give tert-butyl (1R,5S)-3-(7-(8-ethynyl-3-(methoxymethoxy)naphthalen-1-yl)-8-fluoro-2-((2-methylenetetrahydro-1H-pyrrolizin-7a(5H)-yl)methoxy)quinazolin-4-yl)-3,8-diazabicyclo[3.2.1]octane-8-carboxylate (200 mg, yield: 74.9%). ES-API: [M+1]⁺=720.4.

Step 8: tert-butyl (1R,5S)-3-(7-(8-ethynyl-3-(methoxymethoxy)naphthalen-1-yl)-8-fluoro-2-((2-methylenetetrahydro-1H-pyrrolizin-7a(5H)-yl)methoxy)quinazolin-4-yl)-3,8-diazabicyclo[3.2.1]octane-8-carboxylate (200 mg, 0.167 mmol) was dissolved in acetonitrile (5.0 mL), 4 M hydrochloric acid/dioxane solution (2.0 mL, 4 M, 8.0 mmol) was added in an ice bath, and the system reacted for 0.5 h in an ice bath. Upon completion of the reaction, the solvent was rotary dried under reduced pressure, dichloromethane (20 mL) was added, and triethylamine (3.0 mL) was added under an ice-water bath condition. After stirring for 10 min, dichloromethane (100 mL) and water (50 mL) were added for extraction. The dichloromethane phase was dried over anhydrous sodium sulfate, filtered and the filtrate was rotary dried under reduced pressure to give the crude, which was purified by high performance liquid alkaline method preparation to give the target compound 4-(4-(1R,5S)-3,8-diazabicyclo[3.2.1]octan-3-yl)-8-fluoro-2-((2-methylenetetrahydro-1H-pyrrolizin-7a(5H)-yl)methoxy)quinazolin-7-yl)-5-ethynylnaphthalen-2-ol (Z99, 13.78 mg, yield: 14.3%). ES-API: [M+1]⁺=576.3. ¹H NMR (500 MHz, CD₃OD) δ 8.55 (s, 1H), 7.76 (dd, *J* = 20.3, 8.4 Hz, 2H), 7.47 (d, *J* = 6.9 Hz, 1H), 7.41 - 7.33 (m, 1H), 7.28 - 7.19 (m, 2H), 7.03 (d, *J* = 2.4 Hz, 1H), 4.99 (s, 2H), 4.49 (d, *J* = 12.2 Hz, 2H), 4.34 - 4.20 (m, 2H), 3.76 (d, *J* = 14.6 Hz, 1H), 3.64 - 3.55 (m, 4H), 3.24 - 3.13 (m, 1H), 2.79 (ddd, *J* = 24.0, 20.2, 5.5 Hz, 3H), 2.49 (d, *J* = 15.4 Hz, 1H), 2.16 (dd, *J* = 11.7, 5.9 Hz, 1H), 2.05 - 1.76 (m, 8H).

### Example 29: Synthesis of 4-((1R,5S)-3,8-diazabicyclo[3.2.1]octan-3-yl)-2-((2,6-dimethylenetetrahydro-1H-pyrrolizin-7a(5H)-yl)methoxy)-7-(8-ethynylnaphthalen-1-yl)-8-fluoropyrido[4,3-d]pyrimidine (Z190)

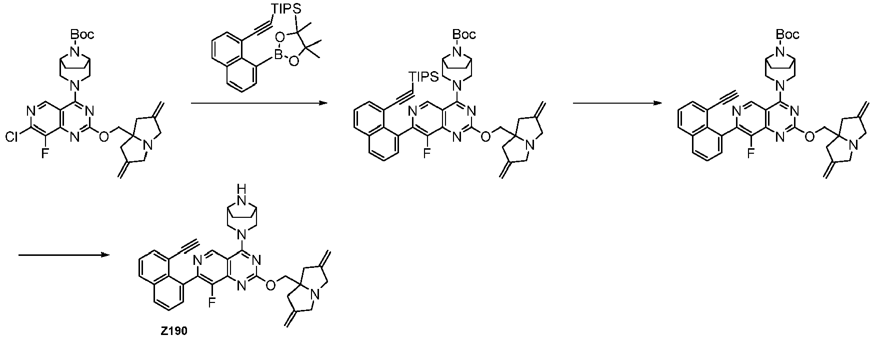

Step 1: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (192 mg, 0.345 mmol), triisopropyl ((8-(4,4,5,5-tetramethyl-1,3,2-dioxoboran-2-yl)naphthalen-1-yl)ethynyl)silane (300 mg, 0.690 mmol), potassium phosphate (220 mg, 1.036 mmol) and[n-butyldi (1-adamantyl)phosphino](2-amino-1,1'-biphenyl-2-yl)palladium (II) methanesulfonate (25 mg, 0.034 mmol) were dissolved in tetrahydrofuran (8 mL) and water (2 mL) and the reaction mixture was heated to 65°C with stirring for 5 h. The reaction was determined to be complete by LCMS and the reaction solution was concentrated under reduced pressure to give the crude which was purified by silica gel column chromatography (dichloromethane: methanol = 0-3.3%) to give tert-butyl (1R,5S)-3-(2-((2,6-dimethylenetetrahydro-1H-pyrrolizin-7a(5H)-yl)methoxy)-8-fluoro-7-(8-((triisopropylsilyl)ethynyl)naphthalen-1-yl)pyrido[4,3-d]pyrimidin-4-yl)-3,8-diazabicyclo[3.2.1]octane-8-carboxylate (222 mg, yield: 78%). ES-API: [M+H]⁺= 829.5.

Step 2: cesium fluoride (407 mg, 2.679 mmol) was added to tert-butyl (1R,5S)-3-(2-((2,6-dimethylenetetrahydro-1H-pyrrolizin-7a(5H)-yl)methoxy)-8-fluoro-7-(8-((triisopropylsilyl)ethynyl)naphthalen-1-yl)pyrido[4,3-d]pyrimidin-4-yl)-3,8-diazabicyclo[3.2.1]octane-8-carboxylate (222 mg, 0.268 mmol) in the N, N-dimethylformamide (10 mL) and the reaction mixture was stirred at room temperature for 2 h. The reaction was determined to be complete by LCMS, the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(2-((2,6-dimethylenetetrahydro-1H-pyrrolizin-7a(5H)-yl)methoxy)-7-(8-ethynylnaphthalen-1-yl)-8-fluoropyrido[4,3-d]pyrimidin-4-yl)-3,8-diazabicyclo[3.2.1]octane-8-carboxylate (180 mg, yield: 99%). ES-API: [M+H]⁺= 673.3. Step 3: a 4 M hydrochloric acid-dioxane solution (10 mL) was slowly added to a solution of tert-butyl (1R,5S)-3-(2-((2,6-dimethylenetetrahydro-1H-pyrrolizin-7a(5H)-yl)methoxy)-7-(8-ethynylnaphthalen-1-yl)-8-fluoropyrido[4,3-d]pyrimidin-4-yl)-3,8-diazabicyclo[3.2.1]octane-8-carboxylate (180 mg, 0.268 mmol) in acetonitrile (5 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 mL/min; chromatographic conditions: 15 mL-35-85-13 min; column temperature: room temperature) to give 4-((1R,5S)-3,8-diazabicyclo[3.2.1]octan-3-yl)-2-((2,6-dimethylenetetrahydro-1H-pyrrolizin-7a(5H)-yl)methoxy)-7-(8-ethynylnaphthalen-1-yl)-8-fluoropyrido[4,3-d]pyrimidine (Z190, 48.23 mg, yield: 31%). ES-API: [M+H]⁺= 573.3. ¹H NMR (500 MHz, DMSO-d₆) δ 9.05 (s, 1 H), 8.16- 8.12 (m, 2 H), 7.73- 7.68 (m, 2 H), 7.59- 7.55 (m, 2 H), 4.94 (d, J= 11.0 Hz, 4 H), 4.49- 4.47 (m, 1 H), 4.32- 4.29 (m, 1 H), 4.06 (s, 2 H), 3.68 (s, 1 H), 3.65- 3.55 (m, 6 H), 3.22- 3.19 (m, 2 H), 2.64- 2.61 (m, 2 H), 2.50- 2.45 (m, 2 H), 1.66 (s, 4 H).

### Example 30: Synthesis of 4-(4-((1R,5S)-3,8-diazabicyclo[3.2.1]octan-3-yl)-8-fluoro-2-((6'-methylenetetrahydrospiro[cyclopropane-1,1'-pyrrolizin]-7a'(5'H)-yl)methoxy)pyrido[4,3-d]pyrimidin-7-yl)-5-ethynylnaphthalen-2-ol (Z106)

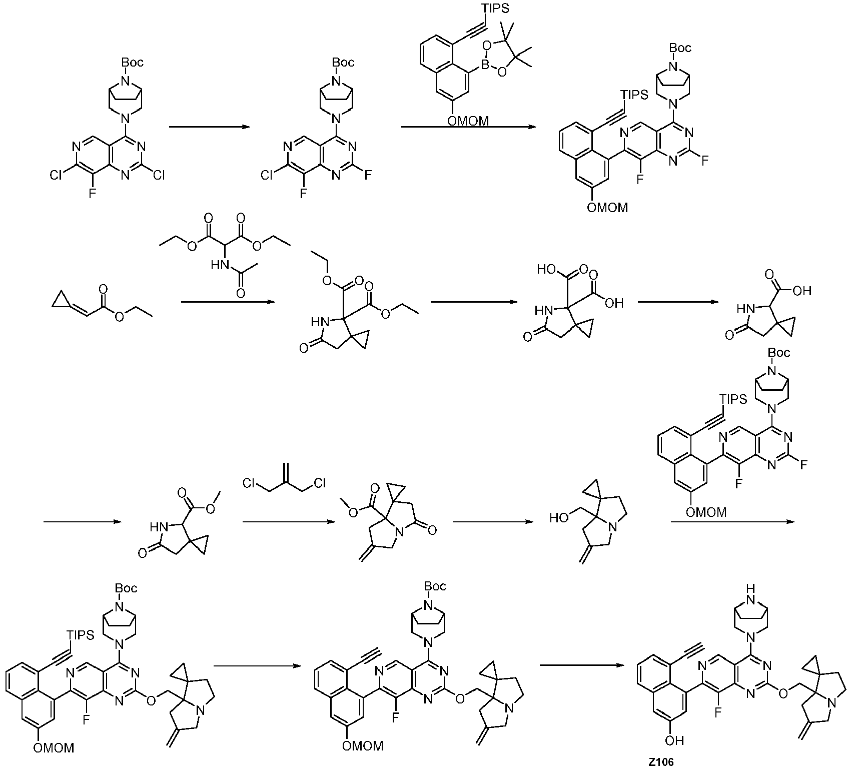

Step 1: tert-butyl (1R,5S)-3-(2,7-dichloro-8-fluoropyrido[4,3-d]pyrimidin-4-yl)-3,8-diazacyclo[3.2.1]octane-8-carboxylate (856 mg, 2.0 mmol) was dissolved in anhydrous dimethylsulfoxide (15 mL) and potassium fluoride (581 mg, 10.0 mmol) was added and the reaction was heated at 120°C under nitrogen protection for 4 h. Diluted with ethyl acetate (200 ml), washed with water (15 mL X2), saturated sodium chloride (20 mL), dried over anhydrous sodium sulfate and concentrated to give the crude which was purified by silica gel column chromatography (petroleum ether/ethyl acetate = 3:1) to give tert-butyl (1R,5S)-3-(7-chloro-2,8-difluoropyrido[4,3-d]pyrimidin-4-yl)-3,8-diazacyclo[3.2.1]octane-8-carboxylate (120 mg, yield: 14.6%).

Step 2: tert-butyl (1R,5S)-3-(7-chloro-2,8-difluoropyrido[4,3-d]pyrimidin-4-yl)-3,8-diazacyclo[3.2.1]potassium phosphate (123 mg, 0.58 mmol), [n-butyl di(1-adamantyl)phosphine](2-amino-1,1'-biphenyl-2-yl)palladium (II) methanesulfonate (24 mg) was added to octane-8-carboxylate (120.0 mg, 0.29 mmol) and triisopropyl ((6-(methoxymethoxy)-8-(4,4,5,5-tetramethyl-1,3,2-dioxaborolan-2-yl)naphthalen-1-yl)ethynyl)silane (216 mg, 0.44 mmol) in tetrahydrofuran (6 mL) and water (1.0 mL). The mixture was nitrogen sparged and reacted at 60°C for 40 min. Upon completion of the reaction, ethyl acetate (200 mL) was added for dilution, washed successively with water (10 mL) and saturated brine (10 mL), dried over anhydrous sodium sulfate, filtered, and the filtrate was concentrated under reduced pressure. The crude was purified by silica gel column chromatography (0-4% methanol/dichloromethane) to give tert-butyl (1R,5S)-3-(2,8-difluoro-7-(3-(methoxymethoxy)-8-((triisopropylsilyl)ethynyl)naphthalen-1-yl)pyrido[4,3-d]pyrimidin-4-yl)-3,8-diazacyclo[3.2.1]octane-8-carboxylate (150 mg, yield: 69.4%). Step 3: ethyl 2-cyclopropylideneacetate (1261 mg, 10.0 mmol) and diethyl 2-acetamidomalonate (1671 mg, 7.69 mmol) were dissolved in ethanol (20 mL), freshly prepared sodium ethoxide (157 mg, 2.31 mmol) was added, and the system reacted under nitrogen protection at 80°C for 3 h. Upon completion of the reaction, the reaction solution was concentrated, ethyl acetate (200 mL) was added, washed with water (20 ml X2), saturated sodium chloride (20 mL), dried over anhydrous sodium sulfate, concentrated, and the crude was purified by silica gel column chromatography (10-50% petroleum ether/ethyl acetate) to give diethyl 6-oxo-5-azaspiro[2.4]heptane-4,4-dicarboxylate (1099 mg, yield: 56%).

Step 4: diethyl 6-oxo-5-azaspiro[2.4]heptane-4,4-dicarboxylate (1099 mg, 4.30 mmol) was dissolved in methanol (20 mL) and 10% sodium hydroxide (5.0 mL) was added, and the system reacted at 60°C for 1 h. The reaction solution was concentrated, diluted with water (20 mL), adjusted to pH 4-5 with 2N hydrochloric acid, precipitated solid, filtered, and dried to give 6-oxo-5-azaspiro[2.4]heptane-4,4-dicarboxylic acid (770 mg, yield: 90.0%).

Step 5: solid 6-oxo-5-azaspiro[2.4]heptane-4,4-dicarboxylic acid (770 mg, 3.87 mmoL) was directly heated to 120°C for 1 h, and the reaction was completed to directly obtain crude 6-oxo-5-azaspiro[2.4]heptane-4-carboxylic acid (550 mg, yield: 91.7%).

Step 6: 6-oxo-5-azaspiro[2.4]heptane-4-carboxylic acid (550 mg, 3.55 mmol) was dissolved in methanol (20 mL) and concentrated sulfuric acid (0.2 mL) was added, and the system reacted for 16 h at 60°C. Upon completion of the reaction, the reaction solution was concentrated, ethyl acetate (200 mL) was added, washed with saturated sodium bicarbonate (20 mL X2) and saturated sodium chloride (10 mL), dried over anhydrous sodium sulfate, and rotary dried. The crude was purified by silica gel column chromatography (10-70% petroleum ether/ethyl acetate) to give methyl 6-oxo-5-azaspiro[2.4]heptane-4-carboxylate (500 mg, yield: 83.3%).

Step 7: methyl 6-oxo-5-azaspiro[2.4]heptane-4-carboxylate (500 mg, 2.96 mmol) and 3-chloro-2-(chloromethyl)prop-1-ene (592 mg, 4.74 mmol) were dissolved in tetrahydrofuran (20 mL). Lithium bistrimethylsilylamide (6.0 mL, 1.0 M tetrahydrofuran) was added at -40°C and allowed to warm to room temperature to react for 16 h. The reaction was quenched by adding ammonium chloride solution (40 mL), extracted with ethyl acetate (50 ml x2), the organic phase was washed with brine, dried over anhydrous sodium sulfate, and concentrated to give the crude, which was purified by silica gel column chromatography (petroleum ether/ethyl acetate = 1/1) to give the product methyl 6'-methylene-3'-oxotetrahydrospiro[cyclopropane-1,1'-pyrrolizine]-7a' (5'H)-carboxylate (207 mg, yield: 31.56%).

Step 8: methyl 6'-methylene-3'-oxotetrahydrospiro[cyclopropane-1,1'-pyrrolizine]-7a' (5'H)-carboxylate (207mg, 0.93 mmol) was dissolved in tetrahydrofuran (10 mL), 1 M lithium aluminum tetrahydride (4.5 mL) was added dropwise in an ice bath and heated to 70°C to react for 3 h. Water (0.2 mL) was added to the reaction solution. The reaction was quenched with 15% sodium hydroxide aqueous solution (0.2 mL), water (0.6 mL), filtered and concentrated to give the crude product (6'-methylenetrahydrospiro[cyclopropane-1,1'-pyrrolizin]-7a'(5'H)-yl) methanol (130 mg, yield: 78.3%).

Step 9: (6'-methylenetetrahydrospiro[cyclopropane-1,1'-pyrrolizin]-7a'(5'H)-yl) methanol (70 mg, 0.39 mmol) was dissolved in anhydrous tetrahydrofuran (10 mL) and 60% sodium hydride (47 mg, 1.17 mmol) was added in an ice bath, and the system reacted at room temperature for 30 min. Tert-butyl (1R,5S)-3-(2,8-difluoro-7-(3-(methoxymethoxy)-8-((triisopropylsilyl)ethynyl)naphthalen-1-yl)pyrido[4,3-d]pyrimidin-4-yl)-3,8-diazacyclo[3.2.1]octane-8-carboxylate (150 mg, 0.20 mmol) was added, and the system reacted at room temperature for 40 min. The reaction was quenched by the addition of saturated ammonium chloride (40 ml), extracted with ethyl acetate, dried over anhydrous sodium sulfate and concentrated to give the crude which was purified by silica gel column chromatography (dichloromethane/methanol = 20:1) to give tert-butyl (1R,5S)-3-(8-fluoro-7-(3-(methoxymethoxy)-8-((triisopropylsilyl)ethynyl)naphthalen-1-yl)-2-((6'-methylenetetrahydrospiro[cyclopropane-1,1'-pyrrolizin]-7a'(5'H)-yl)methoxy)pyrido[4,3-d]pyrimidin-4-yl)-3,8-diazabicyclo[3.2.1]octane-8-carboxylate (100 mg, yield: 55.5%).

Step 10: tert-butyl (1R,5S)-3-(8-fluoro-7-(3-(methoxymethoxy)-8-((triisopropylsilyl)ethynyl)naphthalen-1-yl)-2-((6'-methylenetetrahydrospiro[cyclopropane-1,1'-pyrrolizin]-7a'(5'H)-yl)methoxy)pyrido[4,3-d]pyrimidin-4-yl)-3,8-diazabicyclo[3.2.1]octane-8-carboxylate (100 mg, 0.11 mmol) was dissolved in N, N-dimethylformamide (3.0 mL). Cesium fluoride (84 mg, 0.55 mmol) was added, and the system reacted at room temperature for 30 min. The reaction solution was filtered, purified by reverse phase preparation (chromatographic column: Waters XBridge C18, mobile phase: water/acetonitrile (0.1% trifluoroacetic acid) 0-45%, flow rate: 50 mL/min, column temperature: 25°C) to give the product tert-butyl (1R,5S)-3-(7-(8-ethynyl-3-(methoxymethoxy)naphthalen-1 -yl)-8-fluoro-2-((6'-methylenetetrahydrospiro[cyclopropane-1,1'-pyrrolizin]-7a'(5'H)-yl)methoxy)pyrido[4,3-d]pyrimidin-4-yl)-3,8-diazabicyclo[3.2.1]octane-8-carboxylate (50 mg, yield: 60.9%).

Step 11: tert-butyl (1R,5S)-3-(7-(8-ethynyl-3-(methoxymethoxy)naphthalen-1-yl)-8-fluoro-2-((6'-methylenetetrahydrospiro[cyclopropane-1,1'-pyrrolizin]-7a'(5'H)-yl)methoxy)pyrido[4,3-d]pyrimidin-4-yl)-3,8-diazabicyclo[3.2.1]octane-8-carboxylate (50 mg, 0.067 mmol) was dissolved in acetonitrile (2.0 mL) and 4 M hydrochloric acid/dioxane solution (1 mL) was added in an ice bath, and the system reacted for 0.5 h in an ice bath. Alkalinity was adjusted by saturated sodium bicarbonate and the reaction solution was rotary dried to give the crude, then high performance liquid phase prepraration was performed (formic acid method) to give the product 4-(4-((1R,5S)-3,8-diazabicyclo[3.2.1]octan-3-yl)-8-fluoro-2-((6'-methylenetetrahydrospiro[cyclopropane-1,1'-pyrrolizin]-7a'(5'H)-yl)methoxy)pyrido[4,3-d]pyrimidin-7-yl)-5-ethynylnaphthalen-2-ol (Z106, formate salt, 8.5 mg, yield: 21.2%). ESI: [M+H]⁺=603.4. ¹H NMR (400 MHz, CD₃OD) δ 9.03 (s, 1H), 8.52 (s, 1H), 7.82 (d, *J* = 7.3 Hz, 1H), 7.51 (d, *J* = 6.1 Hz, 1H), 7.43 - 7.36 (m, 1H), 7.33 (d, *J* = 2.6 Hz, 1H), 7.16 (d, *J* = 2.6 Hz, 1H), 5.10 (s, 2H), 4.64 (dd, *J* = 19.7, 12.7 Hz, 2H), 4.42 (p, *J* = 11.7 Hz, 2H), 4.04 (d, *J* = 14.4 Hz, 1H), 3.71 (dd, *J* = 32.5, 13.7 Hz, 5H), 3.54 - 3.40 (m, 1H), 3.08 - 2.95 (m, 2H), 2.72 (d, *J* = 16.5 Hz, 1H), 2.50 (d, *J* = 16.1 Hz, 1H), 2.19 (dt, *J* = 13.7, 7.0 Hz, 1H), 1.91 (ddd, *J* = 34.1, 21.3, 9.1 Hz, 5H), 1.00 - 0.73 (m, 2H), 0.76 - 0.58 (m, 2H).

### Example 31: Synthesis of 4-(4-(3,8-diazabicyclo[3.2.1]octan-3-yl)-2-((2-methylenetetrahydro-1H-pyrrolizin-7a(5H)-yl)methoxy)pyrido[4,3-d]pyrimidin-7-yl)-5-ethynylnaphthalen-2-ol (Z191)

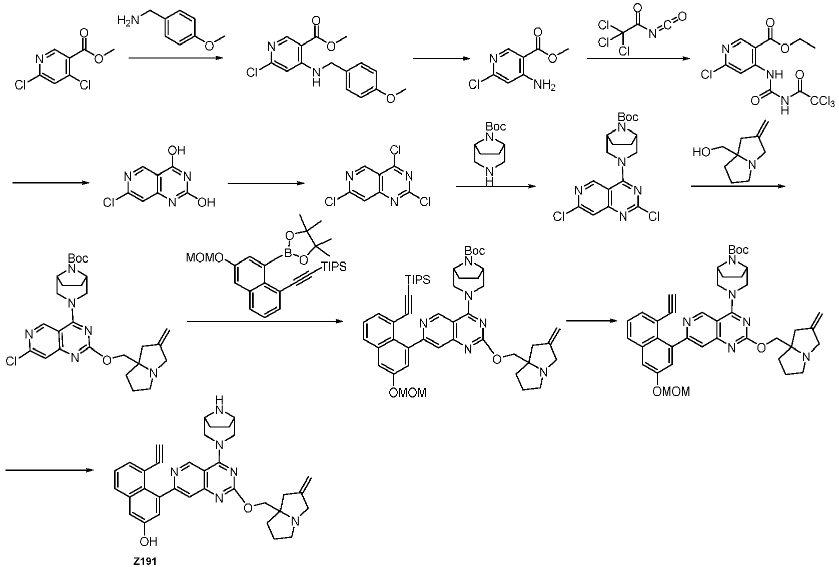

Step 1: methyl 4, 6-dichloropyridine-3-carboxylate (5 g, 24.27 mmol) was dissolved in dimethyl sulfoxide (50 mL) and (4-methoxyphenyl)methanamine (3.33 g, 24.27 mmol) and You (4.91 g, 48.54 mmol) were added thereto, and the system reacted at room temperature for 16 h. Water (200 mL) and ethyl acetate (100 mL) were added to the reaction mixture and liquid separation was performed, and the organic phase was washed with saturated brine (100 mL), dried over anhydrous sodium sulfate, and concentrated to give the crude, which was purified by silica gel column chromatography (petroleum ether: 100-85%, ethyl acetate: 0-15%) to give the product methyl 6-chloro-4-[(4-methoxyphenyl)methylamino]pyridine-3-carboxylate (4.50 g, yield: 60.45%). ES-API: [M+H]⁺= 307.1.

Step 2: methyl 6-chloro-4-[(4-methoxyphenyl)methylamino]pyridine-3-carboxylate (4.5 g, 14.67 mmol) was dissolved in trifluoroacetic acid (30 mL), and the system reacted at 70°C for 16 h. Upon completion of the reaction, the reaction solution was concentrated, adjusted pH to 8-9 with saturated sodium bicarbonate solution, extracted with ethyl acetate (100 mL), the organic phase was washed with brine (100 mL), dried over anhydrous sodium sulfate and concentrated to give the crude, which was purified by silica gel column chromatography (petroleum ether: 100-80%, ethyl acetate: 0-20%) to give 4-amino-6-chloro-pyridine-3-carboxylic acid methyl ester (3.1 g, crude). ES-API: [M+H]⁺= 187.1.

Step 3: 4-amino-6-chloro-pyridine-3-carboxylic acid methyl ester (3.1 g, 16.61 mmol) was dissolved in tetrahydrofuran (30 mL). 2,2,2-trichloroacetyl isocyanate (3.13 g, 16.61 mmol) was added, and the system reacted at room temperature for 2 h. Upon completion of the reaction, the reaction solution was concentrated to obtain a crude which was triturated and purified (petroleum ether: ethyl acetate = 10:1) (20 mL) to give 6-chloro-4-[(2,2,2-trichloroacetyl)carbamoylamino]pyridine-3-carboxylate (4.90 g, yield: 78.65%).[M +H]⁺= 390.1. Step 4: methyl 6-chloro-4-[(2,2,2-trichloroacetyl)carbamoylamino]pyridine-3-carboxylate (4.9 g, 13.07 mmol) was dissolved in methanol (40 mL), 7N ammonia/methanol (40 mL) was added, and the system reacted at room temperature for 30 min. Upon completion of the reaction, the reaction solution was concentrated to obtain a crude which was triturated and purified (petroleum ether: ethyl acetate = 10:1) (10 mL) to give 7-chloropyrido[4,3-d]pyrimidine -2,4-diol (2.40 g, yield: 92.96%). [M +H]⁺= 198.1.

Step 5: 7-chloropyrido[4,3-d]pyrimidine-2,4-diol (2.4 g, 12.15 mmol) was dissolved in toluene (40 mL), phosphorus oxychloride (9.3 g, 60.74 mmol) was added followed by diisopropylethyl amine (7.85 g, 60.74 mmol),, and the system reacted at 110°C for 2 h. The reaction solution was cooled to room temperature and the phosphorus oxychloride was rotary dried to remove under reduced pressure to give crude 2,4,7-trichloropyrido[4,3-d]pyrimidine (3 g, crude). [M +H]⁺= 235.

Step 6: 2,4,7-trichloropyrido[4,3-d]pyrimidine (3 g, crude) was dissolved in dichloromethane (20 mL), diisopropyl ethyl amine (4.9 g, 38.38 mmol) was added, tert-butyl-3,8-azabicyclo[3.2.1]octane-8-carboxylate (2.72 g, 12.79 mmol) was added at -40°C, and the system reacted for 0.5 h at -40°C. Water (40 mL) and dichloromethane (20 mL) were added to the reaction solution and liquid separation was performed, and the organic phase was dried over anhydrous sodium sulfate and rotary dried. The crude was purified by silica gel column chromatography (ethyl acetate/petroleum ether = 10%/100%) to give tert-butyl 3-(2,7-dichloropyrido[4,3-d]pyrimidin-4-yl)-3,8-diazabicyclo[3.2.1]octane-8-carboxylate (2.50 g, yield: 47.62%). ES-API: [M+H]⁺=411.1.

Step 7: tert-butyl 3-(2,7-dichloropyrido[4,3-d]pyrimidin-4-yl)-3,8-diazabicyclo[3.2.1]octane-8-carboxylate (1.3 g, 3.17 mmol) was dissolved in 1,4-dioxane (10 mL) and (6-methylene-2,3,5,7-tetrahydro-1H-pyrrolizin-8-yl) methanol (970.94 mg, 6.34 mmol) and diisopropylethyl amine (1.23 g, 9.51 mmol) were added, and the system reacted under argon protection at 100°C for 16 h. Water (20 mL) and ethyl acetate (20 mL) were added to the reaction solution and liquid separation was performed, and the organic phase was washed with brine (20 mL), dried over anhydrous sodium sulfate, and rotary dried. The crude was purified by silica gel column chromatography (ethyl acetate/petroleum ether = 10%/70%) to give tert-butyl 3-[7-chloro-2-[(6-methylene-2,3,5,7-tetrahydro-1H-pyrrolizin-8-yl)methoxy]pyrido[4,3-d]pyrimidin-4-yl]-3,8-diazabicyclo[3.2.1]octane-8-carboxylate (800.00 mg, yield: 47.91%). ES-API: [M+H]⁺=527.2. Step 8: tert-butyl 3-[7-chloro-2-[(6-methylene-2,3,5,7-tetrahydro-1H-pyrrolizin-8-yl)methoxy]pyrido[4,3-d]pyrimidin-4-yl]-3,8-diazabicyclo[3.2.1]octane-8-carboxylate (200 mg, 0.379 mmol) was dissolved in tetrahydrofuran/water (1.5 mL/0.5 mL) and ((6-(methoxymethoxy)-8-(4,4,5,5-tetramethyl-1,3,2-dioxaborinan-2-yl)naphthalen-1-yl)ethynyl)triisopropylsilane (376.85 mg, 0.759 mmol), potassium phosphate (241.3 mg, 1.14 mmol) and (docosyl-n-butylphosphino) (2'-amino-1,1'-biphenyl-2-yl)palladium (II) methanesulfonate dichloromethane complex (27.63 mg, 0.038 mmol) were added, reacted at 80°C for 3 h. Water (10 mL) and ethyl acetate (10 mL) were added to the reaction solution and liquid separation was performed, and the organic phase was washed with saturated brine (10 mL), dried over anhydrous sodium sulfate and rotary dried to give the crude which was purified by silica gel column chromatography (ethyl acetate = 100%) to give the product tert-butyl-3-(7-(3-(methoxymethoxy)8-((triisopropylsilyl)ethynyl)naphthalen-1-yl)-2-((2-methylene-tetrahydro-1H-pyrrolizin-7a(5H)-yl)methoxy)pyrido[4,3-d]pyrimidin-4-yl-3,8-Azabicyclo[3.2.1]octane)-8-carboxylate (200 mg, yield: 55.08%). ES-API: [M+1]⁺=859.5.

Step 9: tert-butyl-3-(7-(3-(methoxymethoxy)8-((triisopropylsilyl)ethynyl)naphthalen-1-yl)-2-(2-methylene-tetrahydro-1H-pyrrolizin-7a(5H)-yl)methoxy)pyrido[4,3-d]pyrimidin-4-yl-3,8-azabicyclo[3.2.1]octane)-8-carboxylate (100 mg, 0.116 mmol) was dissolved in N, N-dimethylformamide (1 mL). Cesium fluoride (76.9 mg, 1.16 mmol) and a drop of water were added, and the system reacted at room temperature for 4 h. The reaction solution was used directly in the next step without treatment. ES-API: [M+1]⁺=703.3.

Step 10: the above reaction solution was added to acetonitrile (1 mL), 4 M hydrochloric acid/dioxane solution (5 mL) was added in an ice bath, and the reaction was carried out for 0.5 h in an ice bath. Ethyl acetate (5 mL) was added to back-extract, the aqueous phase was adjusted to pH 11-12 with 15% sodium hydroxide solution, extracted with ethyl acetate (10 mL), the organic phase was dried over anhydrous sodium sulfate and rotary dried to obtain a crude which was purified by silica gel column chromatography (dichloromethane/methanol = 85%/15%) to obtain the product 4-(4-(3,8-diazabicyclo[3.2.1]octan-3-yl)-2-((2-methylenetetrahydro-1H-pyrrolizin-7a(5H)-yl)methoxy)pyrido[4,3-d]pyrimidin-7-yl)-5-ethynaphthalen-2-ol (Z191, 11.6 mg, yield: 16.05%). ES-API: [M+1]⁺=559.2.

### Example 32: Synthesis of 4-(4-((1R,5S)-3-azabicyclo[3.2.1]octan-3-yl)-8-fluoro-2-(((2R,7aS)-2-fluorotetrahydro-1H-pyrrolin-7a(5H)-yl)methoxy)pyrido[4,3-d]pyrimidin-7-yl)-5-ethynylnaphthalen-2-ol (Z192)

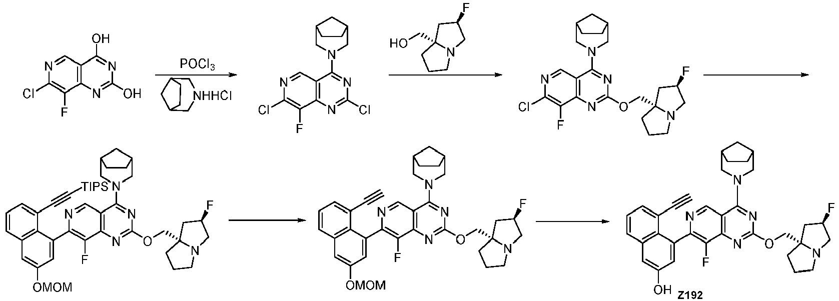

Step 1: 7-chloro-8-fluoropyrido[4,3-d]pyrimidine-2,4-diol (500 mg, 2.32 mmol) was added into the reaction vessel, and then phosphorus oxychloride (1.50 mL) and diisopropyl ethylamine (1.5 g, 11.6 mmol) were added into the reaction vessel successively and allowed to react at 100°C for 2 h. Cooled to room temperature, phosphorus oxychloride was rotary dried to remove under reduced pressure, dichloromethane (5 mL) was added to dissolve, followed by diisopropyl ethyl amine (1.5 g, 11.6 mmol), (1R,5S)-3-azabicyclo[3.2.1]octane hydrochloride (375 mg, 2.55 mmol) was slowly added after cooling below -40°C, and the system reacted at this temperature for 0.5 h. Upon completion of the reaction, it was diluted by adding water (20 mL), extracted with dichloromethane (30 mL X3), the organic phase was washed with saturated brine (20 mL X1), dried over anhydrous sodium sulfate, and rotary dried. The crude was purified by silica gel column chromatography (50-100% ethyl acetate/petroleum ether) to give compound 4-((1R,5S)-3-azabicyclo[3.2.1]octan-3-yl)-2,7-dichloro-8-fluoropyrido[4,3-d]pyrimidine (510 mg, yield: 67.3%). ES-API: [M+H]⁺=327.3.

Step 2: the compound 4-((1R,5S)-3-azabicyclo[3.2.1]octan-3-yl)-2,7-dichloro-8-fluoropyrido[4,3-d]pyrimidine (200 mg, 0.61 mmol) was dissolved in 1,4 dioxane (3 mL). ((2R,7aS)-2-fluorotetrahydro-1H-pyrrolizin-7a(5H)-yl) methanol (195 mg, 1.23 mmol), diisopropylethyl amine (236 mg, 1.83 mmol) and 4A molecular sieves were added, and the system reacted under argon protection at 100°C for 16 h. Water (20 mL) was added to the reaction solution, which was extracted with ethyl acetate (30 mL X3). The organic phase was washed with saturated brine (20 mL X1), dried over anhydrous sodium sulfate, and rotary dried. The crude was purified by silica gel column chromatography (0-5% methanol/dichloromethane) to give compound 4-((1R,5S)-3-azabicyclo[3.2.1]octan-3-yl)-7-chloro-8-fluoro-2-((2R,7aS)-2-fluorotetrahydro-1H-pyrrolin-7a(5H)-yl)methoxy)pyrido[4,3-d]pyrimidine (230 mg, yield: 83.5%). ES-API: [M+H]⁺=500.2.

Step 3: the compound 4-((1R,5S)-3-azabicyclo[3.2.1]octan-3-yl)-7-chloro-8-fluoro-2-((2R,7aS)-2-fluorotetrahydro-1H-pyrrolin-7a(5H)-yl)methoxy)pyrido[4,3-d]pyrimidine (230 mg, 0.51 mmol) was dissolved in tetrahydrofuran/water (4 mL/0.4 mL). Triisopropyl ((6-(methoxymethoxy)-8-(4,4,5,5-tetramethyl-1,3,2-dioxobenzaldehyde-2-yl)naphthalen-1-yl)ethynyl)silane (378 mg, 0.76 mmol), potassium phosphate (217 mg, 1.02 mmol) and[n-butylbis(1-adamantyl)phosphine](2-amino-1,1'-biphenyl-2-yl)palladium (11) methanesulfonate (27.5 mg, 0.05 mmol) were added, and the system reacted at 60°C for 1 h. Water (20 mL) was added to the reaction solution, which was extracted with ethyl acetate (30 mLX3). The organic phase was washed with saturated brine (20 mL X1), dried over anhydrous sodium sulfate, and rotary dried. The crude was purified by silica gel column chromatography (0-10% methanol/dichloromethane) to give 4-((1R,5S)-3-azabicyclo[3.2.1]octan-3-yl)-8-fluoro-2-((2R,7aS)-2-fluorotetrahydro-1H-pyrrolin-7a(5H)-yl)methoxy)-7-(3-(methoxymethoxy)-8-((triisopropylsilyl)ethynyl)naphthalen-1-yl)pyrido[4,3-d]pyrimidine (210 mg, yield: 52.5%). ES-API: [M+H]⁺=782.4.

Step 4: the compound 4-((1R,5S)-3-azabicyclo[3.2.1]octan-3-yl)-8-fluoro-2-((2R,7aS)-2-fluorotetrahydro-1H-pyrrolin-7a(5H)-yl)methoxy)-7-(3-(methoxymethoxy)-8-((triisopropylsilyl)ethynyl)naphthalen-1-yl)pyrido[4,3-d]pyrimidine (210mg, 0.26 mmol) was dissolved in N, N-dimethylformamide (2 mL). Cesium fluoride (118 mg, 0.78 mmol) was added, and the system reacted at room temperature for 15 min. Upon completion of the reaction, the reaction solution was directly filtered and reverse phase preparation was performed (chromatographic column: Waters XBridge C18; mobile phase: water/acetonitrile (0.1% trifluoroacetic acid) 0-60%; flow rate: 50 mL/min; column temperature: 25°C) to give compound 4-((1R,5S)-3-azabicyclo[3.2.1]octan-3-yl)-7-(8-ethynyl-3-(methoxymethoxy)naphthalen-1-yl)-8-fluoro-2-((2R,7aS)-2-fluorotetrahydro-1H-pyrrolin-7a(5H)-yl)methoxy)pyrido[4,3-d]pyrimidine (136 mg, yield: 83%). ES-API: [M+H]+=626.3.

Step 5: the compound 4-((1R,5S)-3-azabicyclo[3.2.1]octan-3-yl)-7-(8-ethynyl-3-(methoxymethoxy)naphthalen-1-yl)-8-fluoro-2-((2R,7aS)-2-fluorotetrahydro-1H-pyrrolin-7a(5H)-yl)methoxy)pyrido[4,3-d]pyrimidine (136 mg, 0.22 mmol) was dissolved in acetonitrile (2.0 mL) and 4 M hydrochloric acid/dioxane solution (0.500 mL, 2.000 mmol) was added and the reaction solution was stirred at 0°C for 1 h. The reaction solution was adjusted to pH 9 with 5 mol/L sodium hydroxide aqueous solution, extracted with ethyl acetate (30 mL X3), the organic phase was washed with saturated brine (20 mL X1), dried over anhydrous sodium sulfate and reverse phase preparation was performed (chromatographic column: Waters XBridgC18; water/acetonitrile (0.1% trifluoroacetic acid) 0-30%; flow rate: 50 mL/min; column temperature: 25°C) to give compound 4-(4-((1R,5S)-3-azabicyclo[3.2.1]octan-3-yl)-8-fluoro-2-(((2R,7aS)-2-fluorotetrahydro-1H-pyrrolin-7a(5H)-yl)methoxy)pyrido[4,3-d]pyrimidin-7-yl)-5-ethynylnaphthalen-2-ol (Z192, trifluoroacetate, 70 mg, yield: 60.3%), brown solid. ES-API: [M+1]+=582.4. ¹H NMR (400 MHz, CD₃OD) δ 9.00 (s, 1H), 7.81 (d, J = 8.3 Hz, 1H), 7.50 (d, J = 7.1 Hz, 1H), 7.39 (t, J = 7.7 Hz, 1H), 7.32 (d, J = 2.4 Hz, 1H), 7.16 (d, J = 2.5 Hz, 1H), 5.34 (d, J = 53.2 Hz, 1H), 4.74 - 4.55 (m, 2H), 4.38 - 4.24 (m, 2H), 3.62 (dd, J = 21.5, 12.2 Hz, 2H), 3.48 - 3.33 (m, 2H), 3.29 - 3.24 (m, 1H), 3.09 (s, 1H), 2.98 (d, J = 7.5 Hz, 2H), 2.45 - 2.15 (m, 5H), 2.09 - 1.89 (m, 4H), 1.73 (s, 3H), 1.59 (d, J = 15.5 Hz, 2H).

### Example 33: Synthesis of 4-((1R,5S)-3,8-diazabicyclo[3.2.1]octan-3-yl)-6-chloro-7-(8-ethynylnaphthalen-1-yl)-8-tluoro-2-((2-methylenetetrahydro-1H-pyrrolizin-7a(SH)-yl)methoxy)quinazoline (Z193)

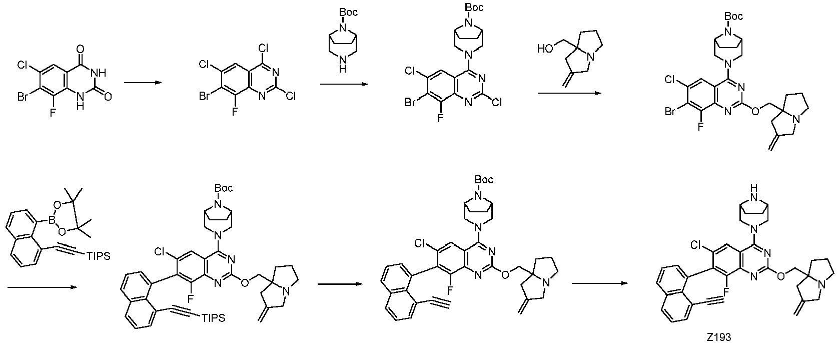

Step 1: 7-bromo-6-chloro-8-fluoroquinazoline-2,4(1H,3H)-dione (1.0 g, 3.4 mmol) was dissolved in 20 mL of toluene, bis-triisopropylethylamine (2.8 mL, 17 mmol) was added to the above solution, and then phosphorus oxychloride (1.5 mL, 17 mmol) was slowly added dropwise to the above solution and stirred at 110°C for 3 h. The reaction was determined to be complete by LCMS, quenched with saturated sodium bicarbonate solution (50 mL), extracted with ethyl acetate (50 mL X3), the organic phase was washed with saturated brine (50 mL X3), dried over anhydrous sodium sulfate, filtered and concentrated. The crude was purified by silica gel column chromatography (petroleum ether: ethyl acetate = 20%) to give 7-bromo-2,4,6-trichloro-8-fluoroquinazoline (1.0 g, yield: 88%). ES-API: [M-55]⁺= 330.3.

Step 2: a solution of 7-bromo-2,4,6-trichloro-8-fluoroquinazoline (900mg, 2.7 mmol), tert-butyl (1R,5S)-3,8-diazabicyclo[3.2.1]octane-8-carboxylate (1.16g, 5.4 mmol) and N, N-diisopropyl ethylenediamine (1.01g, 8.17 mmol) in dioxane (30 mL) was stirred at 110°C for 6 h. The reaction was determined to be complete by LCMS, quenched with saturated ammonium chloride solution (30 mL), extracted with ethyl acetate (20 mL X3), the organic phase was washed with saturated brine (15 mL X3), dried over anhydrous sodium sulfate, filtered and concentrated. The crude was purified by silica gel column chromatography (petroleum ether: ethyl acetate = 50%) to give tert-butyl (1R,5S)-3-(7-bromo-2,6-dichloro-8-fluoroquinazolin-4-yl)-3,8-diazacyclo[3.2.1]octane-8-carboxylate (1.01 g, yield: 75%). ES-API: [M+H]⁺= 507.0.

Step 3: a mixed solution of tert-butyl (1R,5S)-3-(7-bromo-2,6-dichloro-8-fluoroquinazolin-4-yl)-3,8-diazacyclo[3.2.1]octane-8-carboxylate (900mg, 1.8 mmol), (2-methylenetetrahydro-1H-pyrrolizin-7a(5H)-yl) methanol (409 mg, 2.7 mmol), cesium carbonate (1.7 g, 5.3 mmol) and 1,4-diazabicyclo[2.2.2]octane (20 mg, 0.18 mmol) in tetrahydrofuran (5 mL) and N, N-dimethylformamide (5 mL) was stirred at room temperature overnight. The reaction was determined to be complete by LCMS, washed with water (30 mL), extracted with ethyl acetate (20 mL X3), the organic phase was washed with saturated brine (15 mL X3), dried over anhydrous sodium sulfate, filtered and concentrated. The crude was purified by silica gel column chromatography (petroleum ether: ethyl acetate = 50%) to give tert-butyl (1R,5S)-3-(7-bromo-6-chloro-8-fluoro-2-((2-methylenetetrahydro-1H-pyrrolizin-7a(5H)-yl)methoxy)quinazolin-4-yl)-3,8-diazabicyclo[3.2.1]octane-8-carboxylate (801 mg, yield: 72%). ES-API: [M+H]⁺= 623.2.

Step 4: under nitrogen protection, tert-butyl (1R,5S)-3-(7-bromo-6-chloro-8-fluoro-2-((2-methylenetetrahydro-1H-pyrrolizin-7a(5H)-yl)methoxy)quinazolin-4-yl)-3,8-diazabicyclo[3.2.1]octane-8-carboxylate (500 mg, 0.80 mmol), triisopropyl ((8-(4,4,5,5-tetramethyl-1,3,2-dioxaborolan-2-yl)naphthalen-1-yl)ethynyl)silane (1.3 g, 2.4 mmol), potassium phosphate (511 mg, 2.4 mmol) and[n-butylbis(1-adamantyl)phosphine](2-amino-1,1'-biphenyl-2-yl)palladium (II) methanesulfonate (88 mg, 0.12 mmol) were dissolved in tetrahydrofuran (10 mL) and water (2 mL) and the reaction mixture was heated to 65°C and stirred for 3 h. The reaction was determined to be complete by LCMS, the reaction solution was concentrated under reduced pressure and the crude was purified by silica gel column chromatography (dichloromethane: methanol = 20:1) to give tert-butyl (1R,5S)-3-(6-chloro-8-fluoro-2-((2-methylenetetrahydro-1H-pyrrolizin-7a(5H)-yl)methoxy)-7-(8-((triisopropylsilyl)ethynyl)naphthalen-1-yl)quinazolin-4-yl)-3,8-diazabicyclo[3.2.1]octane-8-carboxylate (120 mg, yield: 17%). ES-API: [M+H]⁺= 850.5.

Step 5: cesium fluoride (214 mg, 1.4 mmol) was added to tert-butyl (1R,5S)-3-(6-chloro-8-fluoro-2-((2-methylenetetrahydro-1H-pyrrolizin-7a(5H)-yl)methoxy)-7-(8-((triisopropylsilyl)ethynyl)naphthalen-1-yl)quinazolin-4-yl)-3,8-diazabicyclo[3.2.1]octane-8-carboxylate (120 mg, 0.14 mmol) in N, N-dimethylformamide (5 mL) and the reaction mixture was stirred at room temperature for 2 h. The reaction was determined to be complete by LCMS, the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(6-chloro-7-(8-ethynylnaphthalen-1-yl)-8-fluoro-2-((2-methylenetetrahydro-1H-pyrrolin-7a(5H)-yl)methoxy)quinazolin-4-yl)-3,8-diazacyclo[3.2.1]octane-8-carboxylate (97 mg, yield: 99%). ES-API: [M+H]⁺= 694.3.

Step 6: a 4 M hydrochloric acid-dioxane solution (5 mL) was slowly added to a solution of tert-butyl (1R,5S)-3-(6-chloro-7-(8-ethynylnaphthalen-1-yl)-8-fluoro-2-((2-methylenetetrahydro-1H-pyrrolin-7a(5H)-yl)methoxy)quinazolin-4-yl)-3,8-diazacyclo[3.2.1]octane-8-carboxylate (97 mg, 0.14 mmol) in acetonitrile (2 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase: A: 5 mmol/L ammonium bicarbonate aqueous solution, mobile phase B: acetonitrile; flow rate: 15 mL/min; chromatographic conditions: 15 mL-35-85-13 min; column temperature: room temperature) to give 4-((1R,5S)-3,8-diazabicyclo[3.2.1]octan-3-yl)-6-chloro-7-(8-ethynylnaphthalen-1-yl)-8-fluoro-2-((2-methylenetetrahydro-1H-pyrrolizin-7a(5H)-yl)methoxy)quinazoline (Z193, 35 mg, yield: 42%). ES-API: [M+H]⁺= 594.3. ¹H NMR (500 MHz, CD₃OD) δ 8.50 (s, 1 H), 8.07 (dd, *J* = 16.5, 8.2 Hz, 2 H), 7.88 (s, 1 H), 7.72 (d, *J* = 7.1 Hz, 1 H), 7.65 (t, *J* = 7.7 Hz, 1 H), 7.51 (t, *J* = 7.7 Hz, 1 H), 7.41 (d, *J* = 7.1 Hz, 1 H), 5.22 (s, 2 H), 4.66-4.60 (m, 3 H), 4.59 - 4.50 (m, 1 H), 4.26 (t, *J* = 13.0 Hz, 1 H), 4.05 (d, *J* = 10.0 Hz, 2 H), 3.90 (d, *J* = 12.5 Hz, 1 H), 3.80 (d, *J* = 14.4 Hz, 2 H), 3.70-3.64 (m, 1 H), 3.20-3.09 (m, 1 H), 3.05 (s, 1 H), 3.01 (d, *J* = 16.0 Hz, 1 H), 2.74 (d, *J* = 14 Hz, 1 H), 2.39-2.28 (m, 1 H), 2.21-2.17 (m, 1 H), 2.12-1.98 (m, 6 H).

### Example 34: Synthesis of 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynyl-6-fluoronaphthalen-2-ol (Z194)

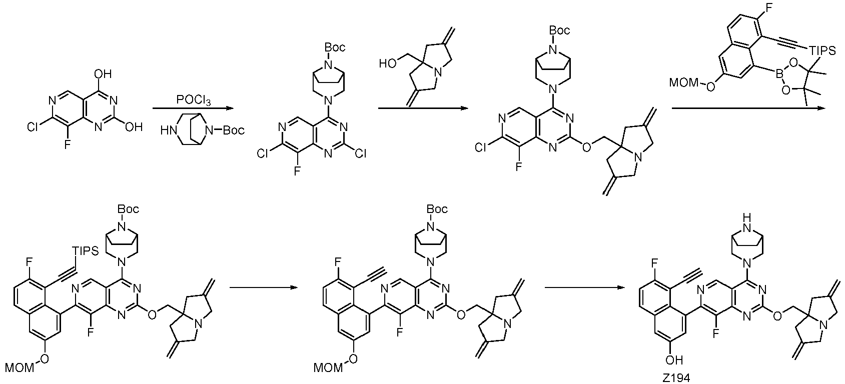

Step 1: 7-chloro-8-fluoropyrido[4,3-d]pyrimidine-2,4-diol (2 g, 9.3 mmol) was added to a reactor containing anhydrous toluene (20 mL), and then phosphorus oxychloride (6 mL) and diisopropyl ethyl amine (6 g, 46 mmol) were successively added to the reactor and allowed to react at 100°C for 2 h. After cooling to room temperature, phosphorus oxychloride was rotary dried to remove under reduced pressure and dissolved in anhydrous dichloromethane (60 mL). Diisopropylethyl amine (6 g, 46 mmol) and tert-butyl 3,8-diazacyclo[3.2.1]octane-8-carboxylate (1.97 g, 9.3 mmol) were added, and the system reacted at -40°C for 0.5 h. Water (60 mL) was added to the reaction solution, which was extracted with dichloromethane (60 mL x2), dried over anhydrous sodium sulfate, and rotary dried. The crude was purified by silica gel column chromatography (50-100% ethyl acetate/petroleum ether) to give tert-butyl (1R,5S)-3-(2,7-dichloro-8-fluoropyrido[4,3-d]pyrimidin-4-yl)-3,8-diazacyclo[3.2.1]octane-8-carboxylate (2 g, yield: 50.3%). ESI: [M+H]⁺=428.3.

Step 2: (2,6-dimethylenetetrahydro-1H-pyrrolizin-7a(5H)-yl) methanol (1.0 g, 6.08 mmol) was dissolved in dry tetrahydrofuran (20 mL) and sodium hydride (800 mg) was added at 0°C followed by tert-butyl (1R,SS)-3-(2,7-dichloro-8-fluoropyrido[4,3-d]pyrimidin-4-yl)-3,8-diazacyclo[3.2.1]octane-8-carboxylate (2 g, 4.68 mmol) while maintaining at 0°C and allowed to react at 0°C for 1 h. Water (20 mL) was added to the reaction solution, which was extracted with ethyl acetate, dried over anhydrous sodium sulfate, and rotary dried. The crude was purified by silica gel column chromatography (50-100% ethyl acetate/petroleum ether) to give compound tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (1.2 g, yield: 46%). ESI: [M+H]⁺=557.3.

Step 3: the compound tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (1.2g, 2.16 mmol) was dissolved in tetrahydrofuran/water (20 mL/2 mL), ((2-fluoro-6-(methoxymethoxy)-8-(4,4,5,5-tetramethyl-1,3,2-dioxoboran-2-yl)naphthalen-1-yl)ethynyl)triisopropylsilane (1.30 g, 2.59 mmol), potassium phosphate (1.37 g, 6.48 mmol) and[n-butylbis(1-adamantyl)phosphine] (2-amino-1,1'-biphenyl-2-yl)palladium (11) methanesulfonate (240 mg) were reacted at 60°C for 2 h. The reaction solution was extracted with ethyl acetate and water, and the organic phase was dried over anhydrous sodium sulfate and rotary dried. The crude was purified by silica gel column chromatography (dichloromethane/methanol = 10/1) to give tert-butyl (1R,5S)-3-(2-((2,6-dimethylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (1.4 g, yield: 71.5%). ESI: [M+H]⁺=907.5.

Step 4: tert-butyl (1R,5S)-3-(2-((2,6-dimethylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (1.4 g, 1.54 mmol) was dissolved in N, N-dimethylformamide (10 mL). Cesium fluoride (456 mg, 3.0 mmol) was added, and the system reacted at room temperature for 2 h. The reaction solution was extracted with ethyl acetate and water, and the organic phase was dried over anhydrous sodium sulfate, rotary dried, and reverse phase preparation was performed (chromatographic column: Waters XBridge C18; mobile phase: water/acetonitrile (0.1% trifluoroacetic acid) 0-50%; flow rate: 50 mL/min; column temperature: 25°C) to give the product tert-butyl (1R,5S)-3-(2-((2,6-dimethylenetetrahydro-1H-pyrrolizin-7a(5H)-yl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (1.0 g, yield: 86.6%). ESI: [M+H]⁺=751.3.

Step 5: tert-butyl (1R,5S)-3-(2-((2,6-dimethylenetetrahydro-1H-pyrrolizin-7a(5H)-yl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (1.0 g, 1.3 mmol) was dissolved in acetonitrile (10 mL), 4 M hydrochloric acid/dioxane solution (5 mL) was added in an ice bath, and the system reacted for 2 h in an ice bath. The reaction solution was adjusted to pH 8 with saturated sodium bicarbonate, extracted with ethyl acetate, rotary dried and the crude was triturated with petroleum ether/ethyl acetate (2/1) to give compound 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynyl-6-fluoronaphthalen-2-ol (Z194, 500 mg, yield: 63.5%) ESI: [M+H]+=607.3. ¹H NMR (400 MHz, CD₃OD) δ 9.00 (s, 1H), 7.85 (dd, *J* = 9.2, 5.6 Hz, 1H), 7.33 (dd, *J* = 14.4, 5.6 Hz, 2H), 7.20 (d, *J* = 2.4 Hz, 1H), 5.02 (d, *J* = 6.8 Hz, 4H), 4.67 - 4.51 (m, 2H), 4.37 - 4.26 (m, 2H), 3.84 - 3.49 (m, 6H), 3.40 - 3.32 (m, 3H), 2.79 (d, *J* = 16.4 Hz, 2H), 2.59 (d, *J* = 16.4 Hz, 2H), 1.96 - 1.59 (m, 4H).

### Example 35: Synthesis of 4-((1R,5S)-3,8-diazabicyclo[3.2.1]octan-3-yl)-7-(7-chloro-8-ethynylnaphthalen-1-yl)-2-((2,6-dimethylenetetrahydro-1H-pyrrolizin-7a(5H)-yl)methoxy)-8-fluoropyrido[4,3-d]pyrimidine (Z195)

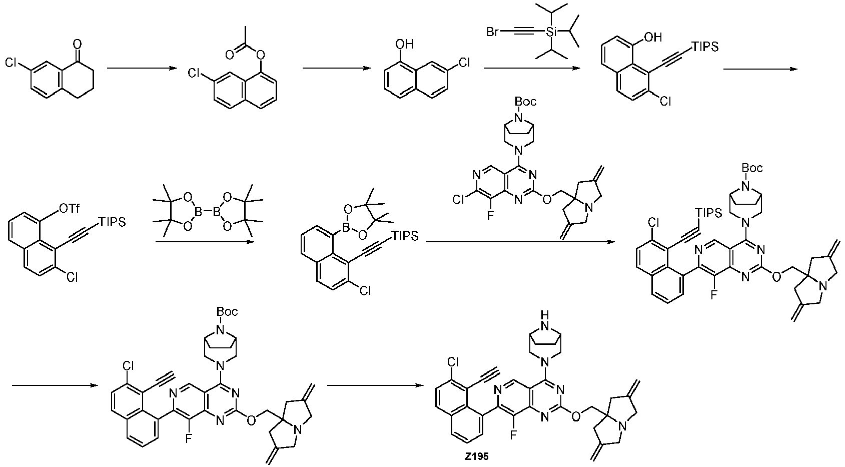

Step 1: 7-chloro-3,4-dihydronaphthalen-1(2H)-one (8 g, 44.28 mmol) was dissolved in isopropenyl acetate (26.6 g, 265.74 mmol). Concentrated sulfuric acid (186.02 mg, 2.22 mmol) was added, and the system reacted under nitrogen protection at 110°C for 16 h. The reaction solution was cooled, filtered, and concentrated to give 7-chloronaphthalene-1-acetate as a crude. The crude material was dissolved in toluene (50 mL), 2,3-dichloro-5,6-dicyanobenzoquinone (16.31 g, 71.86 mmol) was added, and the system reacted under nitrogen protection at 90°C for 2 h. The reaction solution was cooled, filtered, and concentrated to give the crude, which was purified by silica gel column chromatography (petroleum ether: ethyl acetate = 5:1) to give (7-chloro-1-naphthyl) acetate (7.00 g, yield: 88.30%). ES-API: [M+H]⁺= 221.4.

Step 2: potassium hydroxide (6.41 g, 114.21 mmol) was dissolved in ethanol (60 mL) and (7-chloro-1-naphthyl) acetate (7 g, 31.72 mmol) dissolved in ethanol (30 mL) was added in an ice bath, and the system reacted for 30 min in an ice bath. Upon completion of the reaction, the reaction mixture was concentrated to a small volume, liquid separation was performed by adding dichloromethane (50 mL)/water (50 mL), the aqueous layer was adjusted to pH 1 with 6N hydrochloric acid, extracted with ethyl acetate (50 mL), the organic layer was washed with brine (50 mL), dried over anhydrous sodium sulfate and concentrated to give 7-chloronaphthalen-1-ol (6.00 g, crude). ES-API: [M+H]⁺= 179.6.

Step 3: 7-chloronaphthalen-1-ol (3 g, 16.80 mmol) was dissolved in dioxane (30 mL), 2-bromoethynyl (triisopropyl)silane (5.27 g, 20.16 mmol), potassium acetate (3.29 g, 33.59 mmol) and dichlorobis(4-methylisopropylphenyl) ruthenium (II) (1.03 g, 1.68 mmol) were added, displaced with nitrogen for three times, and allowed to react at 110°C for 12 h under nitrogen protection. Upon completion of the reaction, the reaction solution was filtered and concentrated, and the obtained crude was purified by silica gel column chromatography (petroleum ether: 100%) to give 7-chloro-8-(2-triisopropylsilylethynyl) naphthalen-1-ol (6.00 g, yield: 89.56%). ES-API: [M+H]⁺= 359.5.

Step 4: 7-chloro-8-(2-triisopropylsilylethynyl) naphthalen-1-ol (6.5 g, 18.11 mmol) was dissolved in dichloromethane (50 mL), N, N-diisopropylethyl amine (9.36 g, 72.43 mmol) was added and triflic anhydride (15.32 g, 54.32 mmol) was slowly added dropwise at -40°C, and the system reacted for 1 hour at -40°C. Upon completion of the reaction, dichloromethane (200 mL) and water (250 mL) were added and liquid separation was performed, the organic phase was washed with brine (150 mL), dried over anhydrous sodium sulfate, and concentrated to give the crude which was purified by silica gel column chromatography (petroleum ether: 100%) to give[7-chloro-8-(2-triisopropylsilylethynyl)-1-naphthyl]triflate (5.60 g, yield: 56.68%). ES-API: [M+H]+=492.1.

Step 5:[7-chloro-8-(2-triisopropylsilylethynyl)-1-naphthyl]triflate (5.6 g, 11.40 mmol) was dissolved in toluene (50 mL), bis-(pinacolato)diboron (5.79 g, 22.81 mmol), potassium acetate (5.59 g, 57.02 mmol) and[1,1'-bis(diphenylphosphino)ferrocene]dichloropalladium (834.46 mg, 1.14 mmol) were added, displaced with nitrogen for three times and allowed to react at 110°C for 3 h under nitrogen protection. Upon completion of the reaction, the reaction solution was filtered and concentrated, and the obtained crude was purified by silica gel column chromatography (petroleum ether/ethyl acetate = 95%/5%) to give the crude. The crude was triturated with methanol (0.5 mL) to give 2-[2-chloro-8-(4,4,5,5-tetramethyl-1,3,2-dioxaborolan-2-yl)-1-naphthyl]ethynyl-triisopropyl-silane (1.00 g, yield: 16.83%). ES-API: [M+H]+=469.5. ¹H NMR (400 MHz, CDCl₃) δ7.88 - 7.77 (m, 2H), 7.71-7.69 (m, 1H), 7.54 - 7.38 (m, 2H), 1.46 (s, 12H), 1.24-1.19 (m, 21H).

Step 6: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, 0.090 mmol), ((2-chloro-8-(4,4,5,5-tetramethyl-1,3,2-dioxaborolan-2-yl)naphthalen-1-yl)ethynyl)triisopropylsilane (84 mg, 0.180 mmol), potassium phosphate (57 mg, 0.269 mmol) and[n-butyldi (1-adamantyl)phosphino](2-amino-1,1'-biphenyl-2-yl)palladium (II) methanesulfonate (6.5 mg, 0.009 mmol) were dissolved in tetrahydrofuran (2 mL) and water (0.4 mL) and the reaction mixture was heated to 60°C and stirred for 5 h. The reaction was determined to be complete by LCMS, the reaction solution was concentrated under reduced pressure, and the crude was purified by silica gel column chromatography (dichloromethane: methanol = 0-3%) to give tert-butyl (1R,5S)-3-(7-(7-chloro-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (28 mg, yield: 36%). ES-API: [M+H]⁺= 863.3.

Step 7: cesium fluoride (49 mg, 0.324 mmol) was added to tert-butyl (1R,5S)-3-(7-(7-chloro-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (28 mg, 0.032 mmol) in N, N-dimethylformamide (2 mL), the reaction mixture was stirred at room temperature for 1 h. The reaction was determined to be complete by LCMS, the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(7-(7-chloro-8-ethynylnaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (22.9 mg, yield: 99%). ES-API: [M+H]⁺= 707.3.

Step 8: a 4 M hydrochloric acid-dioxane solution (1 mL) was slowly added to a solution of tert-butyl (1R,5S)-3-(7-(7-chloro-8-ethynylnaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (22.9 mg, 0.032 mmol) in acetonitrile (1 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 ml/min; chromatographic conditions: 15 ml - 35-85-13 min; column temperature: room temperature) to give 4-((1R,5S)-3,8-diazabicyclo[3.2.1]octan-3-yl)-7-(7-chloro-8-ethynylnaphthalen-1-yl)-2-((2,6-dimethylenetetrahydro-1H-pyrrolizin-7a(5H)-yl)methoxy)-8-fluoropyrido[4,3-d]pyrimidine (Z195, 5.68 mg, yield: 29%). ES-API: [M+H]⁺= 607.3. ¹H NMR (500 MHz, DMSO) δ 9.05 (s, 1 H), 8.32 (s, 2 H), 8.20- 8.15 (m, 2 H), 7.76- 7.72 (m, 2 H), 7.67- 7.65 (m, 1 H), 4.94 (d, J= 10.0 Hz, 4 H), 4.49- 4.46 (m, 1 H), 4.32- 4.30 (m, 1 H), 4.16 (s, 1 H), 4.07 (s, 2 H), 3.66- 3.57 (m, 6 H), 3.23-3.20 (m, 2 H), 2.64- 2.61 (m, 2 H), 2.50- 2.45 (m, 2 H), 1.68 (s, 4 H).

### Example 36: Synthesis of 4-((1R,5S)-3,8-diazabicyclo[3.2.1]octan-3-yl)-2-((2,6-dimethylenetetrahydro-1H-pyrrolizin-7a(5H)-yl)methoxy)-8-fluoro-7-(5-methyl-1H-indazol-4-yl)pyrido[4,3-d]pyrimidine (Z196)

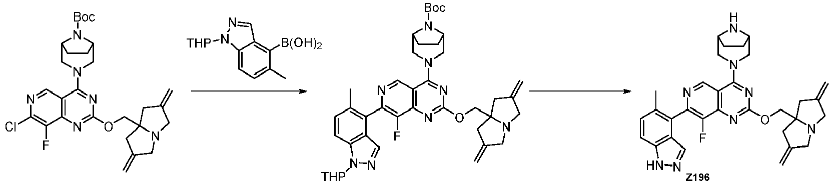

Step 1: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (80 mg, 0.14 mmol), (5-methyl-1-(tetrahydro-2H-pyran-2-yl)-1H-indazol-4-yl)boronic acid (75 mg, 0.29 mmol), cesium carbonate (94 mg, 0.29 mmol) and tetrakis triphenylphosphine palladium (16 mg, 0.014 mmol) were dissolved in dioxane (5 mL) and water (1 mL) and the reaction mixture was heated to 90°C and stirred for 1.5 h. The reaction was determined to be complete by LCMS, the reaction solution was concentrated under reduced pressure and the crude was purified by silica gel column chromatography (dichloromethane: methanol = 20:1) to give tert-butyl (1R,5S)-3-(2-((2,6-dimethylenetetrahydro-1H-pyrrolizin-7a(5H)-yl)methoxy)-8-fluoro-7-(5-methyl-1-(tetrahydro-2H-pyran-2-yl)-1H-indazol-4-yl)pyrido[4,3-d]pyrimidin-4-yl)-3,8-diazabicyclo[3.2.1]octane-8-carboxylate (70 mg, yield: 66%). ES-API: [M+H]⁺= 737.3.

Step 2: a 4 M hydrochloric acid-dioxane solution (5 mL) was slowly added to tert-butyl (1R,5S)-3-(2-((2,6-dimethylenetetrahydro-1H-pyrrolizin-7a(5H)-yl)methoxy)-8-fluoro-7-(5-methyl-1-(tetrahydro-2H-pyran-2-yl)-1H-indazol-4-yl)pyrido[4,3-d]pyrimidin-4-yl)-3,8-diazabicyclo[3.2.1]octane-8-carboxylate (70 mg, 0.09 mmol) in acetonitrile (2 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 mL/min; chromatographic conditions: 15 mL-35-85-13 min; column temperature: room temperature) to give 4-((1R,5S)-3,8-diazabicyclo[3.2.1]octan-3-yl)-2-((2,6-dimethylenetetrahydro-1H-pyrrolizin-7a(5H)-yl)methoxy)-8-fluoro-7-(5-methyl-1H-indazol-4-yl)pyrido[4,3-d]pyrimidine (Z196, 30 mg, yield: 60%). ES-API: [M+H]⁺= 553.3. ¹H NMR (500 MHz, CD₃OD) δ 9.18 (s, 1 H), 8.49 (s, 2 H), 7.69 (d, *J* = 10.1 Hz, 1 H), 7.61 (d, *J* = 8.6 Hz, 1 H), 7.42 (d, *J* = 8.6 Hz, 1 H), 5.15 (s, 4 H), 4.94 (s, 2 H), 4.58 (s, 2 H), 4.13 (d, *J* = 13.8 Hz, 4 H), 3.94 (d, *J* = 13.0 Hz, 2 H), 3.63 (d, *J* = 14.8 Hz, 2 H), 2.95 (d, *J* = 16.5 Hz, 2 H), 2.72 (d, *J* = 16.5 Hz, 2 H), 2.34 (s, 3 H), 2.16-1.96 (m, 4 H).

### Example 37: Synthesis of 4-((1R,5S)-3,8-diazabicyclo[3.2.1]octan-3-yl)-7-(3-(difluoromethyl)-8-ethynylnaphthalen-1-yl)-2-((2,6-dimethylenetetrahydro-1H-pyrrolizin-7a(5H)-yl)methoxy)-8-fluoropyrido[4,3-d]pyrimidine (Z197)

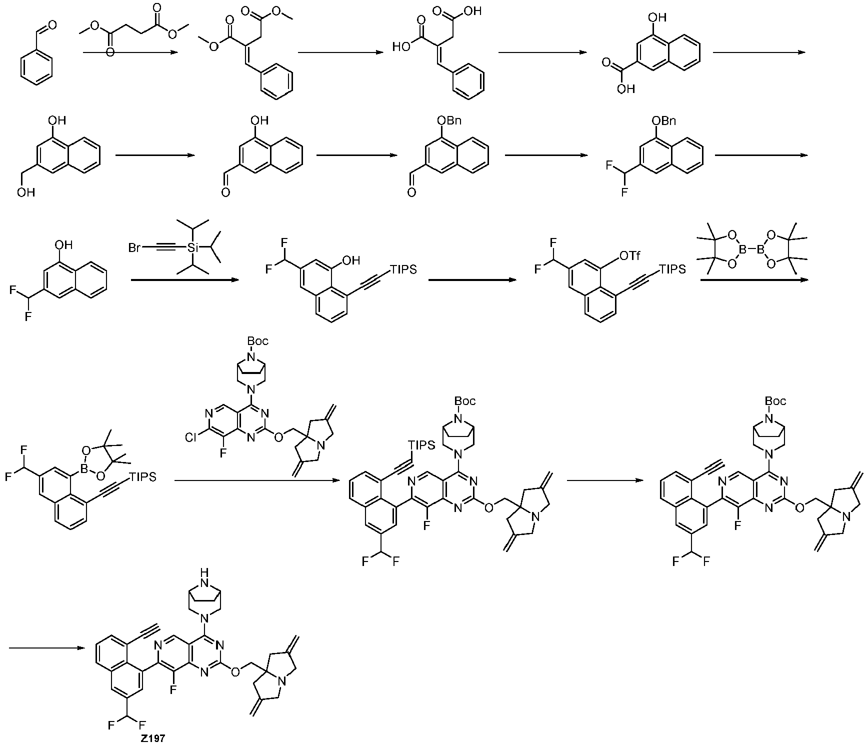

Step 1: dimethyl succinate (37.87 g, 259.14 mmol) was added to a solution of sodium methoxide (15.90 g, 294.47 mmol) in methanol (250 mL) at 80°C, reacted at 80°C for 1 h, followed by adding benzaldehyde (25 g, 235.58 mmol) and reacting at 80°C for 5 h. The dry solvent was concentrated to give (70 g, crude) a crude which was used in the next reaction without purification, ES-API: [M +H]⁺= 235.1.

Step 2: the crude from the previous step was dissolved in 15% sodium hydroxide aqueous solution (250 mL), and the system reacted at 80°C for 4 h. The reaction solution was back-extracted with ethyl acetate (250 mL), the aqueous phase was adjusted to pH 1-2 with 12N hydrochloric acid and a solid was precipitated, and filtered to give the product (E)-2-benzylidene succinic acid (40 g, yield: 82.3%), ES-API: [M +H]⁺= 207.1.

Step 3: (2Z)-2-benzylidenebutanedioic acid (40 g, 193.99 mmol) was dissolved in concentrated sulfuric acid (250 mL) and stirred at 25°C for 4 h. The reaction was slowly poured into ice water, a solid was precipitated, filtered, and then purified by silica gel column chromatography (petroleum ether/ethyl acetate = 1/1, R_{f} = 0.3) to give the product 4-hydroxynaphthalene-2-carboxylic acid (13.00 g, yield: 35.6%), ES-API: [M +H]⁺= 189.1.

Step 4: 4-hydroxynaphthalene-2-carboxylic acid (13 g, 69.08 mmol) was dissolved in tetrahydrofuran (50 mL), borane tetrahydrofuran (130 g, 138.17 mmol) was added, and the system reacted at 25°C for 2 h. The reaction was quenched with 1N diluted hydrochloric acid (50 mL), extracted with ethyl acetate (100 mL), the organic layer was washed with saturated brine (50 mL), dried over anhydrous sodium sulfate and concentrated to give the crude, which was purified by silica gel column chromatography (petroleum ether/ethyl acetate = 1/1, R_{f} = 0.6) to give the product 3-(hydroxymethyl)naphthalen-1-ol (9.50 g, yield: 71.05%). ES-API: [M+H]⁺= 175.1. Step 5: oxalyl chloride (4.37 g, 34.44 mmol) was dissolved in dichloromethane (50 mL), cooled to -78°C, dimethylsulfoxide (5.61 g, 71.76 mmol) was added, stirred at -78°C for 30 min, then 3-(hydroxymethyl)naphthalen-1-ol (5 g, 28.70 mmol) was added, stirred at -78°C for 30 min, triethylamine (14.52 g, 143.52 mmol) was added and the reaction was slowly warmed to 25°C for 1 h. The reaction was quenched with 1N diluted hydrochloric acid (50 mL), extracted with dichloromethane (100 mL), the organic layer was washed with brine (50 mL), dried over anhydrous sodium sulfate and concentrated to give the crude product 4-hydroxynaphthalene-2-carbaldehyde (4.50, yield: 91.8%), ES-API: [M +H]⁺= 173.1.

Step 6: 4-hydroxynaphthalene-2-carbaldehyde (4 g, 23.23 mmol) was dissolved in N, N-dimethylformamide (40 mL), potassium carbonate (6.41 g, 46.46 mmol) was added followed by benzyl bromide (2.50 g, 23.23 mmol), and the system reacted at 25°C for 2 h. The reaction was quenched with water (100 mL), extracted with ethyl acetate (50 mL), the organic layer was washed (50 mL), dried over anhydrous sodium sulfate, and concentrated to give the crude, which was purified by silica gel column chromatography (petroleum ether/ethyl acetate = 10/1, R_{f} = 0.6) to give the product 4-benzyloxy-naphthalene-2-carbaldehyde (4.50g, yield: 70.15%), ES-API: [M +H]⁺= 263.1.

Step 7: 4-benzyloxynaphthalene-2-carbaldehyde (4 g, 15.25 mmol) was dissolved in 1,2-dichloroethane (40 mL), bis(2-methoxyethyl)aminosulfur trifluoride (33.73 g, 152.50 mmol) was added and stirred with heating to 50°C for 16 h. The reaction was quenched with saturated sodium bicarbonate solution (50 mL), extracted with dichloromethane (50 mL), the organic layer was washed with brine (50 mL), dried over anhydrous sodium sulfate and concentrated to give crude 1-benzyloxy-3-(difluoromethyl) naphthalene (3.50 g, yield: 81.4%), ES-API: [M +H]⁺= 285.1.

Step 8: 1-benzyloxy-3-(difluoromethyl) naphthalene (3.4 g, 11.96 mmol) was dissolved in methanol (50 mL). Palladium on carbon (145.24 mg, 1.20 mmol) and palladium hydroxide (167.94 mg, 1.20 mmol) were added, and the system reacted under hydrogen balloon pressure at 25°C for 16 h. The reaction solution was filtered and concentrated to give the crude product 3-(difluoromethyl) naphthalen-1-ol (2.10 g, crude), ES-API: [M +H]⁺= 195.1.

Step 9: 3-(difluoromethyl) naphthalen-1-ol (2 g, 10.30 mmol), 2-bromoethynyl (triisopropyl)silane (3.23 g, 12.36 mmol) were dissolved in 1,4-dioxane (20 mL). Potassium acetate (2.02 g, 20.60 mmol)) and dichlorobis(4-methylisopropylphenyl) ruthenium (II) (630.35 mg, 1.03 mmol) were added, and the system reacted at 100°C for 4 h. The reaction solution was filtered and concentrated to give the crude, which was purified by silica gel column chromatography (petroleum ether/ethyl acetate = 10/1, R_{f} = 0.7) to give the product 3-(difluoromethyl)-8-(2-triisopropylsilylethynyl) naphthalen-1-ol (3.00 g, yield: 77.7%), [M+H]⁺ = 375.2.

Step 10: 3-(difluoromethyl)-8-(2-triisopropylsilylethynyl) naphthalen-1-ol (3 g, 8.01 mmol) was dissolved in dichloromethane (30 mL), N, N-diisopropylethyl amine (3.11 g, 24.03 mmol) was added followed by triflic anhydride (4.52 g, 16.02 mmol) and stirred at -40°C for 0.5 h. Water (30 mL)/dichloromethane (30 mL) was added to the reaction miture and liquid separation was performed, the organic layer was washed with brine (50 mL), dried over anhydrous sodium sulfate, and concentrated to give the crude, which was purified by silica gel column chromatography (petroleum ether/ethyl acetate = 100/1, R_{f} = 0.4) to give the product 3-(difluoromethyl)-8-((triisopropylsilyl)ethynyl)naphthalen-1-yl triflate (2.8 g, yield: 65.5%), [M+H]⁺ = 507.1.

Step 11: 3-(difluoromethyl)-8-((triisopropylsilyl)ethynyl)naphthalen-1-yl triflate (2.8 g, 5.53 mmol) was dissolved in toluene (30 mL). Potassium acetate (867.29 mg, 16.58 mmol), bis-(pinacolato)diboron (2.81 g, 11.05 mmol), and[1,1'-bis(diphenylphosphino)ferrocene]dichloropalladium (404.41 mg, 552.70 µmol) were added, and the system reacted at 110°C for 3 h. The reaction solution was filtered and concentrated to give the crude, which was purified by silica gel column chromatography (petroleum ether/ethyl acetate = 5011, R_{f} = 0.2) to give the product triisopropyl ((6-(difluoromethyl)-8-(4,4,5,5-tetramethyl-1,3,2-dioxaborolan-2-yl)naphthalen-1-yl)ethynyl)silane (1.88 g, yield: 66.70%), [M+H]⁺ = 485.3. ¹H NMR (400 MHz, CDCl₃) δ7.97 - 7.95 (m, 1H), 7.89-7.82 (m, 3H), 7.46 - 7.36 (m, 1H), 6.92-6.62 (m, 1H) ,1.43(s, 12H), 1.15 (m, 21H).

Step 12: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (100 mg, 0.180 mmol), triisopropyl ((6-(difluoromethyl)-8-(4,4,5,5-tetramethyl-1,3,2-dioxoboran-2-yl)naphthalen-1-yl)ethynyl)silane (174 mg, 0.359 mmol), potassium phosphate (13 mg, 0.539 mmol) and[n-butyldi (1-adamantyl)phosphino](2-amino-1,1'-biphenyl-2-yl)palladium (II) methanesulfonate (39 mg, 0.018 mmol) were dissolved in tetrahydrofuran (5 mL) and water (1 mL) and the reaction mixture was heated to 60°C with stirring for 3 h. The reaction was determined to be complete by LCMS, the reaction solution was concentrated under reduced pressure, and the crude was purified by silica gel column chromatography (dichloromethane: methanol = 0-3%) to give tert-butyl (1R,5S)-3-(7-3-(difluoromethyl)-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (121 mg, yield: 77%). ES-API: [M+H]⁺= 879.4.

Step 13: cesium fluoride (209 mg, 1.376 mmol) was added to tert-butyl (1R,5S)-3-(7-(3-(difluoromethyl)-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (121 mg, 0.138 mmol) in N, N-dimethylformamide (10 mL), the reaction mixture was stirred at room temperature for 1 h. The reaction was determined to be complete by LCMS, the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(7-(3-(difluoromethyl)-8-ethynylnaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (99 mg, yield: 99%). ES-API: [M+H]⁺= 723.3. Step 14: a 4 M solution of hydrochloric acid/dioxane solution (2 mL) was slowly added to a solution of tert-butyl (1R,5S)-3-(7-(3-(difluoromethyl)-8-ethynylnaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (99 mg, 0.137 mmol) in acetonitrile (1 mL) under an ice-water bath condition. The reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 ml/min; chromatographic conditions: 15 ml-35-85-13 min; column temperature: room temperature) to give 4-((1R,5S)-3,8-diazabicyclo[3.2.1]octan-3-yl)-7-(3-(difluoromethyl)-8-ethynylnaphthalen-1-yl)-2-((2,6-dimethylenetetrahydro-1H-pyrrolizin-7a(5H)-yl)methoxy)-8-fluoropyrido[4,3-d]pyrimidine (Z197, 23.61 mg, yield: 28%). ES-API: [M+H]⁺= 623.3. ¹H NMR (500 MHz, DMSO-d₆) δ 9.06 (s, 1 H), 8.42 (s, 1 H), 8.28 (d, J= 8.5 Hz, 1 H), 7.84 (d, J= 7.0 Hz, 1 H), 7.70- 7.66 (m, 2 H), 7.40- 7.18 (m, 1 H), 4.94 (d, J= 11.5 Hz, 4 H), 4.48- 4.46 (m, 1 H), 4.33- 4.31 (m, 1 H), 4.07 (s, 2 H), 3.78 (s, 1 H), 3.65- 3.57 (m, 6 H), 3.23- 3.19 (m, 2 H), 2.64- 2.61 (m, 2 H), 2.50- 2.45 (m, 2 H), 1.68 (s, 4 H).

### Example 38: Synthesis of 4-((1R,5S)-3,8-diazacyclo[3.2.1]octan-3-yl)-2-((2,6-dimethyltetrahydro-1H-pyrrolizin-7a(5H)-yl)methoxy)-7-(8-ethynyl-6-fluoronaphthalen-1-yl)-8-fluoropyrido[4,3-d]pyrimidine (Z198)

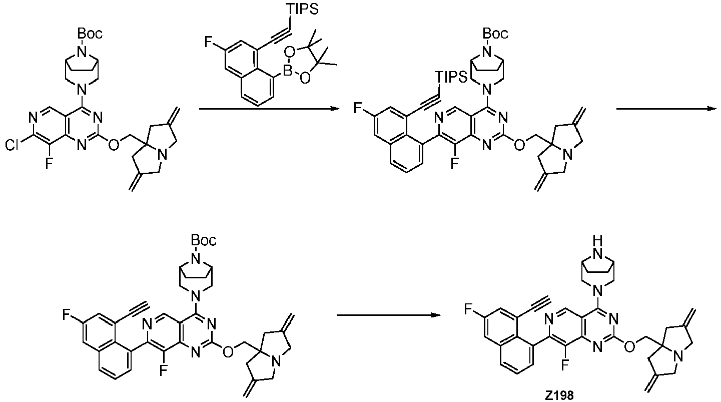

Step 1: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, 0.09mmol), ((3-fluoro-8-(4,4,5,5-tetramethyl-1,3,2-dioxaborolan-2-yl)naphthalen-1-yl)ethynyl)triisopropylsilane (122 mg, 0.27 mmol), potassium phosphate (57 mg, 0.27 mmol) and[n-butyldi (1-adamantyl)phosphino](2-amino-1,1'-biphenyl-2-yl)palladium (II) methanesulfonate (9.8 mg, 0.013 mmol) were dissolved in tetrahydrofuran (5 mL) and water (1 mL) and the reaction mixture was heated to 65°C and stirred for 3 h. The reaction was determined to be complete by LCMS, the reaction solution was concentrated under reduced pressure, the crude was purified by silica gel column chromatography (dichloromethane: methanol = 20:1) to give tert-butyl (1R,5S)-3-(2-((2,6-dimethylenetetrahydro-1H-pyrrolizin-7a(5H)-yl)methoxy)-8-fluoro-7-(6-fluoro-8-((triisopropylsilyl)ethynyl)naphthalen-1-yl)pyrido[4,3-d]pyrimidin-4-yl)-3,8-diazabicyclo[3.2.1]octane-8-carboxylate (30 mg, yield: 39%). ES-API: [M+H]⁺= 847.5.

Step 2: cesium fluoride (54 mg, 0.35 mmol) was added to tert-butyl (1R,5S)-3-(2-((2,6-dimethylenetetrahydro-1H-pyrrolizin-7a(SH)-yl)methoxy)-8-fluoro-7-(6-fluoro-8-((triisopropylsilyl)ethynyl)naphthalen-1-yl)pyrido[4,3-d]pyrimidin-4-yl)-3,8-diazabicyclo[3.2.1]octane-8-carboxylate (30 mg, 0.035mmol) in the N, N-dimethylformamide (3 mL) and the reaction mixture was stirred at room temperature for 2 h. The reaction was determined to be complete by LCMS, the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(2-((2,6-dimethylenetetrahydro-1H-pyrrolizin-7a(5H)-yl)methoxy)-7-(8-ethynyl-6-fluoronaphthalen-1-yl)-8-fluoropyrido[4,3-d]pyrimidin-4-yl)-3,8-diazabicyclo[3.2.1]octane-8-carboxylate (25 mg, yield: 100%). ES-API: [M+H]⁺= 691.3.

Step 3: a 4 M hydrochloric acid-dioxane solution (5 mL) was slowly added to a solution of tert-butyl (1R,5S)-3-(2-((2,6-dimethylenetetrahydro-1H-pyrrolizin-7a(5H)-yl)methoxy)-7-(8-ethynyl-6-fluoronaphthalen-1-yl)-8-fluoropyrido[4,3-d]pyrimidin-4-yl)-3,8-diazabicyclo[3.2.1]octane-8-carboxylate (25 mg, 0.035 mmol) in acetonitrile (2 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 mL/min; chromatographic conditions: 15 mL-35-85-13 min; column temperature: room temperature) to give 4-((1R,5S)-3,8-diazacyclo[3.2.1]octan-3-yl)-2-((2,6-dimethyltetrahydro-1H-pyrrolizin-7a(5H)-yl)methoxy)-7-(8-ethynyl-6-fluoronaphthalen-1-yl)-8-fluoropyrido[4,3-d]pyrimidine (Z198, 9 mg, yield: 44%). ES-API: [M+H]⁺= 591.2.

### Example 39: Synthesis of 4-((1R,5S)-3,8-diazabicyclo[3.2.1]octan-3-yl)-6-chloro-2-((2,6-dimethylenetetrahydro-1H-pyrrolizin-7a(5H)-yl))methoxy)-8-fluoro-7-(5-methyl-1H-indazol-4-yl)quinazoline (Z199)

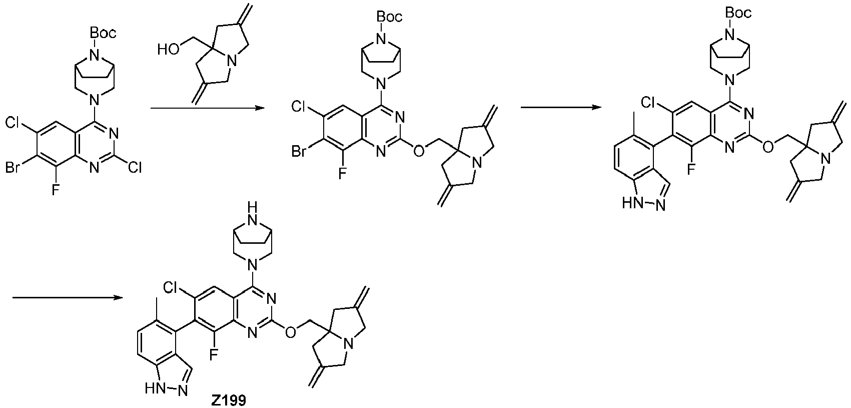

Step 1: the compound tert-butyl (1R,5S)-3-(7-bromo-2,6-dichloro-8-fluoroquinazolin-4-yl)-3,8-diazabicyclo[3.2.1]octane-8-carboxylate (300 mg, 0.60 mmol) was dissolved in 1,4 dioxane (3 mL) and (2,6-dimethylenetetrahydro-1H-pyrrolizin-7a(5H)-yl) methanol (198 mg, 1.2 mmol), cesium carbonate (975 mg, 3.0 mmol) and 4A molecular sieves were added under nitrogen protection, and the system reacted at 100°C for 3 days. Water (20 mL) was added to the reaction solution, which was extracted with ethyl acetate (30 mL X3). The organic phase was washed with saturated brine (20 mL X1), dried over anhydrous sodium sulfate, and rotary dried. The crude was purified by silica gel column chromatography (0-10% methanol/dichloromethane) to give tert-butyl (1R,5S)-3-(7-bromo-6-chloro-2-((2,6-dimethylenetetrahydro-1H-pyrrolizin-7a(5H)-yl)methoxy)-8-fluoroquinazolin-4-yl)-3,8-diazacyclo[3.2.1]octane-8-carboxylate (105 mg, yield: 27.6%). ES-API: [M+H]⁺=634.2.

Step 2: tert-butyl (1R,5S)-3-(7-bromo-6-chloro-2-((2,6-dimethylenetetrahydro-1H-pyrrolizin-7a(5H)-yl)methoxy)-8-fluoroquinazolin-4-yl)-3,8-diazacyclo[3.2.1]octane-8-carboxylate (105 mg, 0.16 mmol) was dissolved in 1,4 dioxane/water (3 mL/0.3 mL). The compound (5-methyl-1H-indazol-3-yl)boronic acid (56 mg, 0.32 mmol), potassium carbonate (44 mg, 0.32 mmol) and tetrakis (triphenylphosphine) palladium (20 mg, 0.016 mmol) were added, and the system reacted at 90°C for 16 h. Upon completion of the reaction, water (20 mL) was added to the reaction solution, which was extracted with ethyl acetate (30 mL X3), and the organic phase was washed with saturated brine (20 mL X1), dried over anhydrous sodium sulfate, and rotary dried. The crude was purified by column chromatography (0-10% methanol/dichloromethane) to give tert-butyl (1R,5S)-3-(6-chloro-2-((2,6-dimethylenetetrahydro-1H-pyrrolizin-7a(5H)-yl)methoxy)-8-fluoro-7-(5-methyl-1H-indazol-4-yl)quinazolin-4-yl)-3,8-diazabicyclo[3.2.1]octane-8-carboxylate (14 mg, yield 12.8%). ES-API: [M+H]⁺= 686.2.

Step 3: tert-butyl (1R,5S)-3-(6-chloro-2-((2,6-dimethylenetetrahydro-1H-pyrrolizin-7a(5H)-yl)methoxy)-8-fluoro-7-(5-methyl-1H-indazol-4-yl)quinazolin-4-yl)-3,8-diazabicyclo[3.2.1]octane-8-carboxylate (14 mg, 0.020 mmol) was dissolved in acetonitrile (2.0 mL) and 4 M hydrochloric acid/dioxane solution (0.500 mL, 2.000 mmol) was added and the reaction solution was allowed to react at 0°C for 1 h. adjusted to pH 9 with 5 mol/L sodium hydroxide aqueous solution, extracted with ethyl acetate (10 mL X3), the organic phase was washed with saturated brine (10 mL X1), dried over anhydrous sodium sulfate, and reverse phase preparation was performed (chromatographic column: Waters XBridge C18; mobile phase: water/acetonitrile (0.1% trifluoroacetic acid) 0-30%; flow rate: 50 mL/min; column temperature: 25°C) to give compound 4-((1R,5S)-3,8-diazabicyclo[3.2.1]octan-3-yl)-6-chloro-2-((2,6-dimethylenetetrahydro-1H-pyrrolizin-7a(5H)-yl)) methoxy)-8-fluoro-7-(5-methyl-1H-indazol-4-yl)quinazoline (Z199, trifluoroacetate, 10.5 mg, yield: 89.5%) as a white solid. ES-API: [M+1]⁺=586.2. ¹H NMR (400 MHz, CD₃OD) δ 8.45 (s, 2H), 8.01 (s, 1H), 7.59 (d, J = 8.6 Hz, 1H), 7.51 (s, 1H), 7.43 (d, J = 8.7 Hz, 1H), 5.13 (s, 4H), 4.65 (s, 2H), 4.51 (s, 2H), 4.08 (d, J = 21.1 Hz, 4H), 3.89 - 3.75 (m, 2H), 3.60 (d, J = 15.1 Hz, 2H), 2.92 (d, J = 17.0 Hz, 2H), 2.70 (d, J = 16.8 Hz, 2H), 2.22 (s, 3H), 2.09 (s, 4H).

### Example 40: Synthesis of 4-((1R,5S)-3,8-diazabicyclo[3.2.1]octan-3-yl)-7-(5-(difluoromethyl)-3-fluoro-2-methylphenyl)-2-((2,6-dimethylenetetrahydro-1H-pyrrolizin-7a(5H)-yl)methoxy)-8-fluoropyrido[4,3-d]pyrimidine (Z200)

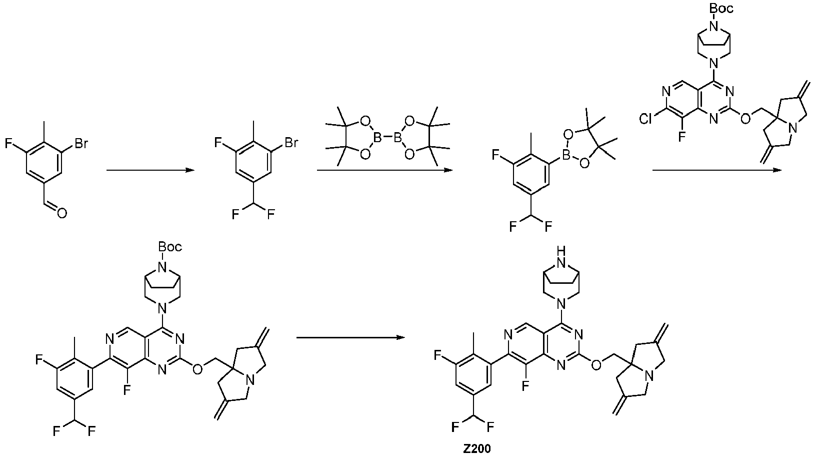

Step 1: 3-bromo-5-fluoro-4-methyl-benzaldehyde (3.9 g, 17.97 mmol) was dissolved in 1,2-dichloroethane (40 mL), bis(2-methoxyethyl)aminosulfur trifluoride (39.75 g, 179.69 mmol) was added, heated to 50°C and stirred for 16 h. The reaction was quenched with saturated sodium bicarbonate solution (50 mL), extracted with dichloromethane (50 mL), the organic layer was washed with brine (50 mL), dried over anhydrous sodium sulfate, and concentrated. The crude was purified by silica gel column chromatography (petroleum ether = 100%, R_{f} = 0.7) to give 1-bromo-5-(difluoromethyl)-3-fluoro-2-methyl-benzene (2.50 g, yield: 55.3%), ES-API: [M+H]⁺= 239.0. Step 2: 1-bromo-5-(difluoromethyl)-3-fluoro-2-methyl-benzene (25 g, 10.46 mmol) was dissolved in toluene (30 mL). Potassium acetate (867.29 mg, 31.38 mmol), bis-(pinacolato)diboron (5.31 g, 20.92 mmol), and[1,1'-bis(diphenylphosphino)ferrocene]dichloropalladium (765.27 mg, 1.05 mmol) were added, and the system reacted at 100°C for 6 h. The reaction solution was filtered, concentrated, and the crude was purified by silica gel column chromatography (petroleum ether/ethyl acetate = 100/1, R_{f} = 0.6) to give the product 2-(5-(difluoromethyl)-3-fluoro-2-methylphenyl)-4,4,5,5-tetramethyl-1,3,2-dioxaborolane (2.4 g, yield: 72.2%), [M+H]⁺ = 287.1. ¹H NMR (400 MHz, Chloroform-d) δ7.84 - 7.80 (m, 1H), 7.24-7.22 (m, 1H), 6.69 - 6.45 (m, 1H), 2.52-2.48 (m, 3H) ,1.34(s, 12H).

Step 3: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, 0.09 mmol), 2-(5-(difluoromethyl)-3-fluoro-2-methylphenyl)-4,4,5,5-tetramethyl-1,3,2-dioxaborolane (77 mg, 0.27 mmol), potassium phosphate (57 mg, 0.27 mmol) and[n-butyldi (1-adamantyl)phosphine](2-amino-1,1'-biphenyl-2-yl)palladium (II) methanesulfonate (10 mg, 0.014 mmol) were dissolved in tetrahydrofuran (5 mL) and water (1 mL) and the reaction mixture was heated to 65°C with stirring for 3 h. The reaction was determined to be complete by LCMS, the reaction solution was concentrated under reduced pressure, and the crude was purified by silica gel column chromatography (dichloromethane: methanol = 20:1) to give tert-butyl (1R,5S)-3-(7-(5-(difluoromethyl)-3-fluoro-2-methylphenyl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, yield: 82%). ES-API: [M+H]⁺= 681.3. Step 4: a 4 M hydrochloric acid-dioxane solution (5 mL) was slowly added to a solution of tert-butyl (1R,5S)-3-(7-(5-(difluoromethyl)-3-fluoro-2-methylphenyl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, 0.07 mmol) in acetonitrile (2 mL) under an ice-water bath condition. The reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase: A: 5 mmol/L ammonium bicarbonate aqueous solution, mobile phase B: acetonitrile; flow rate: 15 mL/min; chromatographic conditions: 15 mL-35-85-13 min; column temperature: room temperature) to give 4-((1R,5S)-3,8-diazabicyclo[3.2.1]octan-3-yl)-7-(5-(difluoromethyl)-3-fluoro-2-methylphenyl)-2-((2,6-dimethylenetetrahydro-1H-pyrrolizin-7a(5H)-yl)methoxy)-8-fluoropyrido[4,3-d]pyrimidine (Z200, 10 mg, yield: 25%). ES-API: [M+H]⁺=581.2.

### Example 41: Synthesis of 4-(4-(3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylquinolin-2-amine (Z201)

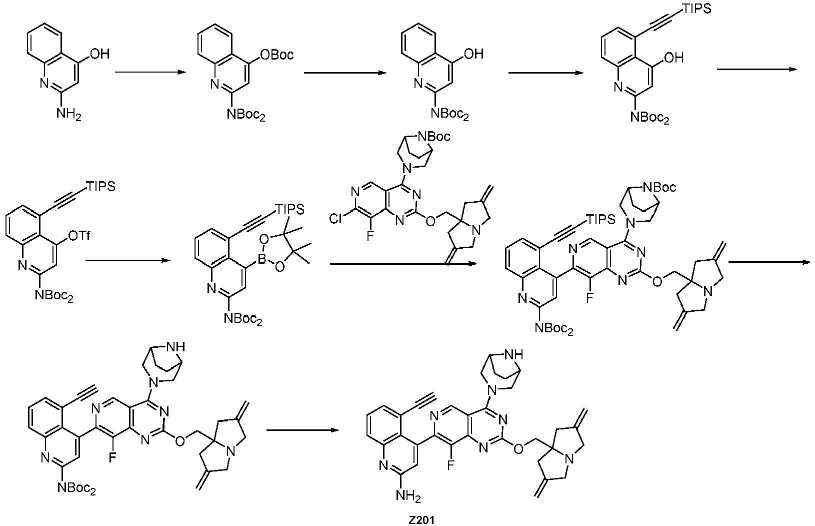

Step 1: di-tert-butyl dicarbonate (6.81 g, 31.2 mmol) followed by 4-dimethylamino pyridine (0.23 g, 1.87 mmol) was added to a suspension of 2-amino-4-hydroxyquinoline hydrate (1.0 g, 6.24 mmol) in dichloromethane (50 mL) at room temperature and the reaction was stirred at room temperature overnight. The reaction solution was concentrated directly and the crude was purified by silica gel column chromatography (ethyl acetate/petroleum ether: 0-10%) to give 2-methylpropan-2-yl ({[(2-methylpropan-2-yl)oxy]carbonyl}[4-({[(2-methylpropan-2-yl)oxy]carbonyl}oxy) quinolin-2-yl]amino) formate (1.60 g, yield: 56%). ES-API: [M+H]⁺= 461.2. Step 2: sodium carbonate (540 mg, 5.1 mmol) was added to a solution of 2-methylpropan-2-yl (f [(2-methylpropan-2-yl)oxy]carbonyll [4-({ [(2-methylpropan-2-yl)oxy]carbonyl}oxy) quinolin-2-yl]amino) carboxylate (1.60 g, 3.47 mmol) in methanol/water (30 mL/6 mL), stirred and allowed to react at room temperature for 2 h. Acetic acid was slowly added dropwise to the reaction solution to adjust the pH until the solid no longer precipitates out, suction filtered, the filter residue was taken, and dried at room temperature overnight to obtain 2-methylpropan-2-yl[(4-hydroxyquinolin-2-yl){ [(2-methylpropan-2-yl)oxy]carbonyl} amino] formate (1.03 g, yield: 82%). ES-API: [M+H]⁺= 361.3.

Step 3: to the sealed tube was added 2-methylpropan-2-yl[(4-hydroxyquinolin-2-yl){[(2-methylpropan-2-yl)oxy]carbonyl}amino]formate (400 mg, 1.11 mmol), potassium acetate (218 mg, 2.22 mmol), dichlorobis(4-methylisopropylphenyl) ruthenium (II) dimer (34 mg, 0.05 mmol), (bromoethynyl)triisopropylsilane (348 mg, 1.33 mmol) and 1,2-dichloroethane (10 mL) successively and the reaction was sealed at 95°C overnight. After cooling to room temperature, suction filtered with diatomite, the filtrate was concentrated, and the crude was purified by flash silica gel column (ethyl acetate/petroleum ether: 0-10%) to give tert-butyl (4-hydroxy-5-((triisopropylsilyl)ethynyl)quinolin-2-yl)(tert-butoxycarbonyl) carbamate (435 mg, yield: 72%). ES-API: [M+H]⁺= 541.3.

Step 4: under nitrogen protection, tert-butyl (4-hydroxy-5-((triisopropylsilyl)ethynyl)quinolin-2-yl)(tert-butoxycarbonyl) carbamate (455 mg, 0.84 mmol) was dissolved in anhydrous dichloromethane (20 mL), N, N-diisopropylethyl amine (0.18 mL, 1.01 mmol) was added thereto and the reaction solution was cooled to -40°C. Triflic anhydride (0.17 mL, 1.01 mmol) was slowly added dropwise thereto and allowed to react at -40°C for 0.5 h. Saturated sodium bicarbonate solution was slowly added thereto and warmed to room temperature. The reaction solution was extracted with dichloromethane 3 times, and the organic phase was washed with saturated brine, dried over anhydrous sodium sulfate, and rotary dried. The crude was purified by silica gel column chromatography (ethyl acetate/petroleum ether: 0-8%) to give 2-((tert-butoxycarbonyl)(tert-butoxycarbonyl)amino)-5-((triisopropylsilyl)ethynyl)quinolin-4-yl triflate (505 mg, yield: 89%). ES-API: [M+H]⁺= 673.3. ¹H NMR (500 MHz, CDCl₃) δ 7.97 (d, *J* = 8.4 Hz, 1H), 7.88 (d, *J* = 7.3 Hz, 1H), 7.69 (t, *J* = 7.9 Hz, 1H), 7.64 (s, 1H), 1.48 (s, 21 H), 1.16 (d, *J* = 6.5 Hz, 18H).

Step 5: a sealed tube was charged with 2-((tert-butoxycarbonyl)(tert-butoxycarbonyl)amino)-5-((triisopropylsilyl)ethynyl)quinolin-4-yl triflate (500 mg, 0.74 mmol), 1,1-bis(diphenylphosphino)ferrocene palladium dichloride (54 mg, 0.07 mmol), potassium acetate (146 mg, 1.49 mmol), bis-(pinacolato)diboron (283 mg, 1.12 mmol) and anhydrous dioxane (7.0 mL) successively, which was displaced with nitrogen for 4 times and reacted at 90°C overnight. After cooling to room temperature, suction filtered with diatomite, the filtrate was concentrated, and the crude was purified by silica gel column chromatography (ethyl acetate/petroleum ether: 0-10%) to give 2-methylpropan-2-yl ({[(2-methylpropan-2-yl)oxy]carbonyl)[4-(trifluoromethyl sulfonyloxy)-5-{[tris (propan-2-yl)silyl]ethynyl}quinolin-2-yl]amino) carboxylate (258 mg, yield: 53%). ES-API: [M+H]⁺= 636.4.

Step 6: a microwave tube was charged with 2-methylpropan-2-yl ({[(2-methylpropan-2-yl)oxy]carbonyl}[4-(4,4,5,5-tetramethyl-1,3,2-dioxaborolan-2-yl)-5-{[tris (propan-2-yl)silyl]ethynyl}quinolin-2-yl]amino) carboxylate (116 mg, 0.18 mmol), tert-butyl 3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, 0.09 mmol), potassium phosphate (57 mg, 0.27 mmol) and[n-butyldi (1-adamantyl)phosphino](2-amino-1,1'-biphenyl-2-yl)palladium (II) methanesulfonate (20 mg, 0.027 mmol), anhydrous tetrahydrofuran (3.0 mL), water (0.6 mL), which was displaced with nitrogen for 4 times, reacted under microwave condition at 60°C for 20 min. After the reaction solution was cooled to room temperature, water was added to the reaction solution and extracted with ethyl acetate 3 times. The organic phase was combined and washed with saturated brine. The organic phase was dried over anhydrous sodium sulfate and filtered. The filtrate was rotary dried under reduced pressure. The crude was purified by silica gel column chromatography (ethyl acetate/petroleum ether: 0-80%) to give the product 2-methylpropan-2-yl {[4-(2-{[(2,6-dimethylmethylene-2,3,5,6,7,7a-hexahydro-1H-pyrrolizin-7a-yl)methyl]oxy}-8-fluoro-4-(8-{[(2-methylpropan-2-yl)oxy]carbonyl}-3,8-diazabicyclo[3.2.1]octan-3-yl)pyrido[4,3-d]pyrimidin-7-yl)-5-{ [tris (propan-2-yl)silyl]ethynyl }quinolin-2-yl] {[(2-methylpropan-2-yl)oxy]carbonyl}amino } carboxylate (95 mg, yield: 100%). ES-API: [M+H]⁺= 1045.7.

Step 7: 2-methylpropan-2-yl {[4-(2-{[(2,6-dimethylmethylene-2,3,5,6,7,7a-hexahydro-1H-pyrrolizin-7a-yl)methyl]oxy} -8-fluoro-4-(8- {[(2-methylpropan-2-yl)oxy]carbonyl }-3,8-diazabicyclo[3.2.1]octan-3-yl)pyrido[4,3-d]pyrimidin-7-yl)-5-{[tris (propan-2-yl)silyl]ethynyl)quinolin-2-yl]{[(2-methylpropan-2-yl)oxy]carbonyl)amino)carboxylate (106 mg, 0.10 mmol) was dissolved in N, N-dimethylformamide (5.0 mL), cesium fluoride (154 mg, 1.01 mmol) was added, and the system reacted at room temperature for 2 h. Upon completion of the reaction, water was added to the reaction solution, which was extracted three times with ethyl acetate, the organic phase was combined, washed with saturated brine, dried over anhydrous sodium sulfate, filtered, and the filtrate was rotary dried under reduced pressure to give crude 2-methylpropan-2-yl {[4-(2-{[(2,6-dimethylene-2,3,5,6,7,7a-hexahydro-1H-pyrazin-7a-yl)methyl]oxy}-8-fluoro-4-(8-{[(2-methylpropan-2-yl)oxy]carbonyl}-3,8-diazabicyclo[3.2.1]octan-3-yl)pyrido[4,3-d]pyrimidin-7-yl)-5-ethynylquinolin-2-yl] {[(2-methylpropan-2-yl)oxy]carbonyl} amino } carboxylate, the crude was used in the next reaction without purification. ES-API: [M+H]⁺=889.4.

Step 8: dissolve crude 2-methylpropan-2-yl {[4-(2-{[(2,6-dimethylene-2,3,5,6,7,7a-hexahydro-1H-pyrazin-7a-yl)methyl]oxy}-8-fluoro-4-(8-{[(2-methylpropan-2-yl)oxy]carbonyl}-3,8-diazabicyclo[3.2.1]octan-3-yl)pyrido[4,3-d]pyrimidin-7-yl)-5-ethynylquinolin-2-yl] {[(2-methylpropan-2-yl)oxy] carbonyl} amino} carboxy late in acetonitrile (5.0 mL), 4 M hydrochloric acid/dioxane solution (3.0 mL, 12.0 mmol) was added in an ice bath, and the system reacted at 50°C for 3 h. Upon completion of the reaction, the solvent was rotary dried under reduced pressure and the crude was purified by preparative HPLC (formic acid method) to give the target compound 4-(4-(3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylquinolin-2-amine (Z201, dicarboxylic acid salt, 32 mg, yield: 55%). ES-API: [M+H]⁺= 589.3. ¹H NMR (500 MHz, DMSO-d₆) δ 9.06 (s, 1H), 8.25 (s, 3H), 7.61 (d, *J* = 7.7 Hz, 1H), 7.52 - 7.44 (m, 1H), 7.29 (d, *J* = 7.1 Hz, 1H), 6.78 (s, 1H), 6.76 (s, 2H), 4.94 (d, *J* = 10.1 Hz, 4H), 4.52 (d, *J* = 12.4 Hz, 1H), 4.34 (d, *J =* 12.5 Hz, 1H), 4.07 (s, 2H), 3.80 - 3.69 (m, 3H), 3.68 - 3.60 (m, 3H), 3.59 (s, 1H), 3.21 (d, *J* = 14.4 Hz, 2H), 2.67 - 2.56 (m, 2H), 2.45 (s, 1H), 2.50 - 2.40 (m, 1H), 1.75 (s, 4H).

### Example 42: Synthesis of 4-(4-((1R,5S)-3,8-diazabicyclo[3.2.1]octan -3-yl)-2-((2,6-dimethylenetetrahydro-1H-pyrrolizin-7a(5H)-yl)methoxy)pyrido[4,3-d]pyrimidin-7-yl)-5-ethynaphthalen-2-ol (Z202)

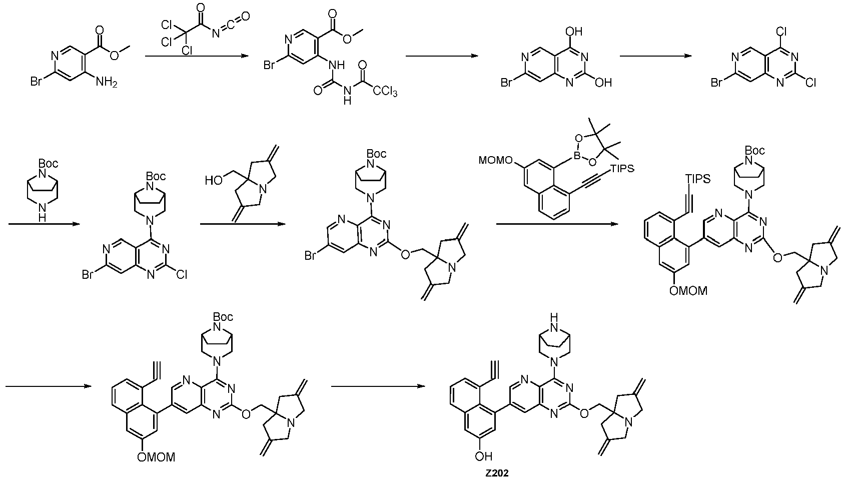

Step 1: methyl 4-amino-6-bromonicotinate (2 g, 8.66 mmol) was dissolved in tetrahydrofuran (30 mL) and 2,2,2-trichloroacetyl isocyanate (2.45 g, 12.98 mmol) was added, and the system reacted at room temperature for 2 h. The reaction solution was concentrated and the residue was triturated with petroleum ether/ethyl acetate = 10/1 (10 mL) to give the product methyl 6-bromo-4-(3-(2,2,2-trichloroacetyl)ureido) nicotinate (3.2 g, yield: 88%) ES-API: [M+H]⁺ = 417.9.

Step 2: methyl 6-bromo-4-(3-(2,2,2-trichloroacetyl)ureido) nicotinate (2.2 g, 5.25 mmol) was dissolved in methanol (25 mL). Ammonia/methanol solution (7 M, 9.74 mL) was added, and the system reacted at room temperature for 1 h. The reaction solution was concentrated and the residue was triturated with petroleum ether/ethyl acetate = 10/1 (10 mL) to give the product 7-bromopyrido[4,3-d]pyrimidine-2,4-diol (1.1 g, yield: 86.7%). ES-API: [M+H]⁺ = 241.8.

Step 3: 7-bromopyrido[4,3-d]pyrimidine-2,4-diol (1 g, 4.13 mmol) was dissolved in toluene (10 mL) and phosphorus oxychloride (3.17 g, 20.66 mmol) was added followed by diisopropylethyl amine (2.67 g, 20.66 mmol) and the mixture was allowed to react at 110°C for 2 h. After cooling to room temperature, phosphorus oxychloride was rotary dried to remove under reduced pressure. The crude was adjusted to pH 6-7 with saturated sodium bicarbonate solution, extracted with ethyl acetate (10 mL). The organic phase was dried over anhydrous sodium sulfate and concentrated. The crude was purified by silica gel column chromatography (ethyl acetate/petroleum ether = 0-25%) to give 7-bromo-2,4-dichloropyrido[4,3-d]pyrimidine (750 mg, yield: 65.2%). ES-API: [M+H]⁺=279.9.

Step 4: 7-bromo-2,4-dichloropyrido[4,3-d]pyrimidine (750 mg, 2.69 mmol) was dissolved in dichloromethane (2 mL), diisopropyl ethyl amine (1.73 g, 13.45 mmol) was added, tert-butyl-3,8-azabicyclo[3.2.1]octane-8-carboxylate (627 mg, 2.96 mmol) was added at -40°C, and the system reacted for 0.5 h at -40°C. Water (20 mL) and dichloromethane (20 mL) were added to the reaction solution and liquid separation was performed. The organic phase was dried over anhydrous sodium sulfate and rotary dried. The crude was purified by silica gel column chromatography (ethyl acetate/petroleum ether = 5/1) to give tert-butyl 3-(7-bromo-2-chloropyrido[4,3-d]pyrimidin-4-yl)-3,8-diazabicyclo[3.2.1]octane-8-carboxylate (700 mg, yield: 57.8%). ES-API: [M+H]⁺=456.

Step 5: sodium hydride (792 mg, 60% dispersion in mineral oil, 5.5 mmol) was dissolved in tetrahydrofuran (5 mL). (2,6-methylene-1H-pyrrolizin-7a(5H)-yl)-methanol (363 mg, 2.2 mmol) was added, and the system reacted for 15 min.Then tert-butyl 3-(7-bromo-2-chloropyrido[4,3-d]pyrimidin-4-yl)-3,8-diazabicyclo[3.2.1]octane-8-carboxylate (500 mg, 1.1 mmol) was added. The system reacted at room temperature for 2 h, quenched with water (10 mL), extracted with ethyl acetate (10 mL), the organic phase was dried over anhydrous sodium sulfate, concentrated, and purified by silica gel column chromatography (ethyl acetate/petroleum ether = 1/1) to give tert-butyl 3-(7-bromo-2-((2,6-dimethylenetetrahydro-1H-pyrrolizin-7a(5H)-yl)methoxy)pyrido[3,2-d]pyrimidin-4-yl)-3,8-diazabicyclo[3.2.1]octane-8-carboxylate (480 mg, 74.97% yield). ES-API: [M+H]⁺=583.2.

Step 6: tert-butyl 3-(7-bromo-2-((2,6-dimethylenetetrahydro-1H-pyrrolizin-7a(5H)-yl)methoxy)pyrido[3,2-d]pyrimidin-4-yl)-3,8-diazabicyclo[3.2.1]octane-8-carboxylate (100 mg, 0.171 mmol) was dissolved in tetrahydrofuran/water (1 mL/0.2 mL). ((6-(methoxymethoxy)-8-(4,4,5,5-tetramethyl-1,3,2-dioxaborinan-2-yl)naphthalen-1-yl)ethynyl)triisopropylsilane (169.5 mg, 0.342 mmol), potassium phosphate (108.9 mg, 0.514 mmol) and (docosyl-n-butylphosphino) (2'-amino-1,1'-biphenyl-2-yl)palladium (II) methanesulfonate dichloromethane complex (12.5 mg, 0.017 mmol) were added, and the system reacted under microwave condition at 80°C for 1 h. Water (10 mL) and ethyl acetate (10 mL) were added to the reaction solution and liquid separation was performed, the organic phase was washed with brine (10 mL), dried over anhydrous sodium sulfate, rotary dried, and purified by column chromatography (ethyl acetate = 100%) to give the product tert-butyl 3-(2-((2,6-dimethylenetetrahydro-1H-pyrrolizin-7a-yl)methoxy)-7-(3-(methoxymethoxy)-8-((triisopropylsilyl)ethynyl)naphthalen-1-yl)pyrido[3,2-d]pyrimidin-4-yl-3,8-diazabicyclo[3.2.1]octane-8-carboxylate (140 mg, yield: 93.99%). ES-API: [M+1]⁺=871.5.

Step 7: tert-butyl 3-(2-((2,6-dimethylenetetrahydro-1H-pyrrolizin-7a-yl)methoxy)-7-(3-(methoxymethoxy)-8-((triisopropylsilyl)ethynyl)naphthalen-1-yl)pyrido[3,2-d]pyrimidin-4-yl-3,8-azabicyclo[3.2.1]octane-8-carboxylate (140 mg, 0.161 mmol) was dissolved in N, N-dimethylformamide (1 mL). Cesium fluoride (106.2 mg, 1.61 mmol) and a drop of water were added, and the system reacted at room temperature for 3 h. Water (5 mL) and ethyl acetate (5 mL) were added to the reaction solution and liquid separation was performed, and the organic phase was washed with brine (5 mL), dried over anhydrous sodium sulfate and rotary dried to give tert-butyl 3-(2-((2,6-dimethylenetetrahydro-1H-pyrrolizin-7a(5H)-yl)methoxy)-7-(8-ethynyl-3-(methoxymethoxy)naphthalen-1-yl)pyrido[3,2-d]pyrimidin-4-yl)-3,8-diazabicyclo[3.2.1]octane-8-carboxylate (100 mg, crude). ES-API: [M+1]⁺=715.4.

Step 8: to tert-butyl 3-(2-((2,6-dimethylenehexahydro-1H-pyrrolizin-7a(5H)-yl)methoxy)-7-(8-ethynyl-3-(methoxymethoxy)naphthalen-1-yl)pyrido[3,2-d]pyrimidin-4-yl)-3,8-diazabicyclo[3.2.1]octane-8-carboxylate (100 mg, 0.139 mmol) in acetonitrile (1 mL) was added 4 M hydrochloric acid/dioxane solution (1 mL) in an ice bath, and the system reacted for 0.5 h in an ice bath. Water (5 mL) and ethyl acetate (5 mL) were added to the reaction solution and liquid separation was performed. The organic phase was dried over anhydrous sodium sulfate and rotary dried. The crude product was triturated and purified (petroleum ether/ethyl acetate = 3/1) to give 4-(4-((1R,5S)-3,8-diazabicyclo[3.2.1]octan-3-yl)-2-((2,6-dimethylenetetrahydro-1H-pyrrolizin-7a(5H)-yl)methoxy)pyrido[4,3-d]pyrimidin-7-yl)-5-ethynylnaphthalen-2-ol (Z202, 35 mg, yield: 41.65%). ES-API: [M+1]⁺=571.3. ¹HNMR (400 MHz, Chloroform-d) δ8.50 (s,1H), 7.96-7.90 (d, *J*=12 Hz, 1H), 7.75 (s,1H), 7.47-7.42 (m,2H), 7.32 (s,1H), 7.07 (s,1H), 4.96(d, *J*=12 Hz, 4H), 4.05-4.03 (m,2H), 3.76-3.75 (m,1H), 3.65-3.64 (m,3H), 3.60-3.56 (m, 2H), 3.23-3.19 (m,3H), 2.66-2.62 (m,2H), 2.51-2.48 (m, 5H), 1.99-1.96 (m,1H), 1.76-1.69 (m,4H).

### Example 43: Synthesis of 4-(4-(3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynyl-1-fluoronaphthalen-2-ol (Z203)

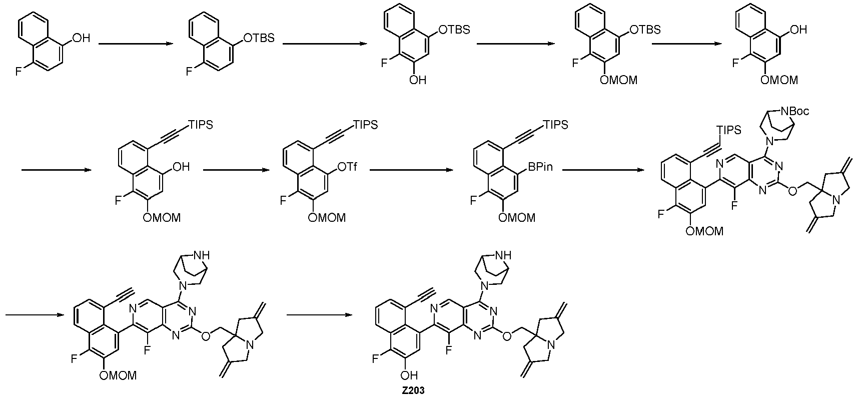

Step 1: a solution of 4-fluoronaphthalen-1-ol (811 mg, 5.0 mmol) in N, N-dimethylformamide (15.0 mL) was cooled to 0°C, and tert-butyldimethylsilyl chloride (904 mg, 6.0 mmol) and imidazole (408 mg, 6.0 mmol) were successively added thereto. After the reaction was warmed to room temperature and stirred for 2 h, saturated sodium bicarbonate aqueous solution was added thereto, and extracted with petroleum ether for 3 times. The organic phase was collected and washed with saturated brine, dried over anhydrous sodium sulfate, and rotary dried. The crude was purified by flash silica gel column (petroleum ether: 100%) to give tert-butyl ((4-fluoronaphthalen-1-yl)oxy) dimethylsilane (965 mg, yield: 70%).

Step 2: a solution of tert-butyl ((4-fluoronaphthalen-1-yl)oxy) dimethylsilane (1.11 g, 4.0 mmol) in anhydrous tetrahydrofuran (20 mL) was cooled to -78°C, and sec-butyllithium (6.2 mL, 1.3 M in n-hexane, 8.0 mmol) was slowly added dropwise thereto. After 0.5 h of reaction, trimethoxyborane (0.67 mL, 6.0 mmol) was added dropwise thereto. After reacting at -78°C for 0.5 h, the reaction was warmed to 0°C, and acetic acid (1.0 mL), 30% hydrogen peroxide (2.0 mL) were successively slowly added thereto, followed by stirring for 1 h, and then warmed to room temperature for 0.5 h. The reaction solution was extracted with dichloromethane 3 times, and the organic phase was collected and washed with saturated brine, dried over anhydrous sodium sulfate, and rotary dried to give 4-((tert-butyldimethylsilyl)oxy)-1-fluoronaphthalen-2-ol, the crude was used in the next step without purification.

Step 3: N,N-diisopropylethyl amine (1.0 mL, 6.0 mmol) followed by bromomethyl methyl ether (0.49 mL, 6.0 mmol) was added dropwise to a solution of crude 4-((tert-butyldimethylsilyl)oxy)-1-fluoronaphthalen-2-ol in dichloromethane (40 mL) at 0°C under nitrogen protection. After reacting for 1 h, saturated sodium bicarbonate aqueous solution was added thereto, the organic phase was separated, the aqueous phase was extracted twice with dichloromethane, the organic phase was collected and washed with saturated brine, dried over anhydrous sodium sulfate, and rotary dried. The crude was purified by flash silica gel column (ethyl acetate/petroleum ether: 0-8%) to give tert-butyl ((4-fluoro-3-(methoxymethoxy)naphthalen-1-yl)oxy) dimethylsilane (442 mg, yield over two steps: 33%).

Step 4: tetrabutylammonium fluoride (2.6 mL, 1.0 Min tetrahydrofuran solution, 2.6 mmol) was slowly added to a solution of tert-butyl ((4-fluoro-3-(methoxymethoxy)naphthalen-1-yl)oxy) dimethylsilane (442 mg, 1.31 mmol) in tetrahydrofuran (10 mL) at room temperature. After stirring at room temperature for 1 h, saturated ammonium chloride aqueous solution was added thereto, extracted three times with dichloromethane, the organic phase was collected and washed with saturated brine, dried over anhydrous sodium sulfate, and rotary dried. The crude was purified by flash silica gel column (ethyl acetate/petroleum ether: 0-35%) to give 4-fluoro-3-(methoxymethoxy)naphthalen-1-ol (261 mg, yield: 89%). ES-API: [M+H]⁺= 223.1.

Step 5: 4-fluoro-3-(methoxymethoxy)naphthalen-1-ol (261 mg, 1.18 mmol), potassium acetate (231 mg, 2.35 mmol), dichlorobis(4-methylisopropylphenyl) ruthenium (II) dimer (36 mg, 0.06 mmol), (bromoethynyl)triisopropylsilane (399 mg, 1.53 mmol) and 1,2-dichloroethane (5 mL) were successively added in a sealed tube, and the system reacted overnight at 95°C in a sealed tube. After cooling to room temperature, suction filtered with diatomite, the filtrate was concentrated, and the crude was purified by flash silica gel column (ethyl acetate/petroleum ether: 0-8%) to give 4-fluoro-3-[(methoxymethyl)oxy]-8-{[tris (propan-2-yl)silyl]ethynyl}naphthalen-1-ol (219 mg, yield: 46%). ES-API: [M+H]⁺= 403.3.

Step 6: 4-fluoro-3-[(methoxymethyl)oxy]-8-{[tris (propan-2-yl)silyl]ethynyl}naphthalen-1-ol (145 mg, 0.36 mmol) was dissolved in anhydrous dichloromethane (10 mL) under nitrogen protection, N, N-diisopropylethyl amine (0.19 mL, 1.08 mmol) was added and the reaction solution was cooled to -40°C, triflic anhydride (0.09 mL, 0.54 mmol) was slowly added dropwise. After reacting for 0.5 h at -40°C, saturated sodium bicarbonate solution was slowly added thereto and warmed to room temperature. The reaction solution was extracted three times with dichloromethane, the organic phase was washed with saturated brine, dried over anhydrous sodium, and rotary dried. The crude was purified by flash silica gel column (ethyl acetate/petroleum ether: 0-8%) to give 4-fluoro-3-(methoxymethoxy)-8-((triisopropylsilyl)ethynyl)naphthalen-1-yl triflate (138 mg, yield: 72%). ¹H NMR (500 MHz, CDCl₃) δ 8.03 (d, *J* = 8.5 Hz, 1H), 7.80 (dd, *J* = 7.2, 0.8 Hz, 1H), 7.54 (d, *J* = 6.9 Hz, 1H), 7.50 (dd, *J* = 8.4, 7.4 Hz, 1H), 5.28 (s, 2H), 3.54 (s, 3H), 1.24 - 1.17 (m, 3H), 1.14 (d, *J* = 6.4 Hz, 18H).

Step 7: 4-fluoro-3-(methoxymethoxy)-8-((triisopropylsilyl)ethynyl)naphthalen-1-yl triflate (213 mg, 0.40 mmol), 1,1-bis(diphenylphosphino)ferrocene palladium dichloride (58 mg, 0.08 mmol), potassium acetate (117 mg, 1.20 mmol), bis-(pinacolato)diboron (202 mg, 0.80 mmol) and anhydrous dioxane (5.0 mL) were successively added in a sealed tube, which was displaced with nitrogen for 4 times, and the system reacted at 110 °C for 24 h. After cooling to room temperature, suction filtered with diatomite, the filtrate was concentrated, and the crude was purified by flash silica gel column (ethyl acetate/petroleum ether: 0-4%) to give ({5-fluoro-6-[(methoxymethyl)oxy]-8-(4,4,5,5-tetramethyl-1,3,2-dioxaborolan-2-yl)-1-naphthyl}ethynyl)[tri (propan-2-yl)]silane (80 mg, yield: 39%). ES-API: [M+H]⁺= 513.3.

Step 8: a microwave tube was charged with ({5-fluoro-6-[(methoxymethyl)oxy]-8-(4,4,5,5-tetramethyl-1,3,2-dioxaborolan-2-yl)-1-naphthyl}ethynyl)[tri (propan-2-yl)]silane (78 mg, 0.15 mmol), tert-butyl 3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (50 mg, 0.09 mmol), potassium phosphate (57 mg, 0.27 mmol) and[n-butyldi (1-adamantyl)phosphino](2-amino-1,1'-biphenyl-2-yl)palladium (II) methanesulfonate (20 mg, 0.027 mmol), anhydrous tetrahydrofuran (3.0 mL), water (0.6 mL), which was displaced with nitrogen for 4 times, reacted under microwave condition at 60°C for 30 min. After the reaction solution was cooled to room temperature, water was added to the reaction solution, which was extracted with ethyl acetate 3 times. The organic phase was combined, washed with saturated brine, dried over anhydrous sodium sulfate, and filtered, and the filtrate was rotary dried under reduced pressure. The crude was purified by preparative thin layer chromatography (ethyl acetate/petroleum ether = 50%) to give the product tert-butyl 3-(2-((2,6-dimethylenetetrahydro-1H-pyrrolizin-7a(5H)-yl)methoxy)-8-fluoro-7-(4-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (10 mg, yield: 10%). ES-API: [M+H]⁺= 907.4.

Step 9: tert-butyl 3-(2-((2,6-dimethylenetetrahydro-1H-pyrrolizin-7a(5H)-yl)methoxy)-8-fluoro-7-(4-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (10 mg, 0.01 mmol) was dissolved in N, N-dimethylformamide (1.0 mL). Cesium fluoride (17 mg, 0.11 mmol) was added, and the system reacted at room temperature for 0.5 h. Upon completion of the reaction, water was added into the reaction solution and extracted with ethyl acetate 3 times, the organic phase was combined and washed with saturated brine, the organic phase was dried over anhydrous sodium sulfate, filtered and the filtrate was rotary dried under reduced pressure to give tert-butyl 3-(2-((2,6-dimethylenetetrahydro-1H-pyrrolizin-7a(5H)-yl)methoxy)-7-(8-ethynyl-4-fluoro-3-(methoxymethoxy)naphthalen-1-yl)-8-fluoropyrido[4,3-d]pyrimidin-4-yl)-3,8-diazabicyclo[3.2.1]octane-8-carboxylate as a crude which was used directly in the next reaction without purification. ES-API: [M+H]⁺=751.3.

Step 10: crude tert-butyl 3-(2-((2,6-dimethylenetetrahydro-1H-pyrrolizin-7a(5H)-yl)methoxy)-7-(8-ethynyl-4-fluoro-3-(methoxymethoxy)naphthalen-1-yl)-8-fluoropyrido[4,3-d]pyrimidin-4-yl)-3,8-diazabicyclo[3.2.1]octane-8-carboxylate was dissolved in acetonitrile (1.0 mL) and 4 M hydrochloric acid/dioxane solution (1.0 mL, 4.0 mmol) was added in an ice bath, reacted at 0°C for 15 min and then warmed to room temperature for 45 min. Upon completion of the reaction, the solvent was rotary dried under reduced pressure and the crude was purified by preparative HPLC (formic acid method) to give the target compound 4-(4-(3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynyl-1-fluoronaphthalen-2-ol (Z203, dicarboxylic acid salt, 2.1 mg, yield: 34%). ES-API: [M+H]⁺= 607.3.

### Example 44: Synthesis of 4-((1R,5S)-3,8-diazabicyclo[3.2.1]octan-3-yl)-2-((2,6-dimethylenetetrahydro-1H-pyrrolizin-7a(5H)-yl)methoxy)-7-(4-ethynyl-1H-indazol-3-yl)-8-fluoroquinazoline (Z204)

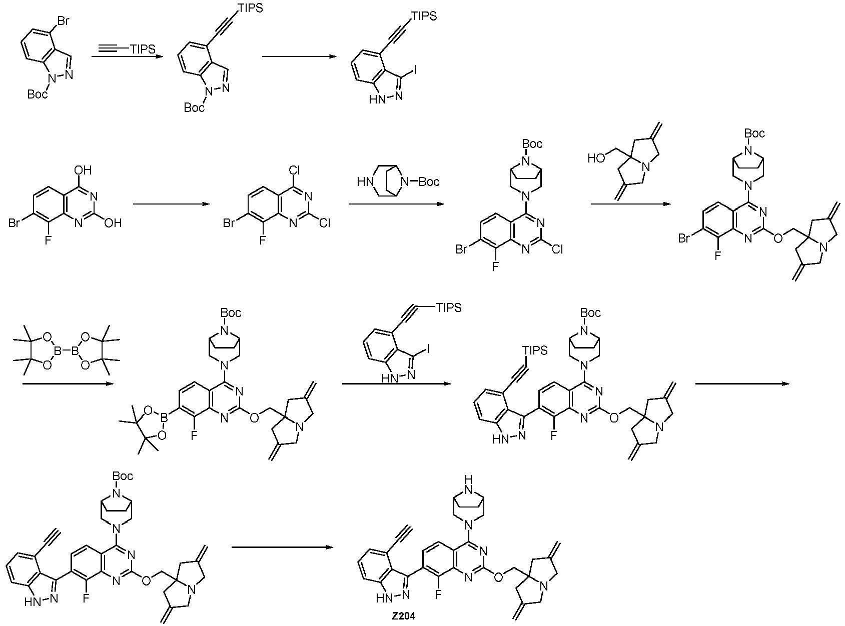

Step 1: the compound tert-butyl 4-bromo-1H-indazole-1-carboxylate (500 mg, 1.70 mmol) was dissolved in triethylamine (3 mL) and cuprous iodide (26 mg, 0.136 mmol), triphenylphosphine (36 mg, 0.136 mmol) and tetrakis (triphenylphosphine) palladium (157 mg, 0.136 mmol) were added and heated to 60°C under nitrogen protection, then ethynyl triisopropylsilane (618 mg, 3.40 mmol) was added, and the system reacted at this temperature for 16 h. Upon completion of the reaction, it was diluted with water (20 mL), extracted with ethyl acetate (30 mL X3), the organic phase was washed with saturated brine (20 mL X1), dried over anhydrous sodium sulfate, and rotary dried. The crude was purified by silica gel column chromatography (0-25% ethyl acetate/petroleum ether) to give tert-butyl 4-(triisopropylsilyl)ethynyl)-1H-indazole-1-carboxylate (640 mg, yield: 94.5%). ES-API: [M+H]⁺=398.2.

Step 2: the compound tert-butyl 4-(triisopropylsilyl)ethynyl)-1H-indazole-1-carboxylate (640 mg, 1.60 mmol) was dissolved in ethanol (10 mL), 6 mol/L sodium hydroxide aqueous solution (1 mL) and elemental iodine (813 mg, 3.2 mmol) were added. The system reacted at room temperature for 16 h. Upon completion of the reaction, it was diluted by adding water (20 mL), extracted with ethyl acetate (30 mL X3), the organic phase was washed with saturated brine (20 mL X1), dried over anhydrous sodium sulfate, rotary dried, and purified by column chromatography (0-25% ethyl acetate/petroleum ether) to give compound 3-iodo-4-((triisopropylsilyl)ethynyl)-1H-indazole (550 mg, yield: 80.6%). ES-API: [M+H]⁺=425.3.

Step 3: 7-bromo-8-fluoroquinazoline-2,4-diol (500 mg, 1.94 mmol) was dissolved in toluene (5 mL), and phosphorus oxychloride (1.50 mL) followed by diisopropylethyl amine (2.5 g, 19.4 mmol) was added to the reaction solution and allowed to react at 110°C for 2 h. After cooling to room temperature, the solvent was rotary dried under reduced pressure and purified by silica gel column chromatography (0-5% methanol/dichloromethane) to give compound 7-bromo-2,4-dichloro-8-fluoroquinazoline (300 mg, yield: 52.6%). ES-API: [M+H]⁺=294.9.

Step 4: 7-bromo-2,4-dichloro-8-fluoroquinazoline (300 mg, 1.02 mmol) was dissolved in dichloromethane (5 mL) and diisopropyl ethylamine (1.3 g, 10.2 mmol) was added, tert-butyl 3,8-diazacyclo[3.2.1]octane-8-carboxylate (217 mg, 1.02 mmol) was added slowly with cooling below -40°C and allowed to react at this temperature for 0.5 h. Upon completion of the reaction, it was diluted by adding water (20 mL), extracted with dichloromethane (30 mL X3), the organic phase was washed with saturated brine (20 mL X1), dried over anhydrous sodium sulfate, and rotary dried. The crude was purified by silica gel column chromatography (0-10% methanol/dichloromethane) to give tert-butyl (1R,5S)-3-(7-bromo-2-chloro-8-fluoroquinazolin-4-yl)-3,8-diazacyclo[3.2.1]octane-8-carboxylate (450 mg, yield: 93.8%). ES-API: [M+H]⁺=471.2. Step 5: the compound tert-butyl (1R,5S)-3-(7-bromo-2-chloro-8-fluoroquinazolin-4-yl)-3,8-diazacyclo[3.2.1]octane-8-carboxylate (270 mg, 0.57 mmol) was dissolved in 1,4-dioxane (3 mL) and (2,6-dimethylenetetrahydro-1H-pyrrolizin-7a(5H)-yl) methanol (188 mg, 1.14 mmol), cesium carbonate (926 mg, 2.85 mmol) and 4A molecular sieves were added under nitrogen protection, and the system reacted at 100°C for 3 days. Water (20 mL) was added to the reaction solution, which was extracted with ethyl acetate (30 mL X3). The organic phase was washed with saturated brine (20 mL X1), dried over anhydrous sodium sulfate, and rotary dried. The crude was purified by silica gel column chromatography (0-10% methanol/dichloromethane) to give tert-butyl (1R,5S)-3-(7-bromo-2-((2,6-dimethylenetetrahydro-1H-pyrrolizin-7a(5H)-yl)methoxy)-8-fluoroquinazolin-4-yl)-3,8-diazabicyclo[3.2.1]octane-8-carboxylate (230 mg, yield: 67.4%). ES-API: [M+H]⁺=600.2.

Step 6: the compound tert-butyl (1R,5S)-3-(7-bromo-2-((2,6-dimethylenetetrahydro-1H-pyrrolizin-7a(5H)-yl)methoxy)-8-fluoroquinazolin-4-yl)-3,8-diazabicyclo[3.2.1]octane-8-carboxylate (230 mg, 0.38 mmol) was dissolved in dry 1,4 dioxane (5 mL). Bis-(pinacolato)diboron (193 mg, 0.76 mmol), potassium carbonate (105 mg, 10.76 mmol) and 1,1'-bis(di-phenylphosphino) ferrocene palladium chloride (50 mg, 0.038 mmol) were added, and the system reacted at 90°C for 16 h. The reaction solution was filtered and concentrated to give the compound crude tert-butyl (1R,5S)-3-(2-((2,6-dimethylenetetrahydro-1H-pyrrolizin-7a(5H)-yl)methoxy)-8-fluoro-7-(4,4,5,5-tetramethyl-1,3,2-dioxaborolan-2-yl)quinazolin-4-yl)-3,8-diazabicyclo[3.2.1]octane-8-carboxylate (210 mg, yield: 85.4%). ES-API: [M+H]⁺=648.4.

Step 7: the compound crude tert-butyl (1R,5S)-3-(2-((2,6-dimethylenetetrahydro-1H-pyrrolizin-7a(5H)-yl)methoxy)-8-fluoro-7-(4,4,5,5-tetramethyl-1,3,2-dioxaborolan-2-yl)quinazolin-4-yl)-3,8-diazabicyclo[3.2.1]octane-8-carboxylate (60mg, 0.09 mmol) was dissolved in 1,4-dioxane/water (5 mL/0.5 mL). The compound 3-iodo-4-((triisopropylsilyl)ethynyl)-1H-indazole (76 mg, 0.18 mmol), potassium carbonate (37 mg, 0.27 mmol) and 1,1'-bis(di-phenylphosphino) ferrocene palladium chloride (20 mg, 0.001 mmol) were added, and the system reacted at 90°C for 16 h. Upon completion of the reaction, water (20 mL) was added to the reaction solution, which was extracted with ethyl acetate (30 mL X3), and the organic phase was washed with saturated brine (20 mL X1), dried over anhydrous sodium sulfate, and rotary dried. The crude was purified by silica gel column chromatography (0-10% methanol/dichloromethane) to obtain tert-butyl (1R,5S)-3-(2-((2,6-dimethylenetetrahydro-1H-pyrrolizin-7a(5H)-yl)methoxy)-8-fluoro-7-(4-((triisopropylsilyl)ethynyl)-1H-indazol-3-yl)quinazolin-4-yl)-3,8-diazabicyclo[3.2.1]octane-8-carboxylate (18 mg, yield: 24.5%). ES-API: [M+H]⁺= 818.4.

Step 8: tert-butyl (1R,5S)-3-(2-((2,6-dimethylenetetrahydro-1H-pyrrolizin-7a(5H)-yl)methoxy)-8-fluoro-7-(4-((triisopropylsilyl)ethynyl))-1H-indazol-3-yl)quinazolin-4-yl)-3,8-diazabicyclo[3.2.1]octane-8-carboxylate (18 mg, 0.022 mmol) was dissolved in N, N-dimethylformamide (1 mL). Cesium fluoride (33 mg, 0.02 mmol) was added, and the system reacted at room temperature for 15 min. Upon completion of the reaction, the reaction solution was directly filtered and reverse phase preparation was performed (chromatographic column: Waters XBridge C18; mobile phase: water/acetonitrile (0.1% trifluoroacetic acid) 0-60%; flow rate: 50 mL/min; column temperature: 25°C) to give the compound tert-butyl (1R,5S)-3-(2-((2,6-dimethylenetetrahydro-1H-pyrrolizin-7a(5H)-yl)methoxy)-7-(4-ethynyl-1H-indazol-3-yl)-8-fluoroquinazolin-4-yl)-3,8-diazabicyclo[3.2.1]octane-8-carboxylate (11 mg, yield: 75.6%). ES-API: [M+H]⁺= 662.3.

Step 9: the compound tert-butyl (1R,5S)-3-(2-((2,6-dimethylenetetrahydro-1H-pyrrolizin-7a(5H)-yl)methoxy)-7-(4-ethynyl-1H-indazol-3-yl)-8-fluoroquinazolin-4-yl)-3,8-diazabicyclo[3.2.1]octane-8-carboxylate (11 mg, 0.017 mmol) was dissolved in acetonitrile (2.0 mL), and 4 M hydrochloric acid/dioxane solution (0.500 mL, 2.000 mmol) was added and the reaction solution reacted at 0°C for 1 h. adjusted to pH 9 with 5 mol/L sodium hydroxide aqueous solution, extracted with ethyl acetate (30 mL X3), the organic phase was washed with saturated brine (20 mL X1), dried over anhydrous sodium sulfate, and reverse phase preparation was performed (chromatographic column: Waters XBridgC18; mobile phase: water/acetonitrile (0.1% trifluoroacetic acid) 0-30%; flow rate: 50 mL/min; column temperature: 25°C) to give compound 4-((1R,5S)-3,8-diazabicyclo[3.2.1]octan-3-yl)-2-((2,6-dimethylenetetrahydro-1H-pyrrolizin-7a(5H)-yl)methoxy)-7-(4-ethynyl-1H-indazol-3-yl)-8-fluoroquinazoline (Z204, trifluoroacetate, 5.5 mg, yield: 57.7%) as a off-white solid. ES-API: [M+1]⁺=562.2. ¹H NMR (400 MHz, CD₃OD) δ 8.68 - 8.39 (m, 1H), 7.85 (d, J = 8.7 Hz, 1H), 7.66 (d, J = 8.0 Hz, 1H), 7.46 (d, J = 20.1 Hz, 2H), 7.35 (d, J = 7.0 Hz, 1H), 5.01 (d, J = 47.1 Hz, 4H), 4.56 (d, J = 12.0 Hz, 2H), 4.41 (s, 2H), 3.92 (d, J = 14.3 Hz, 4H), 3.68 (d, J = 13.6 Hz, 2H), 3.47 (d, J = 14.4 Hz, 2H), 2.86 (d, J = 16.3 Hz, 2H), 2.65 (d, J = 16.5 Hz, 2H), 2.00 (s, 4H).

### Example 45: Synthesis of (1R,5S,8R)-3-(7-(8-ethynyl-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (Z205-1) and (1R, 5S, 8S)-3-(7-(8-ethynyl-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (Z205-2)

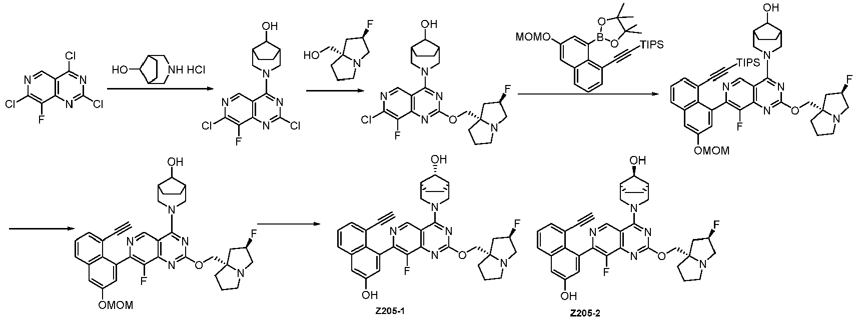

Step 1: 2,4,7-trichloro-8-fluoropyrido[4,3-d]pyrimidine (300 mg, 1.2 mmol) and N, N-diisopropyl ethylenediamine (462 mg, 3.6 mmol) were dissolved in dichloromethane (20 mL), then 3-azabicyclo[3.2.1]octan-8-ol hydrochloride (132 mg, 1.2 mmol) was added to the reaction solution at -40°C and stirred at -40°C for 1 h. The reaction was determined to be complete by LCMS, quenched with saturated ammonium chloride solution (10 mL), extracted with ethyl acetate (20 mL X3), the organic phase was washed with saturated brine (15 mL X3), dried over anhydrous sodium sulfate, filtered and concentrated. The crude was purified by silica gel column chromatography (petroleum ether: ethyl acetate = 50%) to give (1R, 5S, 8s)-3-(2,7-dichloro-8-fluoropyrido[4,3-d]pyrimidin-4-yl)-3-azabicyclo[3.2.1]octan-8-ol (280 mg, yield: 68%). ES-API: [M+H]⁺= 343.1.

Step 2: ((2R,7aS)-2-fluorotetrahydro-1H-pyrrolizin-7a(5H)-yl) methanol (391 mg, 2.46 mmol) was dissolved in tetrahydrofuran (5 mL), sodium hydride (98 mg, 2.46 mmol) was added under an ice-water bath condition, reacted in the ice-water bath for 30 min, a solution of (1R, 5S, 8s)-3-(2,7-dichloro-8-fluoropyrido[4,3-d]pyrimidin-4-yl)-3-azabicyclo[3.2.1]octan-8-ol (280 mg, A solution of 0.82 mmol) in tetrahydrofuran (5 mL) was added to the reaction solution and allowed to react at room temperature for 1 h. The reaction was determined to be complete by LCMS, washed with saturated ammonium chloride solution (20 mL), extracted with ethyl acetate (20 mL X3), the organic phase was washed with saturated brine (15 mL X3), dried over anhydrous sodium sulfate, filtered and concentrated. The crude was purified by silica gel column chromatography (petroleum ether: ethyl acetate = 50%) to give (1R,5S,8R)-3-(7-chloro-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (240 mg, yield: 63%). ES-API: [M+H]⁺= 466.1.

Step 3: under nitrogen protection, (1R,5S,8R)-3-(7-chloro-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (240 mg, 0.52 mmol), triisopropyl ((6-(methoxymethoxy)-8-(4,4,5,5-tetramethyl-1,3,2-dioxoboran-2-yl)naphthalen-1-yl)ethynyl)silane (514 mg, 1.04 mmol), potassium phosphate (331 mg, 1.56 mmol) and[n-butyldi (1-adamantyl)phosphine methanesulfonate](2-amino-1,1'-biphenyl-2-yl)palladium (11) (56 mg, 0.08 mmol) were dissolved in tetrahydrofuran (10 mL) and water (2 mL) and the reaction mixture was heated to 65°C and stirred for 3 h. The reaction was determined to be complete by LCMS, the reaction solution was concentrated under reduced pressure and the crude was purified by silica gel column chromatography (dichloromethane: methanol = 20:1) to give (1R,5S,8R)-3-(8-fluoro-2-(((2R,7aS)-2-fluorotetrahydro-1H-pyrrolizin-7a(SH)-yl)methoxy)-7-(3-(methoxymethoxy)-8-((triisopropylsilyl)ethynyl)naphthalen-1-yl)pyrido[4,3-d]pyrimidin-4-yl)-3-azabicyclo[3.2.1]octan-8-ol (240 mg, yield: 58%). ES-API: [M+H]⁺= 798.3.

Step 4: cesium fluoride (456 mg, 3.0 mmol) was added to a solution of (1R,5S,8R)-3-(8-fluoro-2-(((2R,7aS)-2-fluorotetrahydro-1H-pyrrolizin-7a(SH)-yl)methoxy)-7-(3-(methoxymethoxy)-8-((triisopropylsilyl)ethynyl)naphthalen-1-yl)pyrido[4,3-d]pyrimidin-4-yl)-3-azabicyclo[3.2.1]octan-8-ol (240 mg, 0.3 mmol) in N, N-dimethylformamide (5 mL), the reaction mixture was stirred at room temperature for 2 h. The reaction was determined to be complete by LCMS and the reaction solution was diluted by adding ethyl acetate (30 mL), washed with water (15 mL X2), saturated brine (15 mL X3), dried over anhydrous sodium sulfate, filtered and concentrated to give (1R,5S,8R)-3-(7-(8-ethynyl-3-(methoxymethoxy)naphthalen-1-yl)-8-fluoro-2-(((2R,7aS)-2-fluorotetrahydro-1H-pyrrolizin-7a(5H)-yl)methoxy)pyridine[4,3-d]pyrimidin-4-yl)-3-azabicyclo[3.2.1]octan-8-ol (192 mg, yield: 100%). ES-API: [M+H]⁺= 642.3

Step 5: a 4 M solution of hydrochloric acid/dioxane solution (10 mL) was slowly added to a solution of (1R,5S,8R)-3-(7-(8-ethynyl-3-(methoxymethoxy)naphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (192 mg, 0.3 mmol) in acetonitrile (2 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase: A: 5 mmol/L ammonium bicarbonate aqueous solution, mobile phase B: acetonitrile; flow rate: 15 mL/min; chromatographic conditions: 15 mL-35-85-13 min; column temperature: room temperature) to give two isomeric compounds. One of the isomeric structures was arbitrarily assigned as compound (1R,5S,8R)-3-(7-(8-ethynyl-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (Z205-1, 11 mg, yield: 6%, peak 1, retention time: 6.491 min). ES-API: [M+H]⁺= 598.2. The other isomer was arbitrarily assigned as (1R, 5S, 8S)-3-(7-(8-ethynyl-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3-azabicyclo[3.2.1]octan-8-ol (Z205-2, 16 mg, yield: 9 %, peak 2, retention time: 6.583 min). ES-API: [M+H]⁺= 598.2.

### Example 46: Synthesis of 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylnaphthalen-2-amine (Z206)

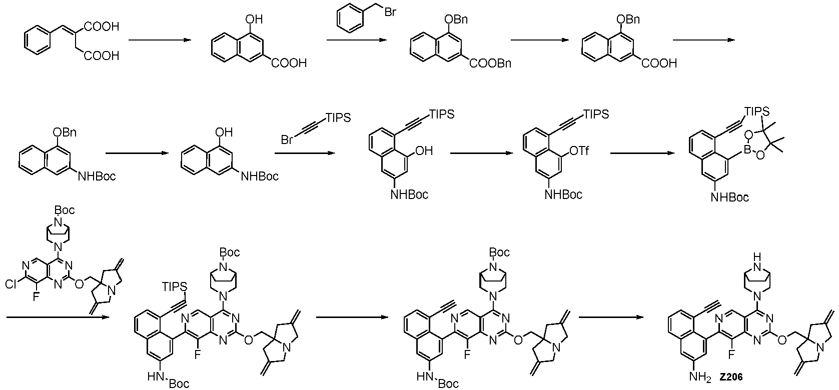

Step 1: 2-(phenylmethylene) butanedioic acid (5 g, 24.248 mmol) was dissolved in concentrated sulfuric acid (20 mL) and stirred at room temperature overnight. The reaction was determined to be complete by LC-MS, the reaction solution was slowly added to ice water (25 mL) and a yellow solid precipitated. After filtration and partial concentration of the filter cake under reduced pressure, the crude was purified by silica gel column chromatography (dichloromethane: methanol = 0-3%) to give 4-hydroxy-2-naphthoic acid (1.175 g, yield: 26%). ES-API: [M+H]⁺= 189.1.

Step 2: 4-hydroxy-2-naphthoic acid (1.175 g, 6.244 mmol), potassium carbonate (2.859 g, 18.732 mmol) and benzyl bromide (2.134 g, 12.488 mmol) were dissolved in N, N-dimethylformamide (15 mL) and the reaction solution was stirred at 70°C for 6 h. The reaction was determined to be complete by LC-MS, the reaction solution was cooled to room temperature, added to ice water (80 mL), extracted with ethyl acetate (20 mL X3), and the combined organic phase was washed with saturated brine (20 mL X2), dried over anhydrous sodium sulfate, filtered and concentrated under reduced pressure. The crude was purified by silica gel column chromatography (petroleum ether/ethyl acetate = 0-2%) to give benzyl 4-(benzyloxy)-2-naphthoate (2.233 g, yield: 97%). ES-API: [M+Na]⁺= 391.1.

Step 3: benzyl 4-(benzyloxy)-2-naphthoate (2.23 g, 6.601 mmol) was dissolved in potassium hydroxide solution (20 wt%, 22 mL) in an ice bath and the reaction solution was slowly warmed to room temperature and then heated to 90°C with stirring overnight. The reaction was determined to be complete by LC-MS. The reaction solution was cooled to room temperature, and the pH of the reaction solution was adjusted to about 1.0 by concentrated hydrochloric acid. A large amount of white solid precipitated, which was filtered, concentrated, and dried under reduced pressure to give 4-(benzyloxy)-2-naphthoic acid (1.504 g, yield: 89%). ES-API: [M+H]⁺= 279.1.

Step 4: 4-(benzyloxy)-2-naphthoic acid (1.504 g, 5.404 mmol), diphenylphosphoryl azide (1.5 mL, 7.025 mmol) and triethylamine (1.1 mL, 8.106 mmol) were dissolved in toluene (10 mL) and the reaction solution was stirred at room temperature for 1 h. The reaction was determined to be complete by LC-MS. Tert-butanol (7 mL) was added to the reaction solution, then the mixture was heated to 90°C and stirred overnight. The reaction solution was cooled to room temperature, concentrated under reduced pressure, and the crude was purified by silica gel column chromatography (petroleum ether/ethyl acetate = 0-3%) to give tert-butyl (4-(benzyloxy) naphthalen-2-yl) carbamate (2.233 g, yield: 97%) as an off-white solid. ES-API: [M+Na]⁺=372.2. Step 5: 10% palladium on carbon (140 mg) was added to a solution of tert-butyl (4-(benzyloxy) naphthalen-2-yl) carbamate (1.365 g, 3.906 mmol) in methanol (50 mL) and the reaction solution was stirred at room temperature overnight under hydrogen protection. The reaction was determined to be complete by LC-MS, the reaction solution was concentrated under reduced pressure to give tert-butyl (4-hydroxynaphthalen-2-yl) carbamate (1.146 g, crude). ES-API: [M-55]⁺= 204.1.

Step 6: under nitrogen protection, tert-butyl (4-hydroxynaphthalen-2-yl) carbamate (573 mg, 2.210 mmol), (bromoethynyl)triisopropylsilane (693 mg, 2.652 mmol), potassium acetate (434 mg, 4.419 mmol) and dichloro (p-cymene) ruthenium (II) dimer (135 mg, 0.221 mmol) were dissolved in 1,4-dioxane (10 mL), and the reaction solution was heated to 100°C and stirred for 4 h. The reaction was determined to be complete by LC-MS. The reaction solution was cooled to room temperature, concentrated under reduced pressure, and the crude was purified by silica gel column chromatography (petroleum ether/ethyl acetate = 0-5%) to give tert-butyl (4-hydroxy-5-((triisopropylsilyl)ethynyl)naphthalen-2-yl) carbamate (786 mg, yield: 81%). ES-API: [M+H]⁺= 440.3.

Step 7: tert-butyl (4-hydroxy-5-((triisopropylsilyl)ethynyl)naphthalen-2-yl) carbamate (786 mg, 1.788 mmol) was dissolved in dichloromethane (15 mL) at -40°C, N, N-diisopropylethyl amine (1.8 mL, 10.728 mmol) and triflic anhydride (757 mg, 2.682 mmol) were added to the reaction solution successively. The reaction solution was slowly raised to 0°C within 1 h. The reaction was determined to be complete by LC-MS and quenched by the addition of saturated sodium bicarbonate solution to the reaction solution (30 mL). It was extracted with dichloromethane (20 mL X3), the organic phase was combined, concentrated under reduced pressure, and the crude was purified by silica gel column chromatography (petroleum ether/ethyl acetate = 0-1%) to give 3-((tert-butoxycarbonyl)amino)-8-((triisopropylsilyl)ethynyl)naphthalen-1-yl triflate (700 mg, yield: 68%). ES-API: [M+Na]⁺=594.2.

Step 8: under nitrogen protection, 3-((tert-butoxycarbonyl)amino)-8-((triisopropylsilyl)ethynyl)naphthalen-1-yl triflate (700 mg, 1.224 mmol), bis-(pinacolato)diboron (933 mg, 3.673 mmol), potassium acetate (360 mg, 3.673 mmol) and [1,1'-bis(diphenylphosphino)ferrocene]dichloropalladium (134 mg, 0.184 mmol) were dissolved in 1,4-dioxane (15 mL) and the reaction solution was heated to 100°C and stirred overnight. The reaction was determined to be complete by LC-MS, the reaction solution was cooled to room temperature, concentrated under reduced pressure, and the crude was purified by silica gel column chromatography (petroleum ether/ethyl acetate = 0-9%) to give tert-butyl (4-(4,4,5,5-tetramethyl-1,3,2-dioxaborolan-2-yl)-5-((triisopropylsilyl)ethynyl)naphthalen-2-yl) carbamate (768 mg) as a pale yellow solid. ES-API: [M+H]⁺= 550.3.

Step 9: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (500 mg, 0.898 mmol), tert-butyl (4-(4,4,5,5-tetramethyl-1,3,2-dioxaborolan-2-yl)-5-((triisopropylsilyl)ethynyl)naphthalen-2-yl) carbamate (197 mg, 0.359 mmol), potassium phosphate (191 mg, 0.897 mmol) and[n-butylbis(1-adamantyl)phosphine](2-amino-1,1'-biphenyl-2-yl)palladium (II) methanesulfonate (26 mg, 0.036 mmol) were dissolved in tetrahydrofuran (10 mL) and water (2 mL). The reaction mixture was stirred under microwave conditions at 90°C for 0.5 h. The reaction was determined to be complete by LC-MS and the reaction solution was concentrated under reduced pressure. The crude was purified by silica gel column chromatography (petroleum ether/ethyl acetate = 0-50%) and then by thin layer chromatography (ethyl acetate/petroleum ether = 3:1) to give tert-butyl (1R,5S)-3-(7-(3-((tert-butoxycarbonyl)amino)-8-((triisopropylsilyl)ethynyl)naphthalen-1-yl)-2-((2,6-dimethyltetrahydro)-1H-pyrrolizin-7a(5H)-yl)methoxy)-8-fluoropyrido[4,3-d]pyrimidin-4-yl)-3,8-diazabicyclo[3.2.1]octane-8-carboxylate (78 mg, yield: 23%). ES-API: [M+H]⁺= 944.5.

Step 10: cesium fluoride (125 mg, 0.826 mmol) was added to tert-butyl (1R,5S)-3-(7-(3-((tert-butoxycarbonyl)amino)-8-((triisopropylsilyl)ethynyl)naphthalen-1-yl)-2-((2,6-dimethyltetrahydro)-1H-pyrrolizin-7a(5H)-yl)methoxy)-8-fluoropyrido[4,3-d]pyrimidin-4-yl)-3,8-diazabicyclo[3.2.1]octane-8-carboxylate (78 mg, 0.083 mmol) in N, N-dimethylformamide (2 mL), the reaction mixture was stirred at room temperature for 2 h. The reaction was determined to be complete by LC-MS, the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(7-(3-((tert-butoxycarbonyl)amino)-8-ethynylnaphthalen-1-yl)-2-((2,6-dimethyltetrahydro-1H-pyrrolizin-7a)(5H)-yl)methoxy)-8-fluoropyrido[4,3-d]pyrimidin-4-yl)-3,8-diazabicyclo[3.2.1]octane-8-carboxylate (65 mg, yield: 99%). ES-API: [M+H]⁺= 788.3.

Step 11: A 4 M solution of hydrochloric acid/dioxane solution (5 mL) was added slowly to a solution of tert-butyl (1R,SS)-3-(7-(3-((tert-butoxycarbonyl)amino)-8-ethynylnaphthalen-1-yl)-2-((2,6-dimethyltetrahydro-1H-pyrrolizin-7a) (5H)-yl)methoxy)-8-fluoropyrido[4,3-d]pyrimidin-4-yl)-3,8-diazabicyclo[3.2.1]octane-8-carboxylate (65 mg, 0.082 mmol) in methanol (2 mL) under an ice-water bath condition. The reaction mixture was stirred at 0°C for 0.5 h. The reaction was determined to be complete by LC-MS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 mL/min; chromatographic conditions: 15 mL-35-85-13 min; column temperature: room temperature) to give 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylnaphthalen-2-amine (Z206, 13.38 mg, yield: 28%). ES-API: [M+H]⁺= 588.3. ¹H NMR (500 MHz, DMSO-d₆) δ 9.01 (s, 1 H), 7.69- 7.68 (m, 1 H), 7.31- 7.26 (m, 2 H), 6.99- 6.97 (m, 2 H), 5.66 (s, 2 H), 4.94 (d, J= 10.5 Hz, 4 H), 4.80- 4.46 (m, 1 H), 4.28- 4.26 (m, 1 H), 4.05 (s, 2 H), 3.63- 3.60 (m, 3 H), 3.53-3.51 (m, 3 H), 3.32 (s, 1 H), 3.22-3.19 (m, 2 H), 2.64- 2.61 (m, 2 H), 1.64 (s, 4 H), 1.23 (s, 2 H).

### Example 47: Synthesis of 4-(2-((2,6-dimethyltetrahydro-1H-pyrrolizin-7a(5H)-yl)methoxy)-8-fluoro-4-((1R, 2R, 5S)-2-methyl-3,8-diazabicyclo[3.2.1]octan-3-yl)pyrido[4,3-d]pyrimidin-7-yl)-5-ethynylnaphthalen-2-ol (Z207)

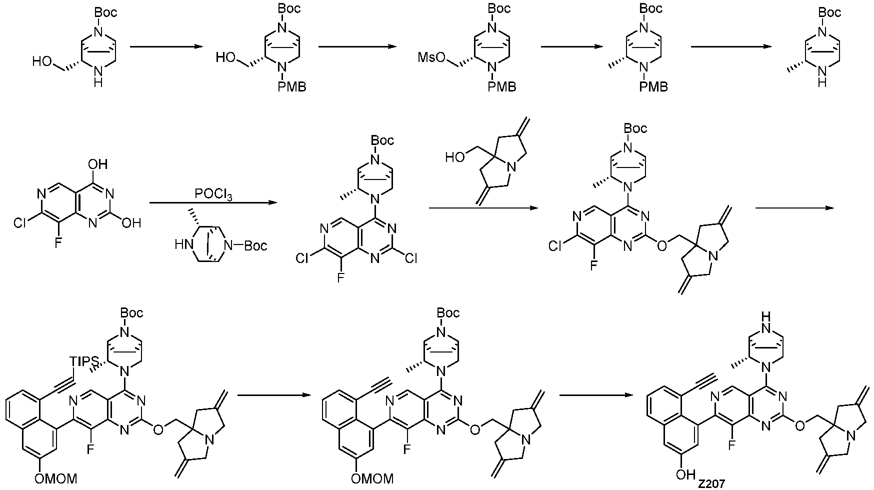

Step 1: tert-butyl (1R, 2S, 5S)-2-(hydroxymethyl)-3,8-diazabicyclo[3.2.1]octane-8-carboxylate (500 mg, 2.04 mmol) was dissolved in acetonitrile (15.0 mL), p-methoxybenzyl chloride (384 mg, 2.46 mmol) and potassium carbonate (704 mg, 5.10 mmol) were added successively, and the system reacted at normal temperature for 16 h. The reaction solution was filtered, washed with ethyl acetate, washed with an appropriate amount of water, dried over anhydrous sodium sulfate, and rotary dried. The crude was purified by silica gel column chromatography (0-40% ethyl acetate/petroleum ether) to give tert-butyl (1R, 2S, 5S)-2-(hydroxymethyl)-3-(4-methoxybenzyl)-3,8-diazabicyclo[3.2.1]octane-8-carboxylate (600 mg, yield: 81.3%).

Step 2: tert-butyl (1R, 2S, 5S)-2-(hydroxymethyl)-3-(4-methoxybenzyl)-3,8-diazabicyclo[3.2.1]octane-8-carboxylate (600 mg, 1.66 mmol) was dissolved in dichloromethane (20.0 mL), and p-toluenesulfonyl chloride (228 mg, 1.99 mmol) and triethylamine (336 mg, 3.32 mmol) were added successively, and the system reacted at normal temperature for 1 h. It was diluted with dichloromethane (200ml), washed with appropriate amount of water, dried over anhydrous sodium sulfate, and rotary dried. The crude was purified by silica gel column chromatography (0-30% ethyl acetate/petroleum ether) to give tert-butyl (1R, 2S, 5S)-3-(4-methoxybenzyl)-2-(((methylsulfonyloxy) methyl)-3,8-diazabicyclo[3.2.1]octane-8-carboxylate (600 mg, yield: 82.2%).

Step 3: tert-butyl (1R, 2S, 5S)-3-(4-methoxybenzyl)-2-((methylsulfonyloxy) methyl)-3,8-diazabicyclo[3.2.1]octane-8-carboxylate (600 mg, 1.36 mmol) was dissolved in anhydrous tetrahydrofuran (15.0 mL) and lithium triethylborohydride (2.0 M tetrahydrofuran, 2.04 ml, 4.08 mmol) was added, and the system reacted at 60°C for 4 h. The reaction was quenched by the addition of saturated ammonium chloride (20 mL), extracted with ethyl acetate, and concentrated, and the crude was purified by silica gel column chromatography (0-30% ethyl acetate/petroleum ether) to give tert-butyl (1R, 2R, 5S)-3-(4-methoxybenzyl)-2-methyl-3,8-diazabicyclo[3.2.1]octane-8-carboxylate (200 mg, yield: 42.5%).

Step 4: tert-butyl (1R, 2R, 5S)-3-(4-methoxybenzyl)-2-methyl-3,8-diazabicyclo[3.2.1]octane-8-carboxylate (200 mg, 0.58 mmol) was dissolved in methanol (4.0 mL) and water (0.4 mL), ammonium formate (380 mg, 5.8 mmol) and palladium on carbon (10%) (40 mg) were added, and the system reacted at 60°C for 2 h. The reaction solution was concentrated, filtered through ethyl acetate, and the filtrate was concentrated and purified by silica gel column chromatography (0-10% methanol/dichloromethane) to give tert-butyl (1R, 2R, 5S)-2-methyl-3,8-diazabicyclo[3.2.1]octane-8-carboxylate (50 mg, yield: 40.5%).

Step 5: 7-chloro-8-fluoropyrido[4,3-d]pyrimidine-2,4-diol (80 mg, 0.37 mmol) was added to a reactor containing anhydrous toluene (2 mL), and then phosphorus oxychloride (0.5 mL) and diisopropyl ethylamine (0.5 g) were successively added to the reactor and allowed to react at 100°C for 2 h. After cooling to room temperature, phosphorus oxychloride was rotary dried to remove under reduced pressure and the mixture was dissolved in anhydrous dichloromethane (2 mL). Then diisopropylethyl amine (0.5 g, 3.8 mmol) and tert-butyl (1R,2R,5S)-2-methyl-3,8-diazabicyclo[3.2.1]octane-8-carboxylate (50 mg, 0.22 mmol) were added, and the system reacted at -40°C for 0.5 h. Water (6 mL) was added to the reaction solution, which was extracted with dichloromethane (20 mL x2), dried over anhydrous sodium sulfate, and rotary dried. The crude was purified by silica gel chromatography (50-100% ethyl acetate/petroleum ether) to give tert-butyl (1R, 2R, SS)-3-(2,7-dichloro-8-fluoropyrido[4,3-d]pyrimidin-4-yl)-2-methyl-3,8-diazabicyclo[3.2.1]octane-8-carboxylate (70 mg, yield: 42.9%). ES-API: [M+H]⁺=442.1.

Step 6: (2,6-dimethylenetetrahydro-1H-pyrrolizin-7a(5H)-yl) methanol (31 mg, 0.19mmol) was dissolved in dry tetrahydrofuran (2 mL) and sodium hydride (80mg) was added at 0°C followed by the compound tert-butyl (1R, 2R, 5S)-3-(2,7-dichloro-8-fluoropyrido[4,3-d]pyrimidin-4-yl)-2-methyl-3,8-diazabicyclo[3.2.1]octane-8-carboxylate (70 mg, 0.16mmol) while maintaining at 0°C and allowed to react at 0°C for 1 h. Water (6 mL) was added to the reaction solution, which was extracted with ethyl acetate, dried over anhydrous sodium sulfate, then rotary dried. The crude was purified by silica gel column chromatography (50-100% ethyl acetate/petroleum ether) to give compound tert-butyl (1R,2R,5S)-3-(7-chloro-2-((2,6-dimethyltetrahydro-1H-pyrrolizin-7a(5H)-yl)methoxy)-8-fluoropyrido[4,3-d]pyrimidin-4-yl)-2-methyl-3,8-diazabicyclo[3.2.1]octane-8-carboxylate (35 mg, yield: 38%). ESI: [M+H]⁺=571.2.

Step 7: the compound tert-butyl (1R,2R,5S)-3-(7-chloro-2-((2,6-dimethyltetrahydro-1H-pyrrolizin-7a(5H)-yl)methoxy)-8-fluoropyrido[4,3-d]pyrimidin-4-yl)-2-methyl-3,8-diazabicyclo[3.2.1]octane-8-carboxylate (35mg, 0.06mmol) was dissolved in tetrahydrofuran/water (2 mL/0.2 mL), ((6-(methoxymethoxy)-8-(4,4,5,5-tetramethyl-1,3,2-dioxoboran-2-yl)naphthalen-1-yl)ethynyl)triisopropylsilane (44 mg, 0.09 mmol), potassium phosphate (25mg, 0.12 mmol) and [n-butylbis(1-adamantyl)phosphine](2-amino-1,1'-biphenyl-2-yl)palladium (11) methanesulfonate (6 mg) were reacted at 60°C for 2 h. The reaction solution was extracted with ethyl acetate and water. The organic phase was dried over anhydrous sodium sulfate and rotary dried. The crude was purified by silica gel column chromatography (dichloromethane/methanol = 10/1) to give tert-butyl (1R, 2R, SS)-3-(2-((2,6-dimethyltetrahydro-1H-pyrrolizin-7a(SH)-yl)methoxy)-8-fluoro-7-(3-(methoxymethoxy)-8-((triisopropylsilyl)ethynyl)naphthalen-1-yl)pyrido[4,3-d]pyrimidin-4-yl)-2-methyl-3,8-diazabicyclo[3.2.1]octane-8-carboxylate (30 mg, yield: 55.4%). ESI: [M+H]⁺=903.4.

Step 8: tert-butyl (1R, 2R, 5S)-3-(2-((2,6-dimethyltetrahydro-1H-pyrrolizin-7a(5H)-yl)methoxy)-8-fluoro-7-(3-(methoxymethoxy)-8-((triisopropylsilyl)ethynyl)naphthalen-1-yl)pyrido[4,3-d]pyrimidin-4-yl)-2-methyl-3,8-diazabicyclo[3.2.1]octane-8-carboxylate (30 mg, 0.033 mmol) was dissolved in N, N-dimethylformamide (1 mL). Cesium fluoride (15 mg, 0.1 mmol) was added, and the system reacted at room temperature for 2 h. The reaction solution was extracted with ethyl acetate and water, and the organic phase was dried over anhydrous sodium sulfate, rotary dried, and the crude was purified by reverse phase preparation (chromatographic column: Waters XBridge C18; mobile phase: 0.1% TFA in water/acetonitrile (0.1% trifluoroacetic acid) 0-50%; flow rate: 50 mL/min; column temperature: 25°C) to give the product tert-butyl (1R, 2R, 5S)-3-(2-((2,6-dimethyltetrahydro-1H-pyrrolizin-7a(5H)-yl)methoxy)-7-(8-ethynyl-3-(methoxymethoxy)naphthalen-1-yl)-8-fluoropyrido[4,3-d]pyrimidin-4-yl)-2-methyl-3,8-diazabicyclo[3.2.1]octane-8-carboxylate (15 mg, yield: 60.1%). ESI: [M+H]⁺=747.4.

Step 9: tert-butyl (1R, 2R, 5S)-3-(2-((2,6-dimethyltetrahydro-1H-pyrrolizin-7a(5H)-yl)methoxy)-7-(8-ethynyl-3-(methoxymethoxy)naphthalen-1-yl)-8-fluoropyrido[4,3-d]pyrimidin-4-yl)-2-methyl-3,8-diazabicyclo[3.2.1]octane-8-carboxylate (15 mg, 0.02 mmol) was dissolved in acetonitrile (1 mL) and 4 M hydrochloric acid/dioxane solution (0.5 mL) was added in an ice bath and the reaction was allowed to react for 2 h in an ice bath. The reaction solution was adjusted to pH 8 with saturated sodium bicarbonate, extracted with ethyl acetate, rotary dried, and the crude was purified by reverse phase preparation (chromatographic column: Waters XBridge C18; water/acetonitrile (0.1% trifluoroacetic acid) 0-30%; flow rate: 50 mL/min; column temperature: 25°C) to give compound 4-(2-((2,6-dimethyltetrahydro-1H-pyrrolizin-7a(5H)-yl)methoxy)-8-fluoro-4-((1R, 2R, 5S)-2-methyl-3,8-diazabicyclo[3.2.1]octan-3-yl)pyrido[4,3-d]pyrimidin-7-yl)-5-ethynylnaphthalen-2-ol (Z207, trifluoroacetate, 4.2 mg, yield: 34.9%). ESI: [M+H]⁺=603.3. ¹H NMR (400 MHz, CD₃OD) δ 9.16 (d, J = 6.8 Hz, 1H), 8.42 (s, 2H), 7.83 (d, J = 8.2 Hz, 1H), 7.51 (dd, J = 6.5, 3.5 Hz, 1H), 7.43 - 7.27 (m, 2H), 7.22 - 7.02 (m, 1H), 5.13 (s, 4H), 4.59 (s, 2H), 4.19 (s, 2H), 4.03 (s, 4H), 3.51 (dd, J = 56.6, 22.0 Hz, 3H), 2.96 (dd, J = 40.2, 28.7 Hz, 3H), 2.71 (d, J = 16.3 Hz, 2H), 2.50 (d, J = 7.6 Hz, 2H), 2.11 (t, J = 63.3 Hz, 2H), 1.37 (s, 3H).

### Example 48: Synthesis of 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylnaphthalen-1-ol (Z208)

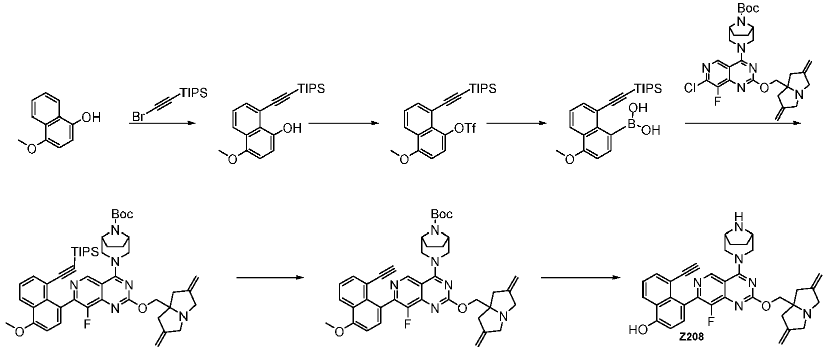

Step 1: (bromoethynyl)triisopropylsilane (5.5 g, 21.050 mmol) and potassium acetate (2.9 g, 34.44 mmol) were added to a solution of 4-methoxynaphthalen-1-ol (3.0 g, 17.222 mmol) in dioxane (50.0 mL), reacted under nitrogen atmosphere at 110°C for 1 h. Upon completion of the reaction, the reaction solution was extracted with ethyl acetate (100mL x2) and saturated brine water (100 mL), and the organic phase was dried over anhydrous sodium sulfate and rotary dried. The crude was purified by a preparative thin layer chromatography [petroleum ether/ethyl acetate = 100:0-90:10 (v/v)] to give the product 4-methoxy-8-((triisopropylsilyl)ethynyl)naphthalen-1-ol (5.0 g, yield: 81.89%). ESI: [M+H]⁺=355.2.

Step 2: triflic anhydride (4.0g, 14.177 mmol) and triethylamine (4.24g, 42.0 mmol) were added to a solution of 4-methoxy-8-((triisopropylsilyl)ethynyl)naphthalen-1-ol (5.0 g, 14.102 mmol) in dichloromethane (30.0 mL) , and the system reacted under nitrogen atmosphere at 25°C for 1.5 h. Upon completion of the reaction, the reaction solution was extracted with ethyl acetate (100mL x2) and saturated brine water (100 mL), and the organic phase was dried over anhydrous sodium sulfate and rotary dried. The crude was purified by a preparative thin layer chromatography [petroleum ether/ethyl acetate = 100:0 - 90:10 (v/v)] to give the product 4-methoxy-8-((triisopropylsilyl)ethynyl)naphthalen-1-yl-triflate (3.0 g, yield: 43.72%). ESI: [M+H]⁺=509.1. Step 3:[1,1'-bis(diphenylphosphino)ferrocene]dichloropalladium dichloromethane (0.25 g, 0.341 mmol), potassium acetate (0.70 g, 8.333 mmol) and bis-(pinacolato)diboron (1.1 g, 4.331 mmol) were added to a solution of 4-methoxy-8-((triisopropylsilyl)ethynyl)naphthalen-1-yl-triflate (1.0 g, 2.055 mmol) in dioxane (20.0 mL) and the reaction was carried out under microwave condition at 90°C for 0.5 h. Upon completion of the reaction, the reaction solution was washed and extracted with ethyl washed (100mL x2) and saturated brine water (100 mL), and the organic phase was dried over anhydrous sodium sulfate and rotary dried. The crude was purified by a preparative thin layer chromatography [petroleum ether/ethyl acetate = 100:0 - 90:10 (v/v)] to give the product (4-methoxy-8-((triisopropylsilyl)ethynyl)naphthalen-1-yl)boronic acid (980 mg, crude). ESI: [M+H]⁺=383.3.

Step 4: the compound (1R,5S)-tert-butyl 3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]oct-6-ene-8-carboxylate (2.10 g, 3.11 mmol) was dissolved in tetrahydrofuran/water (10 mL/2.0 mL) and (4-methoxy-8-((triisopropylsilyl)ethynyl)naphthalen-1-yl)boronic acid (900 mg, 1.937 mmol), potassium phosphate (1233 mg, 05.812 mmol) and[n-butyldi (1-adamantyl)phosphino](2-amino-1,1'-biphenyl-2-yl)palladium (II) methanesulfonate (212 mg, 0.291 mmol) were added, and the system reacted under microwave condition at 90°C for 40 min. The reaction solution was extracted with ethyl acetate (100 mL x2) and water (100 mL), and the organic phase was dried over anhydrous sodium sulfate and rotary dried. The crude was purified by preparative thin layer chromatography (dichloromethane/methanol = 10/1) to give the product tert-butyl (1R,5S)-3-(2-((2,6-dimethylenetetrahydro-1H-pyrrolizin-7a(5H)-yl)methoxy)-8-fluoro-7-(4-methoxy-8-((triisopropylsilyl)ethynyl)naphthalen-1-yl)pyrido[4,3-d]pyrimidin-4-yl)-3,8-diazabicyclo[3.2.1]octane-8-carboxylate (500 mg, yield: 30%). ESI: [M+H]⁺=859.5.

Step 5: tert-butyl (1R,5S)-3-(2-((2,6-dimethylenetetrahydro-1H-pyrrolizin-7a(5H)-yl)methoxy)-8-fluoro-7-(4-methoxy-8-((triisopropylsilyl)ethynyl)naphthalen-1-yl)pyrido[4,3-d]pyrimidin-4-yl)-3,8-diazabicyclo[3.2.1]octane-8-carboxylate (500 mg, 0.582 mmol) was dissolved in N, N-dimethylformamide (10.0 mL). Cesium fluoride (900 mg, 5.921 mmol) was added, and the system reacted at room temperature for 60 min. The reaction solution was extracted with ethyl acetate (100 mL x5) and water (200 mL), and the organic phase was dried over anhydrous sodium sulfate and rotary dried to give tert-butyl (1R,5S)-3-(2-((2,6-dimethylenetetrahydro-1H-pyrrolizin-7a(5H)-yl)methoxy)-7-(8-ethynyl-4-methoxynaphthalen-1-yl)-8-fluoropyrido[4,3-d]pyrimidin-4-yl)-3,8-diazabicyclo[3.2.1]octane-8-carboxylate (80 mg, yield: 19.56%). ESI: [M+H]⁺=703.3. Step 6: the compound tert-butyl (1R,5S)-3-(2-((2,6-dimethylenetetrahydro-1H-pyrrolizin-7a(5H)-yl)methoxy)-7-(8-ethynyl-4-methoxynaphthalen-1-yl)-8-fluoropyrido[4,3-d]pyrimidin-4-yl)-3,8-diazabicyclo[3.2.1]octane-8-carboxylate (80 mg, 0.114 mmol) was dissolved in dry dichloromethane (5 mL) and boron tribromide solution (4 mL of 1 M, 4.0 mmol) was added in an ice bath and allowed to react at room temperature for 1 h. Upon completion of the reaction, the reaction solution was poured into saturated sodium bicarbonate (50 mL) under an ice-water bath condition and extracted with dichloromethane (100 mL x2) and water (200 mL), the organic phase was dried over anhydrous sodium sulfate, the reaction solution was rotary dried and preparative HPLC (formic acid method) was performed to give 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylnaphthalen-1-ol (Z208, formate salt, 13 mg, yield: 18.1%). ESI: [M+H]⁺=589.3.

### Example 49: Synthesis of 4-(4-((1R,5S)-3,8-diazacyclo[3.2.1]octan-3-yl)-2-((2,6-dimethyltetrahydro-1H-pyrrolizin-7a(5H)-yl)methoxy)-8-fluoropyrido[4,3-d]pyrimidin-7-yl)-5-ethynylnaphthalen-1-amine (Z209)

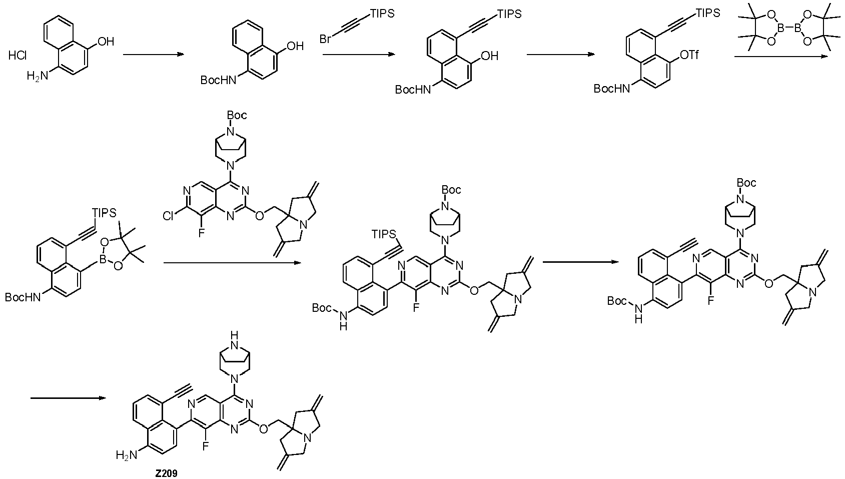

Step 1: di-tert-butyl dicarbonate (4.3 mL, 18.846 mmol) and triethylamine (2.6 mL, 18.846 mmol) were added to a solution of 4-amino-1-naphthol hydrochloride (2.5 g, 15.705 mmol) in dichloromethane (20 mL) and the reaction solution was stirred at room temperature overnight. The reaction was determined to be complete by LCMS, and the reaction solution was concentrated under reduced pressure. The crude was purified by flash silica gel column chromatography (petroleum ether/ethyl acetate = 0-5%) to give tert-butyl (4-hydroxynaphthalen-1-yl) carbamate (2.35 g, yield: 58%). ES-API: [M+Na]⁺=282.1.

Step 2: under nitrogen protection, tert-butyl (4-hydroxynaphthalen-1-yl) carbamate (2350 mg, 9.063 mmol), (bromoethynyl)triisopropylsilane (2841 mg, 10.875 mmol), potassium acetate (1779 mg, 18.125 mmol) and dichloro (p-cymene) ruthenium (II) dimer (555 mg, 0.906 mmol) were dissolved in 1,4-dioxane (40 mL) and the reaction solution was heated to 100°C and stirred overnight. The reaction was determined to be complete by LCMS. The reaction solution was cooled to room temperature, concentrated under reduced pressure, and the crude was purified by flash silica gel column chromatography (petroleum ether/ethyl acetate = 0-5%) to give tert-butyl (4-hydroxy-5-((triisopropylsilyl)ethynyl)naphthalen-1-yl) carbamate (1220 mg, yield: 30%). ES-API: [M+Na]⁺=462.2.

Step 3: tert-butyl (4-hydroxy-5-((triisopropylsilyl)ethynyl)naphthalen-1-yl) carbamate (1098 mg, 2.497 mmol) was dissolved in dichloromethane (20 mL) at -40°C, N, N-diisopropylethyl amine (2.5 mL, 14.984 mmol) and triflic anhydride (1057 mg, 3.746 mmol) were added to the reaction solution successively. The reaction solution was slowly raised to 0°C within 0.5 h. The reaction was determined to be complete by LCMS and quenched by the addition of saturated sodium bicarbonate solution to the reaction solution (30 mL). Dichloromethane (20 mL X3), the organic phase was combined and concentrated under reduced pressure, and the crude was purified by flash silica gel column chromatography (petroleum ether/ethyl acetate = 0-2%) to give 4-((tert-butoxycarbonyl)amino)-8-((triisopropylsilyl)ethynyl)naphthalene -1-trifluoromethane sulfonate (1028 mg, yield: 64%). ES-API: [M+Na]⁺=594.2.

Step 4: under nitrogen protection, 4-((tert-butoxycarbonyl)amino)-8-((triisopropylsilyl)ethynyl)naphthalene -1-trifluoromethane sulfonate (1028 mg, 1.798 mmol), bis-(pinacolato)diboron (913 mg, 3.596 mmol), potassium acetate (529 mg, 5.394 mmol) and[1,1'-bis(diphenylphosphino)ferrocene]dichloropalladium (132 mg, 0.180 mmol) were dissolved in 1,4-dioxane (20 mL), and the reaction solution was heated to 100°C and stirred overnight. The reaction was determined to be complete by LCMS, the reaction solution was cooled to room temperature, concentrated under reduced pressure and the crude was purified by flash silica gel column chromatography (petroleum ether/ethyl acetate = 0-9%) to give tert-butyl (4-(4,4,5,5-tetramethyl-1,3,2-dioxaborolan-2-yl)-5-((triisopropylsilyl)ethynyl)naphthalen-1-yl) carbamate (811 mg, yield: 82%). ES-API: [M+Na]⁺=572.3.

Step 5: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (400 mg, 0.718 mmol), tert-butyl (4-(4,4,5,5-tetramethyl-1,3,2-dioxaboran-2-yl)-5-((triisopropylsilyl)ethynyl)naphthalen-1-yl) carbamate (131 mg, 0.239 mmol), potassium phosphate (152 mg, 0.717 mmol) and[n-butylbis(1-adamantyl)phosphine](2-amino-1,1'-biphenyl-2-yl)palladium (11) methanesulfonate (17 mg, 0.024 mmol) were dissolved in tetrahydrofuran (10 mL) and water (2 mL) and the reaction mixture was stirred under microwave condition at 80°C for 40 min. The reaction was determined to be complete by LCMS, the reaction solution was concentrated under reduced pressure and the crude was purified by flash silica gel column chromatography (petroleum ether/ethyl acetate = 0-50%) and thin layer chromatography (ethyl acetate/petroleum ether = 3:1) to give tert-butyl (1R,5S)-3-(7-(4-((tert-butoxycarbonyl)amino)-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (100 mg, yield: 44%). ES-API: [M+H]⁺= 944.5.

Step 6: cesium fluoride (161 mg, 1.059 mmol) was added to tert-butyl (1R,5S)-3-(7-(4-((tert-butoxycarbonyl)amino)-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (100 mg, 0.106 mmol) in N, N-dimethylformamide (2 mL), the reaction mixture was stirred at room temperature for 2 h. The reaction was determined to be complete by LCMS, the reaction solution was diluted by adding ethyl acetate (20 mL), washed with water (15 X2), saturated brine (15 mL X3), dried over anhydrous sodium sulfate, filtered and concentrated to give tert-butyl (1R, 5S)-3-(7-(4-((tert-butoxycarbonyl)amino)-8-ethynylnaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (83 mg, yield: 99%). ES-API: [M+H]⁺= 788.3.

Step 7: a 4 M solution of hydrochloric acid/dioxane solution (5 mL) was added slowly to a solution of tert-butyl (1R, 5S)-3-(7-(4-((tert-butoxycarbonyl)amino)-8-ethynylnaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (83 mg, 0.105 mmol) in methanol (2 mL) under an ice-water bath condition. The reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 ml/min; chromatographic conditions: 15 ml-50-70-13 min; column temperature: room temperature) to give 4-(4-((1R,5S)-3,8-diazacyclo[3.2.1]octan-3-yl)-2-((2,6-dimethyltetrahydro-1H-pyrrolizin-7a(5H)-yl)methoxy)-8-fluoropyrido[4,3-d]pyrimidin-7-yl)-5-ethynylnaphthalen-1-amine as off-white solid (Z209, 42.22 mg, yield: 68%). ES-API: [M+H]⁺= 588.3.

### Example 50: Synthesis of 4-(4-((1R,5S)-3,8-diazabicyclo[3.2.1]octan-3-yl)-8-fluoro-2-(((1aS,6aS)-4-methylenehexahydrocyclopropa[b]pyrrolizin-5a(3H)-yl)methoxy)pyrido[4,3-d]pyrimidin-7-yl)-5-ethynyl-6-fluoronaphthalen-2-ol (Z210)

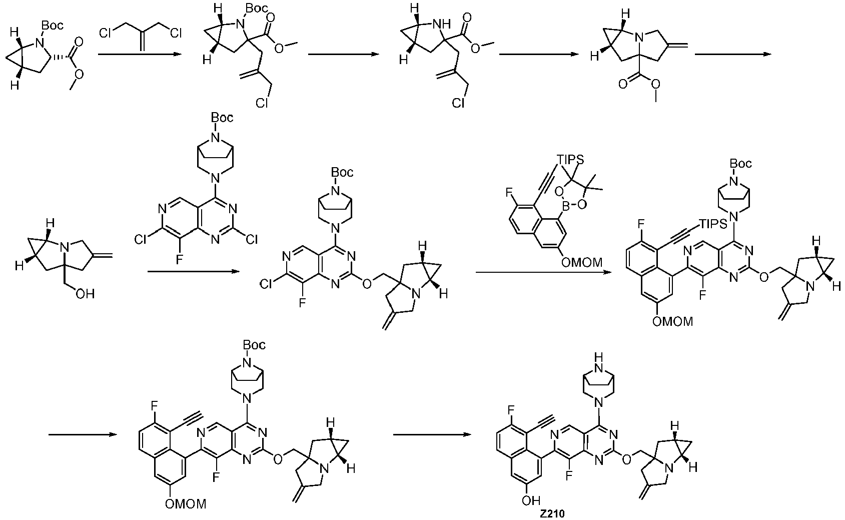

Step 1: under nitrogen protection, 2-(tert-butyl)3-methyl (1S,3S,5S)-2-azabicyclo[3.1.0]hexane-2,3-dicarboxylate (1.0 g, 4.1 mmol) was dissolved in anhydrous tetrahydrofuran (10 mL) and the mixture was placed in a dry ice-acetone bath. Lithium bistrimethylsilylamide solution (8.3 mL, 8.3 mmol, 1 M tetrahydrofuran) was slowly added dropwise to the above solution, stirred at this temperature for 1 h, a solution of 3-chloro-2-(chloromethyl)prop -1-ene (2.59 g, 20.7 mmol) in anhydrous tetrahydrofuran (10 mL) was slowly added via syringe to the above solution, and the reaction solution was further stirred at -78°C for 1 h. The reaction was determined to be complete by LCMS. The reaction was quenched by adding saturated ammonium chloride solution (50 mL), extracted with ethyl acetate (50 mL X3), the organic phase was washed with saturated brine (50 mL X3), dried over anhydrous sodium sulfate, filtered, and concentrated. The crude was purified by flash silica gel column (petroleum ether: ethyl acetate = 20:1) to give 2-(tert-butyl)3-methyl (1S,5S)-3-(2-(chloromethyl)allyl)-2-azabicyclo[3.1.0]hexane-2,3-dicarboxylate (1.28 g, yield: 93%). ES-API: [M-56]⁺= 274.1.

Step 2: a 4 M hydrochloric acid/dioxane solution (15 mL) was slowly added to a solution of 2-(tert-butyl)3-methyl (1S,5S)-3-(2-(chloromethyl)allyl)-2-azabicyclo[3.1.0]hexane-2,3-dicarboxylate (1.28 g, 3.7 mmol) in acetonitrile (3 mL) at room temperature, stirred at room temperature for 0.5 h. The reaction was determined to be complete by LCMS and the reaction solution was concentrated under reduced pressure to give methyl (1S,5S)-3-(2-(chloromethyl)allyl)-2-azabicyclo[3.1.0]hexane-3-carboxylate (0.89 g, yield: 100%). ES-API: [M+H]⁺= 230.2.

Step 3: sodium bicarbonate (1.6 g, 19.5 mmol) and potassium iodide (65 mg, 0.39 mmol) were added to a solution of methyl (1S,5S)-3-(2-(chloromethyl)allyl)-2-azabicyclo[3.1.0]hexane -3-carboxylate (0.89 g, 3.9mmol) in acetonitrile (10 mL) at room temperature and stirred at room temperature for 1.5 h. The reaction was determined to be complete by LCMS, filtered, washed with acetonitrile, and concentrated under reduced pressure, and the crude was purified by flash silica gel column (petroleum ether: ethyl acetate = 15:1) to give methyl (1aS,6aS)-4-methylenehexahydrocyclopropa[b]pyrrolizine-5a(3H)-carboxylate (510 mg, yield: 68%). ES-API: [M+H]⁺= 194.1.

Step 4: methyl (1aS,6aS)-4-methylenehexahydrocyclopropa[b]pyrrolizine-5a(3H)-carboxylate (510 mg, 2.6 mmol) was dissolved in anhydrous tetrahydrofuran (10 mL) under nitrogen protection, the mixture was placed under an ice-water bath condition, lithium tetrahydroaluminum solution (5.4 mL, 5.4 mmol, 1 M tetrahydrofuran) was slowly added dropwise to the above solution, and stirred at this temperature for 0.5 h. The reaction was determined to be complete by LCMS and quenched by the addition of sodium sulfate decahydrate (510 mg), stirred at room temperature for 0.5 h, filtered and the filtrate was concentrated under reduced pressure to give ((1aS,6aS)-4-methylenehexahydrocyclopropa[b]pyrrolizin-5a(3H)-yl) methanol (410 mg, yield: 93%). ES-API: [M+H]⁺= 166.2.

Step 5: ((1aS,6aS)-4-methylenehexahydrocyclopropa[b]pyrrolizin-5a(3H)-yl) methanolic alcohol (35 mg, 0.21 mmol) was dissolved in anhydrous tetrahydrofuran (2 mL), under an ice-water bath condition, sodium hydride (14 mg, 0.35 mmol) was added, under an ice-water bath condition reacted for 30 min, a solution of tert-butyl (1R,5S)-3-(2,7-dichloro-8-fluoropyrido[4,3-d]pyrimidin-4-yl)-3,8-diazacyclo[3.2.1]octane-8-carboxylate (300 mg, 0.7 mmol) in tetrahydrofuran (2 mL) was added dropwise to the above reaction solution and allowed to react at room temperature for 30 min. The reaction was determined to be complete by LCMS, the reaction solution was added and diluted with acetate (20 mL), washed with saturated ammonium chloride solution (15 mL X2), saturated brine (15 mL X3), dried over anhydrous sodium sulfate, filtered, the filtrate was concentrated under reduced pressure, and the crude was purified by flash silica gel column chromatography (dichloromethane: methanol = 20:1) to give tert-butyl (1R,5S)-3-(7-chloro-8-fluoro-2-(((1aS,6aS)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (200 mg, yield: 51%). ES-API: [M+H]⁺= 557.3.

Step 6: under nitrogen protection, tert-butyl (1R,5S)-3-(7-chloro-8-fluoro-2-(((1aS,6aS)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (200 mg, 0.36 mmol), ((2-fluoro-6-(methoxymethoxy)-8-(4,4,5,5-tetramethyl-1,3,2-dioxaboran-2-yl)naphthalen-1-yl)ethynyl)triisopropylsilane (461 mg, 0.9 mmol), potassium phosphate (229 mg, 1.0 mmol) and[n-butyldi (1-adamantyl)phosphine methanesulfonate] (2-amino-1,1 '-biphenyl-2-yl)palladium (II) (39 mg, 0.045 mmol) were dissolved in tetrahydrofuran (10 mL) and water (2 mL) and the reaction mixture was heated to 65°C with stirring for 3 h. The reaction was determined to be complete by LCMS, the reaction solution was concentrated under reduced pressure and the crude was purified by flash silica gel column chromatography (mobile phase, dichloromethane: methanol = 20:1) to give tert-butyl (1R,5S)-3-(8-fluoro-7-(7-fluoro-3-(methoxymethoxy)-8-((triisopropylsilyl)ethynyl)naphthalen-1-yl)-2-((1aS,6aS)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (130 mg, yield: 40%). ES-API: [M+H]⁺= 907.4.

Step 7: cesium fluoride (217 mg, 1.4 mmol) was added to a solution of tert-butyl (1R,5S)-3-(8-fluoro-7-(7-fluoro-3-(methoxymethoxy)-8-((triisopropylsilyl)ethynyl)naphthalen-1-yl)-2-((1aS,6aS)-4-methylenehexahydrocyclopropane[b]pyrrolizin-5a(3H)-yl)methoxy)pyrido[4,3-d]pyrimidin-4-yl)-3,8-diazabicyclo[3.2.1]octane-8-carboxylate (130 mg, 0.14 mmol) in N, N-dimethylformamide (3 mL) and the reaction mixture was stirred at room temperature for 2 h. The reaction was determined to be complete by LCMS and the reaction solution was diluted by adding ethyl acetate (20 mL), washed with water (15 mL X2), saturated brine (15 mL X3), dried over anhydrous sodium sulfate, filtered and concentrated to give tert-butyl (1R,5S)-3-(7-(8-ethynyl-7-fluoro-3-(methoxymethoxy)naphthalen-1-yl)-8-fluoro-2-(((1aS,6aS)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (107 mg, yield: 100%). ES-API: [M+H]⁺= 751.3. Step 8: a 4 M hydrochloric acid-dioxane solution (5 mL) was slowly added to a solution of tert-butyl (1R, SS)-3-(7-(8-ethynyl-7-fluoro-3-(methoxymethoxy)naphthalen-1-yl)-8-fluoro-2-(((1aS,6aS)-4-methylenehexahydrocyclopropa[b]pyrrolizin-5a(3H)-yl)methoxy)pyrido[4,3-d]pyrimidin-4-yl)-3,8-diazabicyclo[3.2.1]octane-8-carboxylate (107 mg, 0.14 mmol) in acetonitrile (2 mL) under an ice-water bath condition and the reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 ml/min; chromatographic conditions: 15 ml -35-85-13 min; column temperature: room temperature) to give 4-(4-((1R,5S)-3,8-diazabicyclo[3.2.1]octan-3-yl)-8-fluoro-2-(((1aS,6aS)-4-methylenehexahydrocyclopropa[b]pyrrolizin-5a(3H)-yl)methoxy)pyrido[4,3-d]pyrimidin-7-yl)-5-ethynyl-6-fluoronaphthalen-2-ol (Z210, 30 mg, yield: 35%). ES-API: [M+H]⁺= 607.3. ¹H NMR (500 MHz, CD₃OD) δ 9.00 (s, 1H), 7.87-7.81 (m, 1H), 7.45 - 7.28 (m, 2H), 7.20 (d, J = 2.4 Hz, 1H), 4.98 (s, 2H), 4.80-4.45 (m, 2H), 4.26 - 4.15 (m, 2H), 3.88-3.81 (m, 1H), 3.77-3.71 (m, 4H), 3.66 - 3.59 (m, 1H), 3.36 (d, J = 2.0 Hz, 1H), 2.78 (dd, J = 15.2, 5.5 Hz, 1H), 2.72-2.68 (m, 1H), 2.65-2.61 (m, 1H), 2.22-2.18 (m, 1H), 2.05 (d, J = 12.0 Hz, 1H), 1.94-1.85 (m, 2H), 1.81 (d, J = 10.0 Hz, 2H), 1.67-1.61 (m, 1H), 1.37-1.28 (m, 1H), 0.81-0.75 (m, 1H), 0.56-0.48 (m, 1H).

### Example 51: Synthesis of 4-(4-((1R,5S)-3,8-diazacyclo[3.2.1]octan-3-yl)-8-chloro-2-((2,6-dimethylenetetrahydro-1H-pyrrolizin-7a(5H)-yl)methoxy)pyrido[4,3-d]pyrimidin-7-yl)-5-ethynyl-6-fluoronaphthalen-2-ol (Z211)

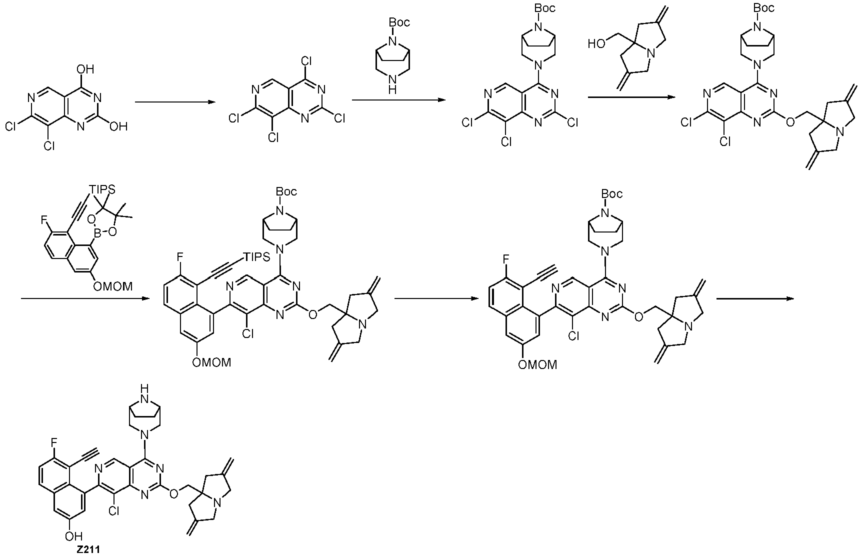

Step 1: to toluene (20.0 mL) was added 7,8-dichloropyrido[4,3-d]pyrimidine-2,4-diol (4.02 g, 17.326 mmol) followed by phosphorus oxychloride (4.844 mL, 51.978 mmol) and N, N-diisopropylethyl amine (9.3 mL, 51.978 mmol) under an ice-water bath condition and heated to 110°C to react for 2 h. Upon completion of the reaction, the solvent was rotary dried under reduced pressure to give 2,4,7,8-tetrachloropyrido[4,3-d]pyrimidine (7.0g, crude), which was used in the next step without purification.

Step 2: to dichloromethane (20.0 mL) was added 2,4,7,8-tetrachloropyrido[4,3-d]pyrimidine (290 mg, 1.078 mmol) followed by N, N-diisopropylethyl amine (1.40 g, 10.78 mmol) and tert-butyl (1R,SS)-3,8-diazabicyclo[3.2.1]octane-8-carboxylate (300 mg, 1.413 mmol), and the system reacted at room temperature for 1 h. The reaction was determined to be complete by LCMS, the reaction solution was added and diluted with ethyl acetate (100 mL), washed with water (50mL X2), saturated brine (60 mL X3), dried over anhydrous sodium sulfate, filtered, and concentrated. The crude was purified by flash silica gel column chromatography[petroleum ether: ethyl acetate = 100:0 - 30:70, (v/v)]to give tert-butyl (1R,5S)-3-(2,7,8-trichloropyrido[4,3-d]pyrimidin-4-yl)-3,8-diazabicyclo[3.2.1]octane-8-carboxylate (232 mg, yield 48.37%). ES-API: [M+H]⁺= 444.1.

Step 3: (2,6-dimethylenetetrahydro-1H-pyrrolizin-7a(5H)-yl) methanol (175 mg, 1.059 mmol) was dissolved in anhydrous tetrahydrofuran (10.0 mL), added sodium hydride (84 mg, 2.118 mmol) under an ice-water bath condition, reacted under an ice-water bath condition for 30 min. A solution of tert-butyl (1R,5S)-3-(2,7,8-trichloropyrido[4,3-d]pyrimidin-4-yl)-3,8-diazabicyclo[3.2.1]octane-8-carboxylate (232 mg, 0.522 mmol) in tetrahydrofuran (2 mL) was added dropwise to the above reaction solution and allowed to react at room temperature for 30 min. The reaction was determined to be complete by LCMS, the reaction solution was diluted by adding ethyl acetate (100 mL), washed with saturated ammonium chloride solution (40 mL X2), saturated brine (60 mL X3), dried over anhydrous sodium sulfate, filtered, the filtrate was concentrated under reduced pressure, and the crude was purified by flash silica gel column chromatography (dichloromethane: methanol = 20:1) to give tert-butyl (1R,5S)-3-(7,8-dichloro-2-((2,6-dimethylenetetrahydro-1H-pyrrolizin-7a(5H)-yl)methoxy)pyrido[4,3-d]pyrimidin-4-yl)-3,8-diazabicyclo[3.2.1]octane-8-carboxylate (300 mg, yield: 100%). ES-API: [M+H]⁺= 573.3

Step 4: under nitrogen protection, tert-butyl (1R,5S)-3-(7,8-dichloro-2-((2,6-dimethylenetetrahydro-1H-pyrrolizin-7a(5H)-yl)methoxy)pyrido[4,3-d]pyrimidin-4-yl)-3,8-diazabicyclo[3.2.1]octane-8-carboxylate (300mg, 0.523 mmol), ((2-fluoro-6-(methoxymethoxy)-8-(4,4,5,5-tetramethyl-1,3,2-dioxaboran-2-yl)naphthalen-1-yl)ethynyl)triisopropylsilane (550 mg, 1.073 mmol), potassium phosphate (333.0 mg, 1.57 mmol) and[n-butylbis(1-adamantyl)phosphine](2-amino-1,1'-biphenyl-2-yl)palladium (11) methanesulfonate (58.2 mg, 0.078 mmol) were dissolved in tetrahydrofuran (10 mL) and water (2 mL), reacted under microwave condition with stirring at 80°C for 40 min. The reaction was determined to be complete by LCMS, the reaction solution was concentrated under reduced pressure and the crude was purified by flash silica gel column chromatography (dichloromethane: methanol = 20:1) to give tert-butyl (1R,5S)-3-(8-chloro-2-((2,6-dimethylenetetrahydro-1H-pyrrolizin-7a(5H)-yl)methoxy)-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (432 mg, yield: 89%). ES-API: [M+H]⁺= 923.3.

Step 5: cesium fluoride (1450 mg, 9.546 mmol) was added to a solution of tert-butyl (1R,5S)-3-(8-chloro-2-((2,6-dimethylenetetrahydro-1H-pyrrolizin-7a(5H)-yl)methoxy)-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (432 mg, 0.468 mmol) in N, N-dimethylformamide (10.0 mL), the reaction mixture was stirred at room temperature for 2 h. The reaction was determined to be complete by LCMS and the reaction solution was diluted by adding ethyl acetate (100 mL), washed with water (50 mL X2), saturated brine (60 mL X3), dried over anhydrous sodium sulfate, filtered and concentrated to give tert-butyl (1R,5S)-3-(8-chloro-2-((2,6-dimethylenetetrahydro-1H-pyrrolizin-7a(5H)-yl)methoxy)-7-(8-ethynyl-7-fluoro-3-(methoxymethoxy)naphthalen-1-yl)pyrido[4,3-d]pyrimidin-4-yl)-3,8-diazabicyclo[3.2.1]octane-8-carboxylate (400 mg, crude). ES-API: [M+H]⁺= 767.3.

Step 6: 4 M hydrochloric acid-dioxane solution (5 mL) was slowly added to a solution of tert-butyl (1R,5S)-3-(8-chloro-2-((2,6-dimethylenetetrahydro-1H-pyrrolizin-7a(5H)-yl)methoxy)-7-(8-ethynyl-7-fluoro-3-(methoxymethoxy)naphthalen-1-yl)pyrido[4,3-d]pyrimidin-4-yl)-3,8-diazabicyclo[3.2.1]octane-8-carboxylate (400 mg, crude) in acetonitrile (15.0 mL) under an ice-water bath condition. The reaction mixture was stirred at 0°C for 0.5 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 ml/min; chromatographic conditions: 15 ml-35-85-13 min; column temperature: room temperature) to give 4-(4-((1R,5S)-3,8-diazabicyclo[3.2.1]octan-3-yl)-8-chloro-2-((2,6-dimethylenetetrahydro-1H-pyrrolizin-7a(5H)-yl)methoxy)pyrido[4,3-d]pyrimidin-7-yl)-5-ethynyl-6-fluoronaphthalen-2-ol (Z211, 46.0 mg, yield: 15.77%). ES-API: [M+H]⁺= 623.2.

### Example 52: Synthesis of 4-((1R,5S)-3,8-diazabicyclo[3.2.1]octan-3-yl)-2-((2,6-dimethylenetetrahydro-1H-pyrrolizin-7a(SH)-yl)methoxy)-7-(1-ethyl-1,2,3,4-tetrahydroquinolin-8-yl)-8-fluoropyrido[4,3-d]pyrimidine (Z212)

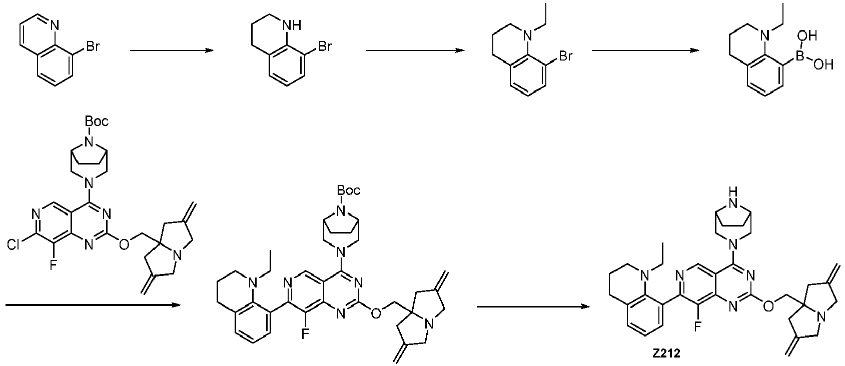

Step 1: elemental iodine (1.27 g, 5.000 mmol) and 8-bromoquinoline (5.20 g, 25 mmol) were placed in a round bottom flask and displaced with nitrogen 3 times. Anhydrous dichloromethane (100 mL) and 4,4,5,5-tetramethyl-1,3,2-dioxaborolane (12.80 g, 100.0 mmol) were added to the above flask, respectively. After the addition was complete, the reaction solution was stirred at room temperature for 72 h. Upon completion of the reaction, an aqueous solution (100 mL) was added to the reaction solution to quench the reaction. After the separation of the organic phase, the aqueous phase was extracted again with dichloromethane (200 mL X2). The organic phase was washed with saturated brine (100 mL), dried over anhydrous sodium sulfate, filtered, and concentrated. The crude was purified by flash silica gel column (ethyl acetate/petroleum ether: 1-10%) to give 8-bromo-1,2,3,4-tetrahydroquinoline (3000 mg, yield 56.58%) as a yellow transparent liquid. ES-API: [M +H]⁺= 212.1, 214.2.

Step 2: a sealed tube reactor was charged with 8-bromo-1,2,3,4-tetrahydroquinoline (2000 mg, 9.430 mmol), iodoethane (7353.95 mg, 47.150 mmol), potassium hydroxide (529.12 mg, 9.430 mmol) and N, N-dimethylacetamide (20 mL). After the sealed tube reactor was sealed, the reaction was stirred at 80°C for 17 h. After cooling to room temperature, water (50 mL) was added to the reaction solution, which was extracted with ethyl acetate (50 mL X3). The combined organic phase was washed with saturated brine (100 mL X3), dried over anhydrous sodium sulfate, filtered, and concentrated. The crude was purified by flash silica gel column (ethyl acetate/petroleum ether: 5-20%) to give 8-bromo-1-ethyl-1,2,3,4-tetrahydroquinoline (1200 mg, yield 52.99%) as a colorless transparent liquid. ES-API: [M +H]⁺= 240.1, 242.2.

Step 3: 8-bromo-1-ethyl-1,2,3,4-tetrahydroquinoline (1200 mg, 4.997 mmol) was dissolved in tetrahydrofuran (20 mL). A solution of n-butyllithium in n-hexane (1.6 M, 2.998 mL) was added to the above solution at -78°C under a nitrogen atmosphere. After the addition was completed, the reaction solution was stirred for another 30 min. A solution of 2-isopropoxy-4,4,5,5-tetramethyl-1,3,2-dioxaborolane (1115.71 mg, 5.997 mmol) in tetrahydrofuran (10 mL) was slowly added to the above solution and stirring was continued at -78°C under nitrogen atmosphere for 30 min. The reaction was quenched by the addition of saturated ammonium chloride (30 mL) to the reaction solution and extracted with dichloromethane/methanol (10:1, v/v, 30 mL X3). The organic phase was washed with saturated brine (100 mL), dried over anhydrous sodium sulfate, filtered, and concentrated to give (1-ethyl-1,2,3,4-tetrahydroquinolin-8-yl)boronic acid (300 mg, yield 29.28%) as a colorless transparent liquid. ES-API: [M +H]⁺= 206.2.

Step 4: tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (100 mg, 0.180 mmol), (1-ethyl-1,2,3,4-tetrahydroquinolin-8-yl)boronic acid (50 mg, 0.244 mmol) were dissolved in tetrahydrofuran (5 mL) and water (1 mL). [n-Butylbis(1-adamantyl)phosphine](2-amino-1,1'-biphenyl-2-yl)palladium (II) methanesulfonate (13.07 mg, 0.018 mmol) and potassium phosphate (114.31 mg, 0.539 mmol) were added to the above solution, and after the addition was completed, the reaction was stirred at 65°C for 5 h. Upon completion of the reaction, water (15 mL) was added to the reaction solution and extracted with ethyl acetate (15 mL X3). The organic phase was washed with saturated brine (50 mL), dried over anhydrous sodium sulfate, filtered, and concentrated. The crude was purified by flash silica gel column (methanol/dichloromethane: 0-5%) to give tert-butyl (1R,5S)-3-(2-((2,6-dimethylenetetrahydro-1H pyrrolizin-7a(SH)-yl)methoxy)-7-(1-ethyl-1,2,3,4-tetrahydroquinolin-8-yl)-8-fluoropyrido[4,3-d]pyrimidin-4-yl)-3,8-diazabicyclo[3.2.1]octane-8-carboxylate (40 mg, 50% pure, 16.34% yield) as a white solid. ES-API: [M+H]⁺= 682.3.

Step 5: (1R,5S)-3-(2-((2,6-dimethylenetetrahydro-1H-pyrrolizin-7a(5H)-yl)methoxy)-7-(1-ethyl-1,2,3,4-tetrahydroquinolin-8-yl)-8-fluoropyrido[4,3-d]pyrimidin-4-yl)-3,8-diazabicyclo[3.2.1]octane-8-carboxylate (40 mg, 50% pure, 0.029 mmol) was dissolved in acetonitrile (2 mL) and cooled to 0°C. Hydrogen chloride/dioxane solution (4 M, 1 mL) was added to the above solution and the reaction was stirred at 0°C for 2 h. The reaction solution was concentrated and redissolved in dichloromethane (3 mL) and alkalified to pH 8 with triethylamine. After concentration of the resulting dichloromethane solution, the crude was purified by preparative HPLC (column type: Waters XBridge C18, 190*250 mm, 5 um; mobile phase system: A: 0.1% formic acid aqueous solution; B: preparative grade acetonitrile; flow rate: 15 ml/min; B%=20%-100%; column temperature: room temperature) to give 4-((1R,5S)-3,8-diazabicyclo[3.2.1]octan-3-yl)-2-((2,6-dimethylenetetrahydro-1H-pyrrolizin-7a(5H)-yl)methoxy)-7-(1-ethyl-1,2,3,4-tetrahydroquinolin-8-yl)-8-fluoropyrido[4,3-d]pyrimidine (Z212, formate salt, 8 mg, yield 40.94%, bisformate salt) as a white solid.

### Example 53: Synthesis of 4-(4-((1R,SS)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynyl-3-fluoronaphthalen-2-amine (Z213)

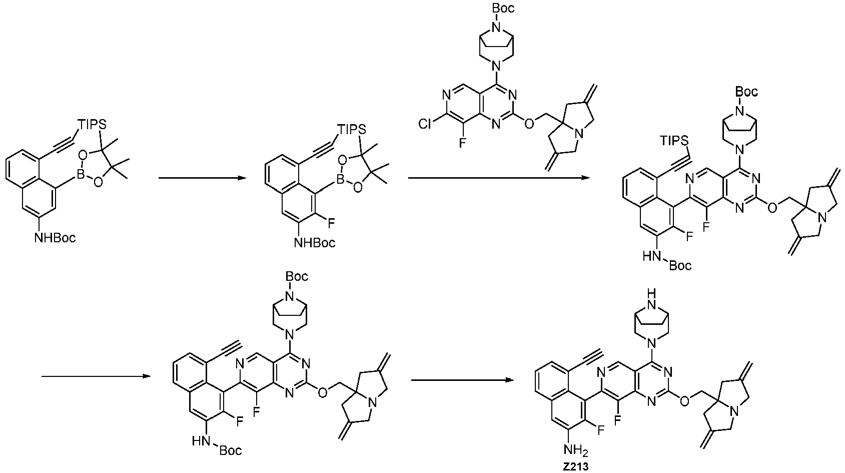

Step 1: 1-chloromethyl-4-fluoro-1,4-diazabicyclo[2.2.2]octane bis(tetrafluoroborate) salt (193 mg, 0.546 mmol) was added to a solution of tert-butyl (4-(4,4,5,5-tetramethyl-1,3,2-dioxaborolan-2-yl)-5-((triisopropylsilyl)ethynyl)naphthalen-2-yl) carbamate (300 mg, 0.546 mmol) in acetonitrile (40 mL) and the reaction solution was stirred at room temperature for 8 h. The reaction was determined to be complete by LCMS, the reaction solution was concentrated under reduced pressure and the crude was purified by flash silica gel column chromatography (petroleum ether/ethyl acetate = 0-1%) to give tert-butyl (3-fluoro-4-(4,4,5,5-tetramethyl-1,3,2-dioxaborolan-2-yl)-5-((triisopropylsilyl)ethynyl)naphthalen-2-yl) carbamate (67 mg, yield: 22%). ES-API: [M+H]⁺= 567.4.

Step 2: under nitrogen protection, tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (60 mg, 0.106 mmol), tert-butyl (3-fluoro-4-(4,4,5,5-tetramethyl-1,3,2-dioxaboran-2-yl)-5-((triisopropylsilyl)ethynyl)naphthalen-2-yl) carbamate (197 mg, 0.359 mmol), potassium phosphate (67 mg, 0.317 mmol) and[n-butylbis(1-adamantyl)phosphine](2-amino-1,1'-biphenyl-2-yl)palladium (II) methanesulfonate (77 mg, 0.106 mmol) were dissolved in tetrahydrofuran (5 mL) and water (1 mL) and the reaction mixture was stirred under microwave condition at 80°C for 0.5 h. The reaction was determined to be complete by LCMS, the reaction solution was concentrated under reduced pressure and the crude was purified by flash silica gel column chromatography (petroleum ether/ethyl acetate = 0-40%) then thin layer chromatography (ethyl acetate/petroleum ether = 3:1) to give tert-butyl (1R, 5S)-3-(7-(3-((tert-butoxycarbonyl)amino)-2-fluoro-8-((triisopropylsilyl)ethynyl)naphthalen-1-yl)-2-((2,6-dimethylenetetrahydro-1H-pyrrolizin-7a(5H)-yl)methoxy)-8-fluoropyridine[4,3-d]pyrimidin-4-yl)-3,8-diazabicyclo[3.2.1]octane-8-carboxylate (69 mg, yield: 67%). ES-API: [M+H]⁺= 962.5.

Step 3: cesium fluoride (109 mg, 0.717 mmol) was added to tert-butyl (1R,5S)-3-(7-(3-((tert-butoxycarbonyl)amino)-2-fluoro-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (69 mg, 0.072 mmol) in N, N-dimethylformamide (5 mL), the reaction mixture was stirred at room temperature for 1 h. The reaction was determined to be complete by LCMS, the reaction solution was diluted by adding ethyl acetate (20 mL), washed with water (15 mL X2), saturated brine (15 mL X3), dried over anhydrous sodium sulfate, filtered and concentrated to give tert-butyl (1R, 5S)-3-(7-(3-((tert-butoxycarbonyl)amino)-8-ethynyl-2-fluoronaphthalen-1-yl)-2-((2,6-dimethylenetetrahydro-1H-pyrrolizin-7a(5H)-yl)methoxy)-8-fluoropyridin[4,3-d]pyrimidin-4-yl)-3,8-diazabicyclo[3.2.1]octane-8-carboxylate (58 mg, yield: 98%). ES-API: [M+H]⁺= 806.3.

Step 4: 4 M hydrochloric acid-dioxane solution (6 mL) was slowly added to tert-butyl (1R,5S)-3-(7-(3-((tert-butoxycarbonyl)amino)-8-ethynyl-2-fluoronaphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (58 mg, 0.072 mmol) in methanol (2 mL) under an ice-water bath condition and the reaction mixture was stirred at 0°C for 3 h. The reaction was determined to be complete by LCMS, the reaction solution was concentrated to dryness under reduced pressure and the residue was purified by preparative HPLC (chromatographic column: Welch Xtimate, 21.2*150 mm, 5 um; mobile phase A: 5 mmol/L ammonium bicarbonate aqueous solution, mobile phase B: acetonitrile; flow rate: 15 ml/min; chromatographic conditions: 15ml-50-60-13 min; column temperature: room temperature) to give 4-(4-((1R,5S)-3,8-diazabicyclo[3.2.1]octan-3-yl)-2-((2,6-dimethylenetetrahydro-1H-pyrrolizin-7a(SH)-yl)methoxy)-8-fluoropyrido[4,3-d]pyrimidin-7-yl)-5-ethynyl-3-fluoronaphthalen-2-amine (Z213, 14.92 mg, yield: 34%) as pale yellow solid. ES-API: [M+H]⁺= 606.3.

### Example 54: Synthesis of 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylnaphthalen-2-amine (Z214)

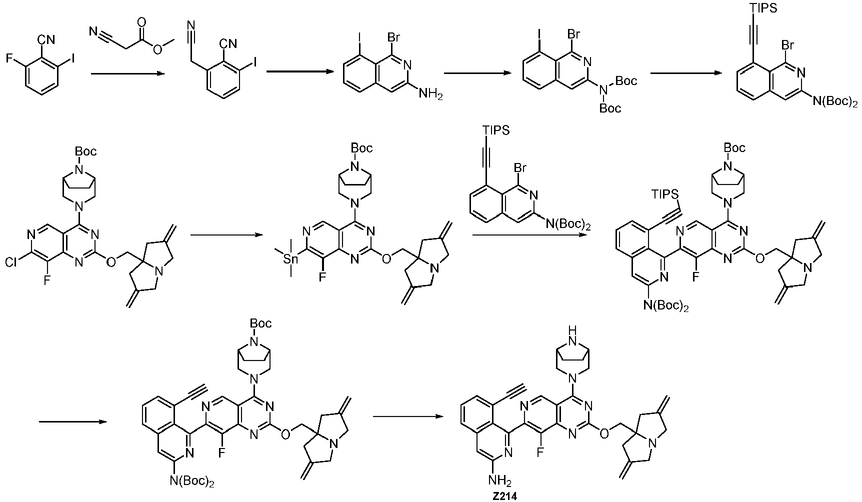

Step 1: sodium hydride (60 wt%, 16 g) was dissolved in dimethylsulfoxide (15 mL) and methyl 2-cyanoacetate (0.03 mol, 3 g) was added at 0°C and stirred at room temperature for 0.5 h. 2-fluoro-6-iodobenzonitrile (5 g, 0.02 mol) dissolved in dimethyl sulfoxide (25 mL) was slowly added dropwise to the above mixture. After the addition was completed, the mixture was reacted at 90°C for 2 h, and water (90 mL) was added and heated at 100°C for 16 h. The reaction was cooled to 0°C, diluted hydrochloric acid (0.1 N/L, 50 mL) was added, stirred for 30 min, filtered and the filter cake was rinsed with clean water to give compound 2-(cyanomethyl)-6-iodobenzonitrile (4.3 g, yield: 80.2%). ES-API: [M+H]⁺= 269.3.

Step 2: 2-(cyanomethyl)-6-iodobenzonitrile (4.3 g, 1.6 mol) was dissolved in dichloroacetic acid (20 mL) and a solution of hydrobromic acid (30% in acetic acid, 16 mL) was added at room temperature and stirred for 16 h. After the reaction was completed, saturated potassium carbonate aqueous solution was added, filtered, and the filter cake was rinsed with clean water to give the compound 1-bromo-8-iodoisoquinolin-3-amine (3.5 g, yield: 62.8%). ES-API: [M+H]⁺= 349.2. Step 3: 1-bromo-8-iodoisoquinolin-3-amine (3.5 g, 0.01 mol) was dissolved in tetrahydrofuran. N, N-diisopropyl ethylenediamine (6.45 g, 0.05 mol), 4-dimethylamino pyridine (122 mg, 0.1 mmol), and di-tert-butyl dicarbonate (4.88 g, 0.04 mmol) were added, and the system reacted at room temperature for 16 h. Upon completion of the reaction, it was rotary dried directly, and the crude was purified by silica gel column chromatography (0%-20% petroleum ether and ethyl acetate) to give tert-butyl (1-bromo-8-iodoisoquinolin-3-yl)(tert-butoxycarbonyl) carbamate (4.7g, yield: 85.7%) as a white solid. ES-API: [M+H]⁺= 549.3.

Step 4: the compound tert-butyl (1-bromo-8-iodoisoquinolin-3-yl)(tert-butoxycarbonyl) carbamate (4.7 g, 8.6 mmol) was dissolved in N, N-dimethylacetamide (15 mL), triethylamine (1.7 g, 17.2 mmol), cuprous iodide (1.6 g, 8.6 mmol) and 1,1-bis(diphenylphosphino)ferrocene dichloride (311 mg, 0.43 mmol) were added, ethynyl triisopropylsilane (3.13 g, 17.2 mmol) was added dropwise with stirring and allowed to react at room temperature for 1 h. Water (30 mL) was added, extracted with ethyl acetate (20 mL X3), washed with saturated brine (10 mL), dried over anhydrous sodium sulfate, filtered, and rotary dried, and the crude was purified by silica gel column chromatography (0-10% petroleum ether in ethyl acetate) to give the compound tert-butyl (1-bromo-8-((triisopropylsilyl)ethynyl)isoquinolin-3-yl)(tert-butoxycarbonyl) carbamate (3.9 g, yield: 45.3%). ES-API: [M+H]⁺= 603.2.

Step 5: tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazacyclo[3.2.1]octane-8-carboxylate (400 mg, 0.72 mmol) was dissolved in anhydrous toluene (10 mL) and lithium chloride (60 mg, 1.44 mmol), tetrakis triphenylphosphine palladium (100 mg, 0.08 mmol) and hexamethylditin (470 mg, 1.44 mmol) were added. The mixture was heated at 110°C for 2 h under argon protection. Upon completion of the sample reaction, it was rotary dried directly to give crude tert-butyl (1R,5S)-3-(2-((2,6-dimethylenetetrahydro-1H-pyrrolizin-7a(5H)-yl)methoxy)-8-fluoro-7-(trimethylstannyl)pyrido[4,3-d]pyrimidin-4-yl)-3,8-diazabicyclo[3.2.1]octane-8-carboxylate which was used in one step reaction without purification. ES-API: [M+H]⁺= 687.3.

Step 6: the crude tert-butyl (1R,5S)-3-(2-((2,6-dimethylenetetrahydro-1H-pyrrolizin-7a(5H)-yl)methoxy)-8-fluoro-7-(trimethylstannyl)pyrido[4,3-d]pyrimidin-4-yl)-3,8-diazabicyclo[3.2.1]octane-8-carboxylate was dissolved in anhydrous N, N-dimethylformamide and cuprous iodide (138 mg, 0.72 mmol), tetrakis triphenylphosphine palladium (80 mg, 0.07 mmol) and (1-bromo-8-((triisopropylsilyl)ethynyl)isoquinolin-3-yl)(tert-butoxycarbonyl) carbamate (867 mg, 1.44 mmol) were added under argon protection and heated at 110°C for 2 h. After the reaction was complete as monitored by LC-MS, water (20 mL) was added, extracted with ethyl acetate (10 mL X3), washed with saturated brine (10 mL), dried over anhydrous sodium sulfate, filtered and rotary dried, and the crude was purified by preparative thin layer chromatography (petroleum ether: ethyl acetate = 1:1) to give the compound tert-butyl (1R,5S)-3-(7-(3-(bis(tert-butoxycarbonyl)amino)-8-((triisopropylsilyl)ethynyl)isoquinolin-1-yl)-2-((2,6-dimethylenetetrahydro-1H-pyrrolizin-7a(5H)-yl)methoxy)-8-fluoropyrido[4,3-d]pyrimidin-4-yl)-3,8-diazacyclo[3.2.1]octane-8-carboxylate (65 mg, yield 8.6%). ES-API: [M+H]⁺= 1045.3.

Step 7: tert-butyl (1R,5S)-3-(7-(3-(bis(tert-butoxycarbonyl)amino)-8-((triisopropylsilyl)ethynyl)isoquinolin-1-yl)-2-((2,6-dimethylenetetrahydro-1H-pyrrolizin-7a(5H)-yl)methoxy)-8-fluoropyrido[4,3-d]pyrimidin-4-yl)-3,8-diazacyclo[3.2.1]octane-8-carboxylate (65 mg, 0.06 mmol) was dissolved in N, N-dimethylformamide (3 mL) and cesium fluoride (91 mg, 0.6 mmol) at room temperature for 1 h. Upon completion of the reaction, the crude was purified by reverse phase preparative HPLC (Waters XBridge C18; water/acetonitrile (0.1% trifluoroacetic acid) 0-100%; 50 mL/min; column temperature: 25°C) to give the compound tert-butyl (1R,5S)-3-(7-(3-(bis(tert-butoxycarbonyl)amino)-8-ethynylisoquinolin-1-yl)-2-((2,6-dimethylenetetrahydro-1H-pyrrolizin-7a(5H)-yl)methoxy)-8-fluoropyrido[4,3-d]pyrimidin-4-yl)-3,8-diazacyclo[3.2.1]octane-8-carboxylate (48 mg, yield: 83.3%). ES-API: [M+H]⁺= 889.4.

Step 8: tert-butyl (1R,5S)-3-(7-(3-(bis(tert-butoxycarbonyl)amino)-8-ethynylisoquinolin-1-yl)-2-((2,6-dimethylenetetrahydro-1H-pyrrolizin-7a(5H)-yl)methoxy)-8-fluoropyrido[4,3-d]pyrimidin-4-yl)-3,8-diazacyclo[3.2.1]octane-8-carboxylate (48 mg, 0.05 mmol) was dissolved in acetonitrile (2 mL) and dioxane hydrochloride (0.5 mL, 4 N/L) was added at 0°C and the reaction was stirred at this temperature for 1 h. After completion, the pH was adjusted to 8-9 with saturated sodium bicarbonate aqueous solution, rotary dried, and the crude was purified by reverse phase preparative HPLC (Waters XBridge C18, water/acetonitrile (0.1% formic acid) 0-30%, 50mL/min, column temperature: 25°C) to give compound 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-5-ethynylnaphthalen-2-amine (Z214, 3.0 mg, yield: 10.2%). ES-API: [M+H]⁺= 589.1. ¹H NMR (400 MHz, CD₃OD) δ 9.08 (s, 1H), 7.70 (d, J = 8.1 Hz, 1H), 7.55 - 7.34 (m, 2H), 6.99 (s, 1H), 5.16 (s, 4H), 4.77 (d, J = 13.2 Hz, 2H), 4.57 (s, 2H), 4.12 (d, J = 14.8 Hz, 4H), 3.92 (t, J = 14.1 Hz, 2H), 3.65 (d, J = 14.6 Hz, 2H), 3.16 (s, 1H), 2.94 (d, J = 16.3 Hz, 2H), 2.73 (d, J = 16.3 Hz, 2H), 2.06 (s, 4H).

### Example 55: Synthesis of 4-(4-((1R,SS)-3,8-diazabicyclo[3.2.1]octan-3-yl)-2-((2-(difluoromethylene)-6-methylenetetrahydro-1H-pyrrolizin-7a(5H))-yl)methoxy)-8-fluoropyrido[4,3-d]pyrimidin-7-yl)-5-ethynyl-6-fluoronaphthalen-2-ol (Z215)

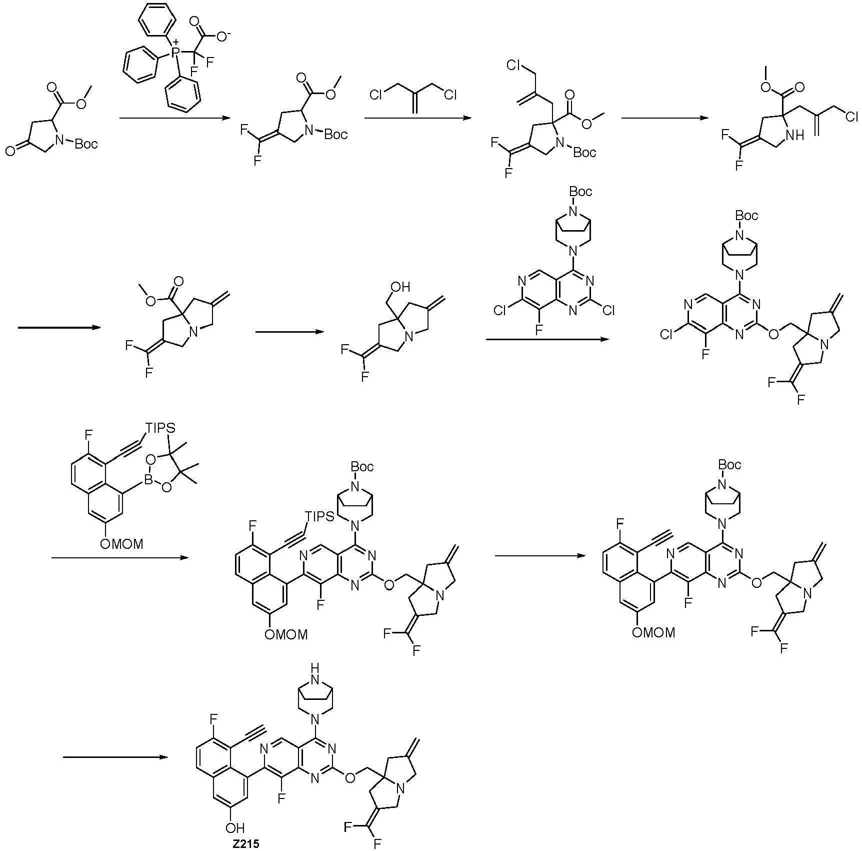

Step 1: 1-(tert-butyl)2-methyl 4-oxopyrrolidine-1,2-dicarboxylate (2.7 g, 11.099 mmol) and (triphenylphosphonium) difluoroacetate (9.89 g, 27.748 mmol) were dissolved in N, N-dimethylformamide (100 mL) and heated to 80°C with stirring for 17 h. Water (100 mL) was added to the reaction solution and extracted with ethyl acetate (100 mL X3). The organic phase was washed with saturated brine (200 mL X3), dried over anhydrous sodium sulfate, filtered and concentrated to give the crude, which was purified by flash silica gel column (ethyl acetate/petroleum ether: 0-5%) to give 1-(tert-butyl)2-methyl 4-(difluoromethylene) pyrrolidine - 1,2-dicarboxylate (1200 mg, yield 38.99%) as a colorless transparent liquid. ES-API: [M-Boc+H]⁺=178.1.

Step 2: 1-(tert-butyl)2-methyl 4-(difluoromethylene) pyrrolidine-1,2-dicarboxylate (500 mg, 1.803 mmol) was dissolved in tetrahydrofuran (5 mL) and a solution of lithium bistrimethylsilylamide in tetrahydrofuran (1 M, 5.410 mL, 5.410 mmol) was added at -78°C under nitrogen atmosphere. After the addition was complete, the reaction was stirred at -78°C for an additional 30 min. Then, 3-chloro-2-(chloromethyl)prop-1-ene (1352.36 mg, 10.820 mmol) was dissolved in tetrahydrofuran (5 mL) and slowly added dropwise to the above reaction solution at -78°C under nitrogen atmosphere. After the addition was complete, the reaction was warmed to room temperature and stirred for 17 h. The reaction solution was added reaction was saturated ammonium chloride (30 mL) to quench the reaction and extracted with ethyl acetate (30 mL X3). The organic phase was washed with saturated brine (100 mL), dried over anhydrous sodium sulfate, filtered, and concentrated to give the crude. The crude was purified by flash silica gel column (ethyl acetate/petroleum ether: 0%-15%) to give 1-(tert-butyl)2-methyl 2-(2-(chloromethyl)allyl)-4-(difluoromethylene) pyrrolidine -1,2-dicarboxylate (500 mg, yield 75.80%) as a colorless transparent liquid. ES-API: [M-Boc+H]⁺=266.0.

Step 3: 1-(tert-butyl)2-methyl 2-(2-(chloromethyl)allyl)-4-(difluoromethylene) pyrrolidine-1,2-dicarboxylate (500 mg, 1.367 mmol) was dissolved in acetonitrile (5 mL) and hydrogen chloride/dioxane solution (4 M, 5 mL) and stirred at room temperature for 2 h. Upon completion of the reaction, the reaction solution was concentrated to give methyl 2-(2-(chloromethyl)allyl)-4-(difluoromethylene) pyrrolidine -2-carboxylate (413 mg, yield 100%, hydrochloride salt) as a white solid. ES-API: [M+H]⁺=266.0.

Step 4: a mixture of methyl 2-(2-(chloromethyl)allyl)-4-(difluoromethylene) pyrrolidine -2-carboxylate (413 mg, 1.367 mmol, hydrochloride salt), potassium iodide (683.26 mg, 4.116 mmol), potassium carbonate (568.87 mg, 4.116 mmol) and N, N-dimethylformamide (6 mL) was stirred at 80°C under nitrogen protection for 3 h. Water (30 mL) was added to the reaction solution and extracted with ethyl acetate (30 mL X3). The organic phase was washed with saturated brine (30 mL X3), dried over anhydrous sodium sulfate, filtered, and concentrated to give the crude. The crude was purified by flash silica gel column (methanol/dichloromethane: 0-6%) to give methyl 2-(difluoromethylene)-6-methylenetetrahydro-1H-pyrrolizine-7a(5H)-carboxylate (220 mg, yield 69.95%) as a colorless transparent oily liquid. ES-API: [M+H]⁺= 230.2.

Step 5: methyl 2-(difluoromethylene)-6-methylenetetrahydro-1H-pyrrolizine-7a(5H)-carboxylate (220 mg, 0.960 mmol) was dissolved in tetrahydrofuran (4 mL) and cooled to 0°C. A solution of lithium borohydride in tetrahydrofuran (2 M, 0.960 mL, 1.919 mmol) was added to the above solution under a nitrogen atmosphere. The reaction was stirred at 0°C for 8 h. The reaction was quenched by adding dilute hydrochloric acid (1 M, 0.1 mL) to the reaction solution, and the reaction solution was adjusted to pH = 8 with 15% sodium hydroxide aqueous solution. The resulting mixed solution was extracted with dichloromethane/methanol (10:1, v/v, 10 mL X3). The combined organic phase was washed with saturated brine (30 mL), dried over anhydrous sodium sulfate, filtered, and concentrated to give (2-(difluoromethylene)-6-methylenetetrahydro-1H-pyrrolizin-7a(5H)-yl) methanol (100 mg, 51.78%) as a colorless transparent oily liquid. ES-API: [M+H]⁺= 202.2.

Step 6: (2-(difluoromethylene)-6-methylenetetrahydro-1H-pyrrolizin-7a(5H)-yl) methanol (46.98 mg, 0.233 mmol) was dissolved in tetrahydrofuran (3 mL) and cooled to 0°C. Sodium hydride (60%, 46.70 mg, 1.167 mmol) was added to the above solution under a nitrogen atmosphere and stirred for 30 min. Then, tert-butyl (1R,5S)-3-(2,7-dichloro-8-fluoropyrido[4,3-d]pyrimidin-4-yl)-3,8-diazabicyclo[3.2.1]octane-8-carboxylate (100 mg, 0.233 mmol) was slowly added to the above solution and stirred at 0°C for 2 h. The reaction was quenched by the addition of water (5 mL) to the reaction solution and extracted with ethyl acetate (20 mL X3). The organic phase was washed with saturated brine (30 mL X3), dried over anhydrous sodium sulfate, filtered, and concentrated to give the crude. The crude was purified by flash silica gel column (ethyl acetate/petroleum ether: 0-40%) to give tert-butyl (1R,5S)-3-(7-chloro-2-((2-(difluoromethylene)-6-methylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (100 mg, 80% pure, yield 57.77%) as a yellow solid. ES-API: [M+H]⁺= 593.1.

Step 7: tert-butyl (1R,5S)-3-(7-chloro-2-((2-(difluoromethylene)-6-methylenetetrahydro-1H-pyrrolizin-7 a(5H)-yl)methoxy) - 8 -fluoropyrido [4,3 -d] pyrimidin-4-yl) -3,8 - diazabicyclo[3.2.1]octane-8-carboxylate (100 mg, 80% pure, 0.135 mmol), ((2-fluoro-6-(methoxymethoxy)-8-(4,4,5,5-tetramethyl-1,3,2-dioxaboran-2-yl)naphthalen-1-yl)ethynyl)triisopropylsilane (138.28 mg, 0.270 mmol) was dissolved in tetrahydrofuran (5 mL) and water (1 mL). [n-Butylbis(1-adamantyl)phosphine](2-amino-1,1'-biphenyl-2-yl)palladium (II) methanesulfonate (9.82 mg, 0.013 mmol) and potassium phosphate (28.63 mg, 0.135 mmol) were added to the above solution, and after the addition was completed, the reaction was stirred at 65°C for 5 h. Upon completion of the reaction, water (15 mL) was added to the reaction solution and extracted with ethyl acetate (15 mL X3). The organic phase was washed with saturated brine (50 mL), dried over anhydrous sodium sulfate, filtered, and concentrated. The crude was purified by flash silica gel column (methanol/dichloromethane: 0-5%) to give tert-butyl (1R,5S)-3-(2-((2-(difluoromethylene)-6-methylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (90 mg, yield 70.74%) as a white solid. ES-API: [M+H]⁺= 944.3.

Step 8: a mixed solution of tert-butyl (1R,5S)-3-(2-((2-(difluoromethylene)-6-methylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (80 mg, 0.085 mmol), cesium fluoride (138 mg, 0.91 mmol) and N, N-dimethylformamide (5 mL) was stirred at room temperature for 2 h. Water (20 mL) was added to the reaction solution and extracted with ethyl acetate (30 mL X2). The organic phase was washed with saturated brine (15 mL X3), dried over anhydrous sodium sulfate, filtered and concentrated to give tert-butyl (1R,5S)-3-(2-((2-(difluoromethylene)-6-methylenetetrahydro-1H-pyrrolizin-7a(5H)-yl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (50 mg, yield 74.92%) as a yellow oily liquid. ES-API: [M+H]⁺= 787.3.

Step 9: tert-butyl (1R,5S)-3-(2-((2-(difluoromethylene)-6-methylenetetrahydro-1H-pyrrolizin-7a(5H)-yl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (50 mg, 0.064 mmol) was dissolved in acetonitrile (2 mL) and cooled to 0°C. Hydrogen chloride/dioxane solution (4 M, 1 mL) was added to the above solution and the reaction was stirred at 0°C for 2 h. The reaction solution was concentrated and redissolved in dichloromethane (3 mL) and alkalified to pH = 8 with 7 M ammonia/methanol solution. After concentration of the resulting solution, the crude was purified by preparative HPLC (column type: Waters XBridge C18, 190*250 mm, 5 um; mobile phase system: A: 0.1% ammonium bicarbonate aqueous solution; B: preparative grade acetonitrile; flow rate: 15 ml/min; B%=20%-100%; column temperature: room temperature) to give 4-(4-((1R,5S)-3,8-diazabicyclo[3.2.1]octan-3-yl)-2-((2-(difluoromethylene)-6-methylenetetrahydro-1H-pyrrolizin-7a(5H))-yl)methoxy)-8-fluoropyrido[4,3-d]pyrimidin-7-yl)-5-ethynyl-6-fluoronaphthalen-2-ol (Z215, 7 mg, yield 16.28%) as a brown solid. ES-API: [M+H]⁺= 643.2.

### Example 56: Synthesis of 4-(4-((1R,SS)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-6-chloro-5-ethynylnaphthalen-2-ol (Z216)

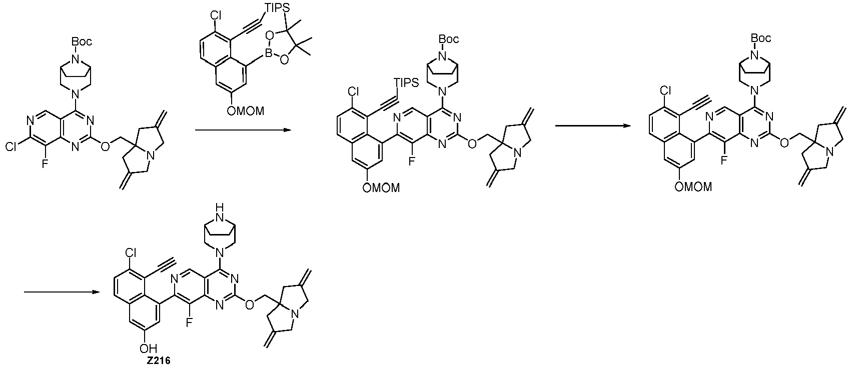

Step 1: tert-butyl (1R,5S)-3-(7-chloro-2-((2,6-dimethylenetetrahydro-1H-pyrrolizin-7a(5H)-yl)methoxy)-8-fluoropyrido[4,3-d]pyrimidin-4-yl)-3,8-diazacyclo[3.2.1]octane-8-carboxylate (150 mg, 0.27 mmol) was dissolved in tetrahydrofuran (10 mL) and water (2.5 mL), ((2-chloro-6-(methoxymethoxy)-8-(4,4,5,5-tetramethyl-1,3,2-dioxaboran-2-yl)naphthalen-1-yl)ethynyl)triisopropylsilane (214 mg, 0.40 mmol), potassium phosphate (114 mg, 0.54 mmol), [n-butylbis(1-adamantyl)phosphino](2-amino-1,1 '-biphenyl-2-yl)palladium (II) methanesulfonate(30 mg, 0.041 mmol) were added under nitrogen protection, and the system reacted at 60°C for 1 h. The reaction solution was directly rotary dried, and the crude was purified by silica gel column chromatography (petroleum ether: ethyl acetate = 0-50%) to give compound tert-butyl (1R,5S)-3-(7-(7-chloro-3-(methoxymethoxy)-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (80 mg, yield: 32.2%). ES-API: [M+H]⁺= 923.4.

Step 2: tert-butyl (1R,5S)-3-(7-(7-chloro-3-(methoxymethoxy)-8-((triisopropylsilyl)ethynyl)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (80 mg, 0.086 mmol) was dissolved in N, N-dimethylformamide (3 mL) and cesium fluoride (130 mg, 0.86 mmol) was added and stirred at room temperature for 1 h. Upon completion of the reaction, the crude was purified by reverse phase preparative HPLC (Waters XBridge C18; water/acetonitrile (0.1% trifluoroacetic acid) 0- 100 %; 50 mL/min; column temperature: 25°C) and lyophilized to give the compound tert-butyl (1R,5S)-3-(7-(7-chloro-8-ethynyl-3-(methoxymethoxy)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (23 mg, yield: 34.9%). ES-API: [M+H]⁺= 767.3.

Step 3: tert-butyl (1R,5S)-3-(7-(7-chloro-8-ethynyl-3-(methoxymethoxy)naphthalen-1-yl)-2-((2,6-dimethylenetetrahydro-1H-pyrrolizin-7a(5H)-yl)methoxy)-8-fluoropyrido[4,3-d]pyrimidin-4-yl)-3,8-diazabicyclo[3.2.1]octane-8-carboxylate (23 mg, 0.03 mmol) was dissolved in acetonitrile (2 mL) and dioxane hydrochloride (0.5 mL, 4 M) was added at 0°C and allowed to react at this temperature for 1 h. After completion, the pH was adjusted to = 8-9 with saturated sodium bicarbonate aqueous solution, rotary dried, and the crude was purified by reverse phase preparative HPLC (Waters XBridge C18, water/acetonitrile (0.1% FA) 0-30%, 50 mL/min, column temperature: 25°C) to give compound 4-(4-((1R,5S)-3,8-diazabicyclo[3.2.1]octan-3-yl)-2-((2,6-dimethylenetetrahydro-1H-pyrrolizin-7a(5H)-yl)methoxy)-8-fluoropyrido[4,3-d]pyrimidin-7-yl)-6-chloro-5-ethynylnaphthalen-2-ol (Z216, formate salt, 7.1 mg, yield: 38.4%). ES-API: [M+H]⁺= 623.2. ¹H NMR (400 MHz, CD₃OD) δ 9.06 (s, 1 H), 8.42 (s, 2 H), 7.81 (d, J = 8.9 Hz, 1 H), 7.52 (d, J = 8.9 Hz, 1 H), 7.34 (d, J = 2.4 Hz, 1 H), 7.20 (d, J = 2.5 Hz, 1 H), 5.14 (s, 4 H), 4.77 (t, J = 12.9 Hz, 2 H), 4.53 (s, 2 H), 4.07 (d, J = 15.6 Hz, 4 H), 3.89 (d, J = 13.4 Hz, 2 H), 3.61 (d, J = 15.1 Hz, 2 H), 2.92 (d, J = 16.1 Hz, 2 H), 2.71 (d, J = 15.8 Hz, 2 H), 2.05 (s, 4 H).

The compounds shown in the following table can be prepared according to the synthetic methods of the above examples or synthesized according to the methods known in the literature.

**Table A**

| Structure | MS [M+H] ⁺ | Structure | MS [M+H] ⁺ | Structure | MS [M+H] ⁺ |
|---|---|---|---|---|---|
| 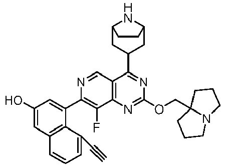 | 564.3 | 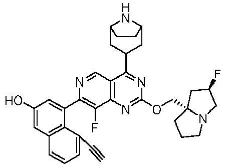 | 582.3 | 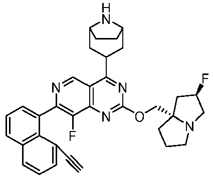 | 566.3 |
| 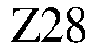 | | 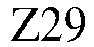 | | 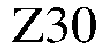 | |
| 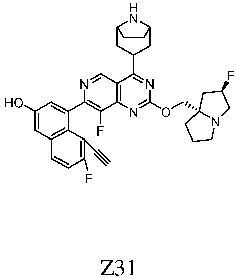 | 582.3 | 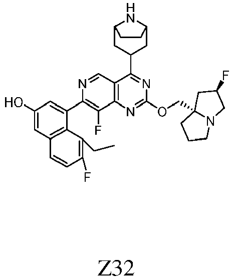 | 604.3 | | |

**Table C**

| Structure | MS [M+H]⁺ | Structure | MS [M+H]⁺ | Structure | MS [M+H]⁺ |
|---|---|---|---|---|---|
| 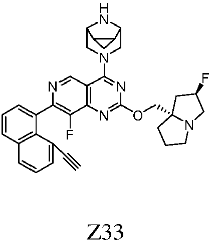 | 579.3 | 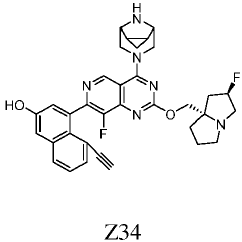 | 595.3 | 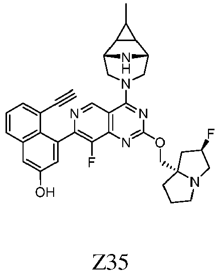 | 609.3 |

**Table D**

| Structure | MS [M+H] ⁺ | Structure | MS [M+H] ⁺ | Structure | MS [M+H] ⁺ |
|---|---|---|---|---|---|
| 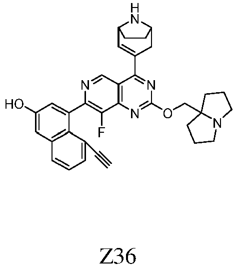 | 562.3 | 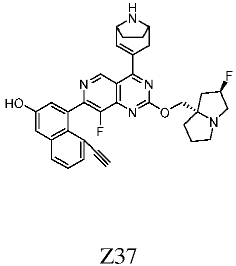 | 580.2 | 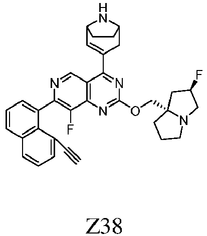 | 564.2 |
| 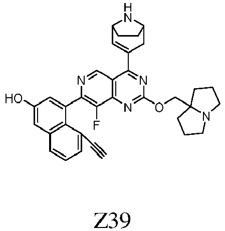 | 562.3 | 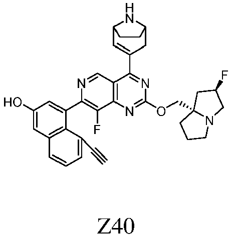 | 580.2 | 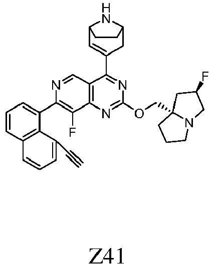 | 564.2 |

**Table E**

| Structure | MS [M+H] ⁺ | Structure | MS [M+H] ⁺ | Structure | MS [M+H] ⁺ |
|---|---|---|---|---|---|
| 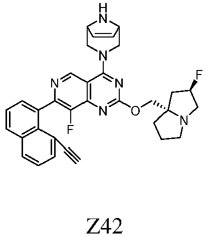 | 565.2 | 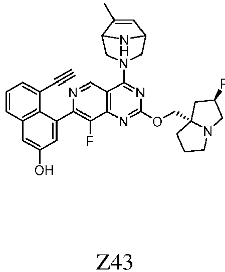 | 595.3 | 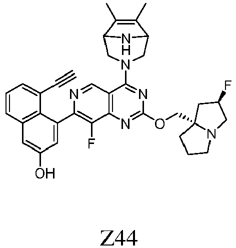 | 609.3 |
| 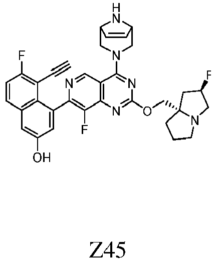 | 599.2 | 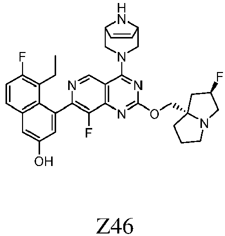 | 603.3 | 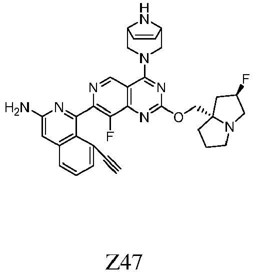 | 581.3 |
| 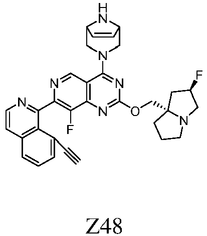 | 566.2 | 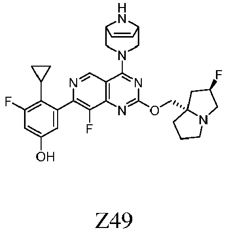 | 565.2 | 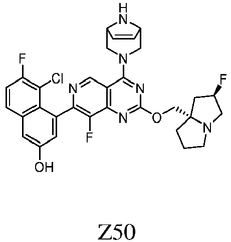 | 609.2 |
| 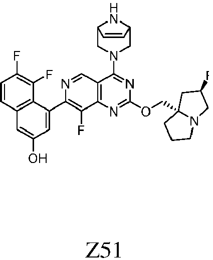 | 593.2 | 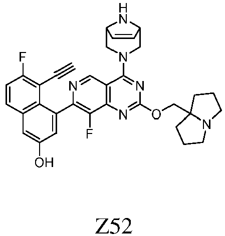 | 581.2 | 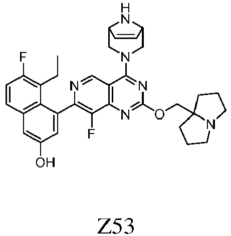 | 585.3 |
| 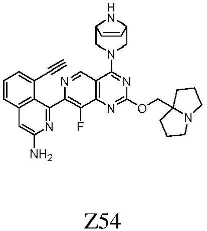 | 563.3 | 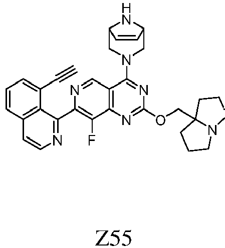 | 548.2 | 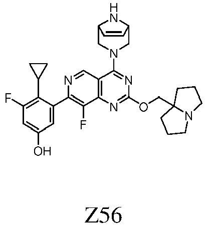 | 547.3 |
| 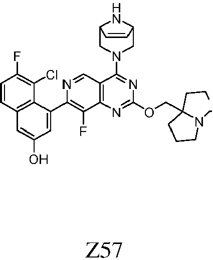 | 591.2 | 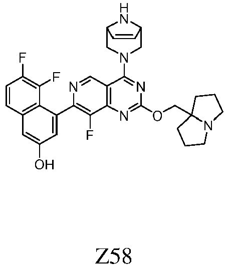 | 575.2 | | |

**Table F**

| Structure | MS [M+H]⁺ | Structure | MS [M+H]⁺ | Structure | MS [M+H]⁺ |
|---|---|---|---|---|---|
| 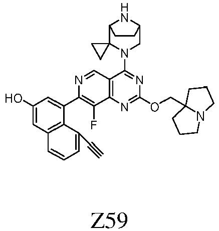 | 591.3 | 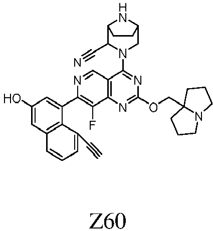 | 590.3 | 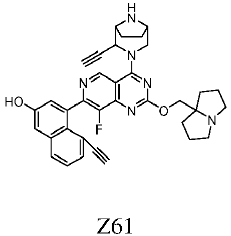 | 589.3 |
| 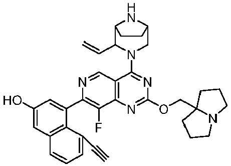 | 591.3 | | | | |
| 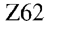 | | | | | |

**Table G**

| Structure | MS [M+H ]⁺ | Structure | MS [M+H] ⁺ | Structure | MS [M+H ]⁺ |
|---|---|---|---|---|---|
| 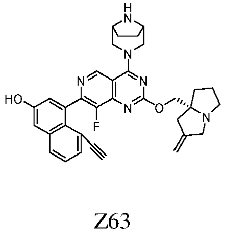 | 577.3 | 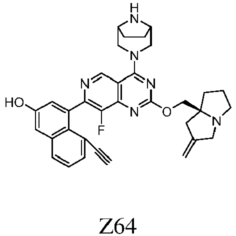 | 577.3 | 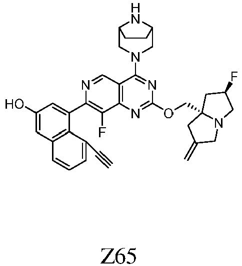 | 595.3 |
| 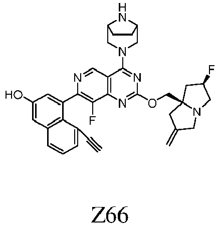 | 595.3 | 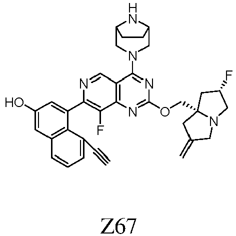 | 595.3 | 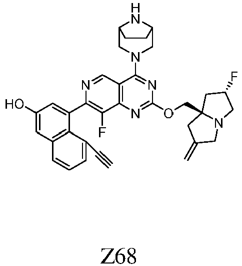 | 595.3 |
| 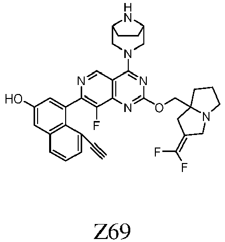 | 613.2 | 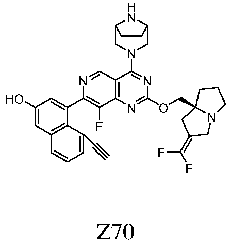 | 613.2 | 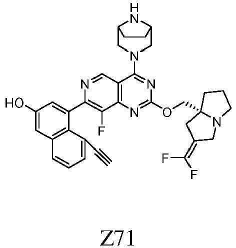 | 613.2 |
| 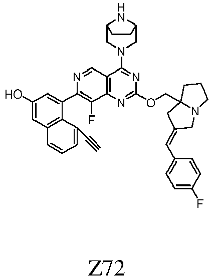 | 671.3 | 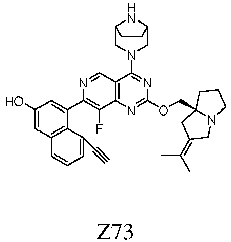 | 605.3 | 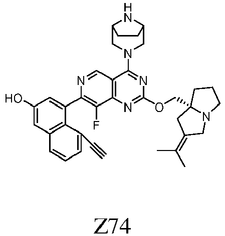 | 605.3 |
| 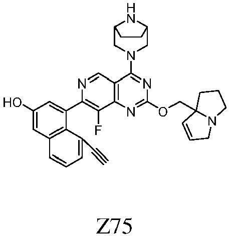 | 563.2 | 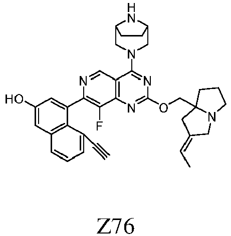 | 591.3 | 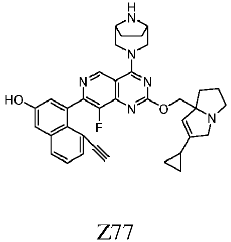 | 603.3 |
| 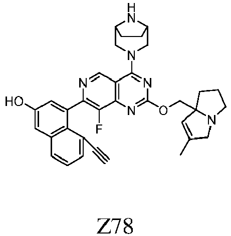 | 577.3 | 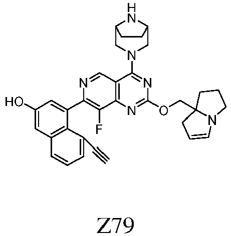 | 563.2 | 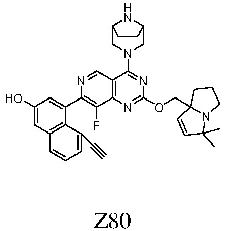 | 591.3 |
| 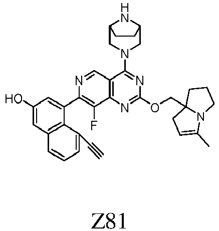 | 577.3 | 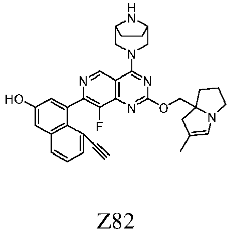 | 577.3 | 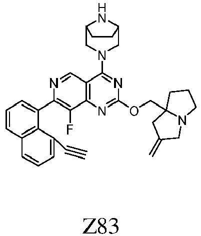 | 561.3 |
| 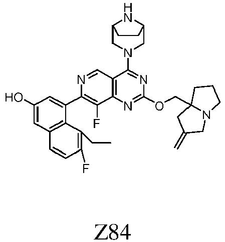 | 599.3 | 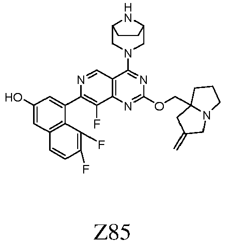 | 589.2 | 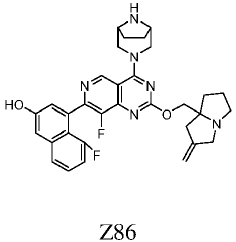 | 571.3 |
| 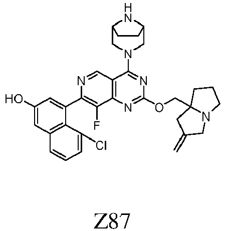 | 587.2 | 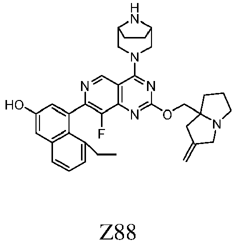 | 581.3 | 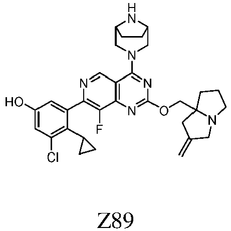 | 577.2 |
| 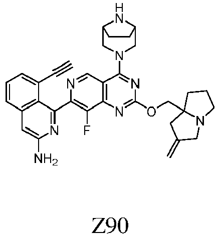 | 577.3 | 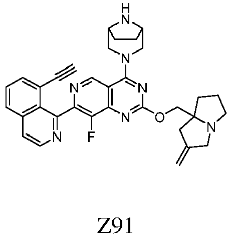 | 562.3 | 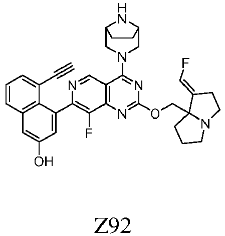 | 595.3 |
| 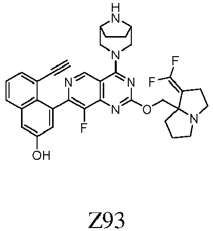 | 613.2 | 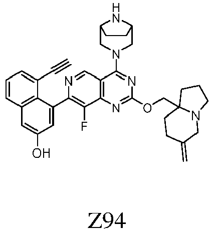 | 591.3 | 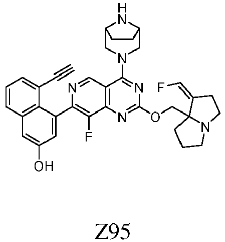 | 595.3 |
| 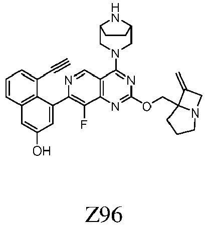 | 563.2 | 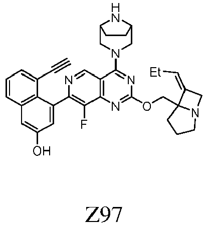 | 591.3 | 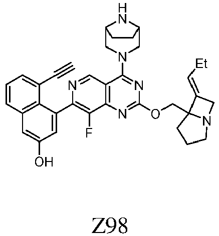 | 591.3 |
| 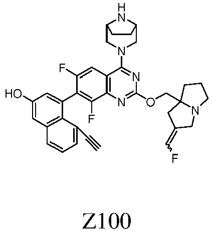 | 612.3 | 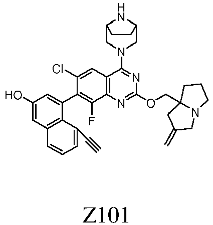 | 610.2 | 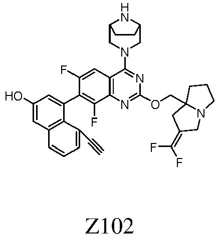 | 630.2 |
| 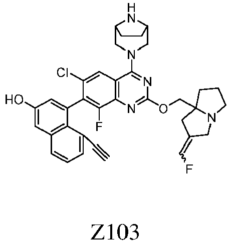 | 628.2 | 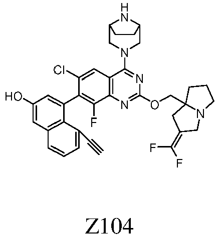 | 646.2 | 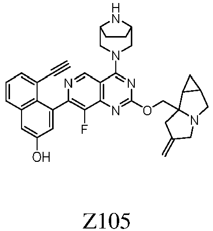 | 589.3 |
| 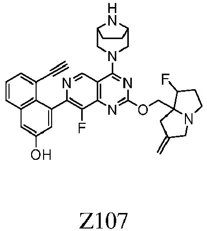 | 595.3 | 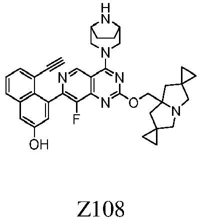 | 617.3 | 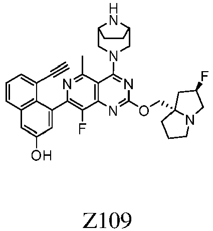 | 579.3 |
| 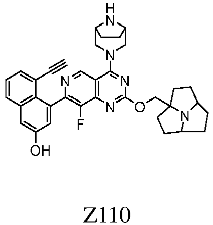 | 591.3 | 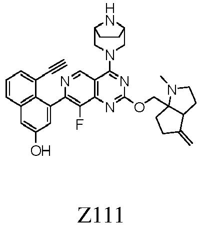 | 591.3 | 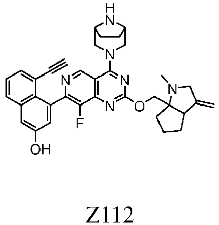 | 591.3 |
| 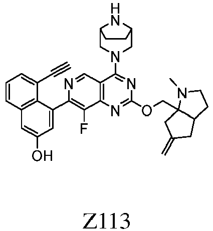 | 591.3 | 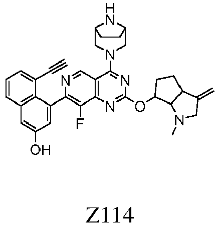 | 577.3 | 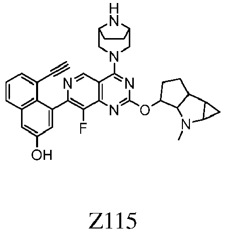 | 577.3 |
| 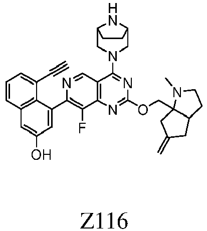 | 591.3 | 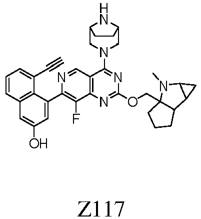 | 591.3 | 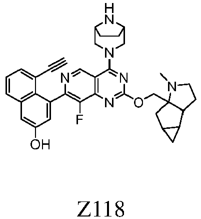 | 591.3 |
| 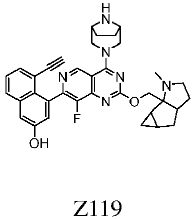 | 591.3 | 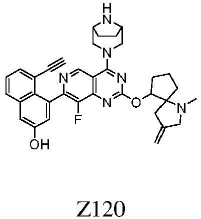 | 591.3 | 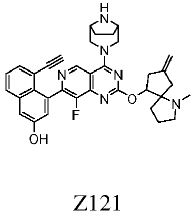 | 591.3 |
| 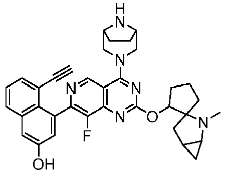 | 591.3 | 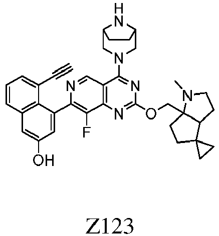 | 605.3 | 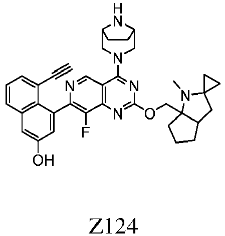 | 605.3 |
| 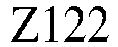 | | | | | |
| 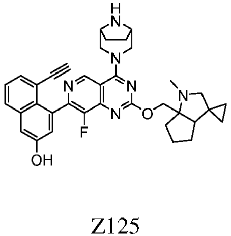 | 605.3 | 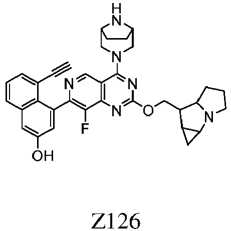 | 577.3 | 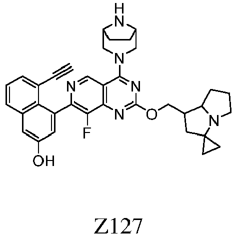 | 591.3 |
| 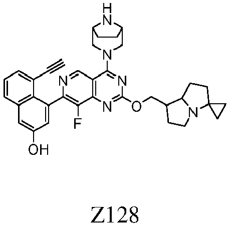 | 591.3 | 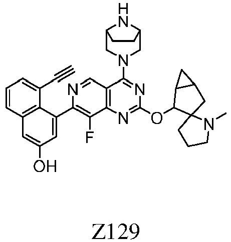 | 591.3 | 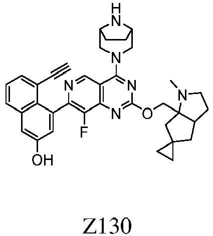 | 605.3 |
| 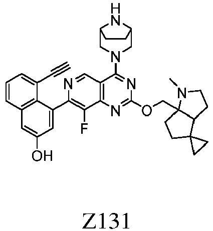 | 605.3 | 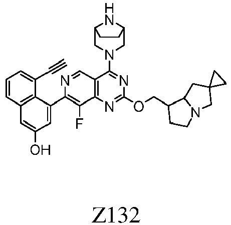 | 591.3 | 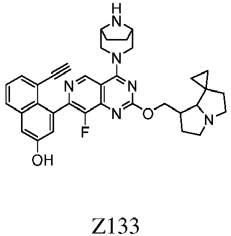 | 591.3 |
| 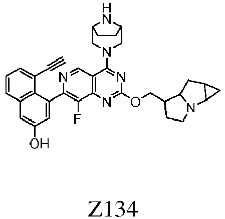 | 577.3 | 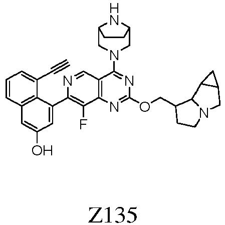 | 577.3 | 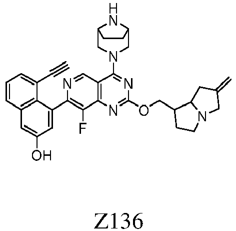 | 577.3 |
| 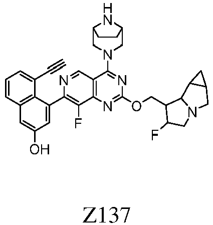 | 595.3 | 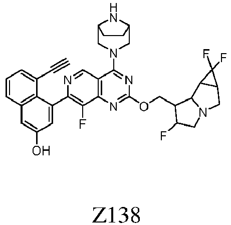 | 631.2 | 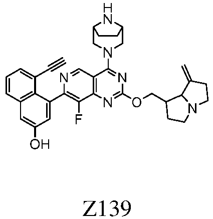 | 577.3 |
| 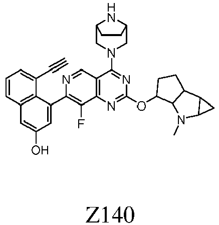 | 577.3 | 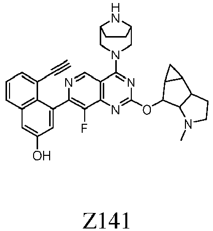 | 577.3 | 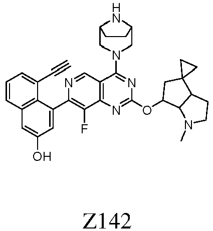 | 591.3 |
| 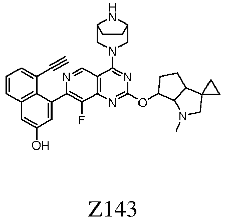 | 591.3 | 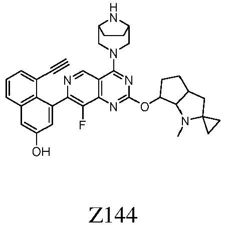 | 591.3 | 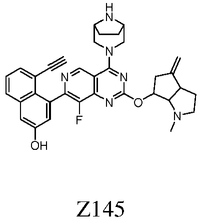 | 577.3 |
| 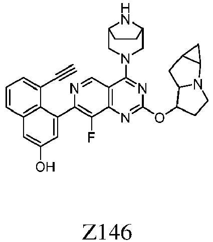 | 563.2 | 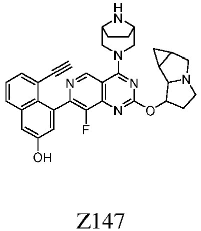 | 563.2 | 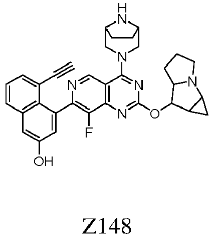 | 563.2 |
| 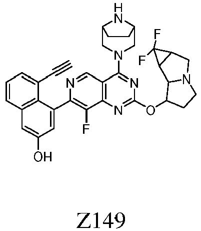 | 599.2 | 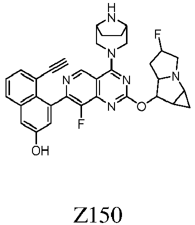 | 581.2 | 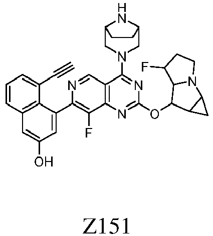 | 581.2 |
| 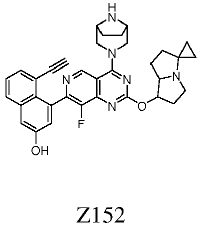 | 577.3 | 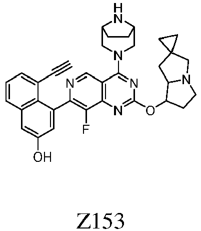 | 577.3 | 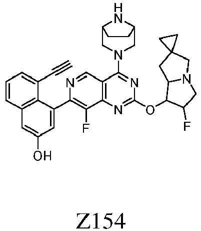 | 595.3 |
| 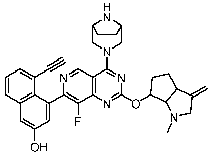 | 577.3 | 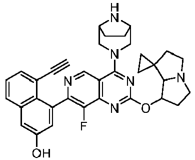 | 577.3 | 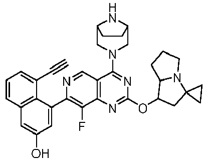 | 577.3 |
| 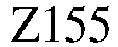 | | 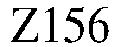 | | 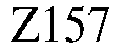 | |
| 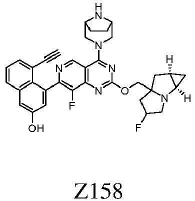 | 595.3 | 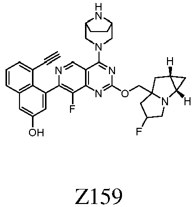 | 595.3 | 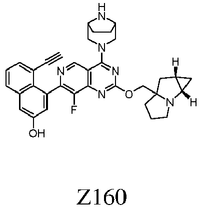 | 577.3 |
| 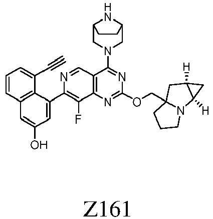 | 577.3 | 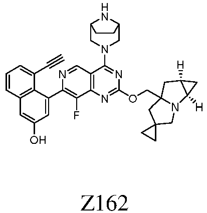 | 603.3 | 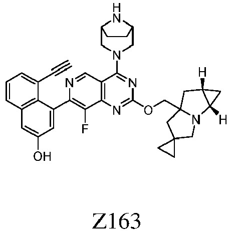 | 603.3 |
| 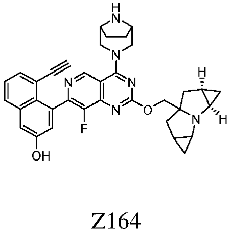 | 589.3 | 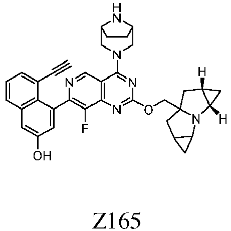 | 589.3 | 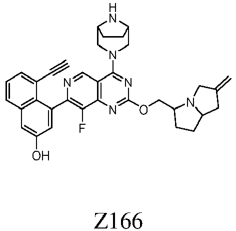 | 577.3 |
| 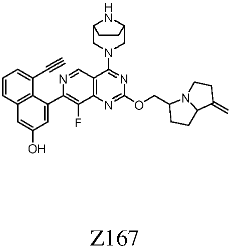 | 577.3 | 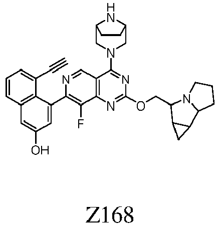 | 577.3 | 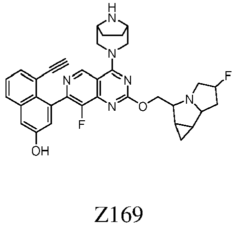 | 595.3 |
| 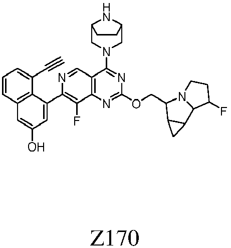 | 595.3 | 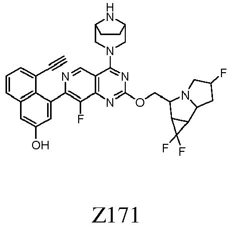 | 631.2 | 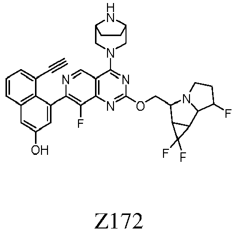 | 631.2 |
| 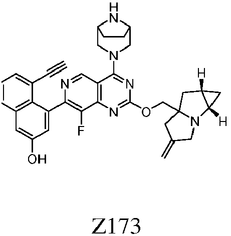 | 589.3 | 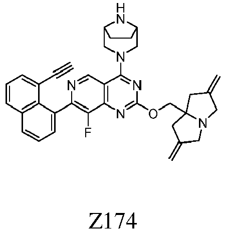 | 573.3 | 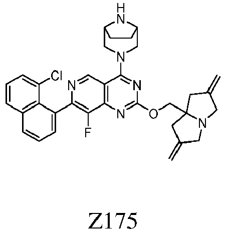 | 583.2 |
| 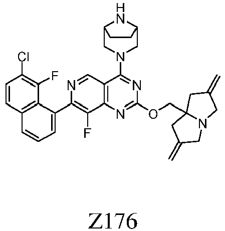 | 601.2 | 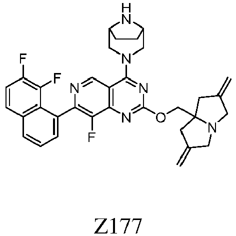 | 585.3 | 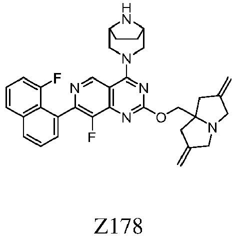 | 567.3 |
| 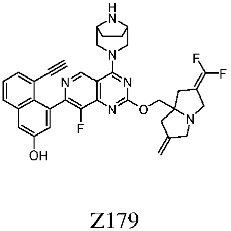 | 625.3 | 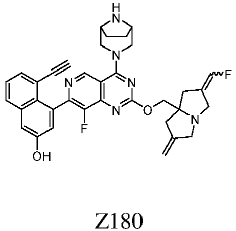 | 607.3 | 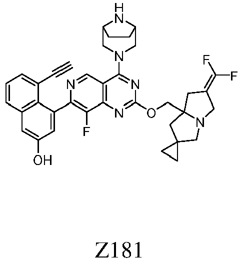 | 639.3 |
| 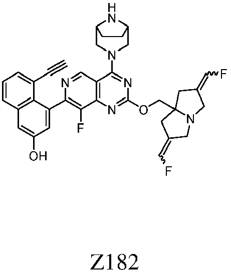 | 625.3 | 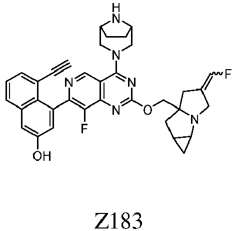 | 607.3 | 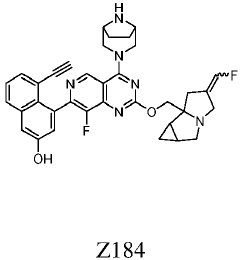 | 607.3 |
| 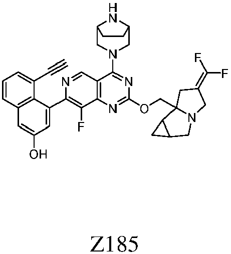 | 625.3 | 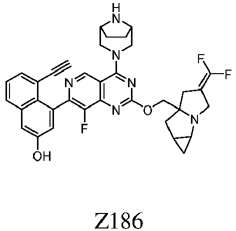 | 625.3 | 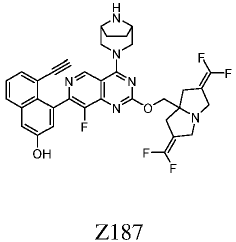 | 661.2 |
| 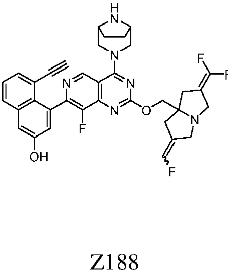 | 643.2 | 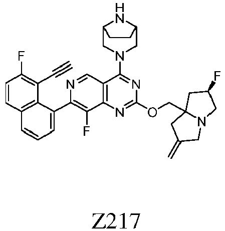 | 597.3 | 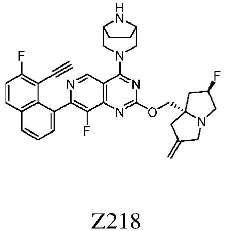 | 597.3 |
| 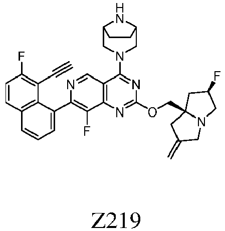 | 597.3 | 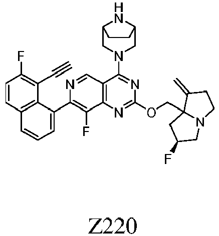 | 597.3 | 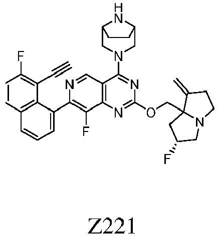 | 597.3 |
| 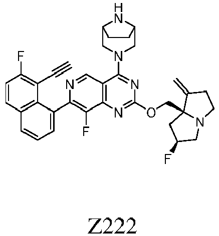 | 597.3 | 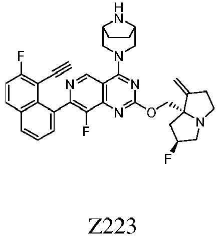 | 597.3 | 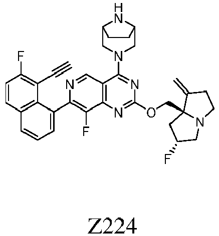 | 597.3 |
| 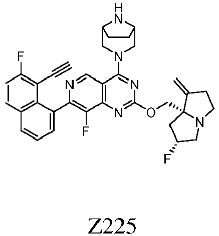 | 597.3 | 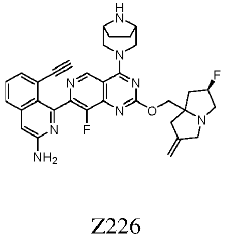 | 595.3 | 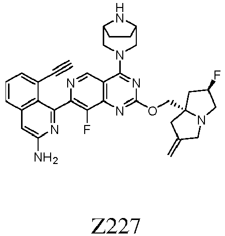 | 595.3 |
| 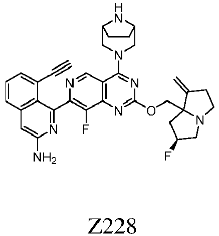 | 595.3 | 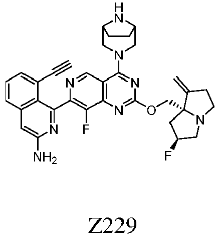 | 595.3 | 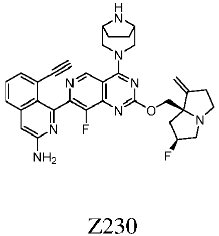 | 595.3 |
| 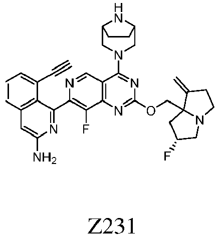 | 595.3 | 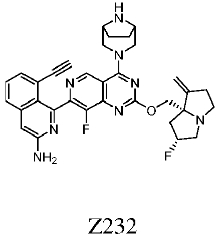 | 595.3 | 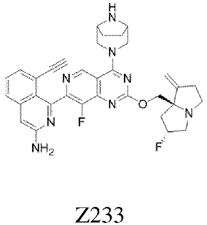 | 595.3 |

### Test Example 1: KRAS-RAF1 binding assay

GST-RAF1-RBD was purchased from BPS Bioscience; Cat. NO.: 100519; KRAS^{G12D} was purchased from BPS Bioscience; Cat. NO.: 100623; KRAS^{wt} was purchased from Reactionbiology; GTP was purchased from Sigma; Cat. NO.: G8877; Mab Anti GST-XL665 was purchased from cisbio; Cat. NO.: 61GSTXLF; Eu-W1024-anti-6XHIS was purchased from PE; Cat. NO.: AD0111; ProxiPlate-384-Plates was purchased from PerkinElmer; Cat. NO.: 6008289; DMSO was purchased from VWR AMRESCO; Cat. NO.: 0231-500ML; microplate reader was purchased from PerkinElmer; Cat. NO.: Envison;
This experiment is intended to investigate the inhibitory effect of compounds on the binding of KRAS^{G12D} mutant protein or KRAS^{wt} (wild type) protein to fragments of the RBD region fragment of RAF1 protein. A 500X 3-fold gradient concentration stock of compound was prepared using DMSO and diluted 50-fold into a 10X stock of compound using reaction buffer. To the reaction wells of an ELISA plate (ProxiPlate-384-Plates), was first added 3 µL of His-KRAS^{G12D} protein or KRAS^{wt} protein, then added 1 µL of 200 µM GTP and incubated for 30 min, then added 2 µL of 10X compound or reaction buffer with 2% DMSO (positive control) and incubated for 30 min, then added 4 µL of GST-RAF1-RBD protein or reaction buffer (negative control) and incubated for 15 min, then added 10 µL of Eu-labeled anti-His antibody and XL665-labeled anti-GST antibody mixed well in advance and incubated for 60 min before reading HTRF signal by microplate reader. The inhibition ratio of compounds on KRAS protein (G12D mutant or wild type) and RAF1 binding was calculated IR (%) = (positive control signal-compound well signal) / (positive control signal-negative control signal) × 100% and IC₅₀ values were calculated by fitting compound concentrations and corresponding inhibition ratios using the Prism 8 four-parameter method. The results show that compared with KRAS^{wt} (wild type) protein, exemplary compounds of the present disclosure have higher inhibition selectivity for the binding of KRAS^{G12D} mutant protein to the RBD region fragment of RAF1 protein. The inhibition IC₅₀ values of some compounds on the binding of KRAS^{G12D} mutant protein to RBD region fragment of RAF1 protein was less than 1 µ M or 500 nM, or even less than 100 nM or 50 nM. While the inhibition IC₅₀ values of the compounds on the binding of KRAS^{wt} (wild type) protein to the RBD region fragment of RAF1 protein was more than 40 nM or 100 nM, or more than 500 nM, or even more than 1 µ M or 2 µ M. Results for some of the exemplary compounds are shown in Table 1 below.

**Table 1**

| No. | KRAS^{G12D}/ RAF1 | KRAS^{wt}/ RAF1 | No. | KRAS^{G12D}/ RAF1 | KRAS^{wt}/ RAF1 |
|---|---|---|---|---|---|
| | IC₅₀ (nM) | IC₅₀ (nM) | | IC₅₀ (nM) | IC₅₀ (nM) |
| Z1 | 255.6 | 2610.8 | Z2 | 185.7 | 788.4 |
| Z3 | 16.2 | 64.5 | Z4 | 20.6 | 392.6 |
| Z5 | 36.3 | 43.7 | Z6 | 51.6 | >10000 |
| Z7 | 72.6 | 1029.50 | Z9 | 79.6 | 260.9 |
| Z10 | 62.91 | 118.76 | Z11 | 45.9 | 263.2 |
| Z13 | 53.56 | 101.27 | Z14 | 39.6 | 109.6 |
| Z15 | 33.0 | >3000 | Z16 | 60.66 | >1000 |
| Z17 | 50.00 | 64.30 | Z18 | 59.43 | >1000 |
| Z19 | 45.98 | 67.77 | Z20 | 104.61 | 110.35 |

### Test Example 2: cell p-ERK detection assay

AGS was purchased from ATCC; Cat. NO.: CRL-1739; F12K was purchased from Gibco; Cat. NO.: 21127-022; FBS was purchased from Gibco; Cat. NO.: 10099-141C; Trypsin (containing EDTA) was purchased from Gibco; Cat. NO.: 25200-072; DMSO was purchased from VWRAMRESCO; Cat. NO.: 0231-500ML; 96-well cell culture plate was purchased from Corning; Cat. NO.: 3599; An white background opaque 96-well plate was purchased from Cisbio; Cat. NO.: 66PL96025; Advance ERK phospho-T202/Y204 kit was purchased from Cisbio; Cat. NO.: 64AERPEH; Cell Counters were purchased from CHEMOMETEC; Model NO.: NC-200; microplate reader was purchased from PerkinElmer; Model NO.: Envison.

AGS is an endogenous human gastric adenocarcinoma cell line containing KRAS^{G12D} mutation, cultured in F12K + 10% FBS medium. On the first day of the experiment, cells in the logarithmic growth phase were taken, digested with trypsin (containing EDTA), collected, and counted, and approximately 20,000 cells/well were inoculated in 96-well cell culture plates and cultured overnight in a 5% CO₂ incubator. A 1000X 3.16-fold gradient concentration stock of the compound was prepared using DMSO, diluted 200-fold into a 5X compound stock using the medium described above, and a 5X compound stock was added to each cell culture well the day after cell inoculating at a final concentration of 1X and a DMSO content of 0.1%. DMSO was used as an experimental control. After the compound was added and the incubation was continued for 3 h, the medium in the culture wells was removed, 50 µl of cell lysis solution (from Advance ERK phospho-T202/Y204 kit, the same below) was added to each well, mixed well and incubated for 30 min, then 16 µl of the mixed solution was transferred to a white background opaque 96-well plate, and another well was taken as a blank well with adding 16 µl of cell lysis solution. After the transfer, 4 µl of p-ERK HTRF antibody mixed solution (from Advance ERK phospho-T202/Y204 kit) was added to each well, after incubation for 4 h, HTRF fluorescence value (RLU) was read by a microplate reader, and p-ERK level inhibition ratio of the compound was calculated IR (%) = (RLU control-RLU compound)/(RLU control-RLU blank) × 100%, compound concentration and corresponding cell p-ERK level inhibition ratio were fitted using Prism 8 four-parameter method, and IC₅₀ value was calculated. The result showed that the compounds of the present disclosure have high inhibitory activity on p-ERK. Inhibition IC₅₀ values of some compounds was less than 1 µM, or less than 500 nM, and even less than 100 nM or 50 nM. Results for some of the example compounds are shown in Table 2 below.

**Table 2**

| No. | AGS p-ERK | No. | AGS p-ERK | No. | AGS p-ERK | No. | AGS p-ERK |
|---|---|---|---|---|---|---|---|
| | IC₅₀ (nM) | | IC₅₀ (nM) | | IC₅₀ (nM) | | IC₅₀ (nM) |
| Z3 | 2.11 | Z4 | 47.15 | Z27 | 1.10 | Z99 | 2.90 |
| Z5 | 1.16 | Z6 | 425.78 | Z190 | 104.60 | Z106 | 1.36 |
| Z7 | 11.97 | Z8 | 2.78 | Z191 | 611.52 | Z205-1 | 612.05 |
| Z9 | 23.73 | Z10 | 214.62 | Z205-2 | 205.50 | Z192 | 93.48 |
| Z11 | 4.91 | Z12 | 1.83 | Z193 | 623.40 | Z194 | 0.69 |
| Z13 | 10.96 | Z14 | 10.08 | Z195 | 161.95 | Z198 | 89.69 |
| Z15 | 60.74 | Z16 | 54.90 | Z201 | 237.74 | Z203 | 25.59 |
| Z17 | 1.16 | Z18 | 7.57 | Z206 | 5.47 | Z207 | 13.40 |
| Z19 | 9.03 | Z20 | 2.86 | Z208 | 9.04 | Z209 | 464.50 |
| Z21 | 0.60 | Z22 | 2.41 | Z210 | 16.58 | Z211 | 5.47 |
| Z23 | 0.76 | Z24 | 3.59 | Z213 | 62.88 | Z214 | 105.38 |
| Z25 | 6.84 | Z26 | 2.76 | Z215 | 1.19 | Z216 | 1.22 |

### Test Example 3: cell proliferation inhibition assay

AsPC-1 was purchased from ATCC; Cat. NO.: CRL-1682; AGS was purchased from ATCC; Cat. NO.: CRL-1739; RPMI1640 was purchased from Gibco; Cat. NO.: 11875-093 2235123; F12K was purchased from Gibco; Cat. NO.: 21127-022; FBS was purchased from Gibco; Cat. NO.: 10099-141C; Trypsin (containing EDTA) was purchased from Gibco; Cat. NO.: 25200-072; CellTiter Glo-3D was purchased from Progema; Cat. NO.: G9683; 384-well sphere plates were purchased from corning; Cat. NO.: 3830; White background opaque 384-well plates were purchased from corning; Cat. NO.: 3570; Cell Counters were purchased from CHEMOMETEC; Model NO.: NC-200; microplate reader was purchased from PerkinElmer; Model NO.: Envison; AsPC-1 is an endogenous human pancreatic cancer cell containing KRAS*^{G12D}* mutation, which is cultured in RPMI-1640 medium containing 10% FBS; AGS human gastric cancer cell line containing KRAS*^{G12D}* mutation, cultured in F-12K medium containing 10% FBS. Cells in the logarithmic growth phase were taken, digested with trypsin (containing EDTA), harvested and counted, and inoculated with 800 AsPC-1 cells/well or 400 AGS cells/well, respectively, in 384-well sphere plates and cultured overnight at 5% CO₂ to establish a 3D cell model. A 3.16-fold gradient concentration stock solution of 1000X compound was prepared using DMSO, diluted 100-fold into a 10X compound stock solution using media, and a 10X compound stock solution was added to each cell culture well the day after cell inoculating at a final concentration of 1X with a DMSO content of 0.1%. DMSO was used as an experimental control (control) and culture medium as a blank control (blank). After the addition of compounds, the cells were cultured for 5 days. 30 µl CellTiter-Glo working solution was added to each well, mixed well, and incubated for 30 min. After standing for 30 min at room temperature, 40 µl of the mixed solution was transferred to a white background opaque 384-well plate. Luminescence chemiluminescence value was read on a microplate reader, and the cell proliferation inhibition ratio was calculated, IR (%) = (RLU control-RLU compound)/(RLU control-RLU blank) × 100%. XLFit four-parameter method was used to fit the gradient dilution concentration of compounds and the corresponding cell proliferation inhibition ratio, and the IC₅₀ value was calculated. The result showed that the compounds of the present disclosure had high inhibitory activity on KRAS*^{G12D}* mutant cells, with some compounds having an IC₅₀ values of less than 1000 nM; or less than 500 nM; even below 100 nM or 50 nM. Results for exemplary of the example compounds are shown in Table 3 below. - indicates not detected.

**Table 3**

| No. | AGS IC₅₀ (nM) | AsPC-1 IC₅₀ (nM) | No. | AGS IC₅₀ (nM) | AsPC-1 IC₅₀ (nM) |
|---|---|---|---|---|---|
| Z3 | 3.0 | 16.0 | Z22 | 10.8 | 75.4 |
| Z4 | 360.5 | - | Z23 | 4.7 | 33.2 |
| Z5 | 4.5 | 29.4 | Z24 | 3.3 | 56.0 |
| Z6 | 814.6 | - | Z25 | 24.9 | 70.7 |
| Z7 | 32.6 | 433.1 | Z27 | 3.1 | 6.9 |
| Z8 | 5.3 | 57.7 | Z99 | 5.2 | 36.6 |
| Z9 | 145.1 | 300 | Z190 | 203.8 | 484.9 |
| Z11 | 2.5 | 50.6 | Z106 | 4.6 | 14.7 |
| Z12 | 2.8 | 12.5 | Z194 | 1.4 | 4.6 |
| Z13 | 13.0 | 67.2 | Z206 | 20.12 | 35.38 |
| Z14 | 27.4 | 143.2 | Z207 | 32 | 91.1 |
| Z15 | 194.0 | 946.9 | Z210 | 26.00 | 176.5 |
| Z16 | 92.6 | 554.7 | Z211 | 4.8 | 71.9 |
| Z17 | 6.8 | 35.7 | Z213 | 57.48 | 334.66 |
| Z18 | 45.3 | 75.5 | Z214 | 106.25 | 611.1 |
| Z19 | 31.7 | 68.3 | Z215 | 5.13 | 49.41 |
| Z20 | 7.7 | 9.3 | Z216 | 3.14 | 43.68 |
| Z21 | 6.5 | 14.7 | | | |

The structure of the reference compound MRTX1133 is

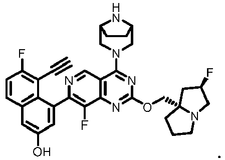

### Test Example 5: Mice pharmacokinetic evaluation experiment

Purpose of the experiment: male ICR mice were used as test animals to determine the drug concentration in plasma at different time points after intravenous and intragastric administration of test compounds by LC/MS/MS method. The pharmacokinetic behavior of the test compound in mice was studied to evaluate its pharmacokinetic characteristics.

Experimental protocol: laboratory animals: twelve healthy adult male ICR mice, weighing about 30 g, were randomly divided into two groups, 6 mice in IV group and 6 mice in PO group, N = 3/time point, with semi-continuous blood collection. Animals were purchased from Vital River Laboratory Animal Technology Co., Ltd.

Drug preparation: IV group: taking 10 mL sample solution as an example, weighing 2 mg sample, followed by adding 0.5 mL DMSO, 10 mL Solutol HS 15, then adding 17 g HP-β-CD, finally dilute to 10 mL with water, stirring and ultrasound to reach 0.2 mg/mL clear state. PO group: weighing an appropriate amount of samples and preparing them according to the solvent ratio of the IV group, and evenly reaching the state of 10 mg/mL after stirring and ultrasound. Administration: after fasting for one night, intravenous administration was performed in the IV group, with an administration volume of 5 mL/kg and a dose of 1 mg/kg: the PO group was administered intragastrically a dose volume of 10 mL/kg and a dose of 100 mg/kg.

Experimental operation: after the test compounds were administered to the male ICR mice in the intravenous injection group and the intragastric administration group, the blood 110 ul were collected at time points of 0.25, 0.5, 1, 2, 4, 7, and 24 h, and stored in a commercial anticoagulant tube pre-filled with K₂EDTA. The plasma was separated by centrifugation for 15 min and stored at-70°C. Animals were fed 4 h after administration. The plasma levels of test compounds following intravenous and intragastric administration in mice were determined by LC/MS/MS method. The linear range of the method was 1-3000 ng/ml; Plasma samples were analyzed after treatment with acetonitrile precipitated protein.

The results of the IV (1 mg/kg) group are shown in Table 4 below; the experimental results of the PO (100 mg/kg) group are shown in Table 5 below.

**Table 4**

| Groups | MRTX1133 | Z194 |
|---|---|---|
| V_{d}(L/kg) | 14.1 | 16.9 |
| T_{1/2} (h) | 5.15 | 11.2 |

Note: V_{d} represents the distribution volume; T_{1/2} represents half-life.

**Table 5**

| Groups | MRTX1133 | Z194 |
|---|---|---|
| Dose | 100 mpk | 100 mpk |
| Cₘₐₓ(ng/mL) | 279 | 560 |
| AUC (hr*ng/mL) | 745 | 2835 |

Note: Cₘₐₓ represents the maximum compound concentration after oral administration; AUC represents the area under curves as administration.

Experimental conclusion: in the mice pharmacokinetic evaluation experiment, the series of compounds of the present disclosure show a longer half-life, higher Cₘₐₓ and AUC than the reference compound MRTX1133.

While particular embodiments of the present invention have been described in detail, it will be apparent to those skilled in the art that various modifications and alternatives to those details could be developed in light of the teachings of the disclosure without departing from the scope of the invention, such variations are within the scope of the present invention. The full scope of the present invention is given by the appended claims and any equivalents thereof.

## Claims

1. A compound of Formula (AI), or a pharmaceutically acceptable salt, a stereoisomer, or a solvate thereof: 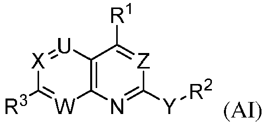
in the formula,
R¹ is selected from the group consisting of the following formulae and stereoisomers thereof: 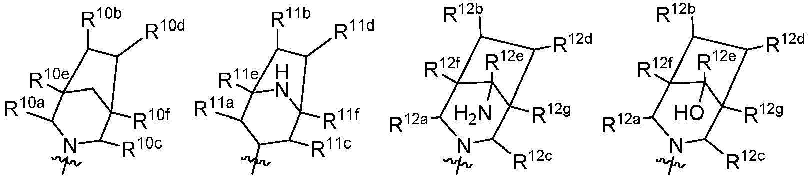 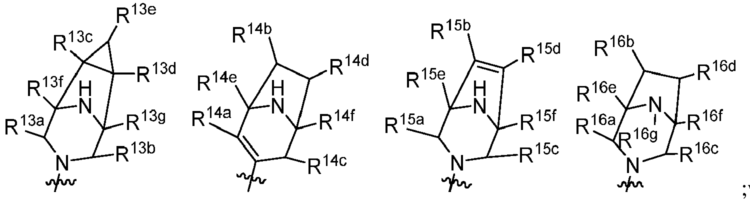 wherein, R^{10a}, R^{10b}, R^{10c}, R^{10d}, R^{10e}, and R^{10f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, -CH₂CH₂-, -CH₂OCH₂CH₂-, or -CH₂CH₂CH₂-;
R^{11a}, R^{11b}, R^{11c}, R^{11d}, R^{11e}, and R^{11f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, -CH₂CH₂-, -CH₂OCH₂CH₂-, or -CH₂CH₂CH₂-;
each R^{12a}, R^{12b}, R^{12c}, R^{12d}, R^{12e}, R^{12f}, and R^{12g} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, -CH₂CH₂-, -CH₂OCH₂CH₂-, or -CH₂CH₂CH₂-;
R^{13a}, R^{13b}, R^{13c}, R^{13d}, R^{13e}, R^{13f}, and R^{13g} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl;
R^{14a}, R^{14b}, R^{14C}, R^{14d}, R^{14e}, and R^{14f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl;
R^{15a}, R^{15b}, R^{15c}, R^{15d}, R^{15e}, and R^{15f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, or 5- or 6-membered heteroaryl;
R^{16a}, R^{16b}, R^{16c}, R^{16d}, R^{16e}, and R^{16f} are each independently hydrogen, C2-C6 alkenyl, C2-C6 alkynyl, cyano, -CH₂CH₂-, -CH₂OCH₂CH₂-, -CH₂CH₂CH₂-, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R₅)₂, -C(O)H, -CO₂R⁵, -CON(R⁵)₂, -CO₂N(R⁵)₂, or 5- or 6-membered heteroaryl;
R^{16g} is hydrogen, hydroxy, or C1-C6 alkyl;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
U is N or C(R⁴⁴); wherein R⁴⁴ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl; the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl; the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4 or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl and the 7- to 11-membered spiroheterocyclyl are each independently saturated or partially unsaturated; when the 6- to 10-membered fused heterocyclyl or the 7- to 11-membered spiroheterocyclyl is partially unsaturated, the ring contain 1 or 2 double bonds;
two hydrogen atoms on the same carbon atom of any one or two carbon atoms of the 6- to 10-membered fused heterocyclyl are optionally simultaneously substituted with =CR^{2a}R^{2b}; the 6- to 10-membered fused heterocyclyl is further optionally substituted with one or more (e.g. 1, 2, 3, 4 or 5) R⁶;
two hydrogen atoms on the same carbon atom of any one or two carbon atoms of the 7- to 11-membered spiroheterocyclyl are optionally simultaneously substituted with =CR^{2a}R^{2b}; the 7- to 11-membered spiroheterocyclyl is further optionally substituted with one or more (e.g. 1, 2, 3, 4 or 5) R6;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is independently 2, 3, or 4;
each m4, m5, m6, m7 is independently 0, 1, 2 or 3; m4 and m5 are not simultaneously 0; m6 and m7 are not simultaneously 0;
or
R² is hydrogen, -N(R⁵)₂, heterocyclyl, C1-C6 alkyl, -L-heterocyclyl, -L-aryl, -L-heteroaryl, -L-cycloalkyl, -L-N(R⁵)₂, -L-NHC(=NH)NH₂, -L-C(O)N(R⁵)₂, -L-C1-C6 haloalkyl, -L-OR⁵, -L-(CH₂OR⁵)(CH₂)ₙOR⁵, -L-NR⁵C(O)-aryl, -L-COOH, or -L-C(O)OC1-C6 alkyl; wherein n is 1, 2, 3, 4 or 5; each of the heterocyclyl, aryl of the -L-NR⁵C(O)-aryl, heterocyclyl of the -L-heterocyclyl, cycloalkyl of the -L-cycloalkyl may be optionally substituted with one or more (e.g. 1, 2, 3, 4 or 5) R⁶; each aryl of the -L-aryl, heteroaryl of the -L-heteroaryl may be optionally substituted with one or more (e.g. 1, 2, 3, 4 or 5) R⁷;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C2-C6 haloalkenyl, C2-C6 haloalkynyl, C1-C6 alkoxy, C1-C6 haloalkoxy, cyano, -Q-phenyl, -Q-phenyl-SO₂F, -NHC(O) phenyl, -NHC(O) phenyl-SO₂F, C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl) C(O)-, oxo, (C1-C3 haloalkyl) C(O)-, -SO₂F, (C1-C3 alkoxy) C1-C3 alkoxy, -CH₂OC(O)N(R⁵)₂, -CH₂NHC(O)OC1-C6 alkyl, -CH₂NHC(O)N(R⁵)₂, - CH₂NHC(O)C1-C6 alkyl, -CH₂ (pyrazolyl), -CH₂NHSO₂C1-C6 alkyl, -C1-C3 alkyl-OC(O) heterocyclyl, -OC(O)N(R⁵)₂, -OC(O)NH (C1-C3 alkyl) O (C1-C3 alkyl)), -OC(O)NH (C1-C3 alkyl) O (C1-C3 alkyl) phenyl (C1-C3 alkyl) N(CH₃)₂, -OC(O)NH (C1-C3 alkyl) O (C1-C3 alkyl) phenyl, -OC(O) heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂ heterocyclyl; wherein the phenyl referred to in the above groups are optionally substituted by-C(O)H or OH; the heterocyclyl in -C1-C3 alkyl-heterocyclyl is optionally substituted with oxo; Q is a bond or O;
each R⁷ is each independently halogen, hydroxy, -C(O)H, C1-C6 alkyl, C1-C6 alkoxy, C1-C6 haloalkyl, C1-C6 hydroxyalkyl, or -N(R⁵)₂;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl, or C1-C6 haloalkyl; or two R⁵ are each independently taken together with the nitrogen atom to which they are attached to form a 3- to 6-membered nitrogen-containing heterocyclyl, the 3- to 6-membered nitrogen-containing heterocyclyl is optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

2. A compound of Formula (AII), or a pharmaceutically acceptable salt, a stereoisomer, or a solvate thereof: 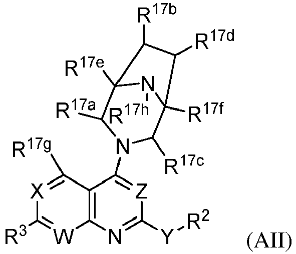 wherein:
R^{17a}, R^{17b}, R^{17c}, R^{17d}, R^{17e}, and R^{17f} are each independently hydrogen, hydroxy, halogen, C1-C6 alkyl, C1-C6 alkoxy, C1-C6 haloalkyl, C1-C6 haloalkoxy, C1-C6 cyanoalkyl, C1-C6 hydroxyalkyl, C1-C6 alkyl-N(R⁵)₂, -C(O)H, -CO₂R⁵, -CON(R⁵)₂, -CO₂N(R⁵)₂, 5- or 6-membered heteroaryl, -CH₂CH₂-, or -CH₂CH₂CH₂-;
R^{17g} is hydrogen, hydroxy, halogen, C1-C6-alkyl, C1-C6-alkoxy, C1-C6-haloalkyl or C1-C6-haloalkoxy;
R^{17h} is hydrogen, hydroxy, or C1-C6 alkyl;
W is N or C(R⁴¹); wherein R⁴¹ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
X is N or C(R⁴²); wherein R⁴² is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Z is N or C(R⁴³); wherein R⁴³ is hydrogen, halogen, cyano, C1-C6 alkyl, C1-C6 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C6 alkyl, C1-C6 alkoxy, C3-C6 monocyclic cycloalkyl, 3- to 6-membered monocyclic heterocyclyl are each independently optionally substituted with halogen or deuterium;
Y is a bond, O or NR⁵;
R² is -L-6- to 10-membered fused heterocyclyl, -L-7- to 11-membered spiroheterocyclyl, -L-spirocyclic ring substituted 6- to 10-membered fused heterocyclyl, -L-spirocyclic ring substituted 7- to 11-membered spiroheterocyclyl, -L-fused ring substituted 6- to 10-membered fused heterocyclyl, -L-fused ring substituted 7- to 11-membered spiroheterocyclyl, or -L-spirocyclic and fused ring substituted 6-to 10-membered fused heterocyclyl;
the spirocyclic ring substituted 6- to 10-membered fused heterocyclyl refers to two hydrogen atoms on the same carbon atom of any one or two carbon atoms of the 6- to 10-membered fused heterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 6- to 10-membered fused heterocyclyl;
the spirocyclic ring substituted 7- to 11-membered spiroheterocyclyl refers to two hydrogen atoms on the same carbon atom of any one or two carbon atoms of the 7- to 11-membered spiroheterocyclyl are simultaneously substituted with-(CH₂)ₘ₃-, -(CH₂)ₘ₄O(CH₂)ₘ₅-, or - (CH₂)ₘ₆NRⁿ(CH₂)ₘ₇ to form a spirocyclic ring substituted 7- to 11-membered spiroheterocyclyl;
the fused ring substituted 6- to 10-membered fused heterocyclyl refers to one hydrogen atom on each carbon atom of the same pair of carbon atoms in any one or two pairs of adjacent carbon atoms of the 6- to 10-membered fused heterocyclyl is simultaneously substituted by -(CH₂)ₘ₃-, - (CH₂)ₘ₄O(CH₂)ₘ₅-, or -(CH₂)ₘ₆NRⁿ(CH₂)ₘ₇ to form a fused ring substituted 6- to 10-membered fused heterocyclyl; or the fused ring substituted 6- to 10-membered fused heterocyclyl refers to a hydrogen atom on any two non-adjacent carbon atoms of the 6- to 10-membered fused heterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 6- to 10-membered fused heterocyclyl;
the fused ring substituted 7- to 11-membered spiroheterocyclyl refers to one hydrogen atom on each carbon atom of the same pair of carbon atoms of any one or two pairs of adjacent carbon atoms on the 7- to 11-membered spiroheterocyclyl is simultaneously substituted with -(CH₂)ₘ₃-, - (CH₂)ₘ₄O(CH₂)ₘ₅-, or -(CH₂)ₘ₆NRⁿ(CH₂)ₘ₇ to form a fused ring substituted 7- to 11-membered spiroheterocyclyl; or the fused ring substituted 7- to 11-membered spiroheterocyclyl refers to a hydrogen atom on any two non-adjacent carbon atoms of the 7- to 11-membered spiroheterocyclyl is substituted with -(CH₂)ₘ₃-, -(CH₂)ₘ₄O(CH₂)ₘ₅-, or -(CH₂)ₘ₆NRⁿ(CH₂)ₘ₇ to form a fused ring substituted 7- to 11-membered spiroheterocyclyl;
the spirocyclic and fused ring substituted 6- to 10-membered fused heterocyclyl refers to one hydrogen atom on each carbon atom of any one pair of adjacent carbon atoms on the 6- to 10-membered fused heterocyclyl is simultaneously substituted by -(CH₂)ₘ₃-, -(CH₂)ₘ₄O(CH₂)ₘ₅-, or - (CH₂)ₘ₆NRⁿ(CH₂)ₘ₇ to form a fused ring substitution and two hydrogen atoms on the other one carbon atom on the 6- to 10-membered fused heterocyclyl is simultaneously substituted by - (CH₂)ₘ₃-, -(CH₂)ₘ₄O(CH₂)ₘ₅-, or -(CH₂)ₘ₆NRⁿ(CH₂)ₘ₇ to form a spirocyclic substitution to form a spirocyclic and fused ring substituted 6- to 10-membered fused heterocyclyl;
the 6- to 10-membered fused heterocyclyl or the 7- to 11-membered spiroheterocyclyl having 1, 2, 3, or 4 heteroatom ring members selected from N(R^{m}), S, S(=O), S(=O)₂ and O;
the spirocyclic ring substituted 6- to 10-membered fused heterocyclyl, the spirocyclic ring substituted 7- to 11-membered spiroheterocyclyl, the fused ring substituted 6- to 10-membered fused heterocyclyl, the fused ring substituted 7- to 11-membered spiroheterocyclyl or the spirocyclic and fused ring substituted 6- to 10-membered fused heterocyclyl having 1, 2, 3, 4, or 5 heteroatom ring members independently selected from N(R^{m}), S, S(=O), S(=O)₂ and O;
Rⁿ is hydrogen, C1-C4 alkyl, deuterated C1-C4 alkyl, halo C1-C4 alkyl, C3-C20 cycloalkyl, or 3-to 20-membered heterocyclyl;
R^{m} is absent, hydrogen, C1-C4 alkyl, deuterated C1-C4 alkyl, halo C1-C4 alkyl, C3-C20 cycloalkyl, or 3- to 20-membered heterocyclyl;
wherein the 6- to 10-membered fused heterocyclyl and the 7- to 11-membered spiroheterocyclyl are each independently saturated or partially unsaturated; when the 6- to 10-membered fused heterocyclyl or the 7- to 11-membered spiroheterocyclyl is partially unsaturated, the ring contain 1 or 2 double bonds;
when R² is -L-6- to 10-membered fused heterocyclyl and R^{17g} is hydrogen, two hydrogen atoms on the same carbon atom of any one or two carbon atoms of the 6- to 10-membered fused heterocyclyl must both be substituted with =CR^{2a}R^{2b} simultaneously; and/or the 6- to 10-membered fused heterocyclyl is optionally substituted with one or more (e.g. 1, 2, 3, 4 or 5) R⁶;
when R² is -L-6- to 10-membered fused heterocyclyl and R^{17g} is not hydrogen, two hydrogen atoms on the same carbon atom of any one or two carbon atoms of the 6- to 10-membered fused heterocyclyl are optionally simultaneously substituted with =CR^{2a}R^{2b}; and/or the 6- to 10-membered fused heterocyclyl is optionally substituted with one or more (e.g. 1, 2, 3, 4 or 5) R⁶;
when R² is -L-7- to 11-membered spiroheterocyclyl and R^{17g} is hydrogen, two hydrogen atoms on the same carbon atom of any one or two carbon atoms of the 7- to 11-membered spiroheterocyclyl must both be substituted with =CR^{2a}R^{2b} simultaneously; and/or the 7- to 11-membered spiroheterocyclyl is optionally substituted with one or more (e.g. 1, 2, 3, 4 or 5) R⁶;
when R² is -L-7- to 11-membered spiroheterocyclyl and R^{17g} is not hydrogen, two hydrogen atoms on the same carbon atom of any one or two carbon atoms of the 7- to 11-membered spiroheterocyclyl are optionally simultaneously substituted with =CR^{2a}R^{2b}; and/or the 7- to 11-membered spiroheterocyclyl is optionally substituted with one or more (e.g. 1, 2, 3, 4 or 5) R⁶;
when R² is -L-spirocyclic ring substituted 6- to 10-membered fused heterocyclyl and R^{17g} is hydrogen and the spirocyclic ring substituted 6- to 10-membered fused heterocyclyl is 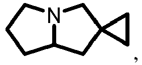 two hydrogen atoms on the same carbon atom of any one or two carbon atoms of the 6- to 10-membered fused heterocyclyl must both be substituted with =CR^{2a}R^{2b} simultaneously; and/or the spirocyclic ring substituted 6- to 10-membered fused heterocyclyl is optionally substituted with one or more (e.g. 1, 2, 3, 4 or 5) R⁶;
when R² is -L-spirocyclic ring substituted 6- to 10-membered fused heterocyclyl and R^{17g} is not hydrogen or the spirocyclic ring substituted 6- to 10-membered fused heterocyclyl is not 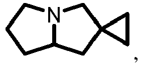 two hydrogen atoms on the same carbon atom of one or two carbon atoms of the spirocyclic ring substituted 6- to 10-membered fused heterocyclyl are optionally simultaneously substituted with =CR^{2a}R^{2b}; and/or the spirocyclic ring substituted 6- to 10-membered fused heterocyclyl is optionally substituted with one or more (e.g. 1, 2, 3, 4 or 5) R⁶;
when R² is -L-spirocyclic ring substituted 7- to 11-membered spiroheterocyclyl, two hydrogen atoms on the same carbon atom of any one or two carbon atoms of the spirocyclic ring substituted 7- to 11-membered spiroheterocyclyl is optionally simultaneously substituted with =CR^{2a}R^{2b}; and/or the spirocyclic ring substituted 7- to 11-membered spiroheterocyclyl is optionally substituted with one or more (e.g. 1, 2, 3, 4 or 5) R⁶;
when R² is -L-fused ring substituted 6- to 10-membered fused heterocyclyl, two hydrogen atoms on the same carbon atom of any one or two carbon atoms of the fused ring substituted 6- to 10-membered fused heterocyclyl are optionally simultaneously substituted with =CR^{2a}R^{2b}; and/or the fused ring substituted 6- to 10-membered fused heterocyclyl is optionally substituted with one or more (e.g. 1, 2, 3, 4 or 5) R⁶;
when R² is -L-fused ring substituted 7- to 11-membered spiroheterocyclyl, two hydrogen atoms on the same carbon atom of any one or two carbon atoms of the fused ring substituted 7- to 11-membered spiroheterocyclyl is optionally simultaneously substituted with =CR^{2a}R^{2b}; and/or the fused ring substituted 7- to 11-membered spiroheterocyclyl is optionally substituted with one or more (e.g. 1, 2, 3, 4 or 5) R⁶;
when R² is -L-spirocyclic and fused ring substituted 6- to 10-membered fused heterocyclyl, two hydrogen atoms on the same carbon atom of any one or two carbon atoms of the spirocyclic and fused ring substituted 6- to 10-membered fused heterocyclyl are optionally simultaneously substituted with =CR^{2a}R^{2b}; and/or the spirocyclic and fused ring substituted 6- to 10-membered fused heterocyclyl is optionally substituted with one or more (e.g. 1, 2, 3, 4 or 5) R⁶;
wherein R^{2a} and R^{2b} are each independently hydrogen, halogen, cyano, C1-C4 alkyl, halo C1-C4 alkyl, C6-C10 aryl, or 5- or 6-membered heteroaryl; the C6-C10 aryl, 5- or 6-membered heteroaryl is each independently optionally substituted with 1, 2, 3 groups selected from halogen, cyano, C1-C4 alkyl, C1-C4 alkoxy, halo C1-C4 alkyl, halo C1-C4 alkoxy; or each pair of R^{2a}, R^{2b} are each independently taken together with the attached carbon atom to form a C3-C6 monocyclic cycloalkyl or 3- to 6-membered monocyclic heterocyclyl; each of the C3-C6 monocyclic cycloalkyl, 3- to 6-membered monocyclic heterocyclyl is independently optionally substituted with 1, 2, 3 groups selected from halogen, cyano, C1-C4 alkyl, C1-C4 alkoxy, halo C1-C4 alkyl, halo C1-C4 alkoxy, C6-C10 aryl, 5- or 6-membered monocyclic heteroaryl;
each m3 is independently 2, 3, or 4;
each m4, m5, m6, m7 is independently 0, 1, 2 or 3; m4 and m5 are not simultaneously 0; m6 and
m7 are not simultaneously 0;
each L is each independently a bond, C1-C4 alkylene or heteroaryl; wherein the C1-C4 alkylene is unsubstituted; or one or two hydrogen atoms on any of the same carbon atoms (denoted as C*¹) of the C1-C4 alkylene are each independently substituted by deuterium, C1-C6-alkyl or C1-C6-haloalkyl; or two hydrogen atoms on any of the same carbon atoms (denoted as C*²) of the C1-C4 alkylene are simultaneously substituted by -(CH₂)ₙ₁-, -(CH₂)ₘ₁-O-(CH₂)ₘ₂-, -(CH₂)ₘ₁-NR⁹-(CH₂)ₘ₂- to form a cyclic substituent; n1 is 1 or 2; each m1 is each independently 0, 1, 2, or 3; each m2 is each independently 0, 1, 2, or 3; and m1 and m2 are not simultaneously 0;
each R⁶ is independently halogen, hydroxy, C1-C6 hydroxyalkyl, C1-C6 alkyl, C2-C6 alkenyl, C2-C6 alkynyl, C1-C6 haloalkyl, C2-C6 haloalkenyl, C2-C6 haloalkynyl, C1-C6 alkoxy, C1-C6 haloalkoxy, cyano, -Q-phenyl, -Q-phenyl-SO₂F, -NHC(O) phenyl, -NHC(O) phenyl-SO₂F, C1-C3 alkyl substituted pyrazolyl, aryl C1-C3 alkyl-, tert-butyldimethylsilyloxy CH₂-, -N(R⁵)₂, (C1-C3 alkoxy) C1-C3 alkyl, (C1-C3 alkyl) C(O)-, oxo, (C1-C3 haloalkyl) C(O)-, -SO₂F, (C1-C3 alkoxy) C1-C3 alkoxy, -CH₂OC(O)N(R⁵)₂, -CH₂NHC(O)OC1-C6 alkyl, -CH₂NHC(O)N(R⁵)₂, - CH₂NHC(O)C1-C6 alkyl, -CH₂ (pyrazolyl), -CH₂NHSO₂C1-C6 alkyl, -C1-C3 alkyl-OC(O) heterocyclyl, -OC(O)N(R⁵)₂, -OC(O)NH (C1-C3 alkyl) O (C1-C3 alkyl)), -OC(O)NH (C1-C3 alkyl) O (C1-C3 alkyl) phenyl (C1-C3 alkyl) N(CH₃)₂, -OC(O)NH (C1-C3 alkyl) O (C1-C3 alkyl) phenyl, -OC(O) heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂ heterocyclyl; wherein the phenyl referred to in the above groups are optionally substituted by-C(O)H or OH; the heterocyclyl in -C1-C3 alkyl-heterocyclyl is optionally substituted with oxo; Q is a bond or O;
R³ is aryl, or heteroaryl; wherein the aryl, heteroaryl are each independently optionally substituted with one or more R⁸; wherein each R⁸ is independently halogen, cyano, hydroxy, C1-C6 alkyl, -S-C1-C6 alkyl, C2-C6 alkenyl, C2-C6 alkynyl, C2-C6 hydroxyalkynyl, C1-C6 cyanoalkyl, triazolyl, C1-C6 haloalkyl, -O-C1-C6 haloalkyl, -S-C1-C6 haloalkyl, C1-C6 alkoxy, C1-C6 hydroxyalkyl, - CH₂C(O)N(R⁵)₂, -C2-C4 alkynyl (NR⁵)₂, -N(R⁵)₂, deuterated C2-C6 alkynyl, (C1-C6 alkoxy) halo C1-C6 alkyl- or C3-C6 monocyclic cycloalkyl; wherein the C3-C6 monocyclic cycloalkyl is optionally substituted with halogen or C1-C3 alkyl;
each of the foregoing R⁵ is independently hydrogen, C1-C6 alkyl, C1-C6 deuterated alkyl or C1-C6 haloalkyl; or two R⁵ are each independently taken together with the nitrogen atom to which they are attached to form a 3- to 6-membered nitrogen-containing heterocyclyl, the 3- to 6-membered nitrogen-containing heterocyclyl is optionally substituted with one or two groups selected from halo, methyl, trifluoromethyl, methoxy, trifluoromethoxy;
R⁹ is hydrogen, C1-C6 alkyl, C1-C6 deuterated alkyl, C1-C6 haloalkyl, C1-C6 alkyl-hydroxy, C1-C6 alkyl-cyano, C1-C6 alkoxy, C1-C6 alkyl-C1-C6 alkoxy, C3-C6 monocyclic cycloalkyl, C3-C6 deuterated cycloalkyl, or C3-C6 halocycloalkyl.

3. The compound of claim 1 or 2 or a pharmaceutically acceptable salt, a stereoisomer, or a solvate thereof, wherein W is N or W is C(R⁴¹); wherein R⁴¹ is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

4. The compound of claim 1 or 2 or a pharmaceutically acceptable salt, a stereoisomer, or a solvate thereof, wherein X is N or X is C(R⁴²); wherein R⁴² is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

5. The compound of claim 1 or 2 or a pharmaceutically acceptable salt, a stereoisomer, or a solvate thereof, wherein Z is N or Z is C(R⁴³); wherein R⁴³ is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

6. The compound of claim 1 or a pharmaceutically acceptable salt, a stereoisomer, or a solvate thereof, wherein U is N or U is C(R⁴⁴); wherein R⁴⁴ is hydrogen, halogen, cyano, C1-C3 alkyl, C1-C3 alkoxy, C3-C6 monocyclic cycloalkyl, C3-C6 monocyclic cycloalkyl-O-, 3- to 6-membered monocyclic heterocyclyl or 3- to 6-membered monocyclic heterocyclyl-O-; the C1-C3 alkyl, C1-C3 alkoxy, C3-C6 monocyclic cycloalkyl, 3- to 6-membered monocyclic heterocyclyl are each independently optionally substituted with halogen or deuterium.

7. The compound of claim 1 or a pharmaceutically acceptable salt, a stereoisomer, or a solvate thereof, wherein R³ is phenyl, naphthyl, 1,2,3,4-tetrahydronaphthalene, 1,2,3,4-tetrahydroquinoline, 2,3-dihydro-1H-indenyl, isoquinolinyl, quinolinyl, indazolyl, or benzo[d][1,3]dioxolane; wherein the phenyl, naphthyl, 1,2,3,4-tetrahydronaphthalene, 1,2,3,4-tetrahydroquinoline, 2,3-dihydro-1H-indenyl, isoquinolinyl, quinolinyl, indazolyl, or benzo[d][1,3]dioxolane are each independently optionally substituted with one, two, or three R⁸; wherein each R⁸ is independently selected from the group consisting of halogen, cyano, hydroxy, methyl, ethyl, cyclopropyl, halomethyl, ethynyl, and amino.

8. The compound of claim 1 or a pharmaceutically acceptable salt, a stereoisomer, or a solvate thereof, wherein R¹ is selected from the group consisting of:

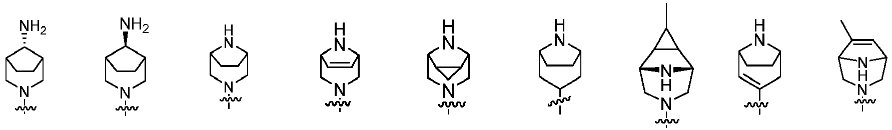

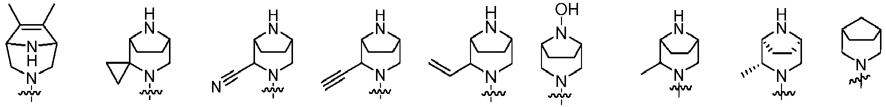

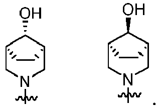

9. The compound of claim 2 or a pharmaceutically acceptable salt, a stereoisomer, or a solvate thereof, wherein R¹ is selected from the group consisting of:

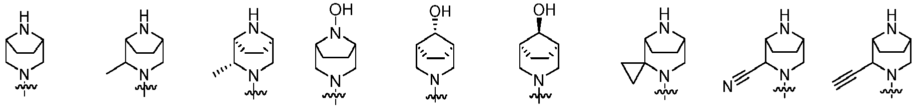

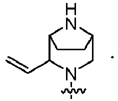

10. The compound of claim 1 or 2 or a pharmaceutically acceptable salt, a stereoisomer, or a solvate thereof, wherein Y is O.

11. The compound of claim 1 or 2 or a pharmaceutically acceptable salt, a stereoisomer, or a solvate thereof, wherein R² is selected from the group consisting of:

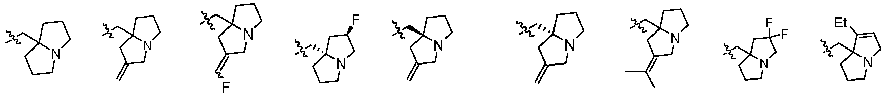

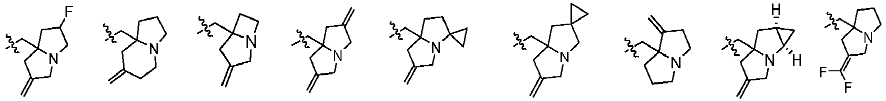

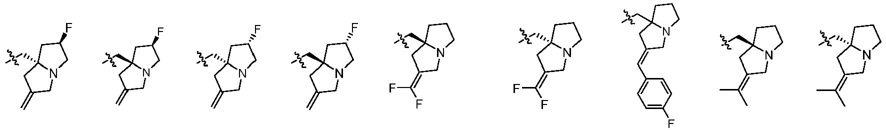

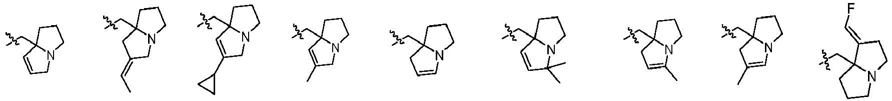

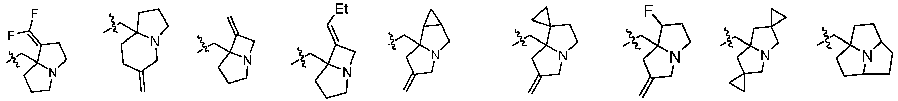

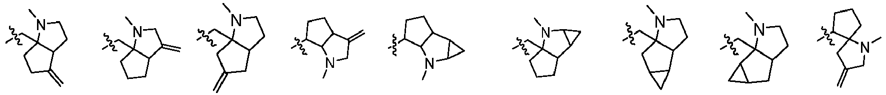

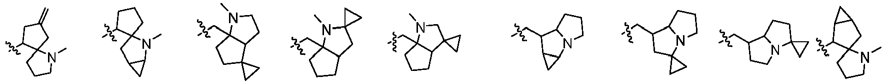

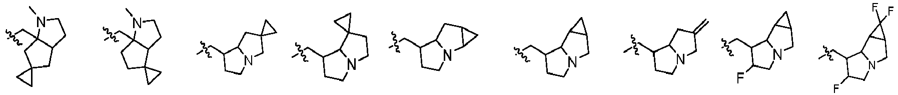

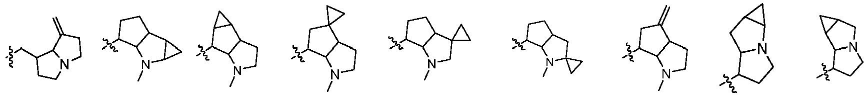

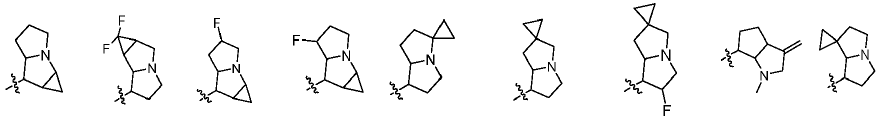

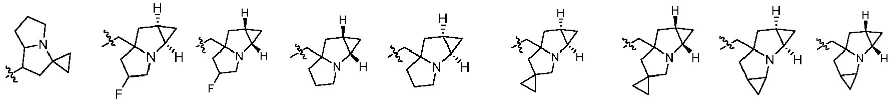

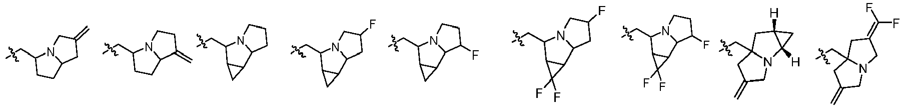

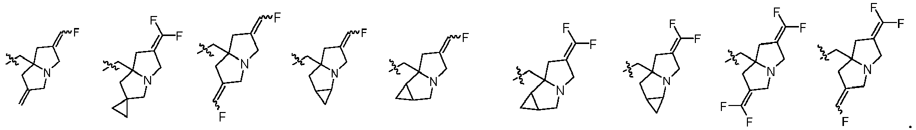

12. The compound claim 1 or 2 or a pharmaceutically acceptable salt, a stereoisomer, or a solvate thereof of, wherein the compound is selected from the group consisting of:

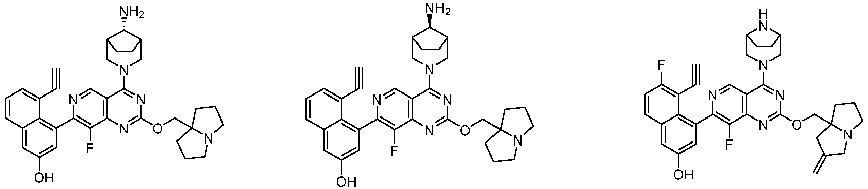

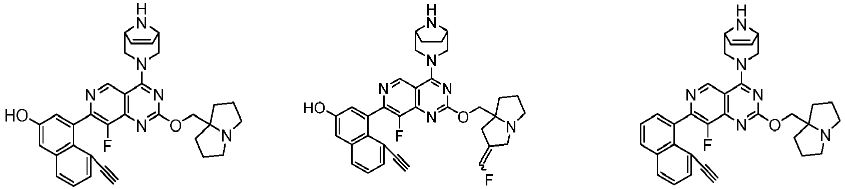

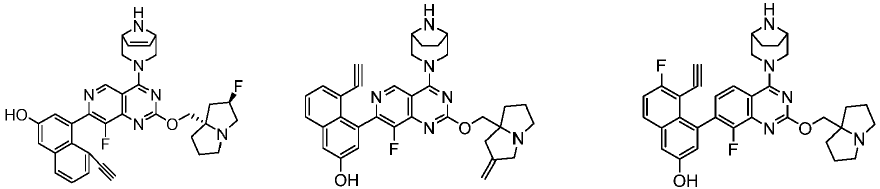

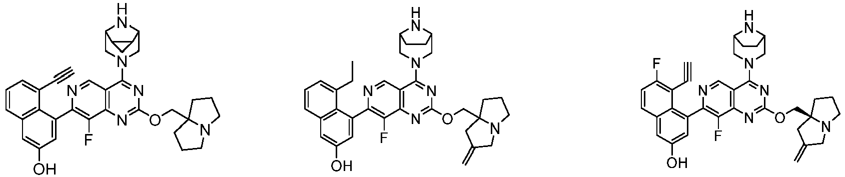

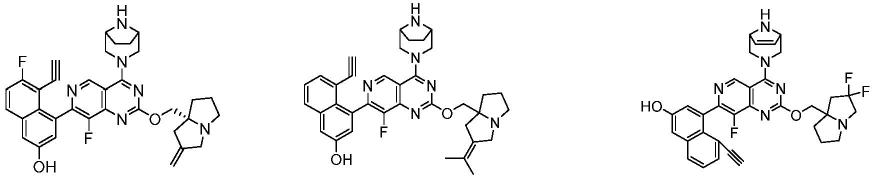

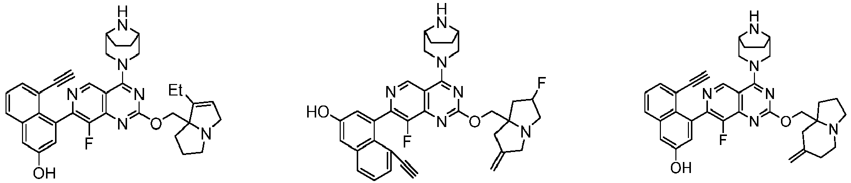

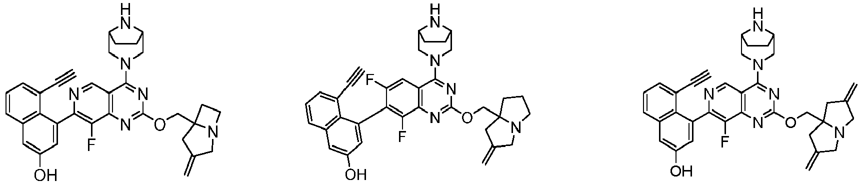

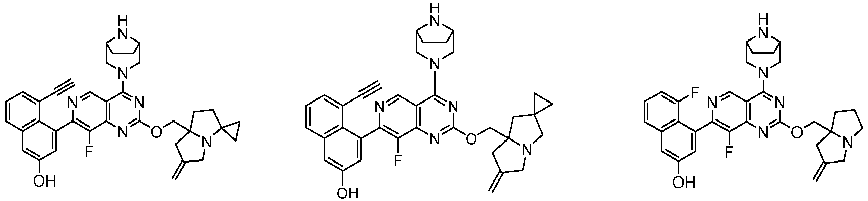

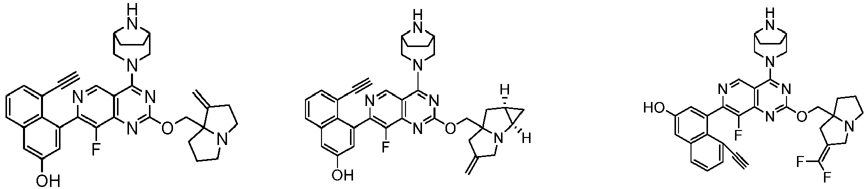

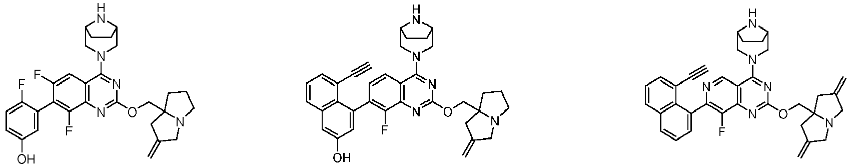

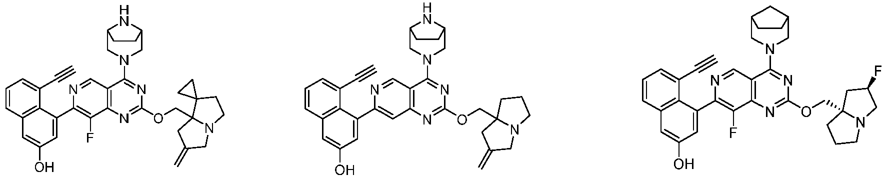

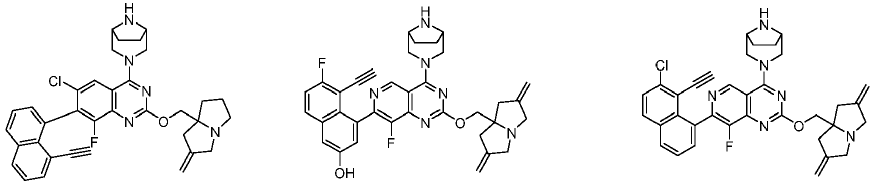

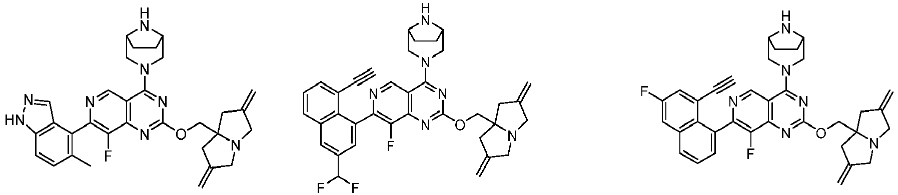

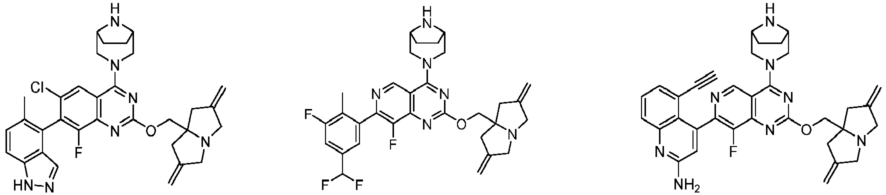

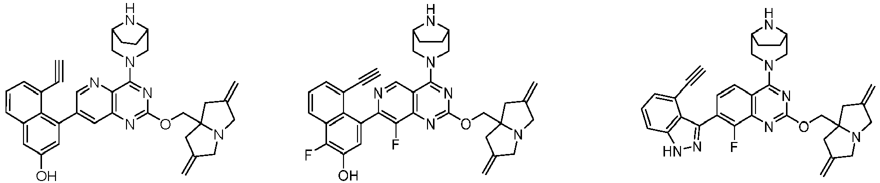

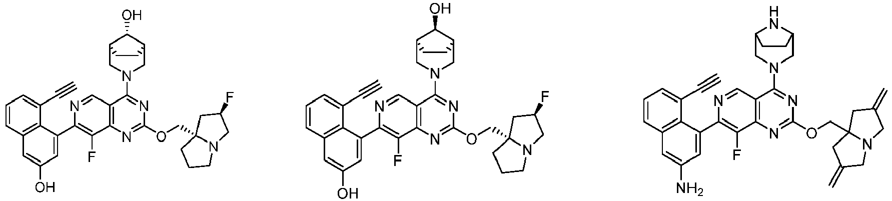

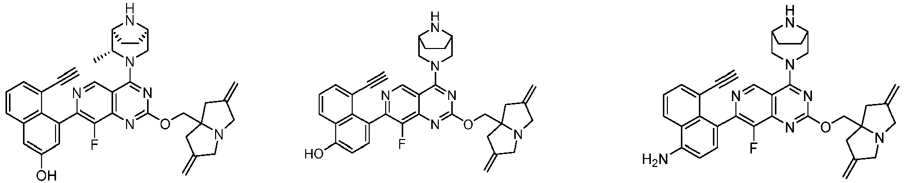

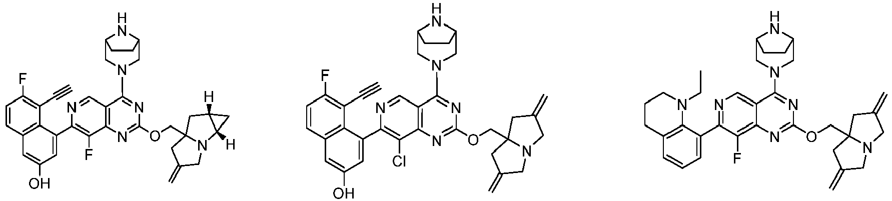

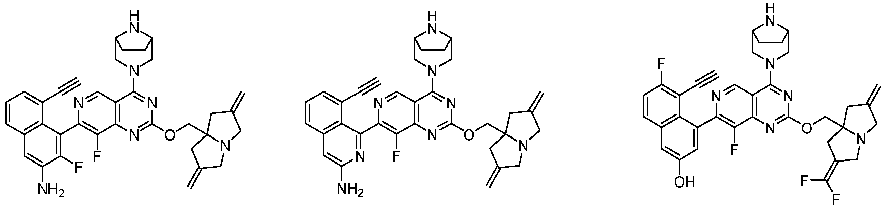

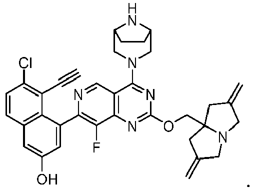

13. A pharmaceutical composition comprising a therapeutically effective amount of the compound of any one of claims 1 to 12 or a pharmaceutically acceptable salt, a stereoisomer, or a solvate thereof; and a pharmaceutically acceptable excipient.

14. Use of the compound of any one of claims 1 to 12 or the pharmaceutically acceptable salt, a stereoisomer, or a solvate thereof, or the pharmaceutical composition of claim 13, for the preparation of a medicament for prevention and/or treatment of a disease or condition; the disease or condition is a KRAS G12D associated disease or disorder.

15. The use of claim 14, wherein the KRAS G12D associated disease or disorder is cancer.
